(19) Europäisches Patentamt
European Patent Office
Office européen des brevets

(11) EP 2 725 024 A1

(12) **EUROPEAN PATENT APPLICATION**
published in accordance with Art. 153(4) EPC

(43) Date of publication:
**30.04.2014 Bulletin 2014/18**

(21) Application number: **12804554.9**

(22) Date of filing: **15.05.2012**

(51) Int Cl.:
*C07D 401/06* (2006.01) *C07D 401/14* (2006.01)
*C07D 403/06* (2006.01) *C07D 403/14* (2006.01)
*C07D 413/06* (2006.01) *C07D 413/14* (2006.01)
*C07D 417/06* (2006.01) *C07D 417/14* (2006.01)
*A61K 31/506* (2006.01) *A61K 31/517* (2006.01)
*A61P 9/10* (2006.01) *A61P 3/10* (2006.01)
*A61P 17/06* (2006.01) *A61P 29/00* (2006.01)

(86) International application number:
**PCT/CN2012/000661**

(87) International publication number:
**WO 2013/000267 (03.01.2013 Gazette 2013/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2011 PCT/CN2011/076402**

(71) Applicant: **Shanghai Institute of Materia Medica, Chinese Academy of Sciences**
**Shanghai 201203 (CN)**

(72) Inventors:
- **SHEN, Jianhua**
  **Shanghai 201203 (CN)**
- **WANG, Yiping**
  **Shanghai 201203 (CN)**
- **WANG, Kai**
  **Shanghai 201203 (CN)**

(74) Representative: **Jansen, Cornelis Marinus et al**
**V.O.**
**Johan de Wittlaan 7**
**2517 JR Den Haag (NL)**

(54) **AZOLE HETEROCYCLIC COMPOUND, PREPARATION METHOD, PHARMACEUTICAL COMPOSITION AND USE**

(57) The present invention relates to the filed of pharmarcutical chemistry, and in particular, to a novel class of azole compounds represented by general formula (I), (II) or (III) amd a preparation method thereof, a pharmarcutical composition with the compounds as active components, and a use of the azole compounds and the pharmarcutical composition in the preparation of a medicament for treatment of diseases associated with Lp-$PLA_2$ enzyme activities, wherein each substituent is as deinfed in the specifictaion.

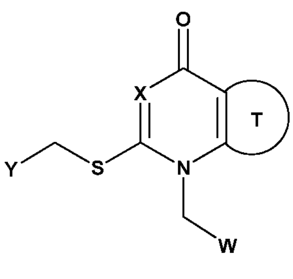

(I) or

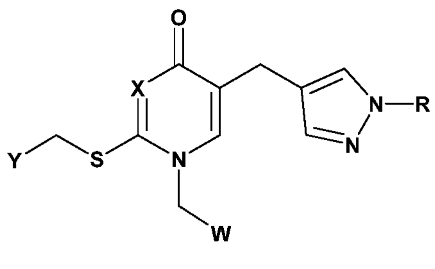

(II) or

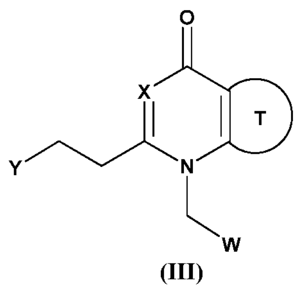

(III)

EP 2 725 024 A1

**Description**

## FIELD OF THE INVENTION

The present invention relates to the field of medicinal chemistry, particularly to novel azole heterocylic compounds, their preparation methods, their pharmaceutical compositions with these compounds as active ingredients and their use in the preparation of medicaments for treating diseases associated with activity of the enzyme Lp-PLA2.

## BACKGROUND OF THE INVENTION

Atherosclerosis is demonstrated to be the pathophysiological basis for the development of cardiovascular diseases. Thrombosis resulting from atherosclerotic plaque rupture is the major cause of cardiovascular events. Hence, it is an important task in urgent need of a solution for modem medicine to prevent or cure atherosclerosis. At present, the standard clinical medication scheme comprises the use of statins for regulating lipid, the use of hypotensors for controlling blood pressure and the concurrent administration of drugs for antiplatelet aggregation. However, a substantial relapse risk still exists in patients despite long-term treatment with such a therapeutic schedule, especially in critical patients.

It is proved that atherosclerosis is a syndrom that relates not only to abnormal lipid level, but also to a variety of inflammatory reactions. Modulation of the related inflammatory factors may be a new approach in treating the disease. Oxidized low density lipoprotein (ox-LDL) is a risk factor in plasma that can accelerate the inflammatory reactions and induces the progression of atherosclerosis.

Furthermore, lipoprotein-associated phospholipase $A_2$ (Lp-$PLA_2$) is reported to play an important role in promoting the inflammatory reactions and inducing the atherosclerosis formation, and thus is a crucial enzyme that mediates the above biological effect of ox-LDL(Zalewski A et al, Arterioscler Thromb Vasc Biol, 2005, 25(5):923-931).

Lp-$PLA_2$, also known as platelet-activating factor (PAF) acetylhydrolase, is a member of the superfamily of $PLA_2$ and belongs to type VII $PLA_2$. The enzyme contains 441 amino acids and has a relative molecule weight of 45kD. In human plasma, 70% Lp-$PLA_2$ is bound to LDL, and 30% Lp-$PLA_2$ is bound to high-density lipoprotein (HDL), indicating that Lp-$PLA_2$ is easily delivered along with LDL to the segments of arterial wall where lesion is formed. Lp-$PLA_2$ can hydrolyze PAF, PAF-like phospholipids, and oxidatively modified phosphatidylcholines. Its enzymatic activity occurs in the absence of divalent cations such as calcium ions, which is different from many other $PLA_2$s. Lp-$PLA_2$ is strongly specific to the short-chain residues of phospholipids at the *sn*-2 position. When the residue at the sn-2 position is acetyl group, it exhibits the maximum hydrolyzing activity. In contrast, it lacks enzymatic activity for phospholipid substrates in which long-chain fatty acids are located at the *sn*-2 position. Gene mutagenesis technique has identified that the enzymatic active center is consisted of three residues in Lp-$PLA_2$, including Ser-273, Asp-296 and His-351.

The atherogenic mechanism of Lp-$PLA_2$ was first proposed by Maphee and co-workers (Macphee CH, Moores KE, Boyd HF, et al. Biochem J 1999; 338:479-87). As a component of LDL, lecithin is oxidatively modified at *sn*-2 position to shorten the long chain and then enters the arterial intima as a substrate of Lp-$PLA_2$. The substrate is rapidly hydrolyzed by Lp-$PLA_2$ into two products—lysophosphatidylcholine (lyso-LPC) and oxidized free fatty acids (oxNEFAs), both of which have potent proinflammatory properties. The two biological mediators exhibit their pre-atherogenic effects by initiating a variety of inflammation-immune responses in several cells (endothelial cells, smooth muscle cells, monocytes, macrophages, T cells and neutrophils, etc.), for example, up-regulation of adhesion molecules, recruitment of monocytes/ macrophages to atherosclerotic plaque region, induction of cytokines (such as interferons) expression, activation of leucocytes, induction of oxidation stress, induction of cell membrane permeability and cellular apoptosis. These effects are deemed to promote atherosclerotic plaque growth and destabilization, and further lead to continuous enlargement of a necrotic core and thinning of a fibrous cap. Thrombus is much likely to be formed once the plaque gets ruptured, ultimately resulting in clinical diseases such as miocardial infarction, coronary heart disease, ischemic stroke and the like.

Therefore, use of Lp-$PLA_2$ inhibitors is expected to reduce aforementioned inflammatory responses and may represent a novel and non lipid-lowering strategy for atherosclerosis treatment. It was observed in human body that selective inhibition of Lp-$PLA_2$ was able to notably reduce the generation of ox-NEFA and the apoptosis of macrophage induced by ox-LDL (Rosenson RS, Vracar-Grabar M, Helenowski I. Cardiovasc Drugs Ther 2008; 22:55-8). The effect of Lp-$PLA_2$ inhibitor was also supported by experimentation on animal models. Wilensky et al. carried out detailed studies on the effects of darapladib as an Lp-$PLA_2$ inhibitor on atherosclerotic lesion area, composition, and gene expression in diabetic/hypercholesterolemic (DM-HC) swine. It was observed that darapladib treatment remarkably reduced the atherosclerotic plaque progression.

Considerable clinical studies have shown a positive association between the level of Lp-$PLA_2$ and the incidence of cardiovascular events. In the West of Scotland Coronary Prevention Study (WOSCOPS)—a nested case-control study involving 580 patients with established miocardial infarction, ischemic reperfusion or coronary heart diseases and 1,168 matched controls, Caslake et al. first proposed the link of Lp-$PLA_2$ level with coronary heart disease events (CHD)

(Packard CJ, O'Reilly DS, Caslake MJ, et al. West of Scotland Coronary Prevention Group. N Engl J Med 2000; 343: 1148-55). Lp-$PLA_2$ was found to be an independent risk factor for predicting future CHD events. Lp-$PLA_2$ was shown to have positive association with LDL, have little association with Fbg, have no association with CRP, WBC and other risk factors, and was not affected by smoking. The incidence of CHD events increases by 22% for each standard deviation increase in Lp-$PLA_2$.

Several studies towards apparently healthy patients have also shown that the concentration of Lp-$PLA_2$ could be used as a diagnostic indicator for early warning to those populations who have relatively low LDL level but have potential risk of CHD events. In the Atherosclerosis Risk in Communities (ARIC) study, Ballantyne et al. examined more than 12,000 middle-aged men and women patients with an LDL level <130mg/dl. It was found that the incidence of cardiovascular events increased with the elevated level of Lp-$PLA_2$. Patients in the highest tertile of Lp-$PLA_2$ level had a hazard ratio of 1.78 for incident CHD events compared to the lowest tertile. But, this association was not evident among those who have an LDL level >130mg/dl. However, in the Rotterdam Study that involved 7,983 patients over 55 years of age without established CHD, Lp-$PLA_2$ is proved to be the predictor of CHD events, as well as a risk marker for ischemic stroke, independently of the level of cholesterol.

In the population with established cardiovascular disease (e.g. secondary prevention patients), Lp-$PLA_2$ also appears to be a risk factor for recurrent cardiovascular events. In a study involving patients who were undergoing clinically indicated coronary angiography, each standard deviation increase in Lp-$PLA_2$ would lead to 30% increase in the risk of cardiovascular event over a four-year follow-up period, independently of traditional risk factors and C-reactive protein (CRP). Another study towards the patients who were participating in a rehabilitation program demonstrated that, patients in the highest tertile of Lp-$PLA_2$ concentrations were associated with twice the risk of recurrent events compared with those in the lowest tertile.

The increase of lysophosphatidylchloline (Lyso-PC), one of the hydrolysis products of ox-LDL, was deemed to be connected with endothelial dysfunction in atherosclerosis patients. Thus, Lp-$PLA_2$ inhibitor is expected to ameliorate such disorder and may have a general application in treating other diseases associated with endothelial dysfunction, for example diabetes, hypertension, angina and ischemic reperfusion.

Lp-$PLA_2$ is expressed in activated inflammatory cells (such as macrophages, lymphocytes, neutrophils, eosnophils). Therefore, Lp-$PLA_2$ inhibitors may be of use in treating any disorder that is associated with inflammatory cells. Such conditions include psoriasis and various airway inflammations, such as asthma and chronic bronchitis.

Additionally, Lp-$PLA_2$ inhibitors may also have a general application in any disorder that involves the hydrolysis of oxidized lipid with the participation of Lp-$PLA_2$ into two inflammation specific compounds. Such diseases may include atherosclerosis, diabetes, hypertension, angina, rheumatoid arthritis, stroke, myocardial infarction, reperfusion injury, acute and chronic inflammation.

Patent applications WO96/13484, WO96/19451, WO97/02242, WO97/21765, WO97/21766, WO97/41098 and WO97/41099 (SmithKline Beecham plc) disclosed a series of monocyclic beta lactam derivatives which are irreversible acetylation inhibitors of Lp-$PLA_2$ (Tew et al, Biochemistry, 37, 10087, 1998).

Researchers from SmithKline Beecham plc developed a class of potent, reversible Lp-$PLA_2$ inhibitors which were characterized by the pyrimidone or pyridone scaffold in the molecules (WO99/24420, WO01/60805, WO02/30911, WO03/016287, WO03/042179, WO03/042206, WO08/048867, et al). Darapladib (SB480848), a representative inhibitor of this class, is undergoing phase III clinical trials at present.

Korean researchers developed a series of novel O-acyloxime derivatives which displayed micromole activities (US7642291).

We have found various novel azole heterocyclic compounds which are demonstrated by pharmacological experimentation to be potent Lp-$PLA_2$ inhibitors.

## SUMMARY OF THE INVENTION

A first object of this invention is to provide pharmaceutically valuable azole heterocyclic compounds of Formula (I), (II) or (III), *cis-trans* isomers, enantiomers, diastereoisomers, racemes, solvates, hydrates, or pharmaceutically acceptable salts thereof.

Another object of this invention is to provide preparation methods of the compounds of Formula (I).

Still another object of this invention is to provide the use of a compound of Formula (I), (II) or (III) as an Lp-$PLA_2$ inhibitor, and as such the use of the compound in manufacturing a medicament for preventing, curing or ameliorating a disease associated with activity of the enzyme Lp-$PLA_2$. The mentioned diseases may include atherosclerosis, stroke, myocardial infarction, angina, myocardial ischemia, reperfusion injury, diabetes, asthma, psoriasis, rheumatoid arthritis, or acute and chronic inflammation.

Yet another object of this invention is to provide a pharmaceutical composition that comprises one or more compounds of Foumula (I), (II) or (III) in therapeutically effective amount, or a pharmaceutically acceptable salt thereof, and a pharmaceutically acceptable excipient.

Yet another object of this invention is to provide a method of preventing, curing or ameliorating a disease associated with activity of the enzyme Lp-$PLA_2$, wherein the method involves the use of the azole heterocyclic compounds, *cis-trans* isomers, enantiomers, diastereoisomers, racemes, solvates, hydrates, or pharmaceutically acceptable salts thereof or the compositions according to this invention.

## DETAILED DESCRIPTION OF THE INVENTION

In the first aspect, the present invention provides azole heterocyclic compounds of Formula (I), (II) or (III), *cis-trans* isomers, enantiomers, diastereoisomers, racemes, solvates, hydrates, or pharmaceutically acceptable salts thereof:

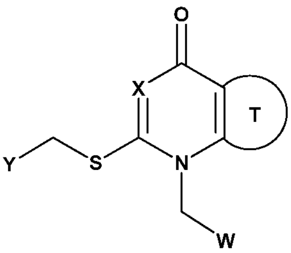
(I)

or

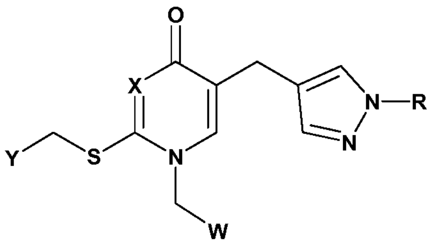
(II)

or

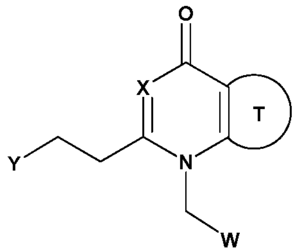
(III)

in which:

T is 4 to 6-membered aliphatic ring or benzene ring;
R is $C_1$-$C_6$ alkyl;
X is CH or N;
Y is phenyl group, optionally substituted by one or more substituents selected from halogen, $C_1$-$C_6$ alkoxyl, $C_1$-$C_6$ alkyl or halogenated $C_1$-$C_6$ alkyl;
W is selected from 12 structures of Formulae (a-1) as follows:

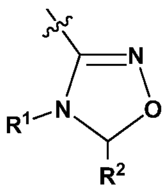
(a)

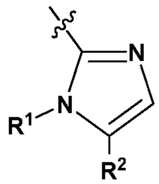
(b)

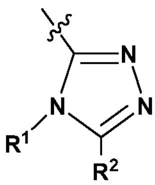
(c)

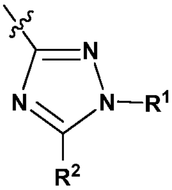
(d)

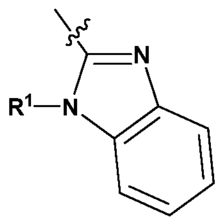
(e)

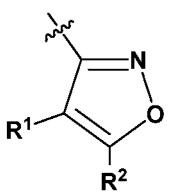
(f)

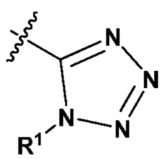
(g )

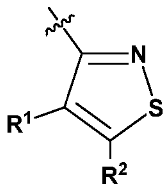
(h)

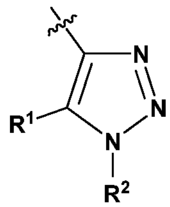
(i)

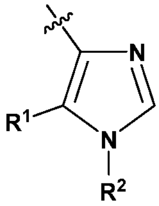
(j)

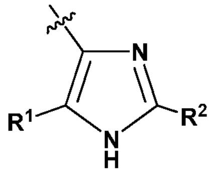 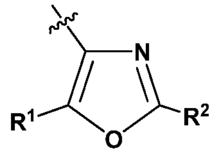

(k) (l)

$R^1$ is selected from H,

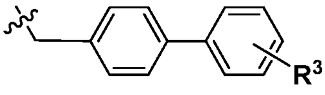

$C_3$-$C_{12}$ alkenyl, $C_1$-$C_{12}$ alkyl, -$NR^4R^5$ substituted $C_2$-$C_4$ alkyl, benzyl or piperidyl which is optionally substituted by -$COOR^4$;
$R^2$ is selected from H,

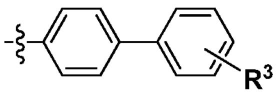 , 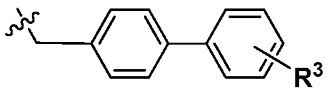 ,

-$COR^4$, -$COOR^4$, -$CONR^4R^5$, -CH=$NNR^4R^5$, -C(=$CH_2$)-OC(=O)$R^4$, $C_1$-$C_{12}$ alkyl, $C_3$-$C_7$ cycloalkyl, phenyl, wherein alkyl, cycloalkyl and phenyl are optionally substituted by halogen, -$NR^4R^5$, -$OR^4$, -$SR^4$, -$SO_2R^4$, -$NHCOR^4$, -$NHSO_2R^4$, -$NHCSNHR^4$,

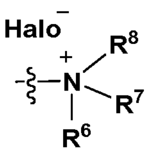 ,

-$N_3$ or phenyl.
$R^3$, optionally at ortho-, meta- or para-positon of the benzene ring, is selected from H, halogen, $C_1$-$C_6$ alkyl or partially or fully halogenated $C_1$-$C_6$ alkyl;
$R^4$ and $R^5$ are independently selected from H, $C_3$-$C_7$ cycloalkyl, straight or branched $C_1$-$C_6$ alkyl, wherein alkyl and cycloalkyl are optionally substituted by -$COOR^9$, -$NR^9R^{10}$, -$OR^9$, -$COR^9$, phenyl, benzyl, aromatic or nonaromatic heterocycle, wherein phenyl, benzyl, aromatic and nonaromatic heterocycle are optionally further substituted by halogen or $C_1$-$C_6$ alkyl; or
$R^4$ and $R^5$ together with the N-atom to which they are attached form 5 to 8-membered nonaromatic heterocycle which may contain another heteroatom selected from the group consisting of N, O and S, and is optionally substituted by halogen, $C_1$-$C_6$ alkyl, -$NR^{11}R^{12}$, -$OR^{11}$, =O or benzyl, wherein $C_1$-$C_6$ alkyl is optionally substituted by -$COOR^4$;
$R^6$, $R^7$ and $R^8$ are independently selected from $C_1$-$C_6$ alkyl, hydroxyl substituted $C_2$-$C_4$ alkyl or benzyl, wherein benzyl is optionally substituted by halogen or $C_1$-$C_6$ alkyl;
$R^9$ and $R^{10}$ are independently selected from H, $C_1$-$C_6$ alkyl; or
$R^9$, $R^{10}$ together with the N-atom to which they are attached form 5 to 8-membered nonaromatic heterocycle which may contain another heteroatom selected from the group consisting ofN, O and S;
$R^{11}$ and $R^{12}$ are independently selected from H, $C_1$-$C_6$ alkyl;
Halo is an abbreviation of halogen.

In a further preferred embodiment, within the compounds of Formula (I) or (III), T is 5-membered aliphatic ring or benzene ring; X is CH or N.

In a further preferred embodiment, within the compounds of Formula (I), when T is 5-membered aliphatic ring,

X is N; when T is benzene ring, X is CH.

In a further preferred embodiment, the compounds of the present invention have the structures of Formula **(IA)-(IG)**, **(IIA)** or **(IIIA)**:

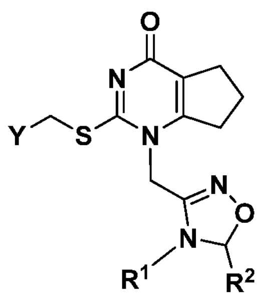

**(IA)**

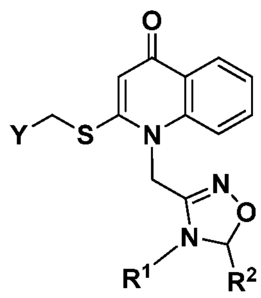

**(IB)**

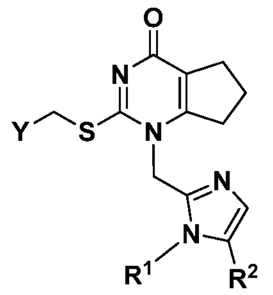

**(IC)**

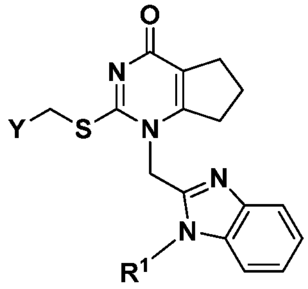

**(ID)**

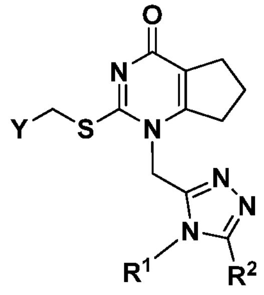

**(IE)**

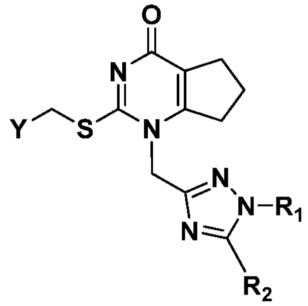

**(IF)**

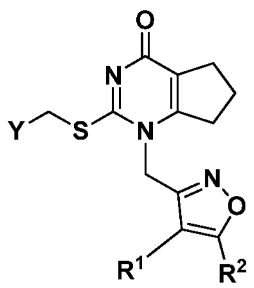

**(IG)**

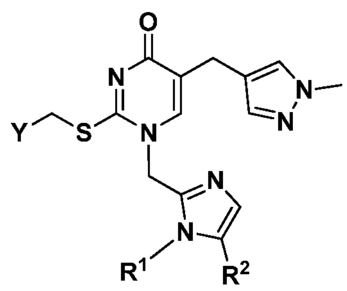

**(IIA)**

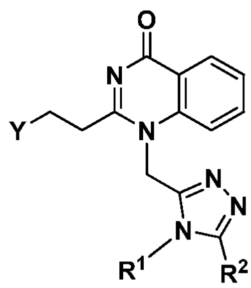

**(IIIA)**

in which:

Y, $R^1$ and $R^2$ are as hereinbefore defined.

In a further preferred embodiment, within the compounds of Formula **(I)**, **(II)**, **(III)**, **(IA)-(IG)**, **(IIA)** or **(IIIA)**:

Y is phenyl ring which is substituted by fluorine atom.

In a further preferred embodiment, within the compounds of Formula **(I), (II), (III)**, **(IA)-(IG)**, **(IIA)** or **(IIIA)**:

Y is 4-fluorophenyl or 2,3-difluorophenyl.

In a further preferred embodiment, within the compounds of Formula **(I)**, **(II)** or **(III)**:

W is selected from 6 structures of Formulae (a-f) as follows:

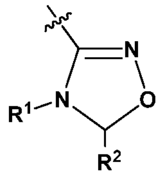
(a)

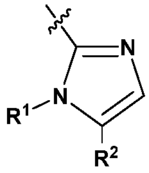
(b)

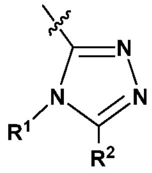
(c)

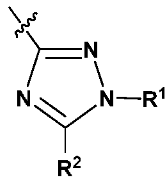
(d)

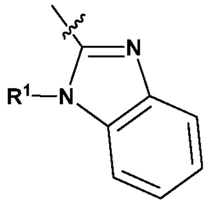
(e)

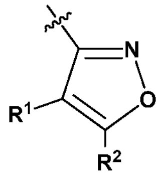
(f)

in which:

$R^1$ and $R^2$ are as hereinbefore defined.

In a further preferred embodiment, within the compounds of Formula **(I)**, **(II)**, **(III)**, **(IA)-(IG)**, **(IIA)** or **(IIIA):**

$R^1$ is

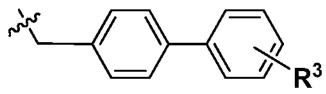

or $-NR^4R^5$ substituted $C_2$-$C_4$ alkyl.

In a further embodiment, within the compounds of Formula **(I)**, **(II)**, **(III)**, **(IA)-(IG)**, **(IIA)** or **(IIIA)**:

$R^1$ is (4-(trifluoromethyl)biphenyl-4-yl)methyl.

In a further preferred embodiment, within the compounds of Formula **(I)**, **(II)**, **(III)**, **(IA)-(IG)**, **(IIA)** or **(IIIA)**:

$R^2$ is $-COR^4$, $-CONR^4R^5$, $C_1$-$C_5$ alkyl or $C_3$-$C_5$ cycloalkyl, wherein alkyl and cycloalkyl are optionally substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

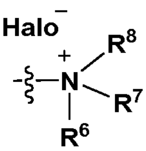
.

In a further preferred embodiment, within the compounds of Formula **(I)**, **(II)**, **(III)**, **(IA)-(IG)**, **(IIA)** or **(IIIA)**:

$R^2$ is $-COR^4$, $-CONR^4R^5$, cyclopropyl or $C_1$-$C_5$ alkyl, wherein alkyl is substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

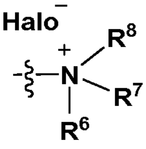
.

In a further preferred embodiment, within the compounds of Formula **(I)**, **(II)**, **(III)**, **(IA)-(IG)**, **(IIA)** or **(IIIA)**:

$R^2$ is $-CONR^4R^5$, cyclopropyl or $C_1$-$C_5$ alkyl, wherein $C_1$-$C_5$ alkyl is substituted by $-NR^4R^5$, ,$-OR^4$, $-SR^4$, $=NNR^4R^5$, or

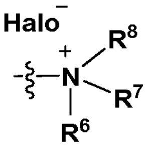.

In a further preferred embodiment, within the compounds of Formula **(I), (II), (III)**, **(IA)-(IG)**, **(IIA)** or **(IIIA)**, $R^2$ is selected from: dimethylcarbamoyl, 2-(diethylamino)ethylcarbamoyl, (2-(diethylamino)ethyl)(methyl)carbamoyl, (dimethylamino)methyl, (diethylamino)methyl, pyrrolidin-1-ylmethyl, ((4-fluorobenzyl)(methyl)amino)methyl, isopropyl, cyclopropyl, 3-(diethylamino)propyl, 4-(diethylamino)butyl, hydroxymethyl, 1-hydroxyethyl, (4-fluorobenzylthio)methyl, (isopropyl(methyl)amino)methyl, ((1-ethylpyrrolidin-2-yl)methylamino)methyl, (4-ethylpiperazin-1-yl)methyl, ((2-(dimethylamino)ethyl)(methyl)amino)methyl, ((2-(diethylamino)ethyl)(methyl)amino)methyl, (((2-(dimethylamino)ethyl)(ethyl)amino)methyl, (((3-(dimethylamino)propyl)(methyl)amino)methyl), (methyl(pyridin-2-ylmethyl)amino)methyl, (4-(dimethylamino)piperidin-1-yl)methyl, (2,2-dimethylhydrazono)methyl, (2-hydroxyethoxy)methyl, (2-(diethylamino)ethoxy)methyl, 1-(2-(dimethylamino)ethylamino)ethyl, 1-((2-(dimethylamino)ethyl)(methyl)amino)ethyl, 1-(2-(diethylamino)ethylamino)ethyl, 1-((2-(diethylamino)ethyl)(methyl)amino)ethyl, 1-((3-(dimethylamino)propyl)(methyl)amino)ethyl, ((methyl(2-(pyrrolidin-1-yl)ethyl)amino)methyl, ((methyl(2-(piperidin-1-yl)ethyl)amino)methyl, 3-(pyrrolidin-1-yl)propyl, 3-(piperidin-1-yl)propyl, 4-(pyrrolidin-1-yl)butyl, 4-(piperidin-1-yl)butyl,

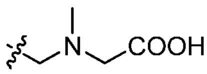, 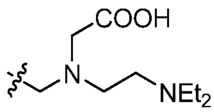, 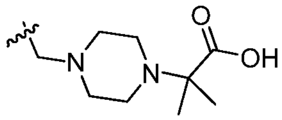, 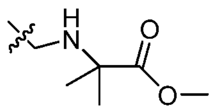,

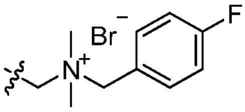, 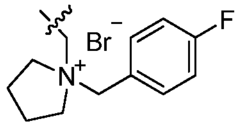, 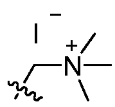, 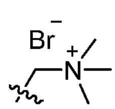, 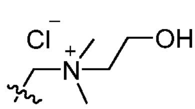.

In a further preferred embodiment, the compound is selected from:

2-((4-fluorobenzylthio)-1-((5-n-heptyl-4,5-dihydro-1,2,4-oxadiazol-3-yl)methyl)-6,7-d ihydro-1*H*-cyclopenta[d]pyrimidin-4(5*H*)-one;

1-(((5-n-decyl-4,5-dihydro-1,2,4-oxadiazol-3-yl)methyl)-2-(4-fluorobenzylthio)-6,7-di hydro-1*H*-cyclopenta[d]pyrimidin-4(5*H*)-one;

2-((4-fluorobenzylthio)-1-((5-n-heptyl-4,5-dihydro-1,2,4-oxadiazol-3-yl)methyl)quinol in-4(1*H*)-one;

2-((4-fluorobenzylthio)-1-((5-(4'-(trifluoromethyl)biphenyl-4-yl)-4,5-dihydro-1,2,4-ox adiazol-3-yl)methyl)-6,7-dihydro-1*H*-cyclopenta[d]pyrimidin-4(5*H*)-one;

(*E*)-2-(4-fluorobenzylthio)-1-((5-n-heptyl-4-(n-oct-1-enyl)-4,5-dihydro-1,2,4-oxadiaz ol-3-yl)methyl)-6,7-dihydro-1*H*-cyclopenta[d]pyrimidin-4(5*H*)-one;

2-((2,3-difluorobenzylthio)-1-((4-(2-morpholinoethyl)-5-(4'-(trifluoromethyl)biphenyl-4-yl)-4,5-dihydro-1,2,4-oxadiazol-3-yl)methyl)-6,7-dihydro-1*H*-cyclopenta[d]pyrimid in-4(5*H*)-one;

ethyl 4-(3-((2-(2,3-difluorobenzylthio)-4-oxo-4,5,6,7-tetrahydro-1*H*-cyclopenta[d]pyrimidi n-1-yl)methyl)-5-(4'-(trifluoromethyl)biphenyl-4-yl)-1,2,4-oxadiazol-4(5*H*)-yl)piperid ine-1-carboxylate;

ethyl 4-(3-((2-(4-fluorobenzylthio)-4-oxo-4,5,6,7-tetrahydro-1*H*-cyclopenta[*d*]pyrimidin-1-yl)methyl)-5-(4'-(trifluoromethyl)biphenyl-4-yl)-1,2,4-oxadiazol-4(5*H*)-yl)piperidine-1-carboxylate;

2-((4-fluorobenzylthio)-1-((4-(2-(piperidin-1-yl)ethyl)-5-(4'-(trifluoromethyl)biphenyl-4-yl)-4,5-dihydro-1,2,4-oxadiazol-3-yl)methyl)-6,7-dihydro-1*H*-cyclopenta[*d*]pyrimid in-4(5*H*)-one;

1-(((4-(2-(diethylamino)ethyl)-5-(4'-(trifluoromethyl)biphenyl-4-yl)-4,5-dihydro-1,2,4-oxadiazol-3-yl)methyl)-2-(4-fluorobenzylthio)-6,7-dihydro-1*H*-cyclopenta[*d*]pyrimidi n-4(5*H*)-one;

2-((2,3-difluorobenzylthio)-1-((4-(2-(piperidin-1-yl)ethyl)-5-(4'-(trifluoromethyl)biphe nyl-4-yl)-4,5-dihydro-1,2,4-oxadiazol-3-yl)methyl)-6,7-dihydro-1*H*-cyclopenta[*d*]pyri midm-4(5*H*)-one;

2-((4-fluorobenzylthio)-1-((5-phenyl-1*H*-imidazol-2-yl)methyl)-6,7-dihydro-1*H*-cyclo penta[*d*]pyrimidin-4(5*H*)-one;

1-(((1*H*-tetrazol-5-yl)methyl)-2-(4-fluorobenzylthio)-6,7-dihydro-1*H*-cyclopenta[*d*]pyr imidin-4(5*H*)-one;

2-((4-fluorobenzylthio)-1-((1-((4'-(trifluoromethyl)biphenyl-4-yl)methyl)-1*H*-imidazol -2-yl)methyl)-6,7-dihydro-1*H*-cyclopenta[*d*]pyrimidin-4(5*H*)-one;

1-(((1-n-dodecyl-1H-imidazol-2-yl)methyl)-2-(4-fluorobenzylthio)-6,7-dihydro-1*H*-cy clopenta[*d*]pyrimidin-4(5*H*)-one;

1-(((1-butyl-1*H*-imidazol-2-yl)methyl)-2-(4-fluorobenzylthio)-6,7-dihydro-1*H*-cyclope nta[*d*]pyrimidin-4(5*H*)-one;

1-(((1-((4'-chlorobiphenyl-4-yl)methyl)-1*H*-imidazol-2-yl)methyl)-2-(4-fluorobenzylth io)-6,7-dihydro-1*H*-cyclopenta[*d*]pyrimidin-4(5*H*)-one;

1-(((1-(biphenyl-4-ylmethyl)-1*H*-imidazol-2-yl)methyl)-2-(4-fluorobenzylthio)-6,7-dih ydro-1*H*-cyclopenta[d]pyrimidin-4(5*H*)-one;

1-(((1-n-butyl-5-(4-chlorophenyl)-1*H*-imidazol-2-yl)methyl)-2-(4-fluorobenzylthio)-6, 7-dihydro-1*H*-cyclopenta[d]pyrimidin-4(5*H*)-one;

1-(((1-n-butyl-5-(4-chlorophenyl)-1*H*-imidazol-2-yl)methyl)-2-(2-nitrobenzylthio)-6,7-dihydro-1*H*-cyclopenta[d]pyrimidin-4(5*H*)-one;

1-(((5-n-decyl-1-(2-(diethylamino)ethyl)-1*H*-imidazol-2-yl)methyl)-2-(4-fluorobenzylt hio)-6,7-dihydro-1*H*-cyclopenta[d]pyrimidin-4(5*H*)-one;1-((1-(2-(diethylamino)ethyl) -5-(4'-(trifluoromethyl)biphenyl-4-yl)-1*H*-imidazol-2-yl)methyl)-2-(4-fluorobenzylthi o)-6,7-dihydro-1*H*-cyclopenta[*d*]pyrimidin-4(5*H*)-one;

1-(((1-benzyl-5-((diethylamino)methyl)-1*H*-imidazol-2-yl)methyl)-2-(4-fluorobenzylth io)-6,7-dihydro-1*H*-cyclopenta[d]pyrimidin-4(5*H*)-one;

2-((4-fluorobenzylthio)-1-((5-methyl-1-((4'-(trifluoromethyl)biphenyl-4-yl)methyl)-1*H* -imidazol-2-yl)methyl)-6,7-dihydro-1*H*-cyclopenta[*d*]pyrimidin-4(5*H*)-one;

1-(((5-((diethylamino)methyl)-1-((4'-(trifluoromethyl)biphenyl-4-yl)methyl)-1*H*-imida zol-2-yl)methyl)-2-(4-fluorobenzylthio)-6,7-dihydro-1*H*-cyclopenta[*d*]pyrimidin-4(5 *H*)-one;

2-((4-fluorobenzylthio)-1-((5-(pyrrolidin-1-ylmethyl)-1-((4'-(trifluoromethyl)biphenyl-4-yl)methyl)-1*H*-imidazol-2-yl)methyl)-6,7-dihydro-1*H*-cyclopenta[d]pyrimidin-4(5*H* )-one;

1-(((5-(((4-fluorobenzyl)(methyl)amino)methyl)-1-((4'-(trifluoromethyl)biphenyl-4-yl) methyl)-1*H*-imidazol-2-yl)methyl)-2-(4-fluorobenzylthio)-6,7-dihydro-1*H*-cyclopenta [d]pyrimidin-4(5*H*)-one;

1-(((5-((4-benzylpiperazin-1-yl)methyl)-1-((4'-(trifluoromethyl)biphenyl-4-yl)methyl)-1*H*-imidazol-2-yl)methyl)-2-(4-fluorobenzylthio)-6,7-dihydro-1*H*-cyclopenta[d]pyrim idin-4(5*H*)-one;

1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[d]pyrimidin-4( 5*H*)-one;

N-(4-fluorobenzyl)-1-(2-((2-(4-fluorobenzylthio)-4-oxo-4,5,6,7-tetrahydro-1*H*-cyclop enta[d]pyrimidin-1-yl)methyl)-1-((4'-(trifluoromethyl)biphenyl-4-yl)methyl)-1*H*-imid azol-5-yl)-N,N-dimethylmethanaminium bromide;

1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[d]pyrimidin-4( 5*H*)-one;

1-((4-fluorobenzyl)-1-((2-((2-(4-fluorobenzylthio)-4-oxo-4,5,6,7-tetrahydro-1*H*-cyclop enta[d]pyrimidin-1-yl)methyl)-1-((4'-(trifluoromethyl)biphenyl-4-yl)methyl)-1*H*-imid azol-5-yl)methyl)pyrrolidinium bromide; ethyl 2-(((2-(4-fluorobenzylthio)-4-oxo-4,5,6,7-tetrahydro-1*H*-cyclopenta[d]pyrimidin-1-yl) methyl)-1-((4'-(trifluoromethyl)biphenyl-4-yl)methyl)-1*H*-imidazole-5-carboxylate;

2-(((2-(4-fluorobenzylthio)-4-oxo-4,5,6,7-tetrahydro-1*H*-cyclopenta[d]pyrimidin-1-yl) methyl)-1-((4'-(trifluoromethyl)biphenyl-4-yl)methyl)-1*H*-imidazole-5-carboxylic acid;

2-(((2-(4-fluorobenzylthio)-4-oxo-4,5,6,7-tetrahydro-1*H*-cyclopenta[d]pyrimidin-1-yl) methyl)-N,N-dimethyl-1-((4'-(trifluoromethyl)biphenyl-4-yl)methyl)-1*H*-imidazole-5-carboxamide;

2-((4-fluorobenzylthio)-1-((1-((4'-(trifluoromethyl)biphenyl-4-yl)methyl)-1*H*-benzo[d] imidazol-2-yl)methyl)-6,7-dihydro-1*H*-cyclopenta[d]pyrimidin-4(5*H*)-one;

1-(((4-ethyl-5-(4'-(trifluoromethyl)biphenyl-4-yl)-4*H*-1,2,4-triazol-3-yl)methyl)-2-(4-fl uorobenzylthio)-6,7-dihydro-1*H*-cyclopenta[d]pyrimidin-4(5*H*)-one;

1-(((4-ethyl-5-(4'-(trifluoromethyl)biphenyl-4-yl)-4*H*-1,2,4-triazol-3-yl)methyl)-2-(4-methoxybenzylthio)-6,7-dihydro-1*H*-cyclopenta[d]pyrimidin-4(5*H*)-one;

2-((2,3-difluorobenzylthio)-1-((4-ethyl-5-(4'-(trifluoromethyl)biphenyl-4-yl)-4*H*-1,2,4-triazol-3-yl)methyl)-6,7-dihydro-1*H*-cyclopenta[d]pyrimidin-4(5*H*)-one;

1-(((4-ethyl-5-(3-phenylpropyl)-4*H*-1,2,4-triazol-3-yl)methyl)-2-(4-fluorobenzylthio)-6,7-dihydro-1*H*-cyclopenta[d]pyrimidin-4(5*H*)-one;

1-(((4-ethyl-5-((4'-(trifluoromethyl)biphenyl-4-yl)methyl)-4*H*-1,2,4-triazol-3-yl)methy l)-2-(4-fluorobenzylthio)-6,7-dihydro-1*H*-cyclopenta[d]pyrimidin-4(5*H*)-one;

2-((4-fluorobenzylthio)-1-((1-((4'-(trifluoromethyl)biphenyl-4-yl)methyl)-1*H*-1,2,4-tria zol-3-yl)methyl)-6,7-dihydro-1*H*-cyclopenta[d]pyrimidin-4(5*H*)-one; methyl 2-((((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-1*H*-1,2,4-triazol-3-yl)meth yl)(methyl)amino)acetate;

2-((((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)meth yl)(methyl)amino)acetic acid;

2-((4-fluorobenzylthio)-1-((5-methyl-4-((4'-(trifluoromethyl)biphenyl-4-yl)methyl)-4*H* -1,2,4-triazol-3-yl)methyl)-6,7-dihydro-1*H*-cyclopenta[d]pyrimidin-4(5*H*)-one;

2-((4-fluorobenzylthio)-1-((5-propyl-4-((4'-(trifluoromethyl)biphenyl-4-yl)methyl)-4*H* -1,2,4-triazol-3-yl)methyl)-6,7-dihydro-1*H*-cyclopenta[d]pyrimidin-4(5*H*)-one;

2-((4-fluorobenzylthio)-1-((5-isopropyl-4-((4'-(trifluoromethyl)biphenyl-4-yl)methyl)-4*H*-1,2,4-triazol-3-yl)methyl)-6,7-dihydro-1*H*-cyclopenta[d]pyrimidin-4(5*H*)-one;

1-(((5-cyclopropyl-4-((4'-(trifluoromethyl)biphenyl-4-yl)methyl)-4*H*-1,2,4-triazol-3-yl )methyl)-2-(4-fluorobenzylthio)-6,7-dihydro-1*H*-cyclopenta[d]pyrimidin-4(5*H*)-one;

1-(((5-(benzylsulfonylmethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;

1-(((5-(3-(diethylamino)propyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;

1-(((5-(4-(diethylamino)butyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;

2-((4-fluorob enzylthio)-1-((5-(hydroxymethyl)-4-((4'-(trifluoromethyl)biphenyl-4-yl)m ethyl)-4H-1,2,4-triazol-3-yl)methyl)-6,7-dihydro-1*H*-cyclopenta[d]pyrimidin-4(5*H*)-o ne;

2-((4-fluorobenzylthio)-1-((4-((4'-(trifluoromethyl)biphenyl-4-yl)methyl)-4*H*-1,2,4-tria zol-3-yl)methyl)-6,7-dihydro-1*H*-cyclopenta[d]pyrimidin-4(5*H*)-one;

5-(((2-(4-fluorobenzylthio)-4-oxo-4,5,6,7-tetrahydro-1*H*-cyclopenta[d]pyrimidin-1-yl) methyl)-4-((4'-(trifluoromethyl)biphenyl-4-yl)methyl)-4*H*-1,2,4-triazole-3-carbaldehy de;

1-(((5-((dimethylamino)methyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;

N-(4-fluorobenzyl)-1-(5-((2-(4-fluorobenzylthio)-4-oxo-4,5,6,7-tetrahydro-1*H*-cyclop enta[d]pyrimidin-1-yl)methyl)-4-((4'-(trifluoromethyl)biphenyl-4-yl)methyl)-4*H*-1,2,4 -triazol-3-yl)-N,N-dimethylmethanaminium bromide;

1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium iodide;

1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium bromide;

1-(((5-((diethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;

1-(((5-(((4-fluorobenzyl)(methyl)amino)methyl)-4-((4'-(trifluoromethyl)biphenyl-4-yl) methyl)-4H-1,2,4-triazol-3-yl)methyl)-2-(4-fluorobenzylthio)-6,7-dihydro-1*H*-cyclope nta[*d*]pyrimidin-4(5*H*)-one;

2-((4-fluorobenzylthio)-1-((5-((isopropyl(methyl)amino)methyl)-4-((4'-(trifluoromethy l)biphenyl-4-yl)methyl)-4*H*-1,2,4-triazol-3-yl)methyl)-6,7-dihydro-1*H*-cyclopenta[*d*]p yrimidin-4(5*H*)-one;

(±)1-((5-(((1-ethylpyrrolidin-2-yl)methylamino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

1-(((5-(((2*S*,6*R*)-2,6-dimethylmorpholino)methyl)-4-((4'-(trifluoromethyl)biphenyl-4-y l)methyl)-4*H*-1,2,4-triazol-3-yl)methyl)-2-(4-fluorobenzylthio)-6,7-dihydro-1*H*-cyclo penta[*d*]pyrimidin-4(5*H*)-one;

methyl-2-((5-((2-(4-fluorobenzylthio)-4-oxo-4,5,6,7-tetrahydro-1*H*-cyclopenta[*d*]pyri midin-1-yl)methyl)-4-((4'-(trifluoromethyl)biphenyl-4-yl)methyl)-4*H*-1,2,4-triazol-3-yl)amino)-2-methylpropanoate;

1-(((5-((4-ethylpiperazin-1-yl)methyl)-4-((4'-(trifluoromethyl)biphenyl-4-yl)methyl)-4 *H*-1,2,4-triazol-3-yl)methyl)-2-(4-fluorobenzylthio)-6,7-dihydro-1*H*-cyclopenta[*d*]pyr imidin-4(5H)-one;

2-((4-fluorobenzylthio)-1-((5-((2-methoxyethylamino)methyl)-4-((4'-(trifluoromethyl) biphenyl-4-yl)methyl)-4*H*-1,2,4-triazol-3-yl)methyl)-6,7-dihydro-1*H*-cyclopenta[*d*]py rimidin-4(5*H*)-one;

methyl-2-(4-((5-((2-(4-fluorobenzylthio)-4-oxo-4,5,6,7-tetrahydro-1*H*-cyclopenta[*d*]p yrimidin-1-yl)methyl)-4-((4'-(trifluoromethyl)biphenyl-4-yl)methyl)-4*H*-1,2,4-triazol-3-yl)methyl)piperazin-1-yl)-2-methylpropanoate;

2-((4-((5-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)-4*H*-1,2,4-triazol-3-yl)m ethyl)piperazin-1-yl)-2-methylpropanoic acid;

1-(((5-(((2-(dimethylamino)ethyl)(methyl)amino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one tartrate;

1-(((5-(((3-(dimethylamino)propyl)(methyl)amino)methyl)-4-((4'-(trifluoromethyl)biph enyl-4-yl)methyl)-4*H*-1,2,4-triazol-3-yl)methyl)-2-(4- fluorobenzylthio)-6,7-dihydro-1 *H*-cyclopenta[*d*]pyrimidin-4(5*H*)-one;

2-((4-fluorobenzylthio)-1-((5-((2-morpholinoethylamino)methyl)-4-((4'-(trifluorometh yl)biphenyl-4-yl)methyl)-4*H*-1,2,4-triazol-3-yl)methyl)-6,7-dihydro-1*H*-cyclopenta[*d*] pyrimidin-4(5*H*)-one;

(*R*)-1-((5-((3-(dimethylamino)pyrrolidin-1-yl)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

(*S*)-1-((5-((3-(dimethylamino)pyrrolidin-1-yl)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

2-((4-fluorobenzylthio)-1-((5-((2-(piperidin-1-yl)ethylamino)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

2-((4-fluorobenzylthio)-1-((5-((2-(pyrrolidin-1-yl)ethylamino)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-((2-(diisopropylamino)ethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-((2-(diethylamino)ethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

2-((4-fluorobenzylthio)-1-((5-((4-methylpiperazin-1-yl)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

2-((4-fluorobenzylthio)-1-((5-((methyl(pyridin-2-ylmethyl)amino)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-((cyclopropyl(methyl)amino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-((4-(dimethylamino)piperidin-1-yl)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-((3,3-difluoropyrrolidin-1-yl)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-((2-(dimethylamino)ethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

(*S*)-1-((5-(((1-ethylpyrrolidin-2-yl)methylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

2-((4-fluorobenzylthio)-1-((5-((2-(2-oxoimidazolidin-1-yl)ethylamino)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-(azidomethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one ;

1-(((5-(aminomethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

N-((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)methyl)ethanesulfonamide;

2-((4-fluorobenzylthio)-1-((5-((2-oxoimidazolidin-1-yl)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)methyl)-3-methylthiourea;

1-(((5-(chloromethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

2-((4-fluorobenzylthio)-1-((5-((4-fluorobenzylthio)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidm-4(5*H*)-one;

(*E*)-1-((5-((2,2-dimethylhydrazono)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

(E)-1-((5-((2-tert-butylhydrazono)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

(*E*)-2-(4-fluorobenzylthio)-1-((5-((piperidin-1-ylimino)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)vinyl acetate;

2-((4-fluorobenzylthio)-1-((5-(1-hydroxyethyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-acetyl-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

N-((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)methyl)-2-hydroxy-*N,N*-dimethylethanaminium chloride;

2-((4-fluorobenzylthio)-1-((5-((2-hydroxyethoxy)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one;

1-(((5-((2-(diethylamino)ethoxy)methyl)-4-((4'-(trifluoromethyl)biphenyl-4-yl)methyl) -4*H*-1,2,4-triazol-3-yl)methyl)-2-(4-fluorobenzylthio)-6,7-dihydro-1*H*-cyclopenta[*d*]p yrimidin-4(5*H*)-one;
1-(((5-(n-butoxymethyl)-4-((4'-(trifluoromethyl)biphenyl-4-yl)methyl)-4*H*-1,2,4-triazo l-3-yl)methyl)-2-(4-fluorobenzylthio)-6,7-dihydro-1*H*-cyclopenta[*d*]pyrimidin-4(5*H*one;
1-(((5-(1-(2-(dimethylamino)ethylamino)ethyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[*d*]pyrimidin-4(5*H*)-one;
1-(((5-(1-((2-(dimethylamino)ethyl)(methyl)amino)ethyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;
1-(((5-(1-(2-(diethylamino)ethylamino)ethyl)-4-((4'-(trifluoromethyl)biphenyl-4-yl)me thyl)-4*H*-1,2,4-triazol-3-yl)methyl)-2-(4-fluorobenzylthio)-6,7-dihydro-1*H*-cyclopenta [*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one tartrate;
1-(((5-(1-((3-(dimethylamino)propyl)(methyl)amino)ethyl)-4-((4'-(trifluoromethyl)bip henyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((5-(((2-(dimethylamino)ethyl)(ethyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
ethyl-2-((2-(diethylamino)ethyl)((3-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl )-4*H*-1,2,4-triazol-5-yl)methyl)amino)acetate;
2-(((2-(diethylamino)ethyl)((3-((2-(4-fluorobenzylthio)-4-oxo-4,5,6,7-tetrahydro-1*H*-c yclopenta[*d*]pyrimidin-1-yl)methyl)-4-((4'-(trifluoromethyl)biphenyl-4-yl)methyl)-4*H*--1,2,4-triazol-5-yl)methyl)amino)acetic acid;
*N*-(2-(diethylamino)ethyl)-2-((2-(4-fluorobenzylthio)-4-oxo-4,5,6,7-tetrahydro-1*H*-cy clopenta[*d*]pyrimidin-1-yl)methyl)-1-((4'-(trifluoromethyl)biphenyl-4-yl)methyl)-1*H*-i midazole-5-carboxamide;
*N*-(2-(diethylamino)ethyl)-2-((2-(4-fluorobenzylthio)-4-oxo-4,5,6,7-tetrahydro-1*H*-cy clopenta[*d*]pyrimidin-1-yl)methyl)-*N*-methyl-1-((4'-(trifluoromethyl)biphenyl-4-yl)me thyl)-1*H*-imidazole-5-carboxamide;
1-(((5-(((2-(diethylammo)ethyl)(methyl)amino)methyl)-1-((4'-(trifluoromethyl)biphen yl-4-yl)methyl)-1*H*-imidazol-2-yl)methyl)-2-(4-fluorobenzylthio)-6,7-dihydro-1*H*-cyc lopenta[*d*]pyrimidin-4(5*H*)-one;
2-((4-fluorobenzylthio)-1-((5-((methyl(2-(pyrrolidin-1-yl)ethyl)amino)methyl)-4-((4'-(t rifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-1*H*-imidazol-2-yl)methyl)-6,7-dihydro-1*H*-cyclopenta[*d*]pyrimidin-4(5*H*)-one;2-(4-fl uorobenzylthio)-1-((5-(3-pyrrolidin-1-yl)propyl)-4-((4'-(trifluoromethyl)biphenyl-4-y l)methyl)-4*H*-1,2,4-triazol-3-yl)methyl)-6,7-dihydro-1*H*-cyclopenta[*d*]pyrimidin-4(5*H* )-one;
2-((4-fluorobenzylthio)-1-((5-(3-(piperidin-1-yl)propyl)-4-((4'-(trifluoromethyl)biphen yl-4-yl)methyl)-4*H*-1,2,4-triazol-3-yl)methyl)-6,7-dihydro-1*H*-cyclopenta[*d*]pyrimidi n-4(5*H*)-one;
2-((4-fluorobenzylthio)-1-((5-(4-(pyrrolidin-1-yl)butyl)-4-((4'-(trifluoromethyl)biphen yl-4-yl)methyl)-4*H*-1,2,4-triazol-3-yl)methyl)-6,7-dihydro-1*H*-cyclopenta[*d*]pyrimidi n-4(5*H*)-one;
2-((4-fluorobenzylthio)-1-((5-(4-(piperidin-1-yl)butyl)-4-((4'-(trifluoromethyl)biphenyl -4-yl)methyl)-4*H*-1,2,4-triazol-3-yl)methyl)-6,7-dihydro-1*H*-cyclopenta[*d*]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
methyl-3-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)isoxazole-5-carboxylate;
2-((4-fluorobenzylthio)-1-((5-(hydroxymethyl)-4-((4'-(trifluoromethyl)biphenyl-4-yl)m ethyl)isoxazol-3-yl)methyl)-6,7-dihydro-1*H*-cyclopenta[*d*]pyrimidin-4(5*H*)-one;
1-(((5-(chloromethyl)-4-((4'-(trifluoromethyl)biphenyl-4-yl)methyl)isoxazol-3-yl)meth yl)-2-(4-fluorobenzylthio)-6,7-dihydro-1*H*-cyclopenta[*d*]pyrimidin-4(5*H*)-one;
1-(((5-((diethylamino)methyl)-4-((4'-(trifluoromethyl)biphenyl-4-yl)methyl)isoxazol-3 -yl)methyl)-2-(4-fluorobenzylthio)-6,7-dihydro-1*H*-cyclopenta[*d*]pyrimidin-4(5*H*)-on e;
1-(((5-(((2-(dimethylamino)ethyl)(methyl)amino)methyl)-4-((4'-(trifluoromethyl)biphe nyl-4-yl)methyl)isoxazol-3-yl)methyl)-2-(4-fluorobenzylthio)-6,7-dihydro-1*H*-cyclop enta[*d*]pyrimidin-4(5*H*)-one;
1-(((5-(((2-(diethylamino)ethyl)(methyl)amino)methyl)-4-((4'-(trifluoromethyl)biphen yl-4-yl)methyl)isoxazol-3-yl)methyl)-2-(4-fluorobenzylthio)-6,7-dihydro-1*H*-cyclope nta[*d*]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)isoxazol-3-yl)methyl)-6,7-dihydro-1*H*-cyclopent a[*d*]pyrimidin-4(5*H*)-one;
2-((4-fluorophenethyl)-1-((4-((4'-(trifluoromethyl)biphenyl-4-yl)methyl)-4*H*-1,2,4-tria zol-3-yl)methyl)quinazolin-4(1*H*)-one.

In the second aspect, the present invention provides a pharmaceutical composition that comprises a therapeutically effective amount of one or more azole heterocylic compounds of the invention, *cis-trans* isomers, enantiomers, diastereoisomers, racemes, solvates, hydrates, or pharmaceutically acceptable salts thereof, and a pharmaceutically acceptable auxiliary.

In a preferred embodiment, the above mentioned pharmaceutical composition may comprise one or more agents selected from the group consisting of anti-hyperlipidaemic, anti-atherosclerotic, anti-diabetic, anti-anginal, anti-inflammatory, anti-hypertension agents and agents for lowering Lp(a).

In the third aspect, the present invention provides the use of said azole heterocylic compounds, *cis-trans* isomers, enantiomers, diastereoisomers, racemes, solvates, hydrates, or pharmaceutically acceptable salts thereof, in manufacturing a medicament as Lp-$PLA_2$ inhibitor.

In the fourth aspect, the present invention provides the use of said azole heterocylic compounds, *cis-trans* isomers, enantiomers, diastereoisomers, racemes, solvates, hydrates, or pharmaceutically acceptable salts thereof, in manufacturing a medicament for preventing, curing or ameliorating diseases associated with activity of the enzyme Lp-$PLA_2$.

In a preferred embodiment, the above mentioned diseases may include atherosclerosis, stroke, coronary heart disease, diabetes, asthma, psoriasis, rheumatoid arthritis, or acute and chronic inflammation.

In the fourth aspect, the present invention provides a method of preventing, curing or ameliorating a disease associated with activity of the enzyme Lp-$PLA_2$. The mentioned method involves treating a patient with said azole heterocylic compounds, *cis-trans* isomers, enantiomers, diastereoisomers, racemes, solvates, hydrates, or pharmaceutically acceptable salts thereof, or a composition of this invention.

In a preferred embodiment, the above mentioned diseases may include atherosclerosis, stroke, coronary heart disease, diabetes, asthma, psoriasis, rheumatoid arthritis, or acute and chronic inflammation.

In the fifth aspect, the present invention provides the preparation methods of the azole heterocyclic compounds of Formula (I), *cis-trans* isomers, enantiomers, diastereoisomers, racemes, solvates, hydrates, or pharmaceutically acceptable salts thereof. The synthesis of the compounds in this invention is featured by employing one of the processes illustrated by synthetic routes (1-4) as follows:

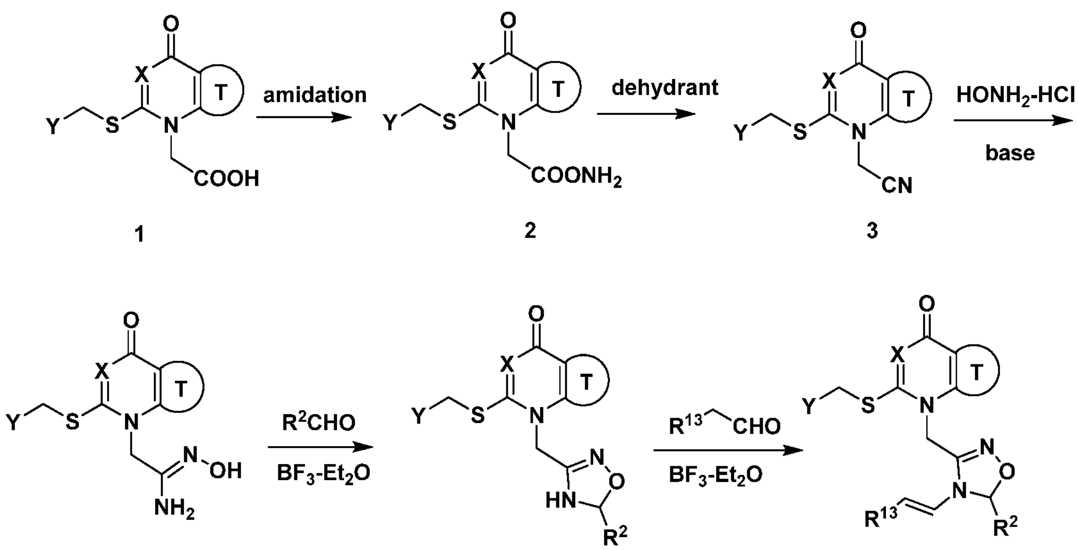

1 2 3 4 5 6

**Synthetic Route 1**

When W is Formula (a) meanwhile $R^1$ is alkenyl in the compounds of Formula (I), the compounds of Formula (I) (i.e. compound **6** in route 1) can be prepared according to the method illustrated by synthetic route 1, in which: $R^{13}$ is $C_1$-$C_{10}$ alkyl; T, X, Y and $R^2$ are as hereinbefore defined;

Compound **1** is converted to compound **2** by amidation which is then dehydrated to give compound **3**; compound **3** reacts with hydroxylamine hydrochloride in the presence of a base to produce compound **4**; catalyzed by boron trifluoride-diethyl ether complex, the reaction of compound **4** with $R^2CHO$ yields compound **5** in an aprotic solvent; compound **5** is then transformed into compound **6** by reaction with $R^{13}CH_2CHO$ in the presence of boron trifluoride-diethyl ether complex as the catalyst;

or,

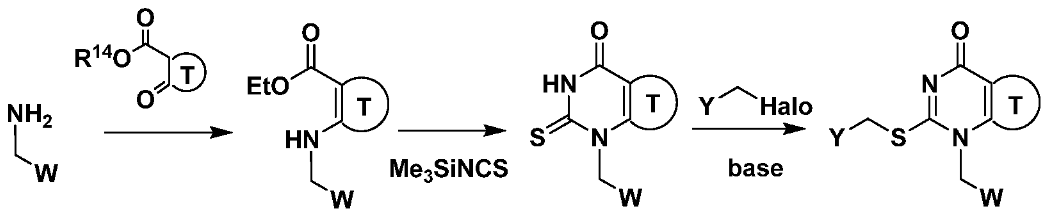


7 8 9 10

**Synthetic Route 2**

When T is 4 to 6-membered aliphatic ring and X=N, meanwhile $R^1$ is not alkenyl in the compounds of Formula (I), the compounds of Formula (I) (i.e. compound **10** in route 2) can be prepared according to the method illustrated by synthetic route 2, in which: $R^{14}$ is methyl or ethyl; Halo, T, W and Y are as hereinbefore defined;

In a polar solvent, compound 7 is condensed with a cycloalkanone carboxylate

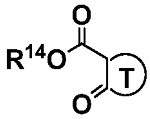

to yield compound **8** in the presence of a dehydrant; compound **8** reacts with $Me_3SiNCS$ to produce compound 9 which is then converted to compound 10 by reaction with Y^Halo in a polar solvent in the presence of a base;
or,

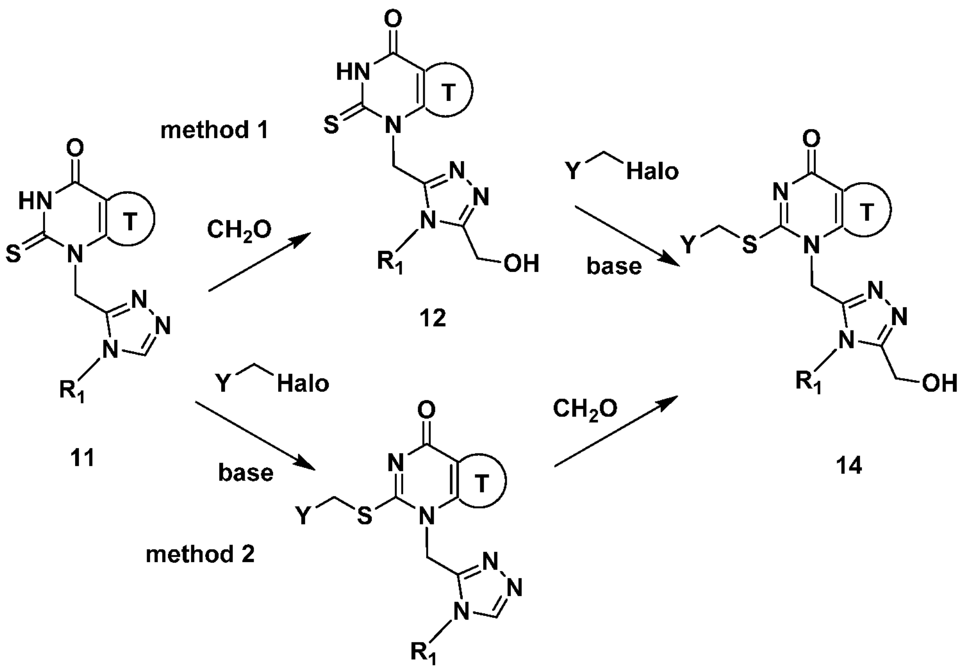


11 12 13 14

**Synthetic Route 3**

When W is Formula (c) meanwhile $R^2$ is hydroxymethyl in the compounds of Formula (I), above mentioned compound 10 can be prepared according to either of the methods illustrated by synthetic route 3, in which Halo, T, Y and $R^1$ are as hereinbefore defined;

According to the first method, compound 11 is heated with aqueous formaldehyde to afford compound 12 which then reacts in the presence of a base with

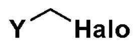

in a polar solvent to give compound **14**;

According to the second method, compound **11** first reacts with

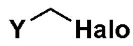

to give compound **13** which is then heated with aqueous formaldehyde to afford compound **14**; or,

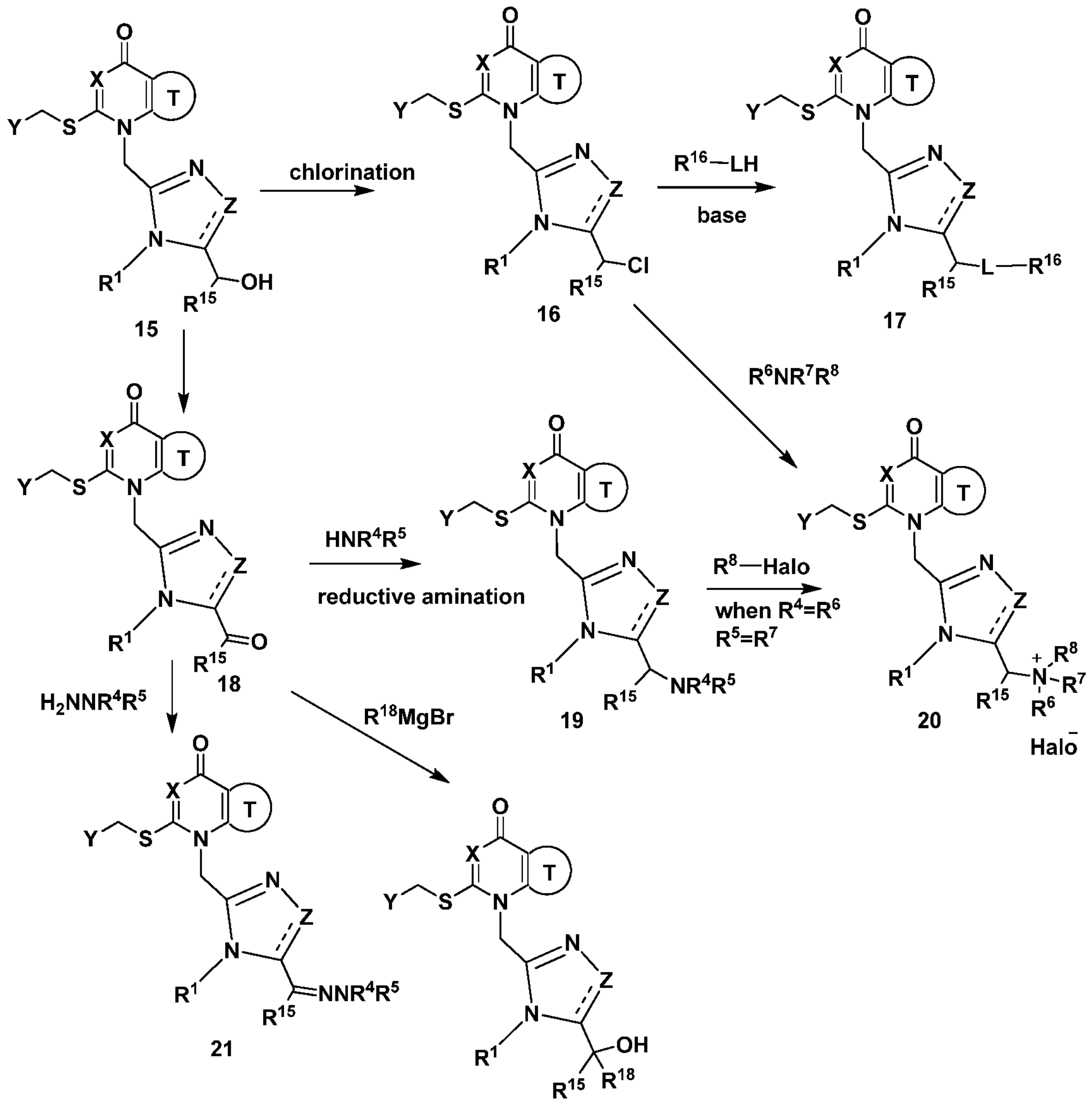


15 16 17 18 19 20 21 22

**Synthetic Route 4**

A compound of Formula (I) can be converted into another structure of the compound of Formula (I) by functional transformation, which is illustrated by route 4 as follows:

When $R^2$ in Formula (I) is $\alpha$-hydroxyalkyl, the compound of Formula (I) can be represented by the structure of compound **15**, in which $R^{15}$ is H or $C_1$-$C_6$ alkyl; $R^{16}$ is $C_1$-$C_6$ alkyl that is optionally substituted by $NR^4R^5$ or phenyl,

wherein phenyl is optionally substituted by halogen; $R^{18}$ is $C_1$-$C_6$ alkyl; L is $NR^4$, O or S; Z is CH, N or O; T, X, Y, $R^1$, $R^4$, $R^5$, $R^6$, $R^7$ and $R^8$ are as hereinbefore defined;

Compound **15** is chlorinated to afford compound **16** which then reacts with $R^{16}$LH to give compound 17 in the presence of a base;

Compound **15** is oxidized to afford compound **18** which then reacts with $HNR^4R^5$ to give compound 19 in the presence of a reductant;

The condensation reaction of compound **16** with $R^6NR^7R^8$ yields compound **20**;

The condensation reaction of compound **19** with $R^8$-Halo also yields compound **20**;

The condensation reaction of compound **18** with $H_2NNR^4R^5$ yields compound 21;

The reaction of compound **18** with $R^{18}$BrMg yields compound **22**.

## DETAILED DISCRIPTION OF THE INVENTION

The term "substituted" in the invention means substituted by one or more groups. In the case where more than one group is selected from the same series of candidate substituents, they may be the same or different.

The term "independently" in the invention means that more than one group as defined may be selected from the same series of candidate groups, they do not influence each other, and they may be the same or different.

The term "aliphatic ring" in this invention refers to a cyclic hydrocarbonyl that has 3 to 12 carbon atoms and may have one or more unsaturated bonds. Of particular interest are 4 to 6-membered aliphatic rings. Examples include, but are not limited to cyclopentane or cyclopentene.

As used hererin, the term "alkyl" or similar terms such as "alkoxy" includes all straight and branched isomers containing a specified number of carbon atoms. Of particular interest is $C_1$-$C_{12}$ alkyl, with $C_1$-$C_6$ alkyl preferred. More preferably, it is $C_1$-$C_4$ alkyl, most preferably $C_1$-$C_3$ alkyl. Examples include, but are not limited to methyl, ethyl, *n*-propyl, isopropyl, *n*-butyl, *sec*-butyl, isobutyl, *tert*-butyl.

As used hererin, the term "alkenyl" refers to all straight and branched isomers that have a specified number of carbon atoms, meanwhile have 1 to 5 double bonds, such as $C_3$-$C_{12}$ alkenyl and $C_3$-$C_8$ alkenyl. Examples include, but are not limited to vinyl and propenyl.

As used hererin, the term "cycloalkyl" refers to a non-aromatic, saturated, cyclic aliphatic groups containing a specified number of carbon atoms. Examples include, but are not limited to cyclopropyl, cyclobutyl, cyclopentyl and cyclohexyl.

Unless otherwise defined, the phrase "nonaromatic heterocycle" refers to a saturated monocyclic ring system that has 2 to 10 carbon atoms and 1 to 4 heteratoms selected from the group consisting of N, O and S. Examples include, but are not limited to aziridine, thiirane, azetidine, tetrahydrofuran, pyrrolidine, piperidine, piperazine and morpholine.

As used hererin, the phrase "aromatic heterocycle" refers to a monocyclic ring system that complies with Hückel's Rule, has 4 to 10 ring atoms and 1 to 4 heteratoms selected from the group consisting of N, O and S. Examples include, but are not limited to pyridine, pyrimidine, pyrazole, furan, thiophene, thiazole and pyrazine.

Unless otherwise indicated, the term "halogen" in this invention includes fluorine, chlorine, bromine and iodine.

Unless otherwise indicated, a substitution on an alkyl, alkenyl or cycloalkyl group in this invention may occur on any carbon atom as long as the substitution on this carbon atom is not saturated.

Unless otherwise indicated, a substitution on a phenyl or a heterocyclic ring in this invention may occur at any position which is not yet occupied by an atom other than hydrogen.

The term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

In certain embodiments, the compounds of Formula (I), (II) or (III) are salts in themselves, for example, when $R^2$ is alkyl which is substituted by

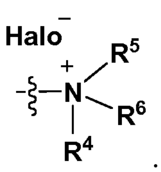

In addition, certain other compounds can form pharmaceutically acceptable salts which are a particularly important portion of the scope of this invention.

The term "pharmaceutically acceptable salts" refers to salts that retain the desired biological activity of the

subject compound and exhibit minimal undesired toxicological effects. These pharmaceutically acceptable salts may be obtained directly in the preparation and purification of the compound, or separately by reacting the compound in its free acid or free base form with a suitable base or acid, respectively.

Specifically, certain compounds of this invention may contain a basic group (for example, but without limitation, when $R^2$ is alkyl which is substituted by-$NR^4R5$), and therefore are capable of forming pharmaceutically acceptable salts by treatment with a suitable acid. Suitable acids include both pharmaceutically acceptable inorganic acids and organic acids. Representative examples of pharmaceutically acceptable salts include, but are not limited to hydrochloride, sulfate, hydrobromide, mesylate, nitrate, phosphate, acetate, oxalate, succinate, tartrate, maleate, arginine salts.

Certain compounds of this invention may contain an acid group (for example, but without limitation, when $R^2$ is alkyl which is substituted by -COOH), and therefore are capable of forming pharmaceutically acceptable salts by treatment with a suitable base. Suitable bases include both pharmaceutically acceptable inorganic bases and organic bases. Representative examples of pharmaceutically acceptable inorganic basic salts include, but are not limited to sodium, potassium, lithium, calcium, aluminium, zinc, and ammonium salts. Examples of organic basic salts include, but are not limited to the salts formed by treatment of an acid group with methylamine, ethylamine, triethylamine, meglumine, tromethamine.

Some compounds or their pharmaceutically acceptable salts in this invention are crystallized or recrystallized from water or organic solvents. The crystalline product may contain solvent molecules that are used. Additionally, different crystallization conditions may result in different polymorphic forms of the crystalline products. Therefore, this invention includes within its scope all crystalline products containing different amounts of solvent as well as all polymorphic forms of the compounds of Formula (I), (II) or (III).

Some compounds of this invention may contain one or more chiral centers. For example, but without limitation, when W is Formula (a), various optical isomers may exist, including racemes, racemic mixtures, enantiomers, diastereoisomers, diastereoisomeric mixtures. This invention includes within its scope all optical isomers of the compounds of Formula (I), (II) or (III). Some compounds of Formula (I), (II) or (III) may be present in the form of *cis-trans* isomers, for example, but without limitation, when $R^1$ is alkenyl. Therefore, this invention includes within its scope the individual *cis*- or *trans*- isomer as well as the mixture of both isomers of a compound. Some compounds of Formula (I), (II) or (III) may have rotational isomers as a result of rotational restriction of certain group within the molecule. This invention includes within its scope the individual rotational isomer as well as the mixture of several rotational isomers of a compound.

As used herein, the term "solvate" refers to a molecular complex of a compound of this invention with one or more stoichiometric solvent molecules that are pharmaceutically acceptable, such as ethanol. When the solvent is water, the term "hydrate" is used.

## COMPOUNDS OF PARTICULAR INTEREST

Within the definition of the substituents of the compounds shown by Formula (I), (II) or (III), the following more detailed description of the groups are of particular interest:

Preferably, T is 5-membered aliphatic ring or benzene ring;
Preferably, X is CH or N;
More preferably, within the compounds of Formula (I), when T is 5-membered aliphatic ring, X is N; when T is benzene, X is CH.

Preferably, Y is phenyl which is substituted by halogen, more preferably by fluorine atoms. Most preferably, Y is 4-fluorophenyl or 2,3-difluorophenyl;
W is selected from 6 structures of Formulae (a-f) as follows:

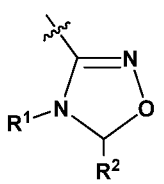
(a)

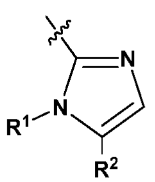
(b)

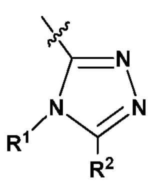
(c)

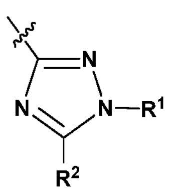
(d)

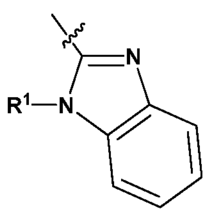
(e)

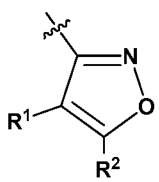
(f)

Preferably, $R^1$ is

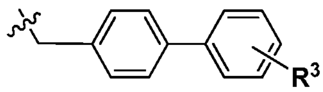

or $-NR^4R^5$ substituted $C_2$-$C_4$ alkyl;
More preferably, $R^1$ is

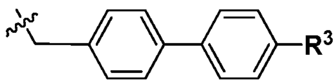

or $-CH_2CH_2NR^4R^5$;
Most preferably, $R^1$ is (4-(trifluoromethyl)biphenyl-4-yl)methyl.

Preferably, $R^2$ is $-COR^4$, $-CONR^4R^5$, $C_1$-$C_5$ alkyl or $C_3$-$C_5$ cycloalkyl, wherein alkyl or cycloalkyl is optionally substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

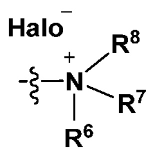
;

More preferably, $R^2$ is $-CONR^4R^5$, cyclopropyl or $C_1$-$C_5$ alkyl, wherein the alkyl is substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

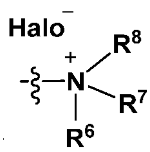
;

Most preferably, $R^2$ is $-CONR^4R^5$, cyclopropyl or $C_1$-$C_5$ alkyl, wherein the $C_1$-$C_5$ alkyl is substituted by $-NR^4R^5$, $-OR^4$, $-SR^4$, $=NNR^4R^5$, or

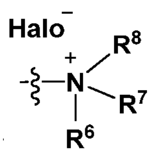
;

Representative examples of $R^2$ include, but are not limited to dimethylcarbamoyl, 2-(diethylamino)ethylcarbamoyl, (2-(diethylamino)ethyl)(methyl)carbamoyl, (dimethylamino)methyl, (diethylamino)methyl, pyrrolidin-1-ylmethyl, ((4-fluorobenzyl)(methyl)amino)methyl, isopropyl, cyclopropyl, 3-(diethylamino)propyl, 4-(diethylamino)butyl, hydroxymethyl, 1-hydroxyethyl, (4-fluorobenzylthio)methyl, (isopropyl(methyl)amino)methyl, ((1-ethylpyrrolidin-2-yl)methylamino)methyl, (4-ethylpiperazin-1-yl)methyl, ((2-(dimethylamino)ethyl)(methyl)amino)methyl, ((2-(diethylamino)ethyl)(methyl)amino)methyl, (((2-(dimethylamino)ethyl)(ethyl)amino)methyl, (((3-(dimethylamino)propyl)(methyl)amino)methyl), (methyl(pyridin-2-ylmethyl)amino)methyl, (4-(dimethylamino)piperidin-1-yl)methyl, (2,2-dimethylhydrazono)methyl, (2-hydroxyethoxy)methyl, (2-(diethylamino)ethoxy)methyl, 1-(2-(dimethylamino)ethylamino)ethyl, 1-((2-(dimethylamino)ethyl)(methyl)amino)ethyl, 1-(2-(diethylamino)ethylamino)ethyl, 1-((2-(diethylamino)ethyl)(methyl)amino)ethyl, 1-((3-(dimethylamino)propyl)(methyl)amino)(methyl)ethyl, ((methyl(2-(pyrrolidin-1-yl)ethyl)amino)methyl, ((methyl(2-(piperidin-1-yl)ethyl)amino)methyl, 3-(pyrrolidin-1-yl)propyl, 3-(piperidin-1-yl)propyl, 4-(pyrrolidin-1-yl)butyl, 4-(piperidin-1-yl)butyl,

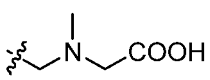
,
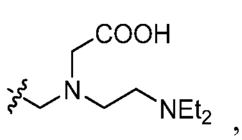
,
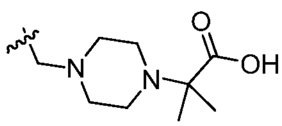
,
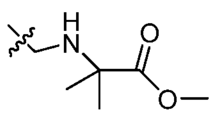
,

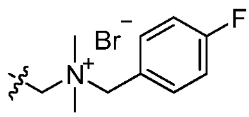, 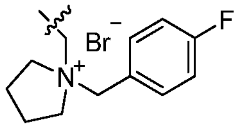, 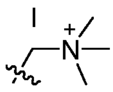, 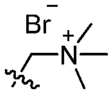, 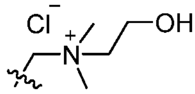.

Compounds of Formula **(I)**, **(II) or (III)** include:

2-((4-fluorobenzylthio)-1-((5-n-heptyl-4,5-dihydro-1,2,4-oxadiazol-3-yl)methyl)-6,7-d ihydro-1*H*-cyclopenta[*d*]pyrimidin-4(5*H*)-one;

1-(((5-n-decyl-4,5-dihydro-1,2,4-oxadiazol-3-yl)methyl)-2-(4-fluorobenzylthio)-6,7-di hydro-1*H*-cyclopenta[*d*]pyrimidin-4(5*H*)-one;

2-((4-fluorobenzylthio)-1-((5-n-heptyl-4,5-dihydro-1,2,4-oxadiazol-3-yl)methyl)quinol in-4(1*H*)-one;

2-((4-fluorobenzylthio)-1-((5-(4'-(trifluoromethyl)biphenyl-4-yl)-4,5-dihydro-1,2,4-ox adiazol-3-yl)methyl)-6,7-dihydro-1*H*-cyclopenta[*d*]pyrimidin-4(5*H*)-one;

(*E*)-2-(4-fluorobenzylthio)-1-((5-n-heptyl-4-(n-oct-1-enyl)-4,5-dihydro-1,2,4-oxadiaz ol-3-yl)methyl)-6,7-dihydro-1*H*-cyclopenta[d]pyrimidin-4(5*H*)-one;

2-((2,3-difluorobenzylthio)-1-((4-(2-morpholinoethyl)-5-(4'-(trifluoromethyl)biphenyl-4-yl)-4,5-dihydro-1,2,4-oxadiazol-3-yl)methyl)-6,7-dihydro-1*H*-cyclopenta[*d*]pyrimid in-4(5*H*)-one;

ethyl 4-(3-((2-(2,3-difluorobenzylthio)-4-oxo-4,5,6,7-tetrahydro-1*H*-cyclopenta[d]pyrimidi n-1-yl)methyl)-5-(4'-(trifluoromethyl)biphenyl-4-yl)-1,2 ,4-oxadiazol-4(5*H*)-yl)piperid ine-1-carboxylate;

ethyl 4-(3-((2-(4-fluorobenzylthio)-4-oxo-4,5,6,7-tetrahydro-1*H*-cyclopenta[*d*]pyrimidin-1-yl)methyl)-5-(4'-(trifluoromethyl)biphenyl-4-yl)-1,2,4-oxadiazol-4(5*H*)-yl)piperidine-1-carboxylate;

2-((4-fluorobenzylthio)-1-((4-(2-(piperidin-1-yl)ethyl)-5-(4'-(trifluoromethyl)biphenyl-4-yl)-4,5-dihydro-1,2,4-oxadiazol-3-yl)methyl)-6,7-dihydro-1*H*-cyclopenta[*d*]pyrimid in-4(5*H*)-one;

1-(((4-(2-(diethylamino)ethyl)-5-(4'-(trifluoromethyl)biphenyl-4-yl)-4,5-dihydro-1,2,4-oxadiazol-3-yl)methyl)-2-(4-fluorobenzylthio)-6,7-dihydro-1*H* cyclopenta[*d*]pyrimidi n-4(5*H*)-one;

2-((2,3-difluorobenzylthio)-1-((4-(2-(piperidin-1-yl)ethyl)-5-(4'-(trifluoromethyl)biphe nyl-4-yl)-4,5-dihydro-1,2,4-oxadiazol-3-yl)methyl)-6,7-dihydro-1*H*-cyclopenta[*d*]pyri midin-4(5*H*)-one;

2-((4-fluorobenzylthio)-1-((5-phenyl-1*H*-imidazol-2-yl)methyl)-6,7-dihydro-1*H*-cyclo penta[*d*]pyrimidin-4(5*H*)-one;

1-(((1H-tetrazol-5-yl)methyl)-2-(4-fluorobenzylthio)-6,7-dihydro-1*H*-cyclopenta[*d*]pyr imidin-4(5*H*)-one;

2-((4-fluorobenzylthio)-1-((1-((4'-(trifluoromethyl)biphenyl-4-yl)methyl)-1*H*-imidazol -2-yl)methyl)-6,7-dihydro-1*H*-cyclopenta[*d*]pyrimidin-4(5*H*)-one;

1-(((1-dodecyl-1*H*-imidazol-2-yl)methyl)-2-(4-fluorobenzylthio)-6,7-dihydro-1*H*-cycl openta[*d*]pyrimidin-4(5*H*)-one;

1-(((1-butyl-1*H*-imidazol-2-yl)methyl)-2-(4-fluorobenzylthio)-6,7-dihydro-1*H*-cyclope nta[*d*]pyrimidin-4(5*H*)-one;

1-(((1-((4'-chlorobiphenyl-4-yl)methyl)-1*H*-imidazol-2-yl)methyl)-2-(4-fluorobenzylth io)-6,7-dihydro-1H-cyclopenta[*d*]pyrimidin-4(5*H*)-one;

1-(((1-(biphenyl-4-ylmethyl)-1*H*-imidazol-2-yl)methyl)-2-(4-fluorobenzylthio)-6,7-dih ydro-1*H*-cyclopenta[*d*]pyrimidin-4(5*H*)-one;

1-(((1-n-butyl-5-(4-chlorophenyl)-1*H*-imidazol-2-yl)methyl)-2-(4-fluorobenzylthio)-6, 7-dihydro-1*H*-cyclopenta[*d*]pyrimidin-4(5*H*)-one;

1-(((1-n-butyl-5-(4-chlorophenyl)-1*H*-imidazol-2-yl)methyl)-2-(2-nitrobenzylthio)-6,7-dihydro-1*H*-cyclopenta[*d*]pyrimidin-4(5*H*)-one;

1-(((5-n-decyl-1-(2-(diethylamino)ethyl)-1*H*-imidazol-2-yl)methyl)-2-(4-fluorobenzylt hio)-6,7-dihydro-1*H*-cyclopenta[*d*]pyrimidin-4(5*H*)-one;

1-(((1-(2-(diethylamino)ethyl)-5-(4'-(trifluoromethyl)biphenyl-4-yl)-1*H*-imidazol-2-yl) methyl)-2-(4-fluorobenzylthio)-6,7-dihydro-1*H*-cyclopenta[*d*]pyrimidin-4(5*H*)-one;

1-(((1-benzyl-5-((diethylamino)methyl)-1*H*-imidazol-2-yl)methyl)-2-(4-fluorobenzylth io)-6,7-dihydro-1*H*-cyclopenta[*d*]pyrimidin-4(5*H*)-one;

2-((4-fluorobenzylthio)-1-((5-methyl-1-((4'-(trifluoromethyl)biphenyl-4-yl)methyl)-1*H*--imidazol-2-yl)methyl)-6,7-dihydro-1*H*-cyclopenta[*d*]pyrimidin-4(5*H*)-one;

1-(((5-((diethylamino)methyl)-1-((4'-(trifluoromethyl)biphenyl-4-yl)methyl)-1*H*-imida zol-2-yl)methyl)-2-(4-fluorobenzylthio)-6,7-dihydro-1*H*-cyclopenta[*d*]pyrimidin-4(5 *H*)-one;

2-((4-fluorobenzylthio)-1-((5-(pyrrolidin-1-ylmethyl)-1-((4'-(trifluoromethyl)biphenyl-4-yl)methyl)-1*H*-imidazol-2-yl)methyl)-6,7-dihydro-1*H*-cyclopenta[*d*]pyrimidin-4(5*H* )-one;

1-(((5-(((4-fluorobenzyl)(methyl)amino)methyl)-1-((4'-(trifluoromethyl)biphenyl-4-yl) methyl)-1*H*-imidazol-2-yl)methyl)-2-(4-fluorobenzylthio)-6,7-dihydro-1*H*-cyclopenta [d]pyrimidin-4(5*H*)-one;

1-(((5-((4-benzylpiperazin-1-yl)methyl)-1-((4'-(trifluoromethyl)biphenyl-4-yl)methyl)-1H-imidazol-2-yl)methyl)-2-(4-

fluorobenzylthio)-6,7-dihydro-1*H*-cyclopenta[*d*]pyrim idin-4(5*H*)-one;
1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[d]pyrimidin-4( 5H)-one;
N-(4-fluorobenzyl)-1-(2-((2-(4-fluorobenzylthio)-4-oxo-4,5,6,7-tetrahydro-1*H*-cyclop enta[d]pyrimidin-1-yl)methyl)-1-((4'-(trifluoromethyl)biphenyl-4-yl)methyl)-1*H*-imid azol-5-yl)-N,N-dimethylmethanaminium bromide;
1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[d]pyrimidin-4( 5H)-one;
1-((4-fluorobenzyl)-1-((2-((2-(4-fluorobenzylthio)-4-oxo-4,5,6,7-tetrahydro-1*H*-cyclop enta[d]pyrimidin-1-yl)methyl)-1-((4'-(trifluoromethyl)biphenyl-4-yl)methyl)-1*H*-imid azol-5-yl)methyl)pyrrolidinium bromide;
ethyl 2-((2-(4-fluorobenzylthio)-4-oxo-4,5,6,7-tetrahydro-1*H*-cyclopenta[d]pyrimidin-1-yl) methyl)-1-((4'-(trifluoromethyl)biphenyl-4-yl)methyl)-1*H*-imidazole-5-carboxylate;
2-(((2-(4-fluorobenzylthio)-4-oxo-4,5,6,7-tetrahydro-1*H*-cyclopenta[*d*]pyrimidin-1-yl) methyl)-1-((4'-(trifluoromethyl)biphenyl-4-yl)methyl)-1*H*-imidazole-5-carboxylic acid;
2-(((2-(4-fluorobenzylthio)-4-oxo-4,5,6,7-tetrahydro-1*H*-cyclopenta[*d*]pyrimidin-1-yl) methyl)-N,N-dimethyl-1-((4'-(trifluoromethyl)biphenyl-4-yl)methyl)-1*H*-imidazole-5-carboxamide;
2-((4-fluorobenzylthio)-1-((1-((4'-(trifluoromethyl)biphenyl-4-yl)methyl)-1*H*-benzo[*d*] imidazol-2-yl)methyl)-6,7-dihydro-1*H*-cyclopenta[d]pyrimidin-4(5*H*)-one;
1-(((4-ethyl-5-(4'-(trifluoromethyl)biphenyl-4-yl)-4*H*-1,2,4-triazol-3-yl)methyl)-2-(4-fl uorobenzylthio)-6,7-dihydro-1*H*-cyclopenta[*d*]pyrimidin-4(5*H*)-one;
1-(((4-ethyl-5-(4'-(trifluoromethyl)biphenyl-4-yl)-4*H*-1,2,4-triazol-3-yl)methyl)-2-(4-methoxybenzylthio)-6,7-dihydro-1*H*-cyclopenta[d]pyrimidin-4(5*H*)-one;
2-((2,3-difluorobenzylthio)-1-((4-ethyl-5-(4'-(trifluoromethyl)biphenyl-4-yl)-4*H*-1,2,4-triazol-3-yl)methyl)-6,7-dihydro-1*H*-cyclopenta[*d*]pyrimidin-4(5*H*)-one;
1-(((4-ethyl-5-(3-phenylpropyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((4-ethyl-5-((4'-(trifluoromethyl)biphenyl-4-yl)methyl)-4*H*-1,2,4-triazol-3-yl)methy 1)-2-(4-fluorobenzylthio)-6,7-dihydro-1*H*-cyclopenta[d]pyrimidin-4(5*H*)-one;
2-((4-fluorobenzylthio)-1-((1-((4'-(trifluoromethyl)biphenyl-4-yl)methyl)-1*H*-1,2,4-tria zol-3-yl)methyl)-6,7-dihydro-1*H*-cyclopenta[d]pyrimidin-4(5H)-one;
methyl 2-(((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)meth yl)(methyl)amino)acetate;
2-((((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)meth yl)(methyl)amino)acetic acid;
2-((4-fluorobenzylthio)-1-((5-methyl-4-((4'-(trifluoromethyl)biphenyl-4-yl)methyl)-4*H* -1,2,4-triazol-3-yl)methyl)-6,7-dihydro-1*H*-cyclopenta[d]pyrimidin-4(1*H*)-one;
2-((4-fluorobenzylthio)-1-((5-propyl-4-((4'-(trifluoromethyl)biphenyl-4-yl)methyl)-4*H* -1,2,4-triazol-3-yl)methyl)-6,7-dihydro-1*H*-cyclopenta[d]pyrimidin-4(5*H*)-one;
2-((4-fluorobenzylthio)-1-((5-isopropyl-4-((4'-(trifluoromethyl)biphenyl-4-yl)methyl)-4*H*-1,2,4-triazol-3-yl)methyl)-6,7-dihydro-1*H*-cyclopenta[d]pyrimidin-4(5H)-one;
1-(((5-cyclopropyl-4-((4'-(trifluoromethyl)biphenyl-4-yl)methyl)-4*H*-1,2,4-triazol-3-yl )methyl)-2-(4-fluorobenzylthio)-6,7-dihydro-1*H*-cyclopenta[d]pyrimidin-4(5*H*)-one;
1-(((5-(benzylsulfonylmethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;
1-(((5-(3-(diethylamino)propyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;1-((5-(4-(diethylamino)butyl)-4-((4'-(trifluoromethyl)biphenyl-4-yl)methy 1)-4H-1,2,4-triazol-3-yl)methyl)-2-(4-fluorobenzylthio)-6,7-dihydro-1*H*-cyclopenta[d] pyrimidin-4(5*H*)-one;
2-((4-fluorob enzylthio)-1-((5-(hydroxymethyl)-4-((4'-(trifluoromethyl)biphenyl-4-yl)m ethyl)-4*H*-1,2,4-triazol-3-yl)methyl)-6,7-dihydro-1*H*-cyclopenta[d]pyrimidin-4(5*H*)-o ne;
2-((4-fluorobenzylthio)-1-((4-((4'-(trifluoromethyl)biphenyl-4-yl)methyl)-4*H*-1,2,4-tria zol-3-yl)methyl)-6,7-dihydro-1*H*-cyclopenta[d]pyrimidin-4(5*H*)-one;
5-(((2-(4-fluorobenzylthio)-4-oxo-4,5,6,7-tetrahydro-1*H*-cyclopenta[d]pyrimidin-1-yl) methyl)-4-((4'-(trifluoromethyl)biphenyl-4-yl)methyl)-4*H*-1,2,4-triazole-3-carbaldehy de;
1-(((5-((dimethylamino)methyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;
N-(4-fluorobenzyl)-1-(5-((2-(4-fluorobenzylthio)-4-oxo-4,5,6,7-tetrahydro-1*H*-cyclop enta[d]pyrimidin-1-yl)methyl)-4-((4'-(trifluoromethyl)biphenyl-4-yl)methyl)-4*H*-1,2,4 -triazol-3-yl)-N,N-dimethylmethanaminium bromide;
1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium iodide;

1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium bromide;

1-(((5-((diethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;

1-(((5-(((4-fluorobenzyl)(methyl)amino)methyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[d]pyrimidin-4(5*H*)-one;

2-((4-fluorobenzylthio)-1-((5-((isopropyl(methyl)amino)methyl)-4-((4'-(trifluoromethy 1)biphenyl-4-yl)methyl)-4H-1,2,4-triazol-3-yl)methyl)-6,7-dihydro-1H-cyclopenta[*d*]p yrimidin-4(5*H*)-one;

(±)1-((5-(((1-ethylpyrrolidin-2-yl)methylamino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

1-(((5-(((2*S*,6*R*)-2,6-dimethylmorpholino)methyl)-4-((4'-(trifluoromethyl)biphenyl-4-y 1)methyl)-4*H*-1,2,4-triazol-3-yl)methyl)-2-(4-fluorobenzylthio)-6,7-dihydro-1H-cyclo penta[*d*]pyrimidin-4(5*H*)-one;

methyl-2-((5-((2-(4-fluorobenzylthio)-4-oxo-4,5,6,7-tetrahydro-1*H*-cyclopenta[*d*]pyri midin-1-yl)methyl)-4-((4'-(trifluoromethyl)biphenyl-4-yl)methyl)-4*H*- 1,2,4-triazol-3-yl)amino)-2-methylpropanoate;

1-(((5-((4-ethylpiperazin-1-yl)methyl)-4-((4'-(trifluoromethyl)biphenyl-4-yl)methyl)-4 *H*-1,2,4-triazol-3-yl)methyl)-2-(4-fluorobenzylthio)-6,7-dihydro-1*H*-cyclopenta[*d*]pyr imidin-4(5*H*)-one;

2-((4-fluorobenzylthio)-1-((5-((2-methoxyethylamino)methyl)-4-((4'-(trifluoromethyl) biphenyl-4-yl)methyl)-4*H*-1,2,4-triazol-3-yl)methyl)-6,7-dihydro-1H-cyclopenta[*d*]py rimidin-4(5*H*)-one;

methyl-2-(4-((5-((2-(4-fluorobenzylthio)-4-oxo-4,5,6,7-tetrahydro-1*H*-cyclopenta[*d*]p yrimidin-1-yl)methyl)-4-((4'-(trifluoromethyl)biphenyl-4-yl)methyl)-4*H*-1,2,4-triazol-3-yl)methyl)piperazin-1-yl)-2-methylpropanoate;

2-((4-((5-((2-(4-fluorobenzylthio)-4-oxo-4,5,6,7-tetrahydro-1H-cyclopenta[*d*]pyrimidi n-1-yl)methyl)-4-((4'-(trifluoromethyl)biphenyl-4-yl)methyl)-4*H*-1,2,4-triazol-3-yl)m ethyl)piperazin-1-yl)-2-methylpropanoic acid;

1-(((5-(((2-(dimethylamino)ethyl)(methyl)amino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one tartrate;

1-(((5-(((3-(dimethylamino)propyl)(methyl)amino)methyl)-4-((4'-(trifluoromethyl)biph enyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

2-((4-fluorobenzylthio)-1-((5-((2-morpholinoethylamino)methyl)-4-((4'-(trifluorometh yl)biphenyl-4-yl)methyl)-4*H*-1,2,4-triazol-3-yl)methyl)-6,7-dihydro-1*H*-cyclopenta[*d*] pyrimidin-4(5*H*)-one;

(*R*)-1-((5-((3-(dimethylamino)pyrrolidin-1-yl)methyl)-4-((4'-(trifluoromethyl)bipheny 1-4-yl)methyl)-4*H*-1,2,4-triazol-3-yl)methyl)-2-(4-fluorobenzylthio)-6,7-dihydro-1*H*-c yclopenta[*d*]pyrimidin-4(5*H*)-one;

(*S*)-1-((5-((3-(dimethylamino)pyrrolidin-1-yl)methyl)-4-((4'-(trifluoromethyl)biphenyl -4-yl)methyl)-4*H*-1,2,4-triazol-3-yl)methyl)-2-(4-fluorobenzylthio)-6,7-dihydro-1*H*-c yclopenta[*d*]pyrimidin-4(5*H*)-one;

2-((4-fluorobenzylthio)-1-((5-((2-(piperidin-1-yl)ethylamino)methyl)-4-((4'-(trifluorom ethyl)biphenyl-4-yl)methyl)-4*H*-1,2,4-triazol-3-yl)methyl)-6,7-dihydro-1*H*-cyclopenta [*d*]pyrimidin-4(5*H*)-one;

2-((4-fluorobenzylthio)-1-((5-((2-(pyrrolidin-1-yl)ethylamino)methyl)-4-((4'-(trifluoro methyl)biphenyl-4-yl)methyl)-4*H*-1,2,4-triazol-3-yl)methyl)-6,7-dihydro-1*H*-cyclope nta[d]pyrimidin-4(5*H*)-one;

1-(((5-((2-(diisopropylamino)ethylamino)methyl)-4-((4'-(trifluoromethyl)biphenyl-4-yl )methyl)-4*H*-1,2,4-triazol-3-yl)methyl)-2-(4-fluorobenzylthio)-6,7-dihydro-1*H*-cyclop enta[*d*]pyrimidin-4(5*H*)-one;

1-(((5-((2-(diethylamino)ethylamino)methyl)-4-((4'-(trifluoromethyl)biphenyl-4-yl)met hyl)-4*H*-1,2,4-triazol-3-yl)methyl)-2-(4-fluorobenzylthio)-6,7-dihydro-1*H*-cyclopenta [*d*]pyrimidin-4(5*H*)-one;

2-((4-fluorobenzylthio)-1-((5-((4-methylpiperazin-1-yl)methyl)-4-((4'-(trifluoromethyl )biphenyl-4-yl)methyl)-4*H*-1,2,4-triazol-3-yl)methyl)-6,7-dihydro-1*H*-cyclopenta[*d*]p yrimidin-4(5*H*)-one;

2-((4-fluorobenzylthio)-1-((5-((methyl(pyridin-2-ylmethyl)amino)methyl)-4-((4'-(triflu oromethyl)biphenyl-4-yl)methyl)-4*H*-1,2,4-triazol-3-yl)methyl)-6,7-dihydro-1*H*-cyclo penta[*d*]pyrimidin-4(5*H*)-one;

1-(((5-((cyclopropyl(methyl)amino)methyl)-4-((4'-(trifluoromethyl)biphenyl-4-yl)meth yl)-4*H*-1,2,4-triazol-3-yl)methyl)-2-(4-fluorobenzylthio)-6,7-dihydro-1*H*-cyclopenta[ *d*]pyrimidin-4(5*H*)-one;

1-(((5-((4-(dimethylamino)piperidin-1-yl)methyl)-4-((4'-(trifluoromethyl)biphenyl-4-yl )methyl)-4*H*-1,2,4-triazol-3-yl)methyl)-2-(4-fluorobenzylthio)-6,7-dihydro-1*H*-cyclop enta[*d*]pyrimidin-4(5*H)*-one;

1-(((5-((3,3-difluoropyrrolidin-1-yl)methyl)-4-((4'-(trifluoromethyl)biphenyl-4-yl)meth yl)-4*H*-1,2,4-triazol-3-yl)methyl)-2-(4-fluorobenzylthio)-6,7-dihydro-1*H*-cyclopenta[ *d*]pyrimidin-4(5*H*)-one;

1-(((5-((2-(dimethylamino)ethylamino)methyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[d]pyrimidin-4(5*H*)-one;

(*S*)-1-((5-(((1-ethylpyrrolidin-2-yl)methylamino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;
2-((4-fluorobenzylthio)-1-((5-((2-(2-oxoimidazolidin-1-yl)ethylamino)methyl)-4-((4'-(t rifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
1-(((5-(azidomethyl)-4-((4'-(trifluoromethyl)biphenyl-4-yl)methyl)-4*H*1,2,4-triazol-3-yl)methyl)-2-(4-fluorobenzylthio)-6,7-dihydro-1*H*-cyclopenta[*d*]pyrimidin-4(5*H*)-one ;
1-(((5-(aminomethyl)-4-((4'-(trifluoromethyl)biphenyl-4-yl)methyl)-4*H*-1,2,4-triazol-3 -yl)methyl)-2-(4-fluorobenzylthio)-6,7-dihydro-1*H*-cyclopenta[*d*]pyrimidin-4(5*H*)-on e;
N-((5-((2-(4-fluorobenzylthio)-4-oxo-4,5,6,7-tetrahydro-1*H*-cyclopenta[*d*]pyrimidin-1 -yl)methyl)-4-((4'-(trifluoromethyl)biphenyl-4-yl)methyl)-4*H*-1,2,4-triazol-3-yl)methy 1)ethanesulfonamide;
2-((4-fluorobenzylthio)-1-((5-((2-oxoimidazolidin-1-yl)methyl)-4-((4'-(trifluoromethyl )biphenyl-4-yl)methyl)-4*H*-1,2,4-triazol-3-yl)methyl)-6,7-dihydro-1H-cyclopenta[*d*]p yrimidin-4(5*H*)-one;
1-(((5-((2-(4-fluorobenzylthio)-4-oxo-4,5,6,7-tetrahydro-1*H*-cyclopenta[*d*]pyrimidin-1 -yl)methyl)-4-((4'-(trifluoromethyl)biphenyl-4-yl)methyl)-4*H*-1,2,4-triazol-3-yl)methy 1)-3-methylthiourea;
1-(((5-(chloromethyl)-4-((4'-(trifluoromethyl)biphenyl-4-yl)methyl)-4*H*-1,2,4-triazol-3 -yl)methyl)-2-(4-fluorobenzylthio)-6,7-dihydro-1*H*-cyclopenta[*d*]pyrimidin-4(5*H*)-on e;
2-((4-fluorobenzylthio)-1-((5-((4-fluorobenzylthio)methyl)-4-((4'-(trifluoromethyl)bip henyl-4-yl)methyl)-4*H*-1,2,4-triazol-3-yl)methyl)-6,7-dihydro-1*H*-cyclopenta[d]pyrim idin-4(5*H*)-one;
(*E*)-1-((5-((2,2-dimethylhydrazono)methyl)-4-((4'-(trifluoromethyl)biphenyl-4-yl)met hyl)-4*H*-1,2,4-triazol-3-yl)methyl)-2-(4-fluorobenzylthio)-6,7-dihydro-1*H*-cyclopenta [*d*]pyrimidin-4(5*H*)-one;
(*E*)-1-((5-((2-tert-butylhydrazono)methyl)-4-((4'-(trifluoromethyl)biphenyl-4-yl)meth yl)-4*H*-1,2,4-triazol-3-yl)methyl)-2-(4-fluorobenzylthio)-6,7-dihydro-1*H*-cyclopenta[ *d*]pyrimidin-4(5*H*)-one;
(*E*)-2-(4-fluorobenzylthio)-1-((5-((piperidin-1-ylimino)methyl)-4-((4'-(trifluoromethyl )biphenyl-4-yl)methyl)-4*H*-1,2,4-triazol-3-yl)methyl)-6,7-dihydro-1*H*-cyclopenta[*d*]p yrimidin-4(5*H*)-one;
1-((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)vinyl acetate;
2-((4-fluorobenzylthio)-1-((5-(1-hydroxyethyl)-4-((4'-(trifluoromethyl)biphenyl-4-yl) methyl)-4*H*-1,2,4-triazol-3-yl)methyl)-6,7-dihydro-1*H*-cyclopenta[*d*]pyrimidin-4(5*H*) -one;
1-(((5-acetyl-4-((4'-(trifluoromethyl)biphenyl-4-yl)methyl)-4*H*-1,2,4-triazol-3-yl)meth yl)-2-(4-fluorobenzylthio)-6,7-dihydro-1*H*-cyclopenta[*d*]pyrimidin-4(5*H*)-one;
N-((5-((2-(4-fluorobenzylthio)-4-oxo-4,5,6,7-tetrahydro-1*H*-cyclopenta[*d*]pyrimidin-1 -yl)methyl)-4-((4'-(trifluoromethyl)biphenyl-4-yl)methyl)-4*H*-1,2,4-triazol-3-yl)methy 1)-2-hydroxy-*N*,*N*-dimethylethanaminium chloride;
2-((4-fluorobenzylthio)-1-((5-((2-hydroxyethoxy)methyl)-4-((4'-(trifluoromethyl)biphe nyl-4-yl)methyl)-4*H*-1,2,4-triazol-3-yl)methyl)-6,7-dihydro-1*H*-cyclopenta[*d*]pyrimid in-4(5*H*)-one;
1-(((5-((2-(diethylamino)ethoxy)methyl)-4-((4'-(trifluoromethyl)biphenyl-4-yl)methyl) -4*H*-1,2,4-triazol-3-yl)methyl)-2-(4-fluorobenzylthio)-6,7-dihydro-1*H*-cyclopenta[*d*]p yrimidin-4(5*H*)-one;
1-(((5-(n-butoxymethyl)-4-((4'-(trifluoromethyl)biphenyl-4-yl)methyl)-4*H*-1,2,4-triazo 1-3-yl)methyl)-2-(4-fluorobenzylthio)-6,7-dihydro-1*H*-cyclopenta[*d*]pyrimidin-4(5*H*)-one;
1-(((5-(1-(2-(dimethylamino)ethylamino)ethyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[*d*]pyrimidin-4(5*H*)-one;
1-(((5-(1-((2-(dimethylamino)ethyl)(methyl)amino)ethyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;
1-(((5-(1-(2-(diethylamino)ethylamino)ethyl)-4-((4'-(trifluoromethyl)biphenyl-4-yl)me thyl)-4*H*-1,2,4-triazol-3-yl)methyl)-2-(4-fluorobenzylthio)-6,7-dihydro-1*H*-cyclopenta [*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one tartrate;
1-(((5-(1-((3-(dimethylamino)propyl)(methyl)amino)ethyl)-4-((4'-(trifluoromethyl)bip henyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((5-(((2-(dimethylamino)ethyl)(ethyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
ethyl-2-((2-(diethylamino)ethyl)((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl )-4*H*-1,2,4-triazol-3-yl)methyl)amino)acetate;
2-(((2-(diethylamino)ethyl)((5-((2-(4-fluorobenzylthio)-4-oxo-4,5,6,7-tetrahydro-1*H*-c yclopenta[*d*]pyrimidin-1-yl)methyl)-4-((4'-(trifluoromethyl)biphenyl-4-yl)methyl)-4*H* -1,2,4-triazol-3-yl)methyl)amino)acetic acid;
*N*-(2-(diethylamino)ethyl)-2-((2-(4-fluorobenzylthio)-4-oxo-4,5,6,7-tetrahydro-1*H*-cy clopenta[*d*]pyrimidin-1-yl)methyl)-1-((4'-(trifluoromethyl)biphenyl-4-yl)methyl)-1*H*-i midazole-5-carboxamide;
*N*-(2-(diethylamino)ethyl)-2-((2-(4-fluorobenzylthio)-4-oxo-4,5,6,7-tetrahydro-1*H*-cy clopenta[*d*]pyrimidin-1-yl)methyl)-*N*-methyl-1-((4'-(trifluoromethyl)biphenyl-4-yl)me thyl)-1*H*-imidazole-5-carboxamide;
1-(((5-(((2-(diethylammo)ethyl)(methyl)amino)methyl)-1-((4'-(trifluoromethyl)biphen yl-4-yl)methyl)-1*H*-imidazol-2-yl)methyl)-2-(4-fluorobenzylthio)-6,7-dihydro-1*H*-cyc lopenta[*d*]pyrimidin-4(5*H*)-one;
2-((4-fluorobenzylthio)-1-((5-((methyl(2-(pyrrolidin-1-yl)ethyl)amino)methyl)-4-((4'-(t rifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-1*H*-imidazol-2-yl)methyl)-6,7-dihydro-1*H*-cyclo penta[*d*]pyrimidin-4(5*H*)-one;
2-((4-fluorobenzylthio)-1-((5-(3-(pyrrolidin-1-yl)propyl)-4-((4'-(trifluoromethyl)biphe nyl-4-yl)methyl)-4*H*-1,2,4-triazol-3-yl)methyl)-6,7-dihydro-1*H*-cyclopenta[*d*]pyrimid in-4(5*H*)-one;
2-((4-fluorobenzylthio)-1-((5-(3-(piperidin-1-yl)propyl)-4-((4'-(trifluoromethyl)biphen yl-4-yl)methyl)-4*H*-,2,4-triazol-3-yl)methyl)-6,7-dihydro-1*H*-cyclopenta[*d*]pyrimidi n-4(5*H*)-one;
2-((4-fluorobenzylthio)-1-((5-(4-(pyrrolidin-1-yl)butyl)-4-((4'-(trifluoromethyl)biphen yl-4-yl)methyl)-4*H*-1,2,4-triazol-3-yl)methyl)-6,7-dihydro-1H-cyclopenta[*d*]pyrimidi n-4(5*H*)-one;
2-((4-fluorobenzylthio)-1-((5-(4-(piperidin-1-yl)butyl)-4-((4'-(trifluoromethyl)biphenyl -4-yl)methyl)-4*H*-1,2,4-triazol-3-yl)methyl)-6,7-dihydro-1*H*-cyclopenta[*d*]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
methyl-3-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)isoxazole-5-carboxylate;
2-((4-fluorobenzylthio)-1-((5-(hydroxymethyl)-4-((4'-(trifluoromethyl)biphenyl-4-yl)m ethyl)isoxazol-3-yl)methyl)-6,7-dihydro-1*H*-cyclopenta[*d*]pyrimidin-4(5*H*)-one;
1-(((5-(chloromethyl)-4-((4'-(trifluoromethyl)biphenyl-4-yl)methyl)isoxazol-3-yl)meth yl)-2-(4-fluorobenzylthio)-6,7-dihydro-1*H*-cyclopenta[*d*]pyrimidin-4(5*H*)-one;
1-(((5-((diethylamino)methyl)-4-((4'-(trifluoromethyl)biphenyl-4-yl)methyl)isoxazol-3 -yl)methyl)-2-(4-fluorobenzylthio)-6,7-dihydro-1*H*-cyclopenta[*d*]pyrimidin-4(5*H*)-on e;
1-(((5-(((2-(dimethylamino)ethyl)(methyl)amino)methyl)-4-((4'-(trifluoromethyl)biphe nyl-4-yl)methyl)isoxazol-3-yl)methyl)-2-(4-fluorobenzylthio)-6,7-dihydro-1*H*-cyclop enta[*d*]pyrimidin-4(5*H*)-one;
1-(((5-(((2-(diethylamino)ethyl)(methyl)amino)methyl)-4-((4'-(trifluoromethyl)biphen yl-4-yl)methyl)isoxazol-3-yl)methyl)-2-(4-fluorobenzylthio)-6,7-dihydro-1*H*-cyclope nta[d]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)isoxazol-3-yl)methyl)-6,7-dihydro-1*H*-cyclopent a[*d*]pyrimidin-4(5*H*)-one;
2-((4-fluorophenethyl)-1-((4-((4'-(trifluoromethyl)biphenyl-4-yl)methyl)-4*H*-1,2,4-tria zol-3-yl)methyl)quinazolin-4(1*H*)-one;
*cis-trans* isomers, enantiomers, diastereoisomers, racemes, solvates, hydrates, or pharmaceutically acceptable salts thereof.

The structures of the above most preferred compounds are shown in the following preparation examples.

Compounds of this invention are effective inhibitors of the enzyme lipoprotein associated phospholipase $A_2$ and are expected to be useful in therapy, especially in the treatment and prevention of acute and chronic coronary events, such as those caused by peripheral vascular atherosclerosis and cerebrovascular atherosclerosis. That is to say, the present invention provides compounds of Formula (I), (II) or (III) that have potential uses in therapy.

Compounds of Formula (I), (II) or (III) according to this invention may inhibit the production of lysophosphatidylchloline (Lyso-PC), and therefore may have a general application in treating diseases associated with endothelial dysfunction, for example atherosclerosis, diabetes, hypertension, angina and ischemic reperfusion. Additionally, the compounds of Formula (I), (II) or (III) may also hava a general application in any disorder involving the hydrolysis process of oxidized lipid with the participation of the enzyme Lp-$PLA_2$. In addition to atherosclerosis and diabetes, such diseases also include ischemia, rheumatoid arthritis, stroke, inflammatory condition of the brain (such as Alzheimer's Disease), myocardial infarction, reperfusion injury, septicemia, acute and chronic inflammation.

The enzyme Lp-$PLA_2$ is expressed in activated inflammatory cells (such as macrophages, lymphocytes, neutrophils, eosnophils), and therefore the compounds of Formula (I), (II) or (III) according to this invention may be of use in treating any disorder that is associated with activated inflammatory cells. Such conditions include psoriasis and various airway inflammations, such as asthma and chronic bronchitis.

Accordingly, the present invention provides the uses of a compound of Formula (I), (II) or (III) in inhibiting the activity of Lp-$PLA_2$, and as such in treating diseases associated with activity of the enzyme Lp-$PLA_2$. Those diseases may be connected with activation of inflammatory cells; with production of phosphatidyl choline and oxidized non-esterified fatty acid; with lipid oxidation with the participation of the enzyme Lp-$PLA_2$; or with endothelial dysfunction.

A compound of Formula (I), (II) or (III) according to the present invention may be of use in treating the above mentioned diseases in combination with an anti-hyperlipidaemic, anti-atherosclerotic, anti-diabetic, anti-anginal, antiinflammatory, or anti-hypertension agent or an agent for lowering Lp(a). Examples of the above agents include, but are not limited to cholesterol synthesis inhibitors such as statins, anti-oxidants such as probucol, insulin sensitisers, calcium channel antagonists, and non-steroidal anti-inflammatory drugs.

A compound of Formula (I), (II) or (III) according to the present invention may be used in combination with cholesterol lowering agents such as statins. The statins are HMG-CoA reductase inhibitors, including atorvastatin, simvarstatin, pravastatin, cerivastatin, fluvastatin, lovstatin and pitavastatin. The two kinds of agents may be administered at the same time or at different times according to the discretion of the physician.

Up to 30% of the patients with high cholesterol level are negative to statin treatment. A compound of Formula (I), (II) or (III) according to this invention is expected to be applied to this population of patients.

In considering that coronary heart diseases account for majority of the death for diabetic patients, the combination of a compound of Formula (I), (II) or (III) according to this invention with an anti-diabetic agent or an insulin sensitizer is of particular interest. Preferably, the insulin sensitizer is a PPAR gamma activator, such as rosiglitazone or pioglitazone.

In therapeutic use, the compounds of this invention are usually administrated in a standard pharmaceutical composition. The present invention therefore provides a pharmaceutical composition that comprises one or more compounds of Formula (I), (II) or (III) in therapeutically effective amount and a pharmaceutically acceptable auxiliary. The pharmaceutically acceptable auxiliary is a pharmaceutically acceptable carrier, excipient or controlled release agent.

The compounds and pharmaceutical compositions of the present invention can be formulated in various dosage forms, for example tablet, capsule, powder, sirup, solution, suspension or aerosol, and can be incorporated into appropriate solid or liquid carriers or diluents. The pharmaceutical compositions of the present invention may be stored in appropriate injection or instillation disinfectors. The pharmaceutical compositions may also contain an odorant, a flavouring agent, etc..

A pharmaceutical composition of the present invention comprises a safe, effective amount (e.g. 0.1-99.9 parts by weight, preferably 1-90 parts by weight) of a compound of Formula (I), (II) or (III) or pharmaceutically acceptable salt thereof, and a balance of a pharmaceutically acceptable excipient, based on 100 parts by weight of the composition in total. In other words, the pharmaceutical composition of this invention contains 0.1-99.9%, preferably 1-90% by weiht of the compound of Formula (I), or (II) or a pharmaceutically acceptable salt thereof , and a balance of a pharmaceutically acceptable auxiliary, based on 100% by weight of the composition in total.

The preferred proportion of the compound of Formula (I), (II) or (III) as an active ingredient is more than 60% by weight. The pharmaceutically acceptable carrier, excipient or controlled release agent as the rest partaccounts for 0-40% by weight, preferably 1-20%, most preferably 1-10%.

The compounds of Formula (I), (II) or (III) according to this invention or pharmaceutical compositions comprising the compounds of Formula (I), (II) or (III) are expected to be administrated to mammal, including human and animal. The routes of administration may include oral, nasal, transdermal, pulmonary or gastrointestinal drug delivery, preferably oral route. Preferably, the composition is in unit dosage form. Such a unit may contain 0.01mg to 200mg of the active ingredient, preferably 0.5mg to 100mg, in a daily dose or sub-dose. Regardless of the method of administration, the individual optimal dose deponds on the condition being treated, and is usually seeked gradually from small dose to high dose.

The pharmaceutical compositions of the present invention may be administrated in oral, intravenous, intramuscular or subcutaneous route. In consideration of the convenience for preparation and administration, a solid composition is preferred, especially tablet and capsule that is packed with solid or liquid materials. The preferred route of administration of the pharmaceutical composition is p.o..

The solid carriers may include starch, lactose, calcium dihydrogen phosphate, microcrystalline cellulose, sucrose and white clay. The liquid carriers may include sterile water, polyethylene glycol (PEG), nonionic surfactant and cooking oil (such as corn oil, peanut oil and sesame oil) as far as the carrier is in accordance with the property of active ingredient and the route of administration. The commonly used adjuvants for preparing the pharmaceutical compositions include spices, pigments, preservatives and antioxidants such as vitamin E, vitamin C, BHT and BHA.

The injections include, but are not limited to sterile, injectable, aqueous or non-aqueous solution, suspension and emulsion. Those injections can be prepared with appropriate parenteral diluent, dispersant, wetting agent or suspending agent. The injections can be sterilized with biofilter that is capable of retaining bacteria, or with bactericide which is dissolved or dispersed in injectable medium, or prepared by the other methods well known in the pharmacy art.

## PREPARATION METHODS

### Abbreviations

DUB—1,8-diazabicyclo[5.4.0]undec-7-ene

DCE—dichloroethane
DCM—dichloromethane
DIPEA—*N,N*-diisopropylethylamine
DMAP—4-dimethylaminopyridine
DME—dimethoxyethane
DMF—dimethyl formamide
DMSO—dimethylsulfoxide
DPPA—diphenyl phosphoryl azide
EA—ethyl acetate
EDCI-1-(3-dimethylaminopropyl)-3-ethylcarbodiimide hydrochloride
$Et_2O$—diethyl ether
EtOH—ethanol
HOBt—1-hydroxybenzotriazole
HOBu-t— tert-butanol
*i*-PrOH—isopropanol
KOBu-t—potassium tert-butoxide
LDA—lithium diisopropylamide
*m*-CPBA—meta-chloroperoxybenzoic acid
MeOH—methanol
NaH—sodium hydride
NaHMDS—sodium bis(trimethylsilyl)amide
*n*-BuLi-*n*-butyllithium
NCS—*N*-chlorosuccinimide
PE—petroleum ether
*s*-BuLi—isobutyllithium
TFFA—trifluoro acetic anhydride
THF—tetrahydrofuran

**Synthetic Routes**

The compounds of this invention can be prepared according to the methods illustrated by the following synthetic route 1, 2, 3 or 4. Unless otherwise indicated, the substituents appeared in the synthetic routes are as hereinbefore defined for Formula (I).

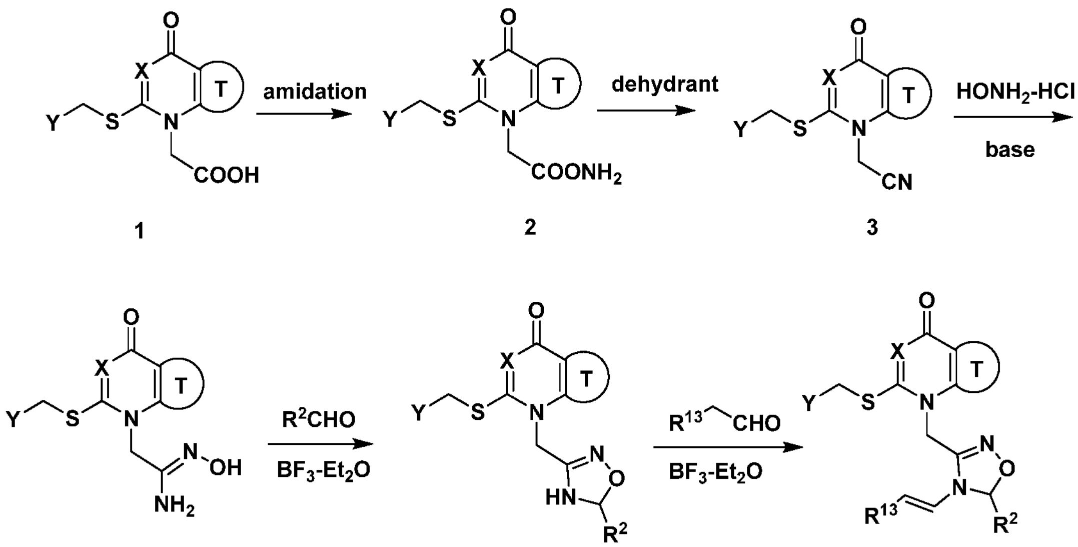


1 2 3 4 5 6

**Synthetic Route 1**

When W is Formula (a) meanwhile $R^1$ is alkenyl in the compounds of Formula (I), the compounds of Formula (I) (i.e. compound **6** in route 1) can be prepared according to the method illustrated by synthetic route 1, in which: $R^{13}$

is $C_1$-$C_{10}$ alkyl; T, X, Y and $R^2$ are as hereinbefore defined:

Compound **1** may be purchased from suppliers or prepared according to the literature methods (such as those disclosed in PCT patent applications WO03016287 and WO03042206, the published contents of which are cited in their entity as references in this invention).

Compound 1 is converted to compound **2** by amidation. In one embodiment, this process may be accomplished by chlorination of the acid compound 1 and subsequent amination of the resulting acyl chloride. The chlorinating agents include thionyl chloride and oxalyl chloride. The amination agents can be excessive concentrated aqueous ammonia or ammonia-methanol solution. The reaction occurs in aprotic solvent, such as DCM, DCE, acetonitrile and THF. The reaction temperature is between -15°C and 0°C. In another embodiment, this process may be accomplished by direct reaction of compound **1** with an ammonium salt in the presence of a condensing agent, wherein the condensing agent includes dicyclohexylcarbodiimide (DCC), diethyl azodicarboxylate/Ph3P, carbonyldiimidazole, 1-(3-dimethylaminopropyl)-3-ethylcarbodiimide hydrochloride/1-hydroxybenzotriazole (EDCI/HOBt), O-(Benzotriazol-1-yl)-N,N,N',N'-tetramethylisouronium tetrafluoroborate (TBTU), and the inorganic ammonium salts include, for example, ammonium chloride, ammonium carbonate and ammonium bicarbonate. The reaction occurs in the presence of an organic base, such as triethylamine, DIPEA and DBU. The reaction occurs in a non-alcohol solvent, such as DCM, DCE, acetonitrile, THF, toluene and DMF; preferably in DCM, acetonitrile or DMF. The reaction temperature is between 0°C and room temperature (rt).

Compound **2** is dehydrated to give compound **3.** The preferred dehydrant is TFFA or $POCl_3$. An organic base, such as pyridine and triethylamine, is optionally added to the reaction solution. The reaction occurs in an aprotic solvent, such as DCM, DCE and THF; preferably in DCM or DMF. The reaction temperature is between -40°C and rt.

Compound **3** reacts with hydroxylamine hydrochloride in the presence of an excessive base to produce compound **4,** wherein the mentioned base is an organic base or an inorganic base, such as triethylamine, potassium carbonate and sodium hydroxide. The reaction occurs in a polar solvent, such as methanol, ethanol, DMF and water or a mixed solvent thereof. The reaction temperature is between 0°C and 60°C, preferably room temperature.

Catalyzed by boron trifluoride-diethyl ether complex ($BF_3$-$Et_2O$), the reaction of compound **4** with 1 equivalent of $R^2$CHO yields compound **5.** More than 1 equivalent (equiv) of $BF_3$-$Et_2O$ is required, typically 2 equiv. The reaction solvents include, for example, $Et_2O$ or THF. The reaction temperature is between 0°C and 40°C, preferably room temperature.

Compound **5** is transformed into compound **6** by reaction with $R^{13}$ $CH_2CHO$ in the presence of $BF_3$-$Et_2O$. More than 1 equiv of $BF_3$-$Et_2O$ is required; typically 2 equiv. The reaction solvent is $Et_2O$ or THF. The reaction temperature is between 0°C and 40°C, preferably room temperature.

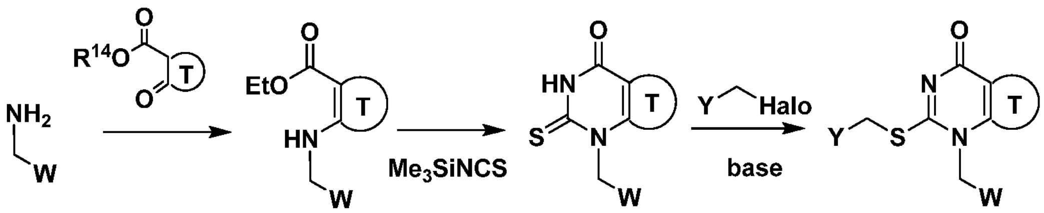


7 8 9 10

**Synthetic Route 2**

When T is 4 to 6-membered aliphatic ring and X=N, meanwhile $R^1$ is not alkenyl in the compounds of Formula (I), the compounds of Formula (I) (i.e. compound **10**) can be prepared according to the method illustrated by synthetic route 2, in which: $R^{14}$ is methyl or ethyl; Halo, T, W and Y are as hereinbefore defined:

Compound **7** or its hydrochloride form is condensed with 1 equiv of a cycloalkanone carboxylate

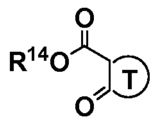

to yield compound **8.** When compound **7** is in hydrochloride form, at least 1 equiv of an organic base is necessary, such as triethylamine and DIPEA. The reaction occurs in the presence of a dehydrant, including such as molecular

sieve, azeotropic toluene and $Si(OEt)_4$. The reaction solvents include such as methanol, ethanol, toluene and acetic acid. The reaction temperature is between 0°C and 140°C, preferably the reflux temperature of the solvent.
Compound **8** reacts with isocyanate via condensation to produce compound **9,** wherein the mentioned isocyanate may include $Me_3SiNCS$,

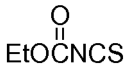 and 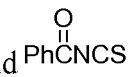 ,

etc., preferably $Me_3SiNCS$, which is usually added at 3.5 equiv of that of compound **8.** The reaction occurs in solvent-free condition or in DMF. The reaction temperature is between 100 °C and 160°C, most preferably 140°C.
Compound **9** is converted to compound **10** by reaction with

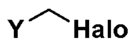 .

More than 1 equiv of an organic or inorganic base is required, such as triethylamine, DBU, DIPEA, potassium carbonate and sodium carbonate. A catalyst is optionally added, such as potassium iodide and tetrabutylammonium iodide. The reaction solvents include acetonitrile, acetone, DME, DCM, DCE, EA, ethanol, methanol, THF, etc.. The reaction temperature is between 0°C and 80°C, preferably the reflux temperature of the solvent.

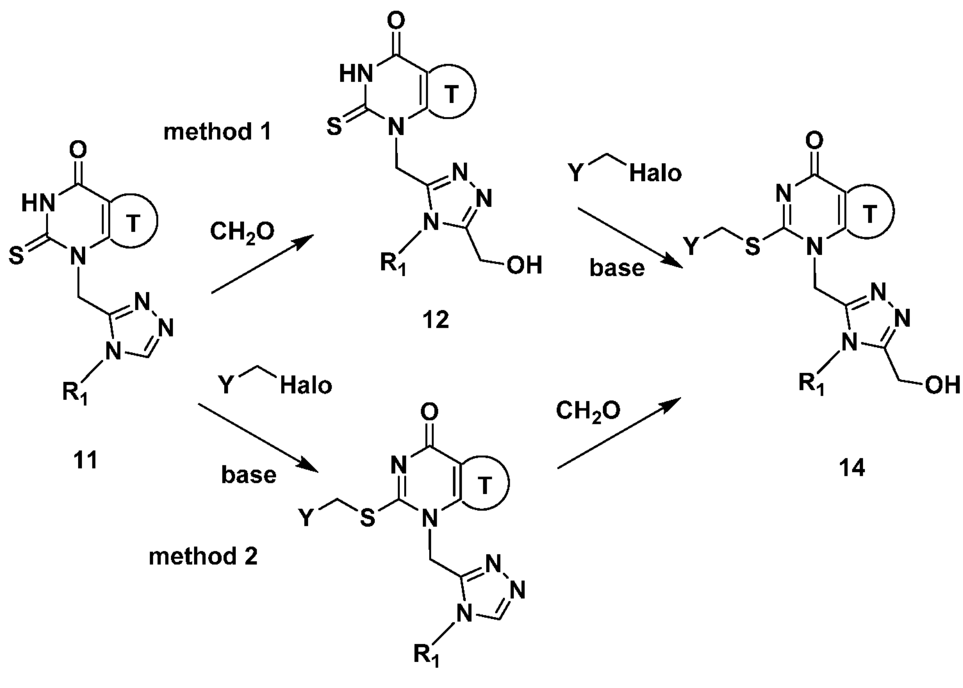


**Synthetic Route 3**

When W is Formula (c) meanwhile $R^2$ is hydroxymethyl in the above mentioned compound **10,** the compound (i.e. compound **14**) can be prepared according to either of the methods illustrated by synthetic route 3, in which Halo, T, Y and $R^1$ are as hereinbefore defined;
Compound **11** is prepared according to a method analogous to that described for compound **9;**
According to the first method, compound **11** is heated with excessive aqueous formaldehyde to afford compound **12.** The reaction occurs in a pressure-proof sealed container or a reflux condenser. The reaction is solvent-free or occurs in dioxane. The reaction temperature is between 100 °C and 160°C. Compound **12** then reacts with

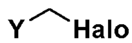

to give compound **14** under a condition analogous to that described for the preparation of compound **10** from compound **9.**

According to the second method, compound **11** first reacts with

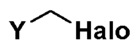

to give compound **13** under a condition analogous to that described for the preparation of compound **10** from compound **9.** Compound **13** is then heated with excessive aqueous formaldehyde to afford compound **14** under a condition analogous to that described for the preparation of compound **14** from compound **12.**

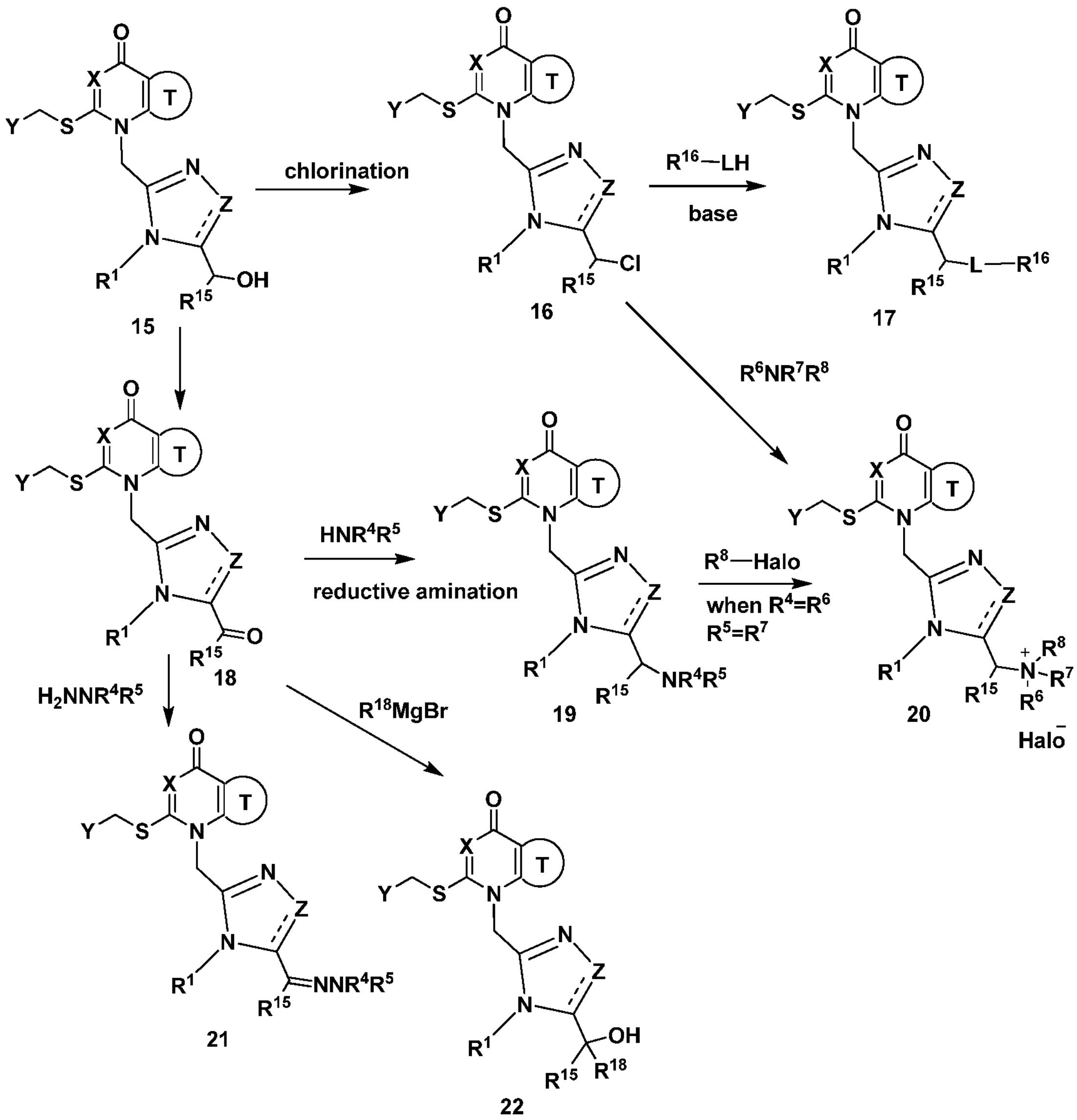


**Synthetic Route 4**

In certain circumstances, a compound of Formula (I) can be converted into another compound of Formula (I) by functional transformation, some examples of which are illustrated by synthetic route 4. It would be specially mentioned that these examples represent only part of the functional transformations of this invention, and therefore are not intended to limit the scope of synthetic methods of the invention in any way. More functional transformation examples will be given in the preparation methods of the specific examples.

The functional transformation methods in route 4 include:

When $R^2$ is α-hydroxyalkyl, a compound of Formula (I) can be represented by the structure of compound **15,** in which $R^{15}$ is H or $C_1$-$C_6$ alkyl; $R^{16}$ is $C_1$-$C_6$ alkyl that is optionally substituted by $NR^4R^5$ or phenyl, wherein phenyl is optionally substituted by halogen; $R^{18}$ is $C_1$-$C_6$ alkyl; L is $NR^4$, O or S; Z is CH, N or O; T, X, Y, $R^1$ $R^4$, $R^5$, $R^6$, $R^7$ and $R^8$ are as hereinbefore defined:

Compound **15** is chlorinated to afford compound **16.** The chlorinating agent may be $SOCl_2$ or $PCl_3$. The reaction occurs in an aprotic solvent, such as DCM, $CCl_4$ and $Et_2O$. The reaction temperature is between 0°C and room temperature;

Compound **16** reacts with $R^{16}LH$ to give compound **17** in the presence of a base, wherein the base may be an inorganic base, such as NaH, n-BuLi, KOBu-t and $K_2CO_3$, or an organic base, such as triethylamine, DIPEA, DBU and DMAP. The reaction solvent is selected from the group consisting of THF, $CH_3CN$, DME, DMF, EtOH, MeOH and HOBu-t, in accordance with the base used. The reaction temperature is between -80°C and 80°C .

Compound **15** is oxidized to afford compound **18** under reaction conditions which have been adequately described in reference books, such as Comprehensive Org. Syn., Vol. 7, pp 251-327. In the present invention, the preferred oxidant is activated $MnO_2$, which is usually added at least 5 equiv of that of compound **15.** The reaction solvent is dioxane or chloroform. The reaction temperature is between room temperature and 80°C, preferably 50-70°C.

Compound **18** reacts with $HNR^4R^5$ to give compound **19** in the presence of a reductant under reaction conditions which have been adequately described in reference books, such as Comprehensive Org. Trains., 2nd ed., Wiley-VCH, NY, 1999, pp. 835-840. In the present invention, the preferred reductant is borohydride, such as $NaBH_4$, $NaBH_3CN/Ti(OPr-i)_4$ or $NaBH(OAc)_3$. The reaction solvents include DCM, DCE, THF, EtOH and MeOH, etc.. The reaction temperature is between 0°C and 80°C, preferably room temperature.

The condensation reaction of compound **16** with $R^6NR^7R^8$ yields compound **20.** When $R^4$=$R^6$ and $R^5$=$R^7$, the reaction of compound **19** with $R^8$-Halo in an appropriate solvent also yields compound **20.** The reaction solvents may include acetone, DCM, THF, DMF and acetonitrile. The reaction temperature is between -20°C and room temperature.

The condensation reaction of compound **18** with $H_2NNR^4R^5$ in an appropriate solvent yields compound **21.** The reaction solvents may include DCM, DCE, THF, EtOH and MeOH. A dehydant such as molecular sieve or anhydrous $MgSO_4$ is optionally added. The reaction temperature is between 0°C and room temperature.

The reaction of compound **18** with $R^{18}BrMg$ yields compound **22**; the reaction occurs in anhydrous THF, $Et_2O$ or DME; the reaction temperature is between -20°C and rt.

The above mentioned intermediate compound 7 can be prepared according to either of the methods illustrated by synthetic route 5 and 6:

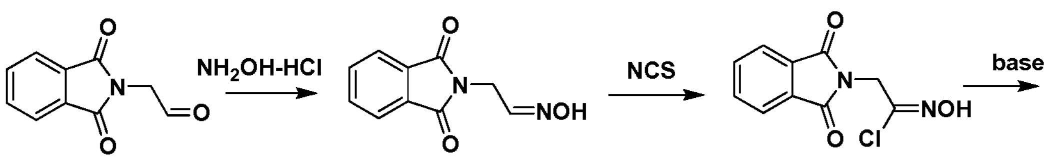


23 24 25

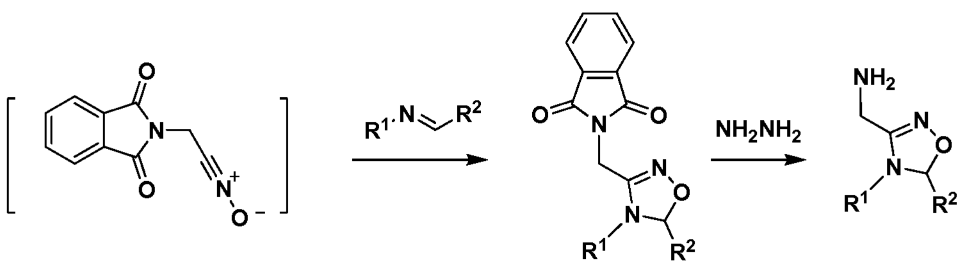


26 27 28

**Synthetic Route 5**

When W is Formula (a) in intermediate compound 7, this compound (i.e. compound **28**) can be prepared according to the method illustrated by synthetic route 5, in which: $R^1$ and $R^2$ are as hereinbefore defined;

Compound **23** may be purchased from suppliers or prepared according to the literature method (Tetrahedron Letters 2001, 42, 315-317). Compound **23** reacts with hydroxylamine hydrochloride via condensation in the presence of a base to produce compound **24,** wherein the mentioned bases include organic bases and inorganic bases, such as triethylamine, potassium carbonate and sodium hydroxide. The reaction occurs in a polar solvent, such as methanol, ethanol, DMF, water or a mixed solvent thereof. The reaction temperature is between 0°C and 40°C.

Compound **24** is chlorinated with equivalent NCS to afford compound **25.** The reaction solvents may include THF and DMF, etc.. The reaction temperature is between 0°C and 60°C.

Dehydrochloration of compound **25** in the presence of a base yields intermediate **26,** followed by addition reaction with

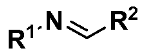

to form compound **27.** The base used is an organic base, such as triethylamine, DIPEA or DBU. The reaction solvents may include THF and DMF, etc.. The reaction temperature is between -40°C and room temperature.

Compound **27** reacts with hydrazine hydrate to give compound **28.** The reaction occurs in a polar solvent, such as THF, MeOH and EtOH. The reaction temperature is between 0°C and 80°C, preferably 50-60°C.

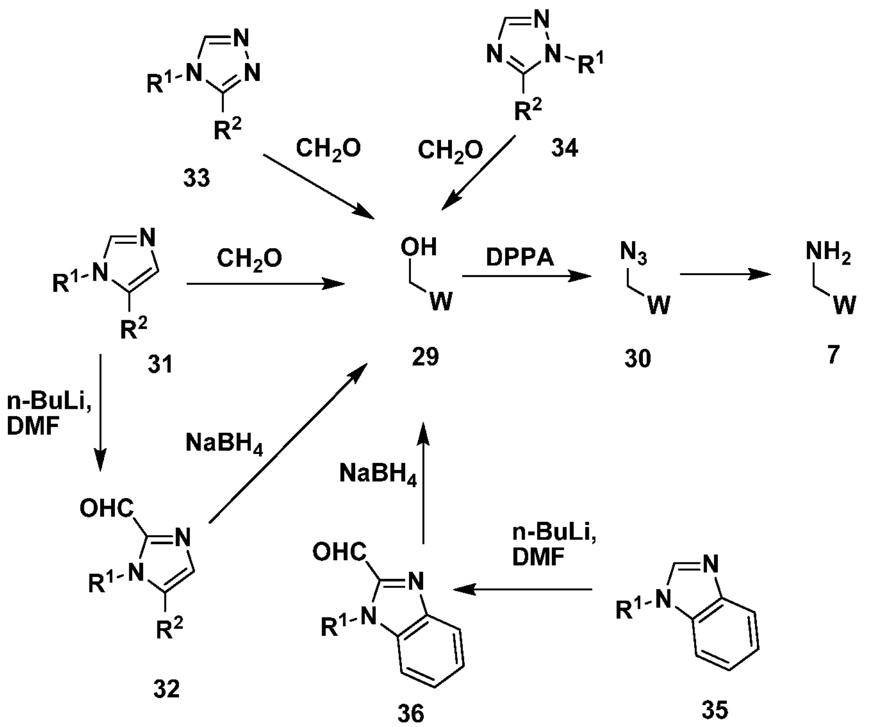


**Synthetic Route 6**

Except for the case where W is Formula (a) in intermediate compound 7 as mentioned above, compound 7 can be prepared by the methods shown in route 6, in which $R^1$ and $R^2$ are as hereinbefore defined;

Compound **29** reacts with DPPA to give compound **30** in the presence of a base, wherein the base is an organic base, such as triethylamine, DIPEA, DBU or DMAP, most preferably DBU and DMAP; the reaction solvent is a polar aprotic solvent, such as THF, $CH_3CN$ and DME, etc., most preferably THF; the reaction temperature is between 0°C and 100°C, most preferably reflux temperature of the solvent.

Compound **30** is converted to compound 7 by hydrogenation reduction or Staudinger reaction (Gololobov, Y. G. Sixty years of Staudinger reaction. Tetrahedron 1981, 37: 437). The hydrogenation reduction is catalyzed by Pd/C in a suitable solvent such as methanol, ethanol, EA or THF. The reaction temperature is between 0°C and 80°C, most preferably room temperature. The Staudinger reaction occurs in the mixed solvent of THF with $H_2O$ in the presence of excessive $Ph_3P$ at a temperature between O°C and 50°C.

The above mentioned intermediate compound **29** can be prepared according to one of the methods as described below:

Compound **31** is heated with excessive aqueous formaldehyde to afford compound **29** under a condition analogous to that described for the preparation of compound **14** from compound **12** in; or

Compound **31** reacts with a formic acid derivative to afford compound **32** in the presence of a strong base, followed by reduction reaction with $NaBH_4$ to form compound **29.** The mentioned strong bases include n-BuLi, s-BuLi, LDA or NaHMDS, etc., most preferably n-BuLi. The mentioned formic acid derivatives include DMF, ethyl formate or methyl formate, etc.. The solvent herein used is THF and the reaction temperature is between -80°C and room temperature.

Compound **33** is heated with excessive aqueous formaldehyde to afford compound **29** under a condition analogous to that described for the preparation of compound **14** from compound **12.**

Compound **34** is heated with excessive aqueous formaldehyde to afford compound **29** under a condition analogous to that described for the preparation of compound **14** from compound **12.**

Compound **35** reacts with a formic derivative to afford compound **36** in the presence of a strong base, followed by reduction reaction with $NaBH_4$ to form compound **29.** The mentioned strong bases include n-BuLi, s-BuLi, LDA or NaHMDS, etc., most preferably n-BuLi. The mentioned formic derivatives include DMF, ethyl formate or methyl formate. The solvent herein used is THF and the reaction temperature is between -80°C and room temperature.

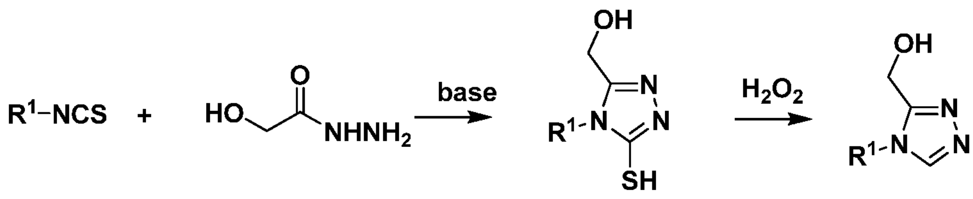


**37** **38**

**Synthetic Route 7**

When W is Formula (c) meanwhile $R^2$ is H in intermediate compound 29, the compound (i.e. compound 38) can be prepared according to the method illustrated by synthetic route 7, in which: $R^1$ is as hereinbefore defined; $R^1$-NCS is condensed with

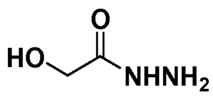

in the presence of a base to produce compound **37,** wherein the mentioned base is an inorganic base, such as potassium carbonate, sodium carbonate or sodium hydroxide, etc.; the reaction occurs in a strong polar protic solvent, such as water, ethanol, etc.; and the reaction temperature is the reflux temperature of the solvent used.

The oxidative desulfurization reaction of compound **37** gives rise to compound **38.** The oxidants include, for example, hydrogen peroxide, $HNO_3/NaNO_2$ or Fe(III) salts, most preferably aqueous hydrogen peroxide solution (30% w/w). AcOH is optionally added as a catalyst. The solvent may be a $H_2O$-DCM mixture or a $H_2O$-AcOH mixture.

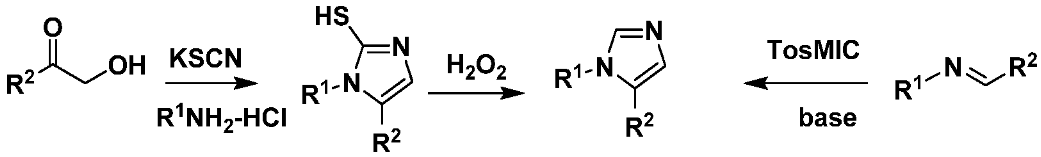


**39** **31**

TosMIC = 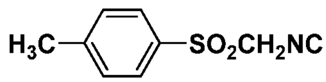

**Synthetic Route 8**

The above mentioned intermediate compound **31** can be prepared according to the method illustrated by synthetic route 8, in which: $R^1$ and $R^2$ are as hereinbefore defined;

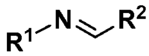

reacts with p-tosylmethyl isocyanide (TosMIC) via condensation in the presence of a base to produce compound **31,** wherein the mentioned base may be an inorganic base such as NaH, KOBu-t, $K_2CO_3$ and $Cs_2CO_3$, etc., or an organic bases such as pyridine, triethylamine, DIPEA and DBU. Alternatively, $R^1NH_2$ can be used as the base directly. The reaction solvents include THF, $CH_3CN$, DME, DMF, EtOH, MeOH, HOBu-t and mixed solvents thereof. The reaction temperature is between 0°C and 80°C; or

The mixture of

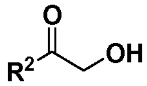

,

$R^1NH_2$-HCl and KSCN is first heated and condensed to produce compound **39,** followed by oxidative desulfurization reaction to yield compound **31.** The condensation reaction occurs in acetonitrile or HOBu-t in the presence of acetic acid or propionic acid as a catalyst at a temperature between 50 and 100°C. The oxidative desulfurization condition is analogous to that described for the preparation of compound **38** from compound **37.**

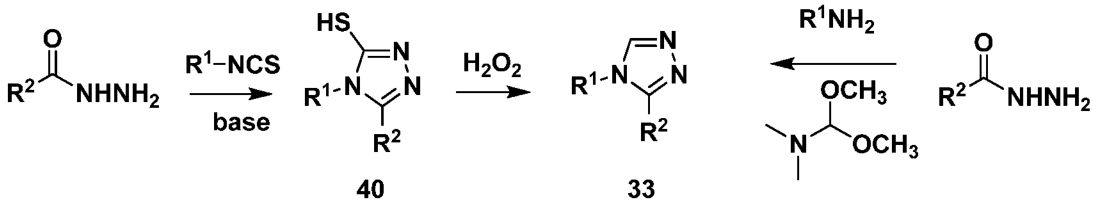


40 33

## Synthetic Route 9

The above mentioned intermediate compound 33 can be prepared according to the methods illustrated by synthetic route 9, in which: $R^1$ and $R^2$ are as hereinbefore defined;

The mixture of

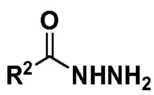

,

1,1-dimethoxy-*N*,*N*-dimethylethylamine and $R^1NH_2$ is heated and condensed to produce compound **33.** The condensation reaction occurs in the mixed solvent of $CH_3CN$ and AcOH at a temperature between 60 and 120°C; or

The condensation reaction of

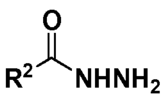

with $R^1NCS$ in the presence of a base yields compound **40,** followed by oxidative desulfurization reaction to form compound **33.** The condensation condition is the same as described for the preparation of compound **37.** The oxidative desulfurization condition is analogous to that described for the preparation of compound **38** from compound **37.**

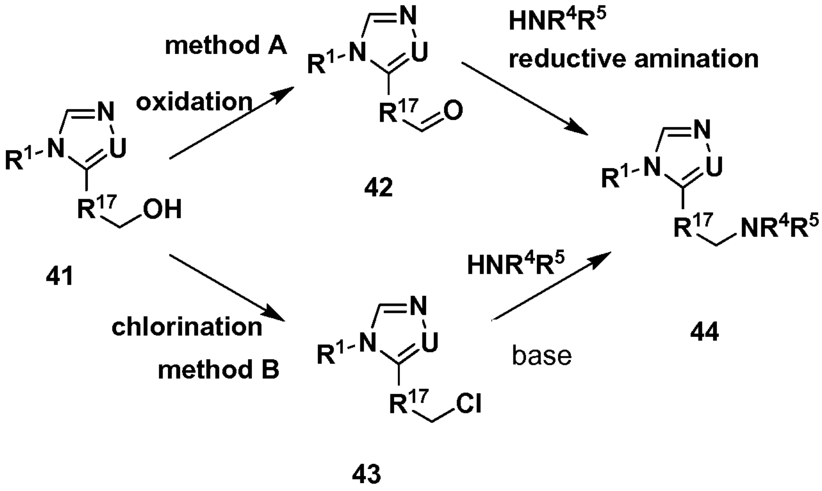


**Synthetic Route 10**

When $R^2$ is specified as

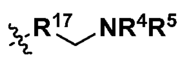

in the above intermediate compound **31** or compound **33,** the compound (i.e. compound **44**) can be prepared according to both methods illustrated by synthetic route 10, in which: $R^{17}$ is a chemical bond, straight or branched $C_1$-$C_4$ alkylene; U is CH or N; $R^1$ , $R^2$, $R^4$ and $R^5$ are as hereinbefore defined;

According to method A, compound **41** is first oxidized to afford compound **42;** the preferred oxidizing condition is activated $MnO_2$ or Swem Oxidation (A. J. Mancuso, S-L. Huang, D. Swern. J. Org. Chem., 1978, 43, 2480); and the use of $MnO_2$ is analogous to that described for the preparation of compound **18** from compound **15.** In this invention, the preferred reagent for Swem Oxidation is the combination of oxalyl chloride, DMSO and $NEt_3$. The reductive amination reaction of compound **42** with $HNR^4R^5$ affords compound **44.** The reaction condition is analogous to that described for the preparation of compound **19** from compound **18.**

According to method B, compound **41** is first chlorinated to afford compound **43**; the reaction condition is analogous to that described for the preparation of compound **16** from compound **15;** compound **43** then reacts with $HNR^4R^5$ to give compound **44** in the presence of a base, such as triethylamine, DIPEA, DBU or $K_2CO_3$; KI is optionally added as a catalyst; the reaction solvents include $CH_3CN$, DCM, DCE, THF or acetone; and the reaction temperature is between room temperature and 90°C, most preferably the reflux temperature of the solvent.

In addition to the preparation methods illustrated by synthetic routes 5 to 10 for the intermediate compounds in the invention, other novel intermediates or raw materials can be prepared according to similar literature methods and will be described in detail with reference to the methods for preparing the compounds in the specific examples. The already known intermediates or raw materials can be purchased from suppliers or prepared according to literature methods.

## Specific Examples

The following synthetic examples are provided to more specifically illustrate the invention. These examples are not intended to limit the scope of the invention, but to give more detailed description to the invention. Unless otherwise indicated, all the parameters and other instructions provided in the invention are based on quality. In general, chromatographic column is packed with silica gel unless otherwise indicated. An experimental method, the condition of which is not specifically described in the invention, is carried out under conventional condition or condition recommended by the manufacturer.

Unless otherwise defined, all professional and scientific terms are in accordance with what are familiarized by the skilled artisan in this area. In addition, any similar or equivalent method and material to that recorded in this invention can be used herein. The preferred examples, methods and materials are used for demonstration purposes only.

**Synthesis Examples**

The number of intermediate in this invention was started with a letter selected from "M, A, B, C, D, E, F", such as intermediate "M1" or "A8"; the number of final compound was started with the word "example", such as "example 18".

**Intermediate M1-4'-(trifluoromethyl)biphenyl-4-carbaldehyde**

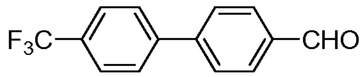

A mixture of 4-(trifluoromethyl)phenylboronic acid (3.80g, 1 equiv), 4-bromobenzaldehyde (3.7g, 1 equiv), palladium acetate (0.225g, 0.05equiv), aq. $Na_2CO_3$ (20ml, 2M solution, 2 equiv) solution in DME (40ml) was purged with nitrogen and then evacuated, then refluxed under nitrogen for 3h. TLC detection showed that the reaction was complete. The insoluble substance was filtered while hot, and then washed with ethyl acetate. The liquid phase was transferred to a separatory funnel and the organic phase was separated and the aqueous phase was extracted with ethyl acetate twice. The organic phases were combined, washed with brine twice, dried over $MgSO_4$ and evaporated under reduced pressure. The residue was purified by column chromatography or recrystallized from petroleum ether and diethyl ether to give the title compound.

**Intermediate M2—methyl2-(4-(tritluoromethylsulfonyloxy)phenyl)acetate**

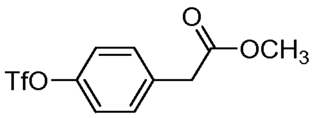

A solution of triflumethanesulfonic anhydride (4.3ml, 26mmol, 1.3equiv) in dichloromethane (100ml) was placed in a low-temperature reactor of -40°C under nitrogen. Pyridine (2.5ml, 30mmol, 1.5equiv) was added dropwise over 10 min via a syringe, during the addition, a precipitate appeared and the mixture was stirred vigorously. After the addition was complete, the mixture was further stirred for 15min before a solution of methyl 4-hydroxyphenylacetate (3.32g, 20mmol, lequiv) in dichloromethane (40ml) was added dropwise via a syringe over 5 min. The refrigeration was stopped and the mixture was stirred for a further 30min, then saturated ammonium chloride solution was added. The organic layer was separated, washed successively with diluted hydrochloric acid, brine and saturated sodium bicarbonate solution, dried over $MgSO_4$, and then evaporated in vacuum to give 3.15 g of oil, which turned to be a crystalline upon standing (3.15g). $^1$H-NMR ($CDCl_3$, 300MHz) $\delta$ 3.65 (s, 2H), 3.71 (s, 3H), 7.24 (d, 2H, J=8.7), 7.37 (d, 2H, J=9.0).

**Intermediate M3—methyl 2-(4'-(trifluoromethyl)biphenyl-4-yl)acetate**

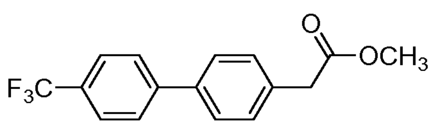

The title compound was prepared by a procedure similar to that described for intermediate M1 except that M2 was used in place of 4-bromobenzaldehyde. $^1$H-NMR ($CDCl_3$, 300MHz) $\delta$ 3.69 (s, 2H), 3.72 (s, 3H), 7.39 (d, 2H, *J*=8.1), 7.56 (m, 2H, *J*=7.8), 7.68 (m, 4H)

**Intermediate M4-biphenyl-4-carbaldehyde**

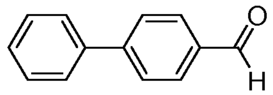

The title compound was prepared by a procedure similar to that described for intermediate M1 except that phenylboronic acid was used in place of 4-(trifluoromethyl)phenylboronic acid. $^{1}H$-NMR ($CDCl_3$, 400MHz) $\delta$ 7.42 (m, 1H), 7.49 (m, 2H), 7.64 (m, 2H), 7.76 (d, 2H, *J*=8.4), 7.96 (d, 2H, *J*=8.4), 10.06 (s, 1H).

**Intermediate M5-4'-methylbiphenyl-4-carbaldehyde**

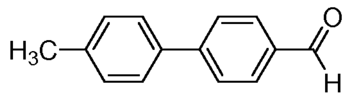

The title compound was prepared by a procedure similar to that described for intermediate M1 except that p-tolylboronic acid was used in place of 4-(trifluoromethyl)phenylboronic acid. $^{1}H$-NMR ($CDCl_3$, 400MHz) $\delta$ 2.42 (s, 3H), 7.29 (d, 2H, *J*=8.0), 7.54 (d, 2H, *J*=8.4), 7.79 (d, 2H, *J*=8.0), 7.94 (d, 2H, *J*=8.0), 10.04 (s, 1H).

**Intermediate M6-4'-chlorobiphenyl-4-carbaldehyde**

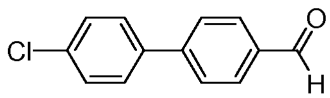

The title compound was prepared by a procedure similar to that described for intermediate M1 except that 4-chlorophenylboronic acid was used in place of 4-(trifluoromethyl)phenylboronic acid. $^{1}H$-NMR ($CDCl_3$, 300MHz) $\delta$ 7.46 (d, 2H, *J*=8.4), 7.58 (d, 2H, *J*=8.4), 7.73 (d, 2H, *J*=8.1), 7.94 (d, 2H, *J*=8.1), 10.07 (s, 1H). The following intermediates were prepared by a procedure similar to that described for intermediate M1.

| Name | Structure | Reactant |
|---|---|---|
| **M7: methyl 4'-(trifluoromethyl)biphenyl-4 -carboxylate** | 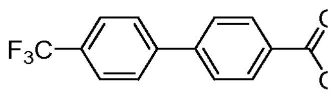 | 4-(trifluoromethyl)phenyl boronic acid, methyl 4-bromobenzoate |
| **M8: methyl biphenyl-4-carboxylate** | 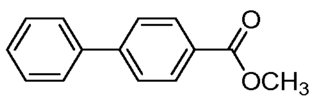 | phenylboronic acid, methyl 4-bromobenzoate |
| **M9: methyl 2-(biphenyl-4-yl)acetate** | 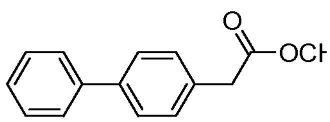 | phenylboronic acid, M2 |

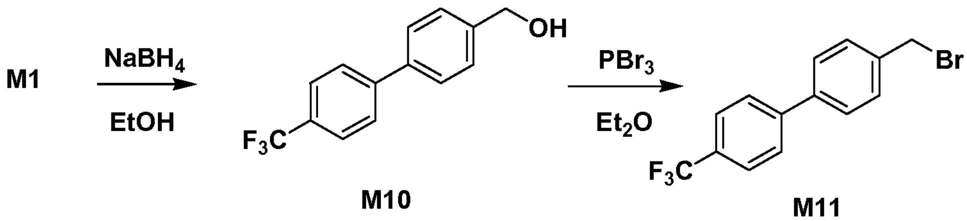


**M10** **M11**

**Intermediate M11-4-(bromomethyl)-4'-(trifluoromethyl)biphenyl**

To an ice cold solution of intermediate M1 (2.5g, lequiv) in absolute ethanol (20ml) was added $NaBH_4$ (190mg) in batches, and then the mixture was stirred at room temperature for 1h. After the reaction was complete, solvent was evaporated and the residue was diluted with water. Concentrated hydrochloric acid was added dropwise to the solution in an ice bath until no more bubbles were generated. Then, sodium bicarbonate solution was added and the mixture was extracted with dichloromethane three times. The combined organic phase was washed with brine twice, dried over $MgSO_4$, and then filtered. The filtrate was evaporated in vacuo to give intermediate M10 as a white solid (2.5g). $^1$H-NMR ($CDCl_3$, 300MHz) $\delta$ 4.77(s, 2H), 7.48 (d, 2H, *J*=8.4), 7.61 (d, 2H, *J*=8.1), 7.70 (s, 4H).

To an ice cold solution of intermediate M10 (2.02g, lequiv) in anhydrous diethyl ether (20ml) protected with a $CaCl_2$ drying tube to insulate moisture was added phosphorus tribromide (0.38ml, 0.5equiv). The mixture was stirred at room temperature for 1.5h to obtain a clear solution. TLC detection showed that the reaction was complete and then the mixture was quenched with saturated sodium bicarbonate solution. The resulting precipitate was filtered off and the filtrate was extracted with dichloromethane twice (40ml), dried over $MgSO_4$, filtered and then evaporated in vacuo to give intermediate M11 (1.77g) as a white solid which could be used in next step without further purification.

The following intermediates were prepared by a procedure similar to that described for intermediate M11.

| Name | Structure | Precusor |
|---|---|---|
| **M12: 4-(bromomethyl)biphenyl** | 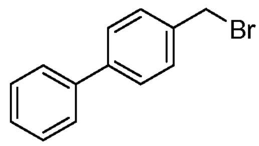 | **M4** |
| **M13: 4-(bromomethyl)-4'-methylbiphenyl** | 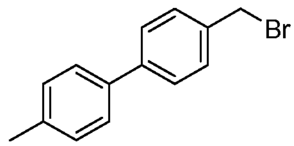 | **M5** |
| **M14: 4-(bromomethyl)-4'-chlorobiphenyl** | 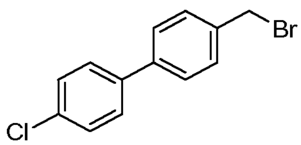 | **M6** |

**Intermediate M15-(E)-3-(1-methyl-1H-pyrazol-4-yl)acrylic acid**

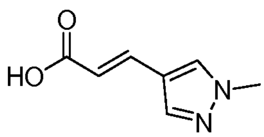

Methylpyrazole (4.1g, 50mmol, lequiv) and anhydrous DMF (11.6ml, 3equiv) were placed in a 100 ml of two-neck flask which was equipped with a Allihn condenser (a CaCl2 drying tube was connected thereto) and a constant-pressure dropping funnel, and the flask was placed in the oil bath of 90 °C. Phosphorus oxychloride (5.6ml, 1.2equiv)) was added dropwise over a period of 1h. After the addition was completed, the mixture was stirred for a further 2h. Then the solution was cooled and poured into a lot of ice water. The resulting mixture was brought to pH 4~5 with a solution of 10% NaOH solution and then extracted with dichloromethane several times until the product in the aqueous phase is little. The combined organic phase was washed with a small amount of brine twice, dried over $MgSO_4$, filtered and then evaporated in vacuo to give the crude product of 1-methyl-1H-pyrazole-4-carbaldehyde, which can be directly used in the next step without further separation.

1-methyl-1H-pyrazole-4-carbaldehyde previously obtained, malonic acid (4.68g, 45mmol), pyridine (4ml, 50mmol) and piperidine (0.09ml, 1mmol) were placed in a 250 ml flask and heated at 110°C for 6h under nitrogen, then the reaction was stopped and cooled. The resulting solution was added with water (100ml), followed by addition of strong

aqua dropwise to dissolve all the solids. The solution was then brought to pH~1 with concentrated hydrochloric acid. The precipitate thus obtained was collected by filtration, washed with water several times and then dried in vacuo to give the title compound (2.9g). $^1$H-NMR ($d_6$-DMSO, 300 MHz) δ3.83 (s, 3H), 6.17 (d, 1H, *J*=16.2), 7.45 (d, 1H, *J*=15.9), 7.83 (s, 1H), 8.07 (s, 1H), 12.07 (s, 1H).

**Intermediate M16-(*E*)-methyl 3-(1-methyl-1H-pyrazol-4-yl)acrylate**

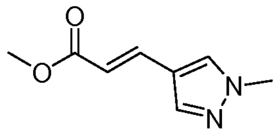

To a stirred slurry of intermediate M15 (2.9g, 19.1mmol) in methanol (30ml) was added concentrated sulfuric acid dropwise (1.9 ml). Heat was released and the mixture was refluxed for 2.5h before it was evaporated in vacuo to remove most of the solvent. Ice water was added to the residue and the resulting solution was neutralized with 10% aqueous sodium hydroxide solution and then extracted with dichloromethane three times. The dichloromethane layer was dried over $MgSO_4$, filtered and then evaporated to give the title compound as a solid (3.08g). $^1$H-NMR ($CDCl_3$, 300 MHz) δ3.75 (s, 3H), 3.89 (s, 3H), 6.14 (d, 1H, *J*=15.9), 7.53 (s, 1H), 7.54 (d, 1H, *J*=16.2), 7.67 (s, 1H).

**Intermediate M17-methyl 3-(1-methyl-1H-pyrazol-4-yl)propanoate**

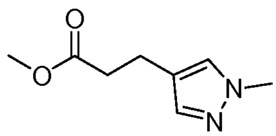

To a solution of M16 (3.08g, 18.55mmol) in absolute methanol (20ml) was added 10% palladium on charcoal (300mg) and the mixture was stirred at 50°C and normal pressure for 3.5h. TLC detection showed that the reaction was complete. The catalyst was filtered off and the filtrate was evaporated to give the title compound as a colorless oil (3.06g). $^1$H-NMR ($CDCl_3$, 300 MHz) δ2.54 (t, 2H, *J*=7.2), 2.77 (t, 2H, *J*=7.2), 3.65 (s, 3H), 3.83 (s, 3H), 7.16 (s, 1H), 7.30 (s, 1H).

**Intermediate M18- methyl 3-hydroxy-2-((1-methyl-1H-pyrazol-4-yl)methyl)acrylate**

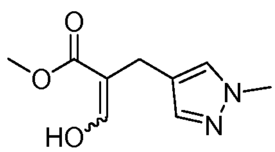

To a solution of intermediate M17 (2.53g, 15.06mmol, lequiv) in anhydrous DME (20ml) was added sodium hydride (55-65% m/m, suspended in mineral oil, 0.75g, 1.25equiv) under nitrogen at room temperature and the mixture was stirred for 1h. Methyl formate (2.74ml, 3equiv) was added and the mixture was stirred at room temperature for a further 15h. Then, anhydrous diethyl ether (100ml) was added and the mixture was stirred for 15min. The solid thus obtained was collected by filtration, washed with diethyl ether and then the filter cake was poured into saturated ammonium chloride solution. The resulting mixture was acidified with concentrated hydrochlolic acid to pH 4~5 in an ice bath and then extracted with dichloromethane three times. The combined organic phase was washed with brine twice, dried over $MgSO_4$, filtered and then evaporated to give the title compound as a solid (0.51g) which could be used in next step without further purification.

**Intermediate M19- *tert*-butyl 4-(1-methoxy-2-methyl-1-oxopropan-2-yl)piperazine-1-carboxylate**

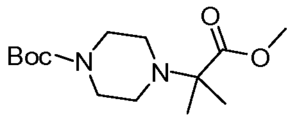

A mixture of *tert*-butyl piperazine-1-carboxylate (3.72g, 2equiv), methyl 2-bromo-2-methylpropanoate (2.58ml, 2equiv), anhydrous $K_2CO_3$ (5.52g, 4equiv) and KI (166mg, 0.1equiv) in acetonitrile (40ml) was refluxed for 8h. Additional methyl 2-bromo-2-methylpropanoate (1.29ml, lequiv) was added and the mixture was refluxed for a further 12h before it was cooled and filtered to remove the insoluble inorganic substances. The filtrate was evaporated and then purified by silicagel column chromatography (petroleum ether/ethyl acetate=2:1) to give the title compound as an oil (2.82g). $^1$H-NMR ($CDCl_3$, 400 MHz) δ1.31 (s, 6H), 1.44 (s, 9H), 2.52 (vbrs, 4H), 3.42 (vbrs, 4H), 3.69 (s, 3H).

**Intermediate M20- methyl 2-methyl-2-(piperazin-1-yl)propanoate hydrochloride**

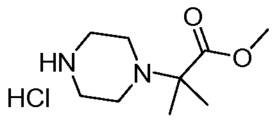

To an ice cold solution of intermediate M19 (2.82g, lequiv) in methanol (20ml) was added acetyl chloride (3.5ml, 5equiv) dropwise and the mixture was stirred for 1h before it was evaporated to remove part of the solvent. White solid precipitated. Diethyl ether (20ml) was added to the residue and the mixture was stirred for a few minutes. The precipitate thus obtained was collected by filtration and dried in vacuo to give the title compound (2.33g). $^1$H-NMR ($d_6$-DMSO, 300 MHz) δ1.49 (s, 6H), 3.33 (vbrs, 8H), 3.73 (s, 3H), 9.52 (s, 1H).

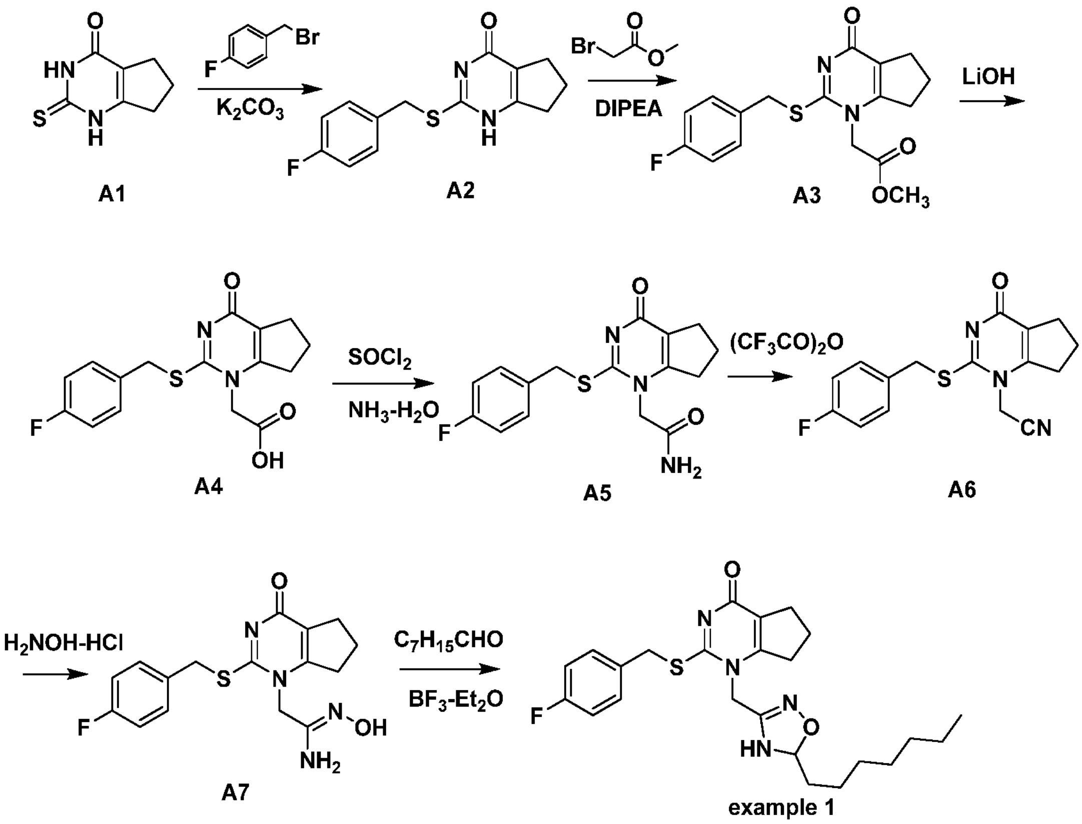

A1 A2 A3 A4 A5 A6 A7 example 1

**Intermediate A1-2-thioxo-2,3,6,7-tetrahydro-1*H*-cyclopenta[*d*]pyrimidin-4(5*H*)-one**

Prepared according to reference method [J. Amer. Chem. Soc., 81, 3108 (1959)].

**Intermediate A2- A2-2-(4-fluorobenzylthio)-6,7-dihydro-1H-cyclopenta[d]pyrimidin-4(5H)-one**

A mixture of intermediate A1 (4.06g, lequiv), 1-(bromomethyl)-4-fluorobenzene (3.2ml, 1.05equiv), anhydrous $K_2CO_3$ (5g, 1.5equiv), KI (0.4g, 0.1equiv) in acetone (40ml) was refluxed for 2h. Then most of the solvent was removed by evaporation in reduced pressure and the residue was poured into water. The resulting mixture was neutralized with con. HCl. The solid thus obtained was collected by filtration, washed with water several times and then the moisture was evacuated. The solid was transferred into a flask, to which methanol (30ml) was added. The mixture was refluxed for 0.5h, cooled and filtered and dried to get the title compound (4.62g) as a solid.

**Intermediate A3- Methyl 2-(2-(4-fluorobenzylthio)-4-oxo-4,5,6,7-tetrahydro-1*H*-cyclopenta[*d*]pyrimidin-1-yl)acetate**

A solution of intermediate A2 (2.67g, 10mmol), methyl 2-bromoacetate (1ml, 11mmol) and DIPEA (1.82ml, 11mmol) in THF (20ml) was refluxed overnight under nitrogen and then quenched with saturated $NH_4Cl$ solution. Then it was extracted with EA twice. The organic phase was washed with water twice, dried over $MgSO_4$, filtered, and then concentrated. The residue was purified by column chromatography (DCM/MeOH=30:1) to give the title compound as a jelly (0.5g). $^1$H-NMR ($CDCl_3$, 300 MHz) $\delta$2.10 (m, 2H), 2.81 (m, 4H), 2.98 (t, 2H, *J*=7.5), 3.78 (s, 3H), 4.49 (s, 2H), 4.57 (s, 2H), 6.97 (t, 2H, *J*=8.4), 7.35 (dd, 2H, *J*=7.8, 6.0).

**Intermediate A4- 2-(2-(4-fluorobenzylthio)-4-oxo-4,5,6,7-tetrahydro-1H-cyaopenta[d]pyrimidin-1-yl)acetic acid**

A solution of intermediate A3 (6.9g, 1equiv), $LiOH{\cdot}H_2O$ (2.5g, 3equiv) in isopropanol/water (1:2, 60ml) was stirred at room temperature for 2h to obtain a clear solution. The solution was acidified with concd. HCl to pH<3 in an ice bath, the solid thus obtained was collected by filtration, washed with water several times and the moisture was evacuated as much as possible. The solid was transferred to a 100 mL flask and 30 ml acetone was added to obtain a mixture, which was refluxed for 0.5 h. Then the mixture was placed in refrigerator for several hours until the recrystallization was complete. The solid obtained was filtered and washed by acetone, then dried to afford the title compound as a white solid (4.3g). $^1$H-NMR ($d_6$-DMSO, 300 MHz) $\delta$1.96(m 2H), 2.58 (t, 2H, *J*=7.2), 2.82 (t, 2H, *J*=7.5), 4.42 (s, 2H), 4.69 (s, 2H), 7.13 (t, 2H, *J*=9.0), 7.47 (dd, 2H, *J*=8.9 ,5.6), 13.5 (vbrs, 1H); MS (ESI): 333 (M-H).

**Intermediate A5- 2-(2-(4-fluorobenzylthio)-4-oxo-4,5,6,7-tetrahydro-1*H*-cyaopenta[*d*]pyrimidin-1-yl)acetamide**

To an ice cold suspension of intermediate A4 (2.0g, lequiv) in dichloromethane (15ml) (wherein a drying tube was used to insulate moisture) were added thionyl chloride (0.47ml) and DMF (2 drops, catalyst). The mixture was stirred for 0.5h to get a brown clear solution and then added slowly to a flask containing strong aqua (20ml) over 10min in an ice bath with vigorous stirring. The solid thus formed was collected by filtration, washed to white with water and ethanol, and then dried in vacuo to give the title compound (1.0g). MS (ESI): 334(M+H).

**Intermediate A6- 2-(2-(4-fluorobenzylthio)-4-oxo-4,5,6,7-tetrahydro-1H-cyclopenta[d]pyrimidin-1-yl)acetonitrile**

To an ice-salt cold (-15°C) suspension of intermediate A5 (10mmol) in anhydrous THF (20ml) was added trifluoroacetic anhydride (20mmol) dropwise via a syringe under nitrogen over 10min. The reaction mixture was allowed to warm to room temperature, quenched with saturated $NaHCO_3$ solution and extracted with EA twice. The combined organic phase was washed with brine twice, dried and purified by column chromatography (DCM/MeOH=30:1) to give the title compound. $^1$H-NMR ($CDCl_3$, 300 MHz) $\delta$2.16 (m, 2H), 2.82 (t, 2H, *J*=7.2), 2.98 (t, 2H, *J*=7.5), 4.53 (s, 2H), 4.76 (s, 2H), 5.67 (t, 1H, *J*=4.8), 7.00 (t, 2H, *J*=8.7), 7.39 (dd, 2H, *J*=8.3, 5.3); MS (ESI): 316 (M+H).

**Intermediate A7- (Z)-2-(2-(4-fluorobenzylthio)-4-oxo-4,5,6,7-tetrahydro-1H-cyclopenta[d]pyrimidi n-1-yl)-N'-hydroxyacetimidamide**

To a solution of intermediate A6 (10mmol) in absolute ethanol (20ml) were added hydroxylamine hydrochloride (13mmol) and $K_2CO_3$ (15mmol). The mixture was stirred at room temperature for 12h. TLC detection showed that the reaction was complete. Then the mixture was evaporated to remove most of the solvent. Water (10ml) and EA (10ml) was added to the residue and the resulting mixture was stirred for a 1h. The solid thus obtained was collected by filtration,

washed with water and EA, and then dried in vacuo to give the title compound. $^1$H-NMR ($d_6$-DMSO, 300 MHz) δ1.9 (m, 2H), 2.59 (t, 2H, *J*=7.3), 2.90 (t, 2H, *J*=7.3), 4.43 (s, 2H), 4.55 (s, 2H), 5.72 (s, 2H), 7.17 (t, 2H, *J*=8.8), 7.49 (dd, 2H, *J*=8.8 ,5.6), 9.44 (s, 1H); MS (ESI): 349(M+H).

**Example 1- 2-(4-fluorobenzylthio)-1-((5-n-heptyl-4,5-dihydro-1,2,4-oxadiazol-3-yl)methyl)-6, 7-dihydro-1H-cyclopenta[d]pyrimidin-4(5H)-one**

To a mixture of intermediate A7 (1mmol) and octyl aldehyde (1.1mmol) in anhydrous THF (5ml) was added $BF_3$-$Et_2O$ solution (2mmol) under nitrogen. The solution was stirred at room temperature until completion of the reaction monitored by TLC. The reaction mixture was then poured into saturated $NaHCO_3$ solution and extracted with EA. The EA solution was dried over $MgSO_4$ and then purified by column chromatography to give the title compound. $^1$H-NMR ($CDCl_3$, 300 MHz) δ0.84 (t, 3H, *J*=6.9), 1.22-1.32 (m, 10H), 1.70 (m, 2H), 2.13 (m, 2H), 2.69 (t, 2H, *J*=7.5), 3.00 (m, 2H), 4.52 (s, 2H), 4.76 (2x d, 2H, *J*=16.5), 5.67 (t, 1H, *J*=4.8), 6.93 (t, 2H, *J*=9.0), 7.10 (s, 1H), 7.48 (dd, 2H, *J*=8.7, 5.1); MS (ESI): 459(M+H).

**Example 2- 1-((5-n-decyl-4,5-dihydro-1,2,4-oxadiazol-3-yl)methyl)-2-(4-fluorobenzylthio)-6,7-dihydro-1H-cyclopenta[d]pyrimidin-4(5H)-one**

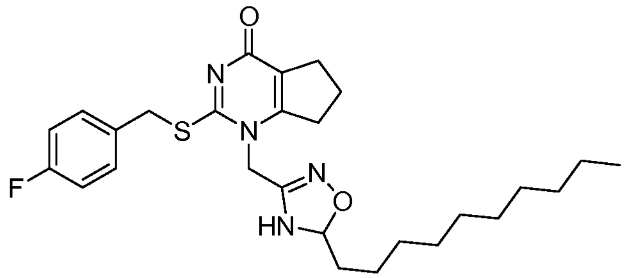

Following a procedure similar to that described for the preparation of example 1 except that n-undecylic aldehyde was used in place of octyl aldehyde. $^1$H-NMR ($CD_3OD$, 400 MHz) δ0.89 (t, 3H, *J*=6.6), 1.27 (m, 16H), 1.60 (m, 2H), 2.12 (m, 2H), 2.75 (t, 2H, *J*=7.2), 3.00 (t, 2H, *J*=7.6), 4.52 (s, 2H), 4.86 (s, 2H), 5.56 (t, 1H, *J*=4.8), 7.01 (t, 2H, *J*=8.4), 7.48 (dd, 2H, *J*=8.0, 5.6); MS (ESI): 501(M+H).

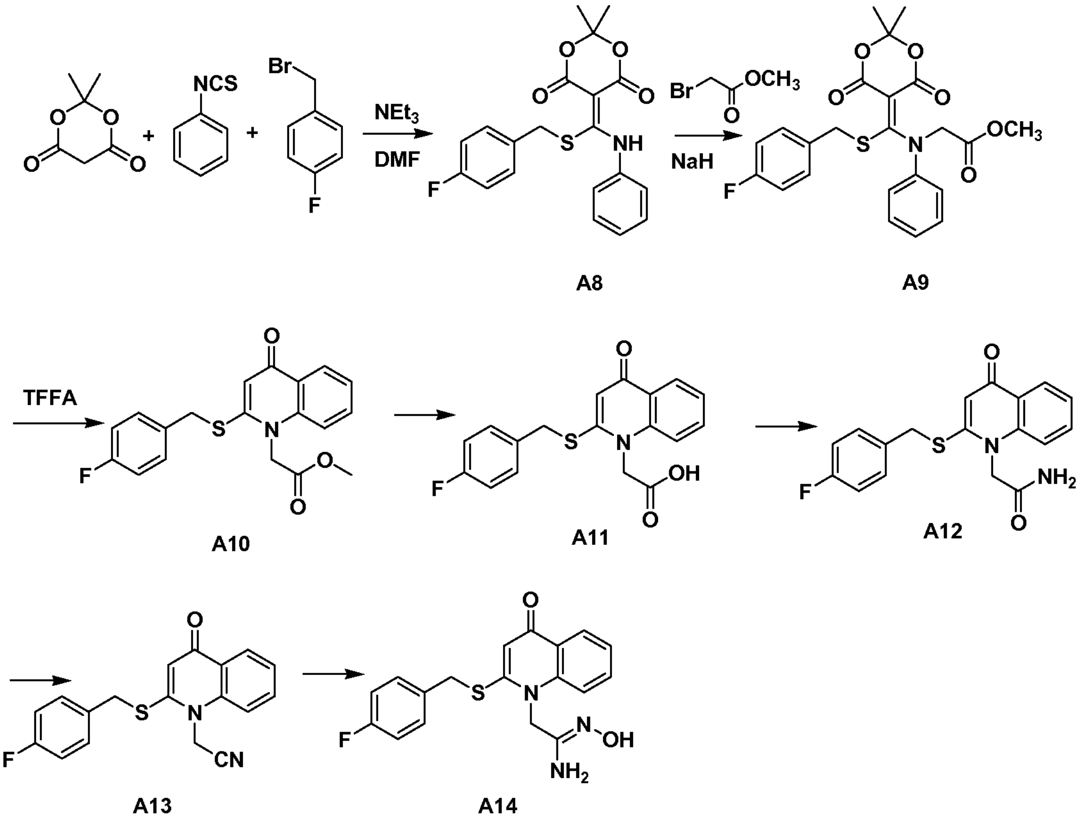


A8 A9 A10 A11 A12 A13 A14

**Intermediate A8 - 5-((4-fluorobenzylthio)(phenylamino)methylene)-2,2-dimethyl-1,3-dioxane-4,6-di one**

To a solution of 2,2-dimethyl-1,3-dioxane-4,6-dione (1.5g, lequiv) in DMF (10ml) was added triethylamine (2.7ml, 2equiv) and the mixture was stirred at room temperature for 0.5h to obtain a light yellow solution before phenyl isocyanate (1.2ml, lequiv) was added. The resulting mixture was stirred for a further 5h at 40~45°C and then placed in an ice bath. 1-(bromomethyl)-4-fluorobenzene (1.25ml, lequiv) was added and the mixture was stirred at room temperature overnight. After the reaction was complete, the mixture was poured into water, extracted with EA three times. The combined organic phase was washed with brine several times, dried over $MgSO_4$ and concentrated. The solid thus obtained was collected by filtration and dried to give the title compound (2.2g). $^1$H-NMR ($d_6$-DMSO, 300 MHz) δ1.63 (s, 6H), 4.09 (s, 2H), 7.14 (t, 2H, *J*=8.7), 7.29 (dd, 2H, *J*=8.9, 5.6), 7.44 (m, 5H), 12.13 (s, 1H); MS (ESI): 388(M+H).

**Intermediate A9- methyl 2-(((2,2-dimethyl-4,6-dioxo-1,3-dioxan-5-ylidene)(4-fluorobenzylthio)methyl)(phe nyl)amino)acetate**

To an ice cold solution of intermediate A8 (13.55g, lequiv) in anhydrous DMF (40ml) was added NaH (2.8g, 2equiv) in batches under nitrogen and the mixture was stirred for 20 min. Methyl 2-bromoacetate (6.7ml, 2equiv) was added and the solution was stirred at room temperature for 2h and then at 60°C overnight. After the reaction was complete as monitored by TLC, the mixture was poured into saturated $NH_4Cl$ solution and extracted with 200ml of EA three times. The organic phase was washed with brine five times, dried over $MgSO_4$, and purified by column chromatography (PE/EA=2:1) to give the title compound as a jelly (8.2g). $^1$H-NMR ($CDCl_3$, 400 MHz) δ1.56 (s, 6H), 3.72 (s, 3H), 4.31 (s, 2H), 4.82 (s, 2H), 6.98 (t, 2H, *J*=8.6), 7.21 (m, 2H), 7.39 (m, 5H).

**Intermediate A10- methyl 2-(2-(4-fluorobenzylthio)-4-oxoquinolin-1(4H)-yl)acetate**

A mixture of intermediate A9 (8.2g, 17.86mmol) and trifluoroacetic anhydride (10ml) was stirred at room temperature overnight. Excess trifluoroacetic anhydride was removed under reduced pressure. The residue was quenched with saturated aq. $NaHCO_3$ solution (20ml) and extracted with dichloromethane (3*10ml). The combined organic phase was dried over $MgSO_4$, and then purified by column chromatography (PE/EA=1:3) to give the title compound (5.5g). $^1$H-NMR ($CDCl_3$, 400 MHz) δ3.79 (s, 3H), 4.23 (s, 2H), 5.09 (s, 2H), 6.44 (s, 1H), 7.02 (t, 2H, *J*=8.6), 7.18 (d, 1H, *J*=8.7), 7.33 (dd, 2H, *J*=8.8, 5.2), 7.39 (t, 1H, *J*=7.4), 7.63 (ddd, 1H, *J*=8.8, 7.2, 1.6), 8.41 (dd, 1H, *J*=8.2, 1.4).

**Intermediate A11-2-(2-(4-fluorobenzylthio)-4-oxoquinolin-1(4H)-yl)acetic acid**

Following a procedure similar to that described for the preparation of intermediate A4 except that intermediate A10 was used as a starting material. $^1$H-NMR ($d_6$-DMSO, 300 MHz) δ4.45 (s, 2H), 5.18 (s, 2H), 6.25 (s, 1H), 7.19 (t, 2H, *J*=8.7), 7.37 (t, 1H, *J*=7.2), 7.50 (dd, 2H, *J*=8.7, 5.4), 7.60 (d, 1H, *J*=8.7), 7.69 (ddd, 1H, *J*=8.7, 7.2, 1.7), 8.13 (dd, 1H, *J*=8.0, 1.8).

**Intermediate A12-2-(2-(4-fluorobenzylthio)-4-oxoquinolin-1(4H)-yl)acetamide**

To an ice cold suspension of intermediate A11 (1.37g, lequiv) in anhydrous DMF (6ml) were added EDCI (1.15g, 1.5equiv) and HOBt (0.81g, 1.5equiv) under nitrogen and the mixture was stirred for 0.5h to obatin a yellow clear solution. Then, DIPEA (2.64ml, 4equiv) and $NH_4Cl$ (0.43g, 2equiv) were added and the mixture was stirred at room temperature overnight. Then the mixture was diluted with water (20ml) and EA (20ml), stirred for 1h. The solid thus obtained was collected by filtration, washed with water and EA, and dried to obtain the title compound (0.54g). Aqueous phase of the filtrate was separated and acidified with concd. HCl to recycle the starting materials. $^1$H-NMR ($d_6$-DMSO, 300 MHz) δ4.42 (s, 2H), 5.04 (s, 2H), 6.23 (s, 1H), 7.19 (t, 2H, *J*=8.9), 7.36 (t, 1H, *J*=7.4), 7.49 (m, 4H), 7.68 (t, 1H, *J*=7.4), 7.79 (s, 2H), 8.13 (d, 1H, *J*=7.8); MS (ESI) found (M+H) = 343.

**Intermediate A13-2-(2-(4-fluorobenzylthio)-4-oxoquinolin-1(4H)-yl)acetonitrile**

Following a procedure similar to that described for the preparation of intermediate A6 except that intermediate A12 was used as a starting material. $^1$H-NMR ($CDCl_3$, 300 MHz) δ4.27 (s, 2H), 5.22 (s, 2H), 6.43 (s, 1H), 7.03 (t, 2H, *J*=8.6), 7.33 (dd, 2H, *J*=8.7, 5.1), 7.41 (d, 1H, *J*=8.7), 7.45 (dd, 1H, *J*=7.5, 7.5), 7.75 (ddd, 1H, *J*=8.7, 7.2, 1.5), 8.41 (dd, 1H, *J*=8.0, 1.7).

**Intermediate A14-(Z)-2-(2-(4-fluorobenzylthio)-4-oxoquinolin-1(4H)-yl)-N'-hydroxyacetimidamide**

Following a procedure similar to that described for the preparation of intermediate A7 except that intermediate A13 was used as a starting material. $^1$H-NMR ($d_6$-DMSO, 300 MHz) δ4.42 (s, 2H), 5.04 (s, 2H), 5.68 (s, 2H), 6.21 (s, 1H), 7.19 (t, 2H, *J*=8.7), 7.33 (t, 1H, *J*=7.4), 7.47-7.55 (m, 3H), 7.68 (m, 1H), 8.09 (dd, 1H, *J*=7.8, 1.5).

**Example 3-**2**-(4-fluorobenzylthio)-1-((5-n-heptyl-4,5-dihydro-1,2,4-oxadiazol-3-yl)methyl)qui nolin-4(1H)-one**

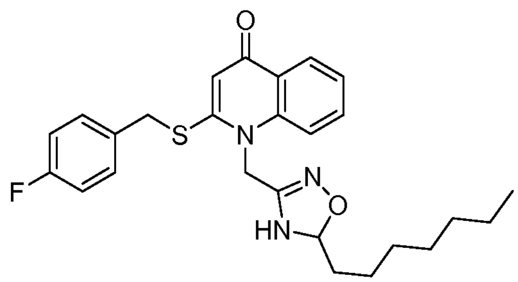

Following a procedure similar to that described for the preparation of example 1 except that intermediate A14 and octyl aldehyde were used as starting materials. $^1$H-NMR ($CDCl_3$, 400 MHz) δ0.83 (t, 3H, *J*=7.0), 1.18-1.30 (m, 10H), 1.70 (m, 2H), 4.08 (s, 2H), 5.21 (s, 2H), 5.59 (t, 1H, *J*=5.2), 5.71 (s, 1H), 6.04 (s, 1H), 7.03 (t, 2H, *J*=8.6), 7.33 (m, 3H), 7.67 (m, 2H), 8.25 (d, 1H, *J*=7.6).

**Example 4-1-((5-n-butyl-4,5-dihydro-1,2,4-oxadiazol-3-yl)methyl)-2-(4-fluorobenzylthio)-6,7-dihydro-1H-cyclopenta[d]pyrimidin-4(5H)-one**

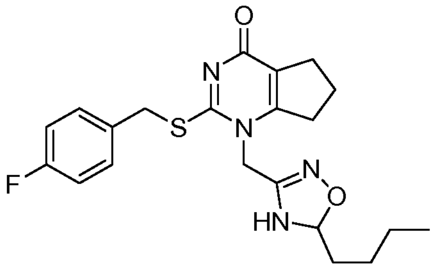

Following a procedure similar to that described for the preparation of example 1 except that n-valeraldehyde was used in place of octyl aldehyde. $^1$H-NMR ($CDCl_3$, 400 MHz) δ 0.85 (t, 3H, J=7.2), ~1.30 (m, 4H), 1.68 (m, 2H), 2.13 (m, 2H), 2.70 (t, 2H, J=7.2), 3.00 (t, 2H), 4.45 (s, 2H), 4.75 (2x d, 2H, J=16.4), 5.66 (t, 1H, J=4.8), 6.94 (t, 2H, J=8.4), 7.29 (dd, 2H, J=8.4, 4.8).

**Example 5-1-((5-n-decyl-4,5-dihydro-1,2,4-oxadiazol-3-yl)methyl)-2-(4-fluorobenzylthio)qui nolin-4(1H)-one**

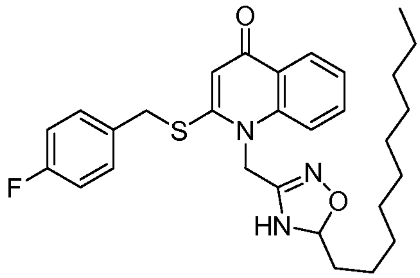

Following a procedure similar to that described for the preparation of example 1 except that intermediate A14 and n-undecanal was used as staring materials. $^1$H-NMR ($CDCl_3$, 400 MHz) δ0.86 (t, 3H), 1.20 (m, 16H), 1.71 (m, 2H), 4.11 (s, 2H), 5.21 (s, 2H), 5.58 (t, 1H), 5.71 (s, 1H), 6.09 (s, 1H), 7.03 (t, 2H), 7.34 (m, 3H), 7.67 (m, 2H), 8.27 (d, 1H).

The following compounds were prepared by a procedure similar to that described for example 1.

| **Name** | **Structure** | **Precusor** |
|---|---|---|
| **Example 6: 2-(4-fluorobenzylthio)-1-((5 -n-propyl-4,5-dihydro-1,2,4 -oxadiazol-3-yl)methyl)-6,7-dihydro-1H-cyclopenta[d]p yrimidin-4(5H)-one** | 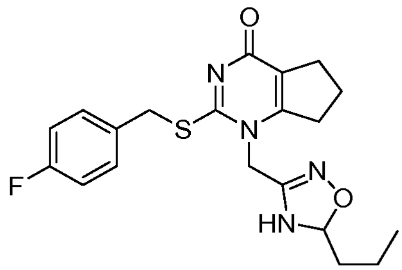 | A7,n-butanal |

(continued)

| Name | Structure | Precusor |
|---|---|---|
| **Example 7: 2-(4-fluorobenzylthio)-1-((5 -(4'-(trifluoromethyl)biphe nyl-4-yl)-4,5-dihydro-1,2,4-oxadiazol-3-yl)methyl)-6,7-dihydro-1H-cyclopenta[d]p yrimidin-4(5H)-one** | 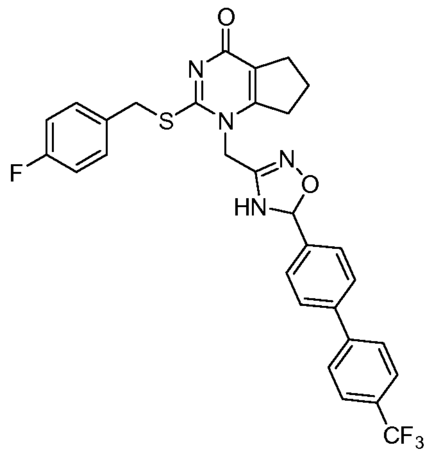 | A7,M1 |

**Example 8-(*E*)-2-(4-fluorobenzylthio)-1-((5-n-heptyl-4-(n-oct-1-enyl)-4,5-dihydro-1,2,4-oxadi azol-3-yl)methyl)-6,7-dihydro-1H-cyclopenta[d]pyrimidin-4(5H)-one**

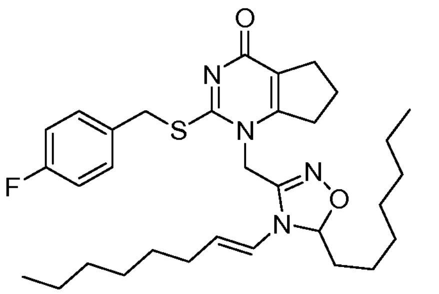

To a mixture of example 1 (1equiv) and octyl aldehyde (2equiv) in anhydrous THF was added boron trifluoride-ether complex (3equiv) under nitrogen. The mixture was stirred for 2h at room temperature. About a half of the starting materials were converted and there was no significant conversion of starting materials as time increases. The mixture was quenched with saturated aq. $NaHCO_3$ solution and extracted with EA. The organic phase was dried over $MgSO_4$, purified by column chromatography to give the title compound. $^1$H-NMR ($d_6$-DMSO, 400 MHz) $\delta$0.84 (m, 6H), 1.19-1.32 (m, 18H), 1.68 (m, 2H), 1.96(m, 4H), 2.57 (t, 2H, *J*=7.2), 2.88 (M, 2H), 4.42 (2x d, 2H, *J*=13.4), 4.75 (dt, 1H, *J*=13.6, 6.8), 5.07 (s, 2H), 5.87 (m, 1H), 7.13 (t, 2H, *J*=8.8), 7.47 (dd, 2H, *J*=8.4 ,5.6); MS (ESI): 569(M+H).

The following compounds were prepared by a procedure similar to that described for example 8.

| Name | Structure | Precusor |
|---|---|---|
| **Example 9: (E)-2-(4-fluorobenzylthio) -1-((4-(n-pent-1-enyl)-5-n-propyl-4,5-dihydro-1,2,4-o xadiazol-3-yl)methyl)-6,7-dihydro-1H-cyclopenta[d] pyrimidin-4(5H)-one** | 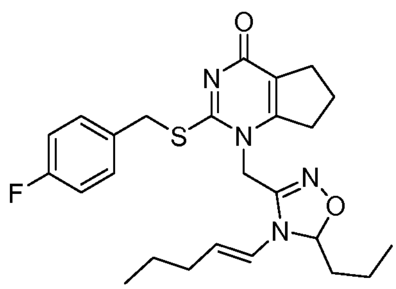 | Example 4,n-valeraldehyde |

(continued)

| Name | Structure | Precusor |
|---|---|---|
| **Example 10: (E)-1-((4-(n-but-1-enyl)-5-n-propyl-4,5-dihydro-1,2,4 -oxadiazol-3-yl)methyl)-2-(4-fluorobenzylthio)-6,7-dihydro-1H-cyclopenta[d]pyrimidin-4(5H)-one** | 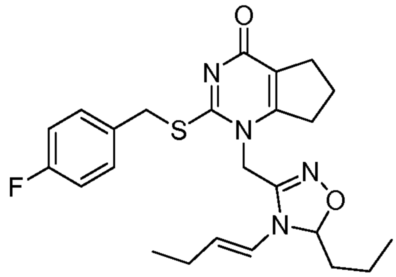 | Example 4,n-butyl aldehyde |
| **Example 11: (E)-1-((5-n-butyl-4-(n-hex-1-enyl)-4,5-dihydro-1,2,4-oxadiazol-3-yl)methyl)-2-(4-fluorobenzylthio)-6,7-dihydro-1H-cyclopenta[d]pyrimidin-4(5H)-one** | 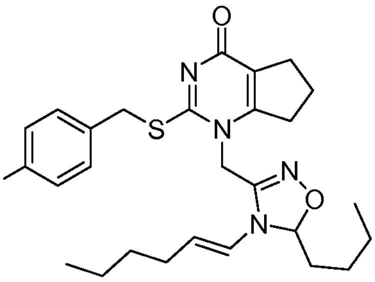 | Example 5,n-hexanal |
| **Example 12: (E)-1-((4-(n-but-1-enyl)-5-n-butyl-4,5-dihydro-1,2,4-oxadiazol-3-yl)methyl)-2-(4-fluorobenzylthio)-6,7-dihydro-1H-cyclopenta[d]pyrimidin-4(5H)-one** | 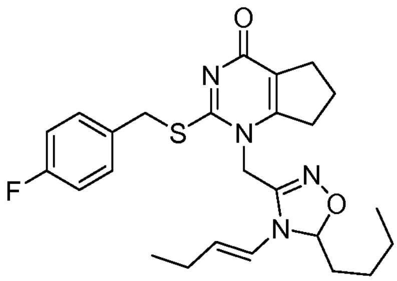 | Example 5,n-butyl aldehyde |
| **Example 13: (E)-2-(4-fluorobenzylthio)-1-((4-(n-hex-1-enyl)-5-propyl-4,5-dihydro-1,2,4-oxad iazol-3-yl)methyl)-6,7-dihydro-1H-cyclopenta[d]pyrimidin-4(5H)-one** | 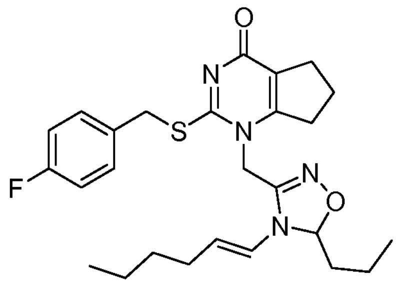 | Example 4,n-hexanal |
| **Example 14: (E)-1-((5-n-butyl-4-(n-pent-1-enyl)-4,5-dihydro-1,2,4-oxadiazol-3-yl)methyl)-2-(4-fluorobenzylthio)-6,7-dihydro-1H-cyclopenta[d]pyrimidin-4(5H)-one** | 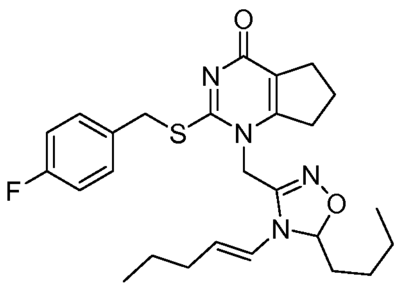 | Example 5,n-valeraldehyde |

(continued)

| Name | Structure | Precusor |
|---|---|---|
| **Example 15:** **(E)-2-(4-fluorobenzylthio)-1-((4-(n-oct-1-enyl)-5-propyl-4,5-dihydro-1,2,4-oxadiazol-3-yl)methyl)-6,7-dihydro-1H-cyclopenta[d]pyrimidin-4(5H)-one** | 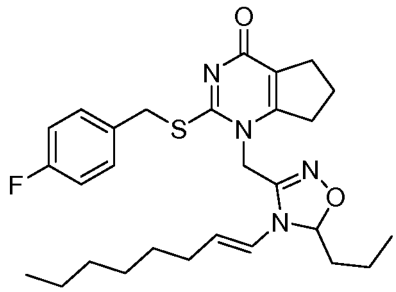 | Example 4, octyl aldehyde |
| **Example 16:** **(E)-2-(4-fluorobenzylthio)-1-((5-n-heptyl-4-(n-pent-1-enyl)-4,5-dihydro-1,2,4-o xadiazol-3-yl)methyl)-6,7-dihydro-1H-cyclopenta[d]pyrimidin-4(5H)-one** | 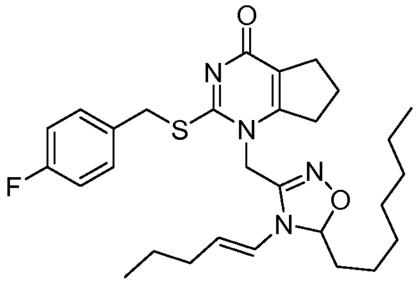 | Example 1, n-valeraldehyde |
| **Example 17:** **(E)-1-((4-(n-but-1-enyl)-5-n-heptyl-4,5-dihydro-1,2,4-oxadiazol-3-yl)methyl)-2-(4-fluorobenzylthio)-6,7-dihydro-1H-cyclopenta[d]pyrimidin-4(5H)-one** | 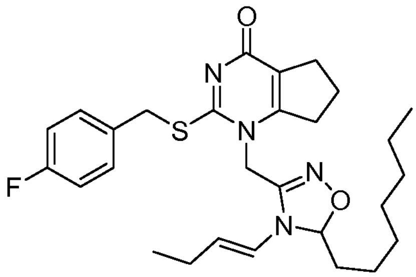 | Example 1, n-butyl aldehyde |
| **Example 18:** **(E)-2-(4-fluorobenzylthio)-1-((5-n-heptyl-4-(n-hex-1-enyl)-4,5-dihydro-1,2,4-oxadiazol-3-yl)methyl)-6,7-dihydro-1H-cyclopenta[d]py rimidin-4(5H)-one** | 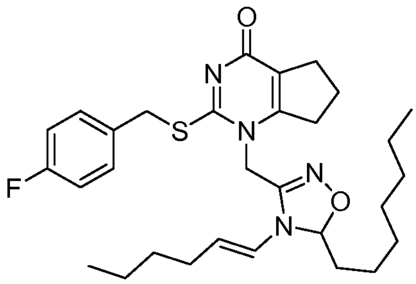 | Example 1, n-hexanal |

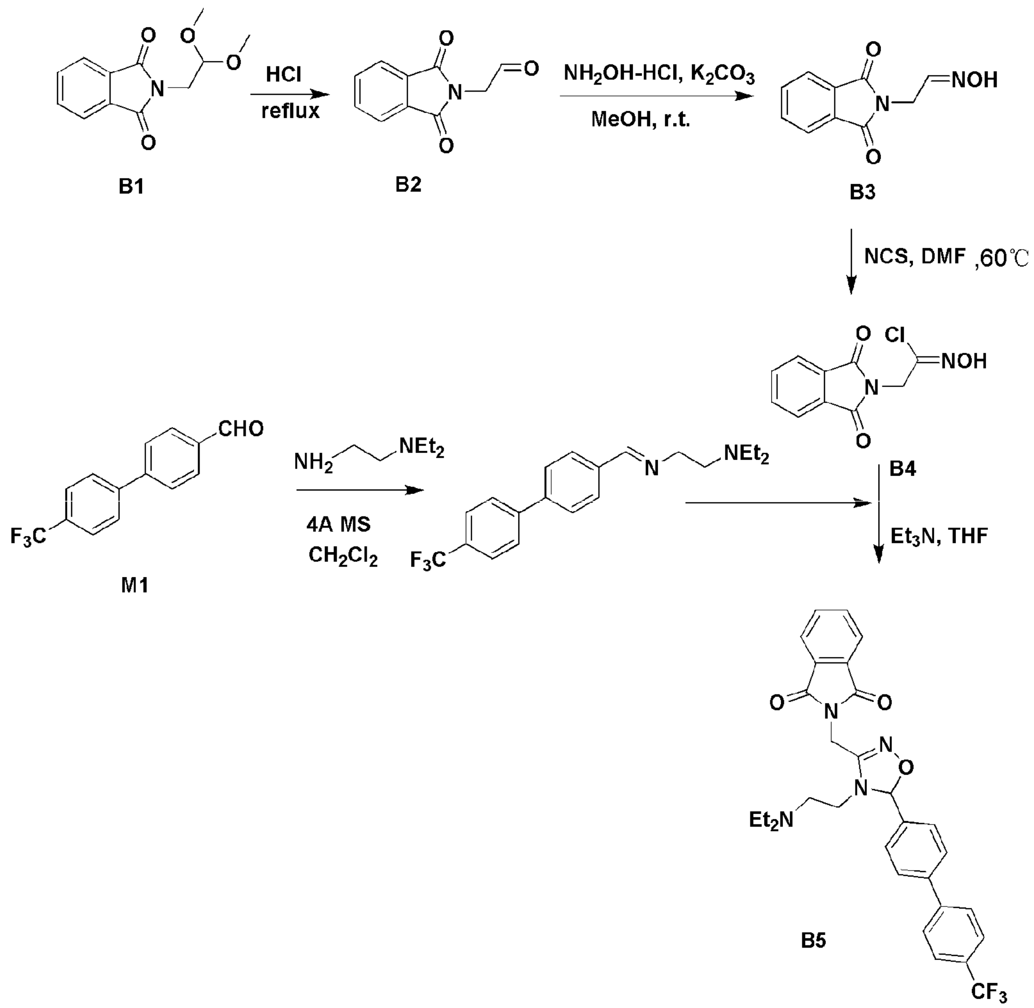


B1 B2 B3 B4 M1 B5

**Intermediate B1-2-(2,2-dimethoxyethyl)isoindoline-1,3-dione**

Refer to [Eur. J. Org. Chem., 2008, 895-913.]

**Intermediate B2-2-(1,3-dioxoisoindolin-2-yl)acetaldehyde**

To a suspension of intermediate B1 (4.18g) in water (20ml) was added concd.HCl (4ml). The mixture was refluxed for 1h. TLC detection showed that the reaction was complete. The mixture was extracted with dichloromethane threee times. The combined organic phase was washed with brine twice, saturated aq.$NaHCO_3$ solution once, dried over $MgSO_4$, decolored with active carbon and purified by column chromatography (PE/EA=2:1) to give the title compound as a white solid (2.93g). $^1$H-NMR ($CDCl_3$, 300 MHz) δ4.55 (s, 2H), 7.75 (m, 2H), 7.88 (m, 2H), 9.65 (s, 1H).

**Intermediate B3-2-(1,3-dioxoisoindolin-2-yl)acetaldehyde oxime**

A mixture of intermediate B2 (1.46g, 1equiv), hydroxylamine hydrochloride (0.64g, 1.2equiv), anhydrous $K_2CO_3$ (1.6g, 1.5equiv) in absolute methanol (10ml) was stirred at room temperature overnight. The solvent was evaporated under reduced pressure and the residue was diluted with water, extracted with dichloromethane three times. The organic phase was combined and washed with brine, dried and solvent evaporated to give the title compound as a white solid (0.75g). $^1$H-NMR ($d_6$-DMSO, 400 MHz, ca 1:1 cis-trans isomer) δ4.30/4.37 (2x d, 2H, *J*=4.0), 6.81/7.35 (2x t, 1H, *J*=4.0), 7.81-7.88 (m, 4H), 10.86/11.34 (2x s, 1H); MS (EI) *m*/*z*: 204 ($M^+$).

**Intermediate B4-2-(1,3-dioxoisoindolin-2-yl)-N-hydroxyacetimidoyl chloride**

A mixture of intermediate B3 (0.43g, 1equiv) and NCS (0.28g, 1equiv) in DMF (5ml) was heated at 60 °C for 2h. After cooled, the mixture was diluted with EA (20ml), washed with brine five times to wash out DMF, dried and

evaporated to dryness to give the title compound as white powder (0.42g). $^1H$-NMR ($d_6$-DMSO, 300 MHz) δ4.60 (s, 2H), 7.89-7.97 (m, 4H), 12.02 (s, 1H).

**Intermediate B5-2-((4-(2-(diethylamino)ethyl)-5-(4'-(trifluoromethyl)biphenyl-4-yl)-4,5-dihydro-1, 2,4-oxadiazol-3-yl)methyl)isoindoline-1,3-dione**

4Å molecular sieves (3g) was added to a mixture of intermediate M1 (10mmol), 2-diethylaminoethylamine (10mmol) in dichloromethane (10ml). The mixture was heated under reflux for 2h, using $CaCl_2$ drying tube to insulate moisture, to afford the intermediate imine solution. Heating was stopped and the mixture was cooled for use. To an ice-salt cold solution of intermediate B4 (10mmol) in anhydrous THF (15ml) was added triethylamine (13mmol) dropwise over 5 min under nitrogen and vigorous stirring. After the addition, the mixture was further stirred for 15min. Then, the solution of intermediate imine in DCM was added rapidly and the mixture was stirred at room temperature for 2h. Then the reaction mixture was poured into saturated aq.$NH_4Cl$ solution and extracted with dichloromethane twice. The combined organic phase was washed with brine twice, dried and then purified by column chromatography to give the title compound as a jelly. $^1H$-NMR ($CDCl_3$, 400 MHz) δ1.02 (t, 6H, *J*=7.2), 2.44-2.53 (m, 6H), 3.14 (m, 1H), 3.25 (m, 1H), 4.75 (2x d, 2H, *J*=16.0), 6.38 (s, 1H), 7.63 (2x d, 4H, *J*=8.4), 7.71 (s, 4H), 7.77 (m, 2H), 7.93 (m, 2H); MS (ESI): 551(M+H).

**Intermediate B6-2-((4-(2-morpholinoethyl)-5-(4'-(trifluoromethyl)biphenyl-4-yl)-4,5-dihydro-1,2,4 -oxadiazol-3-yl)methyl)isoindoline-1,3-dione**

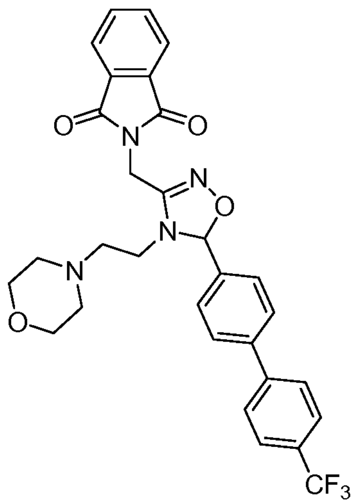

Following a procedure similar to that described for the preparation of intermediate B5 except that 2-morpholinoethanamine was used in place of 2-diethylaminoethylamine. $^1H$-NMR ($CDCl_3$, 300MHz) δ 2.40 (m, 6H), 3.18 (dt, 1H, *J*=15.0, 6.0), 3.29 (dt, 1H, *J*=15.0, 6.3), 3.68 (t, 4H, *J*=4.7), 4.72 (2x d, 2H, *J*=15.9), 6.35 (s, 1H), 7.56 (d, 2H, *J*=8.4), 7.62 (d, 2H, *J*=8.4), 7.68 (m, 4H), 7.75 (m, 2H), 7.90 (m, 2H).

The following intermediates were prepared by a procedure similar to that described for intermediate B5.

| Name | Structure | Starting material |
|---|---|---|
| **Intermediate B7:**<br>**ethyl 4-(3-((1,3-dioxoisoindolin-2-yl)methyl)-5-(4'-(trifluoromethyl)biphenyl-4-yl)-1,2,4-oxadiazol-4(5H)-yl)piperidine-1-carboxylate** | 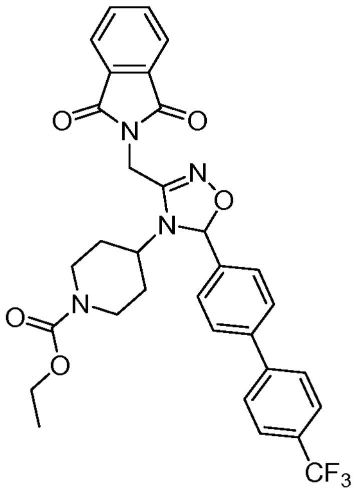 | B4, M1,<br>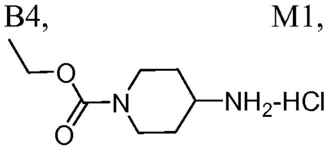<br>, $NEt_3$ |
| **Intermediate B8:**<br>**2-((4-(2-(piperidin-1-yl)ethyl)-5-(4'-(trifluoromethyl)biphenyl-4-yl)-4,5-dihydro-1,2,4-oxadiazol-3-yl)methyl)isoindoline-1,3-dione** | 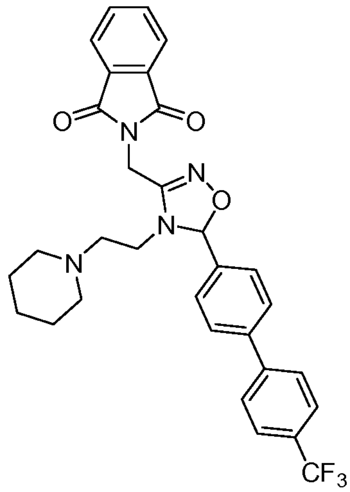 | B4, M1,<br>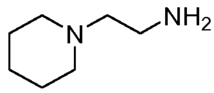 |

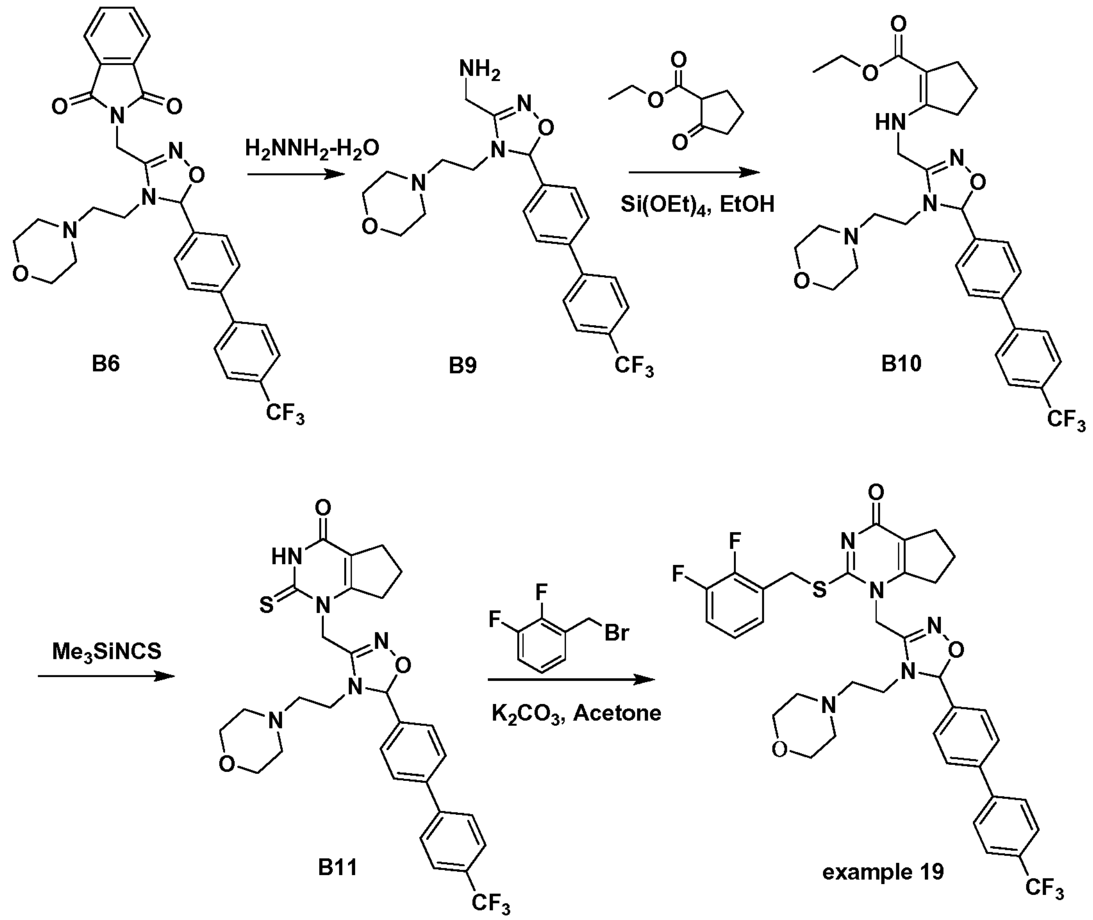


B6 B9 B10

B11 example 19

**Intermediate B10-ethyl 2-((4-(2-morpholinoethyl)-5-(4'-(trifluoromethyl)biphenyl-4-yl)-4,5-dihydro-1,2,4 -oxadiazol-3-yl)methylamino)cyclopent-1-enecarboxylate**

To a solution of intermediate B6 (2.57g, 1equiv) in THF(15ml) was added hydrazine hydrate (85%, 0.78ml, 3equiv) and the mixture was stirred at 50°C for 5h. TLC detection showed that the reaction was complete. Then the mixture was cooled and filtered to remove the insoluble white solid. The filtrate was evaporated to dryness unfrt reduced pressure. Then toluene was added to remove the trace amount of water and this procedure was repeated twice to give intermediate B9 (1.82g) as an oil.

A mixture of intermediate B9 (1.82g, 1equiv), ethyl 2-oxocyclopentanecarboxylate (0.65ml, 1.05equiv), $Si(OEt)_4$ (1.86ml), 2equiv) in absolute ethanol was refluxed for 4h under nitrogen until the reaction was complete. The solution was mixed with an amount of silica gel, evaporated to dryness and purified by column chromatography to give the title compound as a jelly (1.37g). $^1$H-NMR ($CDCl_3$, 300MHz) $\delta$ 1.26 (t, 3H, *J*= 7.2), 1.87 (m, 2H), 2.34 (m, 6H), 2.54 (t, 2H, *J*= 7.2), 2.72 (t, 2H, *J*= 7.2), 3.20 (m, 2H), 3.63 (t, 4H, *J*= 4.5), 4.13 (q, 2H, *J*= 7.2), 4.18 (d, 2H, *J*= 6.6), 6.37 (s, 1H), 7.54 (d, 2H, *J*= 8.4), 7.63 (d, 2H, *J*= 8.4), 7.70 (m, 5H).

**Intermediate B11-1-((4-(2-morpholinoethyl)-5-(4'-(trifluoromethyl)biphenyl-4-yl)-4,5-dihydro-1,2,4 -oxadiazol-3-yl)methyl)-2-thioxo-2,3,6,7-tetrahydro-1H-cyclopenta[d]pyrimidin-4 (5H)-one**

A mixture of intermediate B10 (1.36g, 1equiv), $Me_3SiNCS$ (1.33ml, 4equiv) and anhydrous DMF (2ml) was heated at 140 °C for 4h under nitrogen. TLC detection showed that the reaction was complete. Then, the solution was cooled in an ice bath, quenched with saturated aq. $NaHCO_3$ solution and then diluted with EA (20ml). The mixture was stirred for 0.5h before transferred into a separating funnel. The organic layer was separated, washed with brine twice, dried over $MgSO_4$, decolored with active carbon, and then the solution was concentrated to a small amount and then silica gel was added and purified by column chromatography to collect the target product and then recrystallized from PE-EA to give the title compound as a white solid (100mg). $^1$H-NMR ($d_6$-DMSO, 400MHz) $\delta$ 2.05 (m, 2H), 2.32 (m, 6H), 2.62 (t, 2H, *J*=7.6), 3.04 (m, 3H), 3.35 (m, 1H), 3.56 (m, 4H), 5.38 (s, 2H), 6.44 (s, 1H), 7.64 (d, 2H, *J*= 8.4), 7.83 (d, 2H, *J*= 8.4), 7.93 (d, 2H, *J*= 8.4), 12.65 (s, 1H); MS (ESI): 586(M+H).

**Example 19-2-(2,3-difluorobenzylthio)-1-((4-(2-morpholinoethyl)-5-(4'-(trifluoromethyl)biphe nyl-4-yl)-4,5-dihydro-1,2,4-oxadiazol-3-yl)methyl)-6,7-dihydro-1H-cyclopenta[d] pyrimidin-4(5H)-one**

A mixture of intermediate B11 (30mg, 1equiv), 1-(bromomethyl)-2,3-difluorobenzene (7.2μl, 1.1equiv), anhydrous $K_2CO_3$ (11mg, 1.5equiv) in acetone (2ml) was heated to reflux for 0.5h. Then the mixture was filtered to remove the inorganic salts and the filtrate was purified by preparative TLC (DCM/MeOH=15:1) and the target product portion was collected to give the title compound as a solid (20mg). $^1$H-NMR ($CDCl_3$, 300MHz) δ 2.13 (m, 2H), 2.38 (m, 6H), 2.83 (t, 2H, *J*=7.2), 3.07 (m, 4H), 3.64 (m, 4H), 4.65 (2x d, 2H, *J*=12.9), 4.84 (s, 2H), 6.37 (s, 1H), 7.05 (m, 2H), 7.38 (m, 1H), 7.52 (d, 2H, *J*=6.9), 7.63 (d, 2H, *J*=7.5), 7.70 (4H, m); MS (ESI): 712 (M+H).

**Example 20-2-(4-fluorobenzylthio)-1-((4-(2-(piperidin-1-yl)ethyl)-5-(4'-(trifluoromethyl)biphe nyl-4-yl)-4,5-dihydro-1,2,4-oxadiazol-3-yl)methyl)-6,7-dihydro-1H-cyclopenta[d] pyrimidin-4(5H)-one**

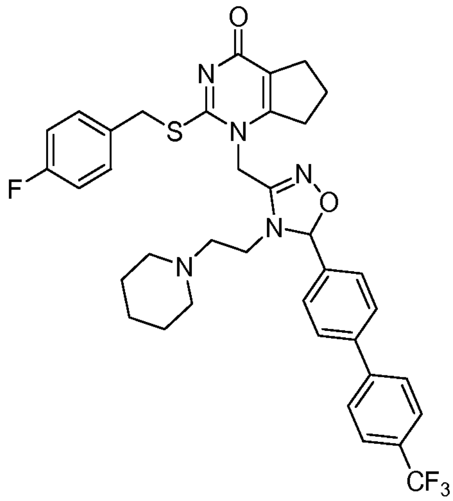

Following a procedure similar to that described for the preparation of example 19 except that intermediate B8 and 1-(bromomethyl)-4-fluorobenzene was used in place of intermediate B6 and 1-(bromomethyl)-2,3-difluorobenzene. $^1$H-NMR ($CDCl_3$, 300MHz) δ 1.45 (m, 2H), 1.62 (m, 4H), 2.12 (m, 2H), 2.47 (m, 6H), 2.83 (t, 2H, *J*=6.9), 3.03 (t, 2H, *J*=7.2), 3.30 (m, 2H), 4.55 (2x d, 2H, *J*=12.9), 5.00 (2x d, 2H, *J*=18.0), 6.31 (s, 1H), 6.99 (d, 2H, *J*=8.7), 7.40 (dd, 2H, *J*=7.2, 5.7), 7.52 (d, 2H, *J*=8.1), 7.59 (d, 2H, *J*=8.1), 7.66 (d, 2H, *J*=8.4), 7.72 (d, 2H, *J*=8.4); MS (ESI): 692(M+H).

**Example 21-ethyl 4-(3-((2-(2,3-difluorobenzylthio)-4-oxo-4,5,6,7-tetrahydro-1H-cyclopenta[d]pyrim idin-1-yl)methyl)-5-(4'-(trifluoromethyl)biphenyl-4-yl)-1,2,4-oxadiazol-4(5H)-yl)p iperidine-1-carboxylate**

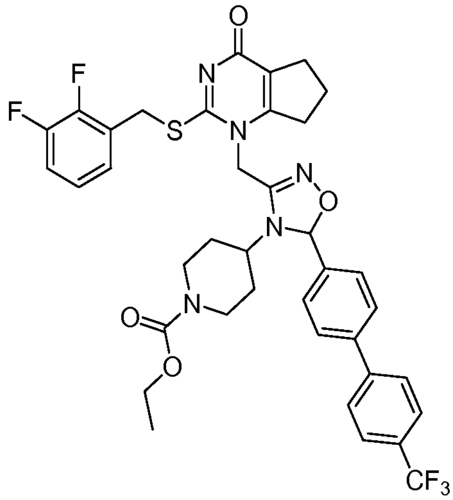

Following a procedure similar to that described for the preparation of example 19 except that intermediate B7 and 1-(bromomethyl)-4-fluorobenzene was used in place of intermediate B6 and 1-(bromomethyl)-2,3-difluorobenzene. $^1$H-NMR ($CDCl_3$, 300MHz) δ 1.16 (m, 2H), 1.23 (t, 3H, *J*=6.9), 1.57 (m, 1H), 1.84 (m, 1H), 2.09 (m, 2H), 2.53 (t, 1H, *J*=13.5), 2.70 (t, 1H, *J*=12.6), 2.80 (t, 2H, *J*=6.9), 2.96 (m, 2H), 3.41 (t, 1H, *J*=12.3), 4.10 (m, 3H), 4.30 (m, 1H), 4.65 (2x d, 2H, *J*=13.8), 4.80 (2x d, 2H *J*=17.4), 6.39 (s, 1H), 7.05 (m, 2H), 7.38 (t, 1H, *J*=6.3), 7.47 (d, 2H, *J*=8.1), 7.59 (d, 2H, *J*=8.1), 7.69 (4H, m); MS (ESI): 754(M+H)$^+$.

**Example 22-ethyl 4-(3-((2-(4-fluorobenzylthio)-4-oxo-4,5,6,7-tetrahydro-1H-cyclopenta[d]pyrimidi n-1-yl)methyl)-5-(4'-(trifluoromethyl)biphenyl-4-yl)-1,2,4-oxadiazol-4(5H)-yl)pip eridine-1-carboxylate**

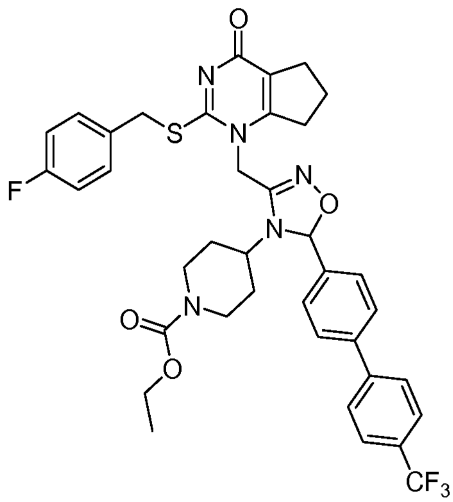

Following a procedure similar to that described for the preparation of example 19 except that intermediate B7 was used in place of intermediate B6. $^1$H-NMR ($CDCl_3$, 300MHz) δ 1.26 (m, 5H), 1.54 (m, 1H), 1.82 (m, 1H), 2.10 (m, 2H), 2.50 (t, 1H, *J*=12.0), 2.66 (t, 1H, *J*=13.2), 2.83 (t, 2H, *J*=7.2), 2.97 (m, 2H), 3.39 (t, 1H, *J*=12.3), 4.11 (m, 3H), 4.30 (m, 1H), 4.52 (d, 1H, *J*=13.5), 4.65 (d, 1H, *J*=13.5), 4.80 (2x d, 2H *J*=17.4), 6.40 (s, 1H), 7.00 (t, 2H, *J*=8.4), 7.40 (dd, 2H, *J*=8.4, 5.1), 7.47 (d, 2H, *J*=8.9), 7.57 (d, 2H, *J*=8.4), 7.67 (d, 2H, *J*=8.4), 7.71 (d, 2H, *J*=8.7); MS (ESI): 736(M+H)$^+$.

**Example 23-2-(2,3-difluorobenzylthio)-1-((4-(2-(piperidin-1-yl)ethyl)-5-(4'-(trifluoromethyl)bi phenyl-4-yl)-4,5-dihydro-1,2,4-oxadiazol-3-yl)methyl)-6,7-dihydro-1H-cyclopenta [d]pyrimidin-4(5H)-one**

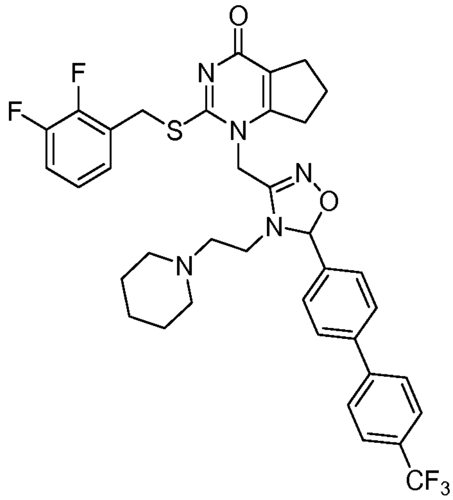

Following a procedure similar to that described for the preparation of example 19 except that intermediate B8 and 1-(bromomethyl)-4-fluorobenzene was used in place of intermediate B6 and 1-(bromomethyl)-2,3-difluorobenzene. $^1$H-NMR ($CDCl_3$, 300MHz) $\delta$ 1.46 (m, 2H), 1.61 (m, 4H), 2.13 (m, 2H), 2.46 (m, 6H), 2.84 (t, 2H), 3.01 (t, 2H), 3.25 (m, 2H), 4.63 (2x d, 2H), 4.94 (2x d, 2H), 6.32 (s, 1H), 7.04 (m, 2H), 7.38 (m, 1H), 7.55 (d, 2H), 7.63 (d, 2H), 7.71 (4H, m); MS (ESI): 710$(M+H)^+$.

The following compounds were prepared by a procedure similar to that described for example 19 from B6.

| Name | Structure | Precusor |
|---|---|---|
| **Example 24: 1-((4-(2-(diethylamino)ethyl)-5-(4'-(trifluoromethyl)biphenyl-4-yl)-4,5-dihydro-1,2,4-oxadiazol-3-yl)methyl)-2-(4-fluorobenzylthio)-6,7-dihydro-1H-cyclopenta[d]pyrimidin-4 (5H)-one** | 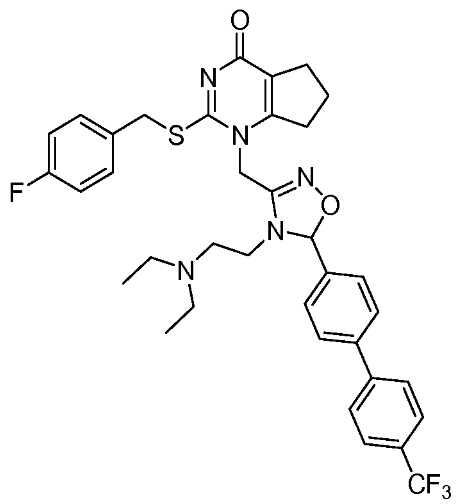 | B5, 1-(bromomethyl)-4-fluorobenzene |

(continued)

| Name | Structure | Precusor |
|---|---|---|
| **Example 25: 2-(4-fluorobenzylthio)-1-((4-(2-morpholinoethyl)-5-(4'-(trifluoromethyl)biphenyl-4-yl)-4,5-dihydro-1,2,4-oxadiazol-3-yl)met hyl)-6,7-dihydro-1H-cyclopenta[d]pyrimidin-4(5H)-one** | 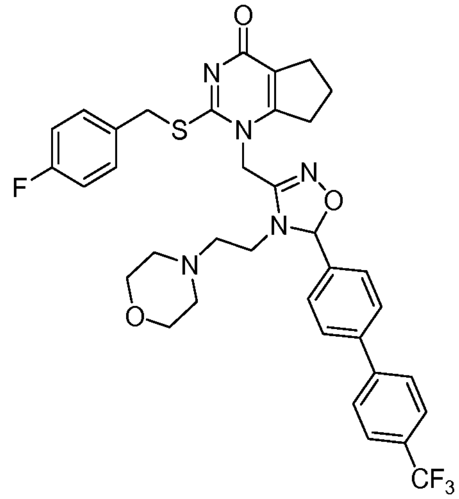 | B6, 1-(bromomethyl)-4-fluorobenzene |

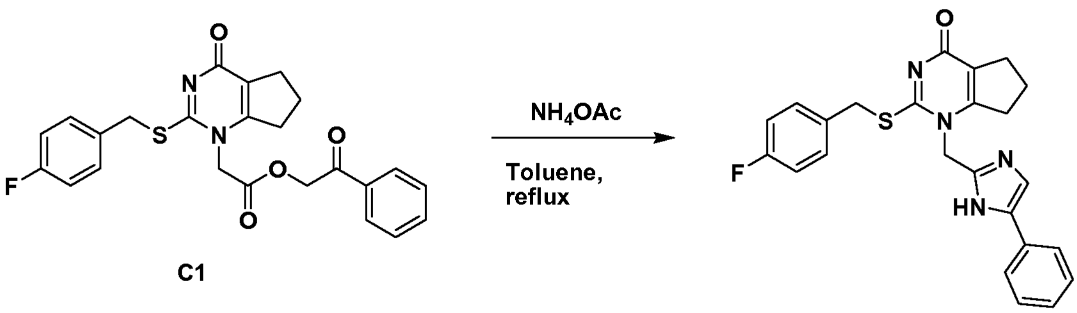


C1

**Intermediate C1-2-oxo-2-phenylethyl 2-(2-(4-fluorobenzylthio)-4-oxo-4,5,6,7-tetrahydro-1H-cyclopenta[d]pyrimidin-1-yl)acetate**

A mixture of intermediate A4 (1.67g, 1equiv), 2-bromo-1-phenylethanone (1g, 1equiv), anhydrous $K_2CO_3$ (1.38g, 2equiv) in acetone (20ml) was stirred at room temperature for 6h. TLC detection showed that the reaction was complete. Solvent was evaporated under recuded pressure and the residue was diluted with water and EA. The mixture was stirred for 10min and the solid so obtained was collected by filtration, washed with water and EA, and then dried to give the title compound as a white solid (1.14g). Then organic layer of the filtrate was separated and evaporated to remove most of the solvent to give a further batch of the title compound (0.75g). 1H-NMR ($d_6$-DMSO, 300 MHz) $\delta$1.99 (m, 2H), 2.60 (t, 2H, J=7.3), 2.95 (t, 2H, J=7.5), 4.45 (s, 2H), 5.02 (s, 2H), 5.66 (s, 2H), 7.15 (t, 2H, J=8.8), 7.49 (dd, 2H, J=8.8 ,5.6), 7.56 (t, 2H, J=7.3), 7.69 (t, 1H, J=7.2), 7.95 (d, 1H, J=6.6); MS (ESI): 453(M+H).

**Example 26-2-(4-fluorobenzylthio)-1-((5-phenyl-1H-imidazol-2-yl)methyl)-6,7-dihydro-1H-cy clopenta[d]pyrimidin-4(5H)-one**

A mixture of intermediate C1 (665mg, 1.47mmol) and ammonium acetate (560mg, 5equiv) in toluene was refluxed under nitrogen in a Dean-Stark trap for 1.5h. Toluene was evaporated and the residue was dissolved in methanol, mixed with silica gel, concentrated to dryness and purified by column chromatography. The column was first eluted with DCM/MeOH (30:1) to recycle material and then eluted with DCM/MeOH (15:1) to obtain the title compound (67mg). $^1$H-NMR ($d_6$-DMSO, 300 MHz) $\delta$2.00 (m, 2H), 2.61 (t, 2H, *J*=7.5), 3.02 (t, 2H, *J*=6.9), 4.41 (s, 2H), 5.17 (s, 2H), 7.11 (t, 2H, *J*=9.0), 7.34 (m, 3H), 7.45 (dd, 2H, *J*=8.6, 5.6), 7.68 (d, 2H, *J*=6.0); MS (ESI): 433(M+H).

**Example 27-1-((1H-tetrazol-5-yl)methyl)-2-(4-fluorobenzylthio)-6,7-dihydro-1H-cyclopenta[d ]pyrimidin-4(5H)-one**

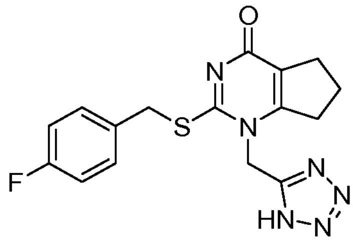

A mixture of intermediate A6 (95mg, 1equiv), sodium azide (21mg, 1.06equiv) and ammonium chloride (18mg, 1.1equiv) in DMF (2ml) was heated at 90-100°C for 2h. The mixture was diluted with water, acidified to pH 3~4 with concd. HCl and extracted with EA three times. The combined organic phase was washed with brine to neutral, dried over $MgSO_4$ and solvent evaporated. The residue was recrystallized from MeOH-EA to give the title compound (11mg) as a solid. $^1$H-NMR ($d_6$-DMSO, 300 MHz) δ1.97 (m, 2H), 2.58 (t, 2H, *J*=7.4), 2.91 (t, 2H, *J*=7.6), 4.36 (s, 2H), 5.46 (s, 2H), 7.10 (t, 2H, *J*=9.0), 7.40 (dd, 2H, *J*=8.8, 5.6); MS (ESI): 359(M+H).

**Intermediate C2-1H-imidazole-2-carbaldehyde**

Refer to [J. Het. Chem., 32, 611 (1995). ]

**Intermediate C3-1-((4'-(trifluoromethyl)biphenyl-4-yl)methyl)-1H-imidazole-2-carbaldehyde**

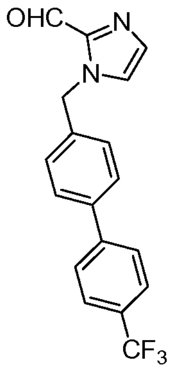

A mixture of intermediate M11 (1.26g, 1equiv), intermediate C2 (396mg, 1equiv) and anhydrous $K_2CO_3$ (830mg, 1.5equiv) in acetonitrile (10ml) was refluxed for 1h. TLC detection showed that the reaction was complete. The mixture was filtered to remove the insoluble substances and washed with EA. The filtrate was concentrated and then purified by column chromatography (PE/EA=2:1) to give the title compound as a white solid (1.14g). $^1$H-NMR ($CDCl_3$, 300 MHz) δ5.67 (s, 2H), 7.20 (s, 1H), 7.30 (d, 2H, *J*=8.4), 7.33 (s, 1H), 7.57 (d, 2H, *J*=8.4), 7.67 (q, 4H), 9.86 (s, 1H); MS (ESI): 331(M+H).

The following intermediates were prepared by a procedure similar to that described for intermediate C3.

| Name | Strcuture | Precusor |
|---|---|---|
| **Intermediate C4: 1-n-dodecyl-1H-imidazole-2-carbaldehyde** | 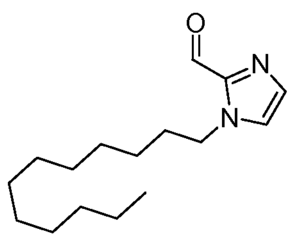 | C2, bromododecane |

(continued)

| Name | Strcuture | Precusor |
|---|---|---|
| **Intermediate C5: 1-n-butyl-1H-imidazole-2-carbaldehyde** | 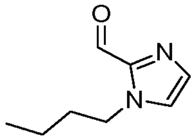 | C2, 1-bromobutane |
| **Indtermediate C6: 1-(biphenyl-4-ylmethyl)-1H-imidazole-2-carbaldehyde** | 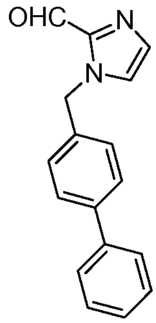 | C2, M4 |
| **Intermediate C7: 1-((4'-methylbiphenyl-4-yl)methyl)-1H-imidazole-2-carbaldehyde** | 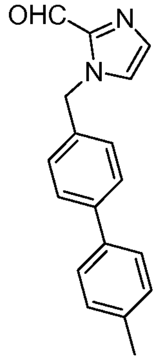 | C2, M5 |
| **Intermediate C8: 1-((4'-chlorobiphenyl-4-yl)methyl)-1H-imidazole-2-carbaldehyde** | 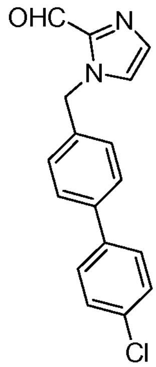 | C2, M6 |

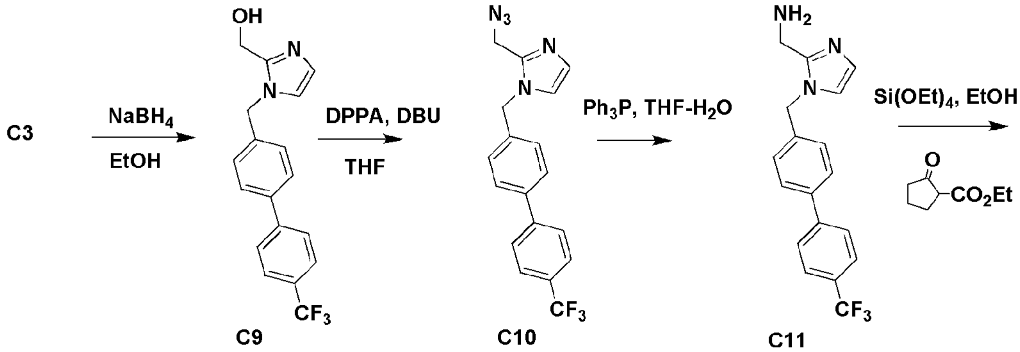


C9 C10 C11

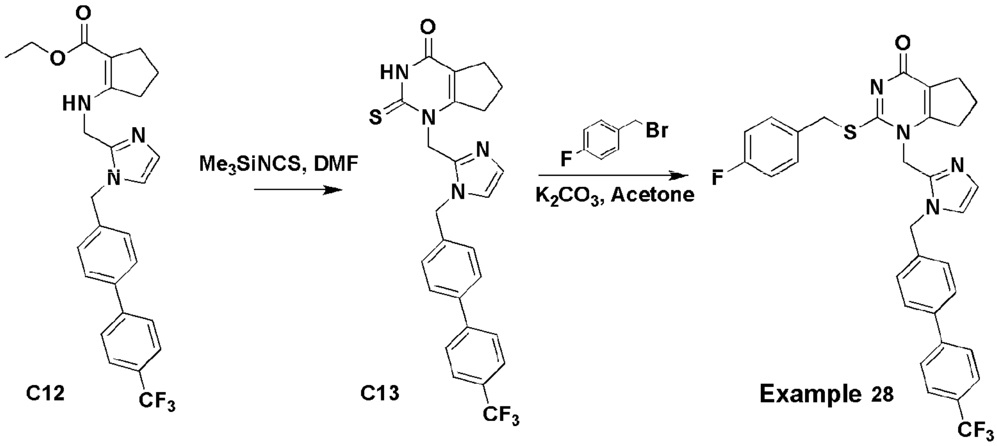


C12 C13 Example 28

**Intermediate C9-(1-((4'-(trifluoromethyl)biphenyl-4-yl)methyl)-1H-imidazol-2-yl)methanol**

Following a procedure similar to that described for the preparation of intermediate M10 except that intermediate C3 was used in place of intermediate M1. $^1$H-NMR ($CDCl_3$, 300 MHz) δ4.68 (s, 2H), 5.29 (s, 2H), 6.88 (s, 1H), 6.97 (s, 1H), 7.25 (d, 2H, *J*=8.1), 7.57 (d, 2H, *J*=8.1), 7.65 (d, 2H, *J*=9.0), 7.69 (d, 2H, *J*=9.0); MS (ESI): 333(M+H).

**Intermediate C10-2-(azidomethyl)-1-((4'-(trifluoromethyl)biphenyl-4-yl)methyl)-1H-imidazole**

A mixture of intermediate C9 (1.05g, 1equiv), DPPA (0.89ml, 1.3equiv) and DBU (0.66ml, 1.4equiv) in THF (10ml) was refluxed for 3h under nitrogen. TLC detection showed that the reaction was complete. After cooled, the solution was poured into saturated $NH_4Cl$ solution and extracted with EA. The organic phase was washed with brine, dried and then purified by column chromatography (PE/EA=1:2) to give the title compound as a white solid (1.10g). $^1$H-NMR ($CDCl_3$, 300 MHz) δ4.41 (s, 2H), 5.23 (s, 2H), 6.97 (d, 1H, *J*=1.5), 7.08 (d, 1H, *J*=0.9), 7.20 (d, 2H, *J*=8.4), 7.59 (d, 2H, *J*=8.4), -7.68 (m, 4H).

**Intermediate C11-(1-((4'-(trifluoromethyl)biphenyl-4-yl)methyl)-1H-imidazol-2-yl)methanamine**

To a solution of intermediate C10 (0.50g, 1equiv) in THF-$H_2O$ (5-0.5ml) was added $Ph_3P$ (550mg, 1.5equiv) and the mixture was stirred at room temperature for 4h. Then the solution was mixed with silica gel, evaporated to dryness and loaded on a short silica column. The column was first eluted with EA to get rid of $Ph_3P$ and $Ph_3P$=O and then eluted with methanol to afford the target product portion. The methanol solution so collected was evaporated to dryness and the residue was added a mixed solvent of DCM/MeOH (15:1). Insoluble silica gel was removed by filtration and the filtrate was evaporated to dryness to give the title compound as a yellow solid (0.45g), which can be used directly in the next reaction.

**Intermediate C12-ethyl 2-((1-((4'-(trifluoromethyl)biphenyl-4-yl)methyl)-1H-imidazol-2-yl)methylamino) cyclopent-1-enecarboxylate**

To a solution of intermediate C11 (0.45g, 1equiv) in absolute ethanol (5ml) were added ethyl 2-oxocyclopentanecarboxylate (210μl, 1.05equiv) and tetraethyl orthosilicate (610μl, 2equiv). The mixture was refluxed under nitrogen

for 3h until the completion of the reaction monitored by TLC. Silica gel was added to the solution and the mixture was evaporated to dryness, and then purified by column chromatography (PE/EA=1:3) to give the title compound as a jelly (0.56g). $^1$H-NMR ($CDCl_3$, 300 MHz) δ1.22 (t, 3H, *J*=7.1) 1.77 (m, 2H), 2.48 (m, 4H), 4.08 (q, 2H, *J*=7.2), 4.37 (d, 2H, *J*=5.7), 5.21 (s, 2H), 6.93 (d, 1H, *J*=1.2), 7.03 (d, 1H, J=0.9), 7.15 (d, 2H, *J*=8.4), 7.55 (d, 2H, *J*=8.1), 7.67 (m, 5H); MS (ESI) found (M+H) = 470.

**Intermediate C13-2-thioxo-1-((1-((4'-(trifluoromethyl)biphenyl-4-yl)methyl)-1H-imidazol-2-yl)meth yl)-2,3,6,7-tetrahydro-1H-cyclopenta[d]pyrimidin-4(5H)-one**

A mixture of intermediate C12 (0.5 g, 1equiv), isothiocyanatotrimethylsilane (620μl, 3.5equiv) in anhydrous DMF (1ml) was heated at 140°C for 4h under nitrogen. Then, the solution was cooled in an ice bath, quenched with saturated $NaHCO_3$ solution, and stirred with EA (15ml) and water (10ml) for 10 minutes. The organic layer was separated and the aqueous layer was extracted with 10 ml of EA again. The combined organic phase was washed with brine twice, dried over anhydrous $MgSO_4$, decolored with active carbon and filtered to remove the insoluble substances one hour later. The filtrate was concentrated to about 3ml and stayed overnight after cooled. The white solid so obtained was collected by filtration and dried to give the title compound (100mg). If no solid precipitated out from EA, the crude product could be purified by column chromatography. $^1$H-NMR ($d_6$-DMSO, 300 MHz) δ1.93 (m, 2H), 2.52 (t, 2H), 2.88 (t, 2H, *J*=7.6), 5.43 (s, 2H), 5.52 (s, 2H), 6.90 (d, 1H, *J*=0.6), 7.27 (d, 3H), 7.71 (d, 2H, *J*=8.4), 7.81 (d, 2H, *J*=8.4), 7.87 (d, 2H, *J*=8.1), 12.50 (s, 1H); MS (ESI) found (M+H) = 483.

**Example 28-2-(4-fluorobenzylthio)-1-((1-((4'-(trifluoromethyl)biphenyl-4-yl)methyl)-1H-imid azol-2-yl)methyl)-6,7-dihydro-1H-cyclopenta[d]pyrimidin-4(5H)-one**

A mixture of intermediate C13 (30mg, 1equiv), 1-(bromomethyl)-4-fluorobenzene (9μl, 1.1equiv) and anhydrous $K_2CO_3$ (13mg, 1.5equiv) in acetone (2ml) was refluxed for 0.5h. TLC detection showed that the reaction was complete. The inorganic salt was removed by filtration and the filtrate was purified by column chromatography or preparative TLC (spread with DCM/MeOH=20:1) to collect the target product portion, obtaining the title compound as a solid (20mg). $^1$H-NMR ($CDCl_3$, 300 MHz) δ1.97 (m, 2H), 2.68 (m, 4H), 4.47 (s, 2H), 5.01 (s, 2H), 5.19 (s, 2H), 6.90-7.03 (m, 5H), 7.10 (s, 1H), 7.33 (t, 2H), 7.48 (d, 2H, *J*=7.8), 7.63 (d, 2H, *J*=7.5), 7.48 (d, 2H, *J*=8.1); MS (ESI): 591(M+H).

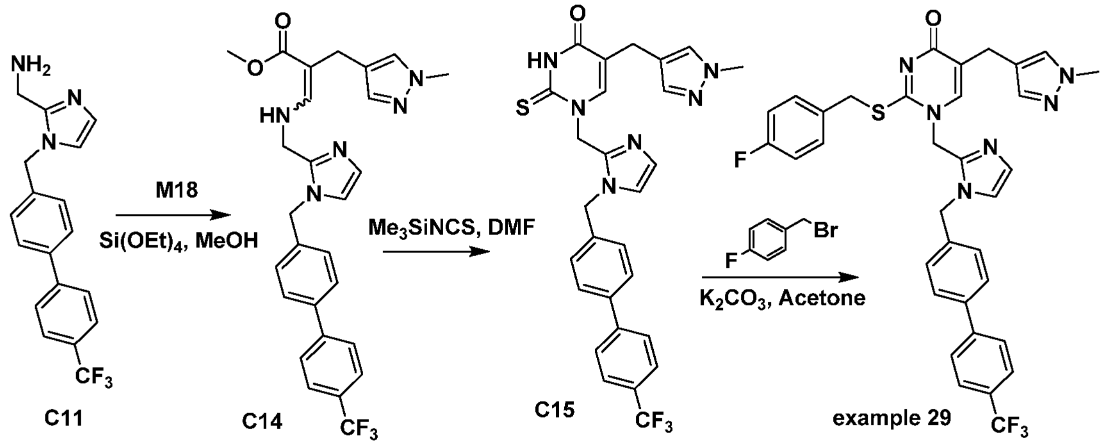


C11 C14 C15 example 29

**Intermediate C14-methyl 2-((1-methyl-1H-pyrazol-4-yl)methyl)-3-((1-((4'-(trifluoromethyl)biphenyl-4-yl)m ethyl)-1H-imidazol-2-yl)methylamino)acrylate**

To a solution of intermediate C11 (0.23 g, 1equiv) in anhydrous methanol (3ml) were added intermediate M18 (200mg, 1.43equiv) and tetraethyl orthosilicate (445μl, 2equiv) and the mixture was refluxed under nitrogen for 3h. TLC detection showed that the reaction was complete. The solution was mixed with silica gel, evaporated to dryness and purified by column chromatography (eluted with DCM/MeOH=30:1) to give the title compound as a jelly (0.35g). $^1$H-NMR ($CDCl_3$, 300 MHz, ca 2.3:1 *cis-trans* isomer) δ3.18/3.24 (2x s, 2H), 3.62/3.64 (2x s, 3H), 3.80/3.77 (2x s, 3H), 4.33/4.34 (2x d, 2H, *J*=5.4), 5.17/5.13 (2x s, 2H), 6.56/7.32 (2x d, 1H, *J*=12.9), 6.95/6.97 (2x d, 1H, *J*=1.2), 7.06-7.13 (m, 4H), 7.22/7.23 (2x s, 1H), 7.55/7.57 (2x d, 2H, *J*=8.1), 7.64 (m, 4H), 7.84 (m, 1H).

**Intermediate C15-5-((1-methyl-1H-pyrazol-4-yl)methyl)-2-thioxo-1-((1-((4'-(trifluoromethyl)biphen yl-4-yl)methyl)-1H-imidazol-2-yl)methyl)-2,3-dihydropyrimidin-4(1H)-one**

A mixture of intermediate C14 (0.34g, 1equiv), isothiocyanatotrimethylsilane (340μl, 3.5equiv) in anhydrous DMF (0.6ml) was heated at 140°C for 3h under nitrogen. Then, the solution was cooled in an ice bath, quenched with saturated $NaHCO_3$ solution, diluted with EA (5ml) and water (5ml), and then stirred for a further 20min to dissolve all the product (if the product could not be dissolved, a small amount of methanol may be added) before it was stored in refrigerator. The precipitate so obtained was collected by filtration and dried to give the title compound (130mg). $^1H$-NMR ($d_6$-DMSO, 300 MHz) δ3.29 (s, 2H), 3.74 (s, 3H), 5.39 (s, 2H), 5.45 (s, 2H), 6.94 (d, 1H, *J*=0.9), 7.20 (s, 1H), 7.22 (d, 2H, *J*=8.7), 7.33 (d, 1H, *J*=0.9), 7.43 (s, 1H), 7.47 (s, 1H), 7.66 (d, 2H, *J*=8.4), 7.81 (d, 2H, *J*=8.7), 7.85 (d, 2H, *J*=8.4), 12.60 (s, 1H); MS (ESI): 537(M+H).

**Example 29-2-(4-fluorobenzylthio)-5-((1-methyl-1H-pyrazol-4-yl)methyl)-1-((1-((4'-(trifluoro m**ethyl)biphenyl-4-yl)methyl)-1H-imidazol-2-yl)methyl)pyrimidin-4(1H)-one

Following a procedure similar to that described for the preparation of example 28 except that intermediate C15 was used in place of intermediate C13. $^1H$-NMR ($CDCl_3$, 300 MHz) δ3.38 (s, 2H), 3.79 (s, 3H), 4.44 (s, 2H), 4.92 (s, 2H), 5.11 (s, 2H), 6.90-6.98 (m, 5H), 7.05 (s, 1H), 7.12 (s, 1H), 7.19 (s, 1H), 7.22 (s, 1H), -7.37 (m, 4H), 7.62 (d, 2H, *J*=8.4), 7.70 (d, 2H, *J*=8.1); MS (ESI): 645(M+H).

**Example 30-1-((1-n-dodecyl-1H-imidazol-2-yl)methyl)-2-(4-fluorobenzylthio)-6,7-dihydro-1H-cyclopenta[d]pyrimidin-4(5H)-one**

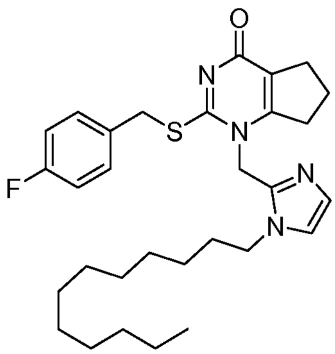

Following a procedure similar to that described for the preparation of example 28 except that intermediate C4 was used in place of intermediate C3. $^1H$-NMR ($CDCl_3$, 300 MHz) δ 0.85 (t, 3H), 1.23 (m, 18H), 1.69 (m, 2H), 2.08 (m, 2H), 2.86 (m, 4H), 3.86 (t, 2H), 4.48 (s, 2H), 5.05 (s, 2H), 6.88 (s, 1H), 6.96 (t, 2H), 6.99 (s, 1H), 7.30 (dd, 2H); MS (ESI): 525(M+H).

**Example 31-1-((1-n-butyl-1H-imidazol-2-yl)methyl)-2-(4-fluorobenzylthio)-6,7-dihydro-1H-cy clopenta[d]pyrimidin-4(5H)-one**

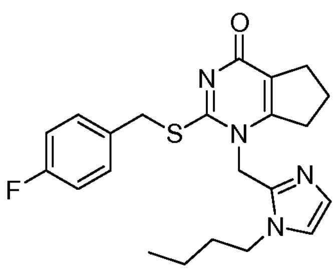

Following a procedure similar to that described for the preparation of example 28 except that intermediate C5 was used in place of intermediate C3. $^1$H-NMR ($CDCl_3$, 300 MHz) δ0.89 (t, 3H, *J*=7.5), 1.28 (m, 2H), 1.66 (m, 2H), 2.08 (m, 2H), 2.82 (t, 2H, *J*=7.5), 2.89 (t, 2H, *J*=7.8), 3.87 (t, 2H, *J*=7.5), 4.48 (s, 2H), 5.05 (s, 2H), 6.88 (s, 1H), 6.95 (t, 2H, *J*=9.0), 6.98 (s, 1H), 7.32 (dd, 2H, *J*=8.7, 5.8); MS (ESI): 413(M+H).

**Example 32-1-((1-((4'-chlorobiphenyl-4-yl)methyl)-1H-imidazol-2-yl)methyl)-2-(4-fluorobenz ylthio)-6,7-dihydro-1H-cyclopenta[d]pyrimidin-4(5H)-one**

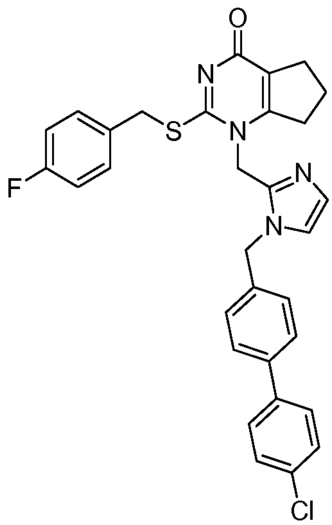

Following a procedure similar to that described for the preparation of example 28 except that intermediate C8 was used in place of intermediate C3. $^1$H-NMR ($CDCl_3$, 300 MHz) δ1.96 (m, 2H), 2.68 (m, 4H), 4.46 (s, 2H), 5.00 (s, 2H), 5.17 (s, 2H), 6.91-7.03 (m, 5H), 7.09 (s, 1H), 7.32 (dd, 2H, *J*=8.1, 5.4), 7.43 (m, 6H); MS (ESI): 557(M+H).

**Example 33-1-((1-(biphenyl-4-ylmethyl)-1H-imidazol-2-yl)methyl)-2-(4-fluorobenzylthio)-6,7-dihydro-1H-cyclopenta[d]pyrimidin-4(5H)-one**

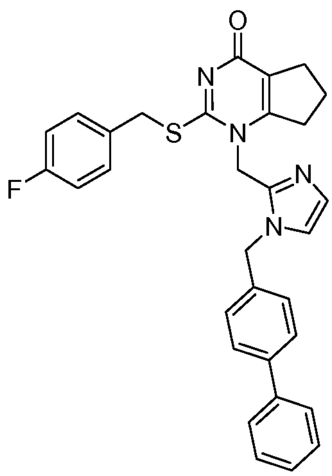

Following a procedure similar to that described for the preparation of example 28 except that intermediate C6 was used in place of intermediate C3. $^1$H-NMR ($CDCl_3$, 300 MHz) δ1.98 (m, 2H), 2.71 (m, 4H), 4.46 (s, 2H), 4.98 (s, 2H), 5.17 (s, 2H), 6.92 (t, 2H, *J*=8.4), 7.02 (d, 2H, *J*=8.1), 7.04 (s, 1H), 7.09 (s, 1H), 7.30 (dd, 2H, *J*=8.4, 5.4), 7.39 (m, 1H), 7.50 (m, 6H); MS (ESI): 523 (M+H).

**Example 34-2-(4-fluorobenzylthio)-5-((1-methyl-1H-pyrazol-4-yl)methyl)-1-((1-((4'-methylbip henyl-4-yl)methyl)-1H-imidazol-2-yl)methyl)pyrimidin-4(1H)-one**

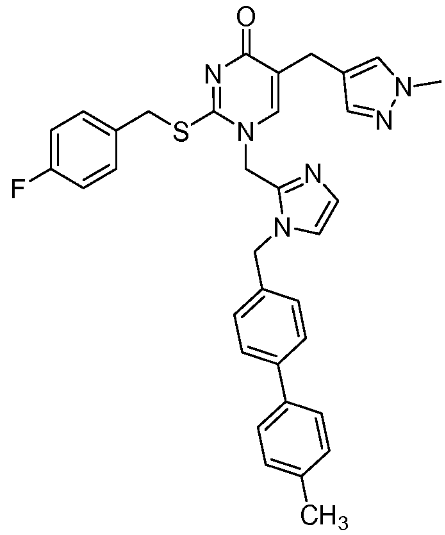

Following a procedure similar to that described for the preparation of example 29 except that intermediate C7 was used in place of intermediate C3. $^1$H-NMR ($CDCl_3$, 300 MHz) δ2.40 (s, 3H), 3.43 (s, 2H), 3.80 (s, 3H), 4.46 (s, 2H), 4.90 (s, 2H), 5.08 (s, 2H), 6.90-6.93 (m, 5H), 7.04 (s, 1H), 7.11 (s, 1H), 7.22-7.30(m, 4H), -7.34 (m, 2H), 7.43 (m, 4H); MS (ESI): 591 (M+H).

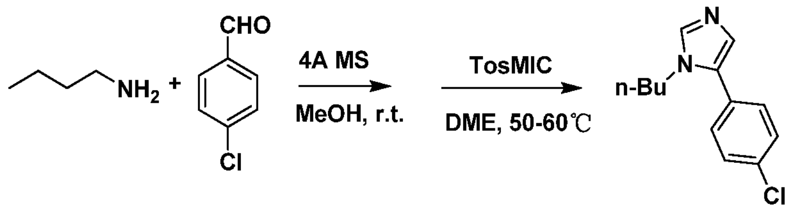


**C16**

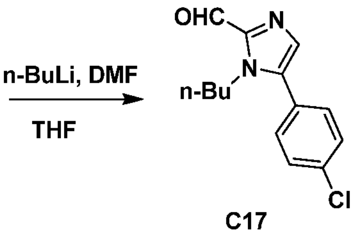


**C17**

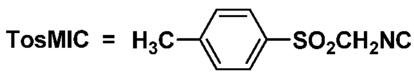

**Intermediate C16-1-n-butyl-5-(4-chlorophenyl)-1H-imidazole**

To a mixture of *n*-butylamine (2.2ml, 2.2equiv) and 4-chlorobenzaldehyde (1.4g, 1equiv) in anhydrous methanol (10ml) was added 4Å molecular sieve (5g) and the mixture was stayed for 3h with occasional shake of the flask equipped with $CaCl_2$ drying tube. Then, DME (10ml) and 1-(isocyanomethylsulfonyl)-4-methylbenzene (2.34g, 1.2equiv) were added and the mixture was heated at 50~60°C for 12h. After cooled, the mixture was filtered to remove molecular sieve and the filtrate was mixed with silica gel, and evaporated to dryness and then purified by column chromatography to give the title compound as a jelly (0.98g). $^1$H-NMR ($CDCl_3$, 300 MHz) δ0.83 (t, 3H, *J*=7.4), 1.22 (m, 2H), 1.59 (m, 2H), 3.93 (t, 2H, *J*=7.4), 7.04 (d, 1H, *J*=1.2), 7.29 (d, 2H, *J*=9.0), 7.40 (d, 2H, *J*=8.7), 7.55 (d, 1H, *J*=0.9); MS (EI) *m/z*: 234 ($M^+$).

**Intermediate C17-1-n-butyl-5-(4-chlorophenyl)-1H-imidazole-2-carbaldehyde**

A two-necked flask containing a solution of intermediate C16 (0.97g, 1equiv) in anhydrous THF (5ml) was backfilled with nitrogen and then placed in a -80°C low-temperatuer reactor. n-Butyl lithium (1.72ml, 1.6M solution in hexane, 1.2equiv) was added dropwise over 5min and then the mixture was stirred for 1h before anhydrous DMF (0.64ml, 2equiv) was added dropwise. After the addition, the solution was allowed to warm to room temperature slowly and stirred for a few hours. The reaction was quenched with saturated $NH_4Cl$ solution and extracted with EA twice. The combined organic phase was washed with brine, dried over $MgSO_4$ and purified by column chromatography to give the title compound as an oil (0.78g). $^1$H-NMR ($CDCl_3$, 300 MHz) δ0.82 (t, 3H, *J*=7.4), 1.21 (m, 2H), 1.62 (m, 2H), 4.34 (t, 2H, *J*=7.6), 7.29 (s, 1H), 7.33 (d, 2H, *J*=8.4), 7.48 (d, 2H, *J*=8.7), 9.85 (s, 1H); MS (EI) *m*/*z*: 262 ($M^+$).

**Intermediate C18-5-n-decyl-1-(2-(diethylamino)ethyl)-1H-imidazole-2-carbaldehyde**

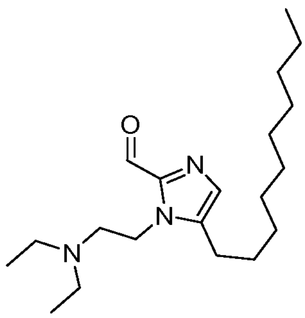

Following a procedure similar to that described for the preparation of intermediate C17 except that decyl aldehyde and *N^1^,N^1^*-diethylethane-1,2-diamine were used as starting materials. $^1$H-NMR ($CDCl_3$, 300 MHz) δ 0.87 (t, 3H), 1.03 (t, 6H), 1.26 (m, 14H), 1.69 (m, 2H), 2.66 (m, 8H), 4.40 (t, 2H), 7.08 (s, 1H), 9.70 (s, 1H).

**Intermediate C19-1-(2-(diethylamino)ethyl)-5-(4'-(trifluoromethyl)biphenyl-4-yl)-1H-imidazole-2-c arbaldehyde**

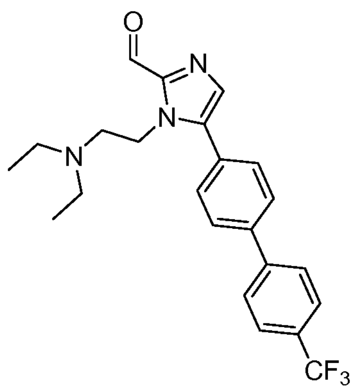

Following a procedure similar to that described for the preparation of intermediate C17 except that intermediate M1 and *N^1^,N^1^*-diethylethane-1,2-diamine was used as starting materials. $^1$H-NMR ($CDCl_3$, 300 MHz) δ0.84 (t, 6H, *J*=7.2), 2.42 (q, 4H, *J*=7.2), 2.65 (t, 2H, *J*=6.9), 4.51 (t, 2H, *J*=6.9), 7.36 (s, 1H), 7.58 (d, 2H, *J*=8.4), 7.72 (d, 2H, *J*=8.1), 7.73 (s, 4H), 9.85 (s, 1H); MS (ESI): 416(M+H).

**Example 35-1-((1-n-butyl-5-(4-chlorophenyl)-1H-imidazol-2-yl)methyl)-2-(4-fluorobenzylthio) -6,7-dihydro-1H-cyclopenta[d]pyrimidin-4(5H)-one**

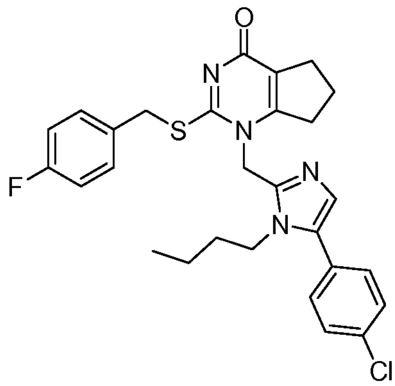

Following a procedure similar to that described for the preparation of example 28 except that intermediate C17 was used in place of intermediate C3. $^1$H-NMR ($CDCl_3$, 300 MHz) δ0.77 (t ,3H, *J*=7.2), 1.14 (m, 2H), 1.48 (m, 2H), 2.12 (m, 2H), 2.85 (t, 2H, *J*=7.5), 2.95 (t, 2H, *J*=7.5), 3.89 (t, 2H, *J*=7.6), 4.50 (s, 2H), 5.10 (s, 2H), 6.95 (t, 2H, *J*=8.7), 6.97 (s, 1H), 7.24 (d, 2H, *J*=8.7), 7.33 (dd, 2H, *J*=8.7, 5.3), 7.41 (d, 2H, *J*=8.1); MS (ESI): 523(M+H).

**Example 36-1-((1-n-butyl-5-(4-chlorophenyl)-1H-imidazol-2-yl)methyl)-2-(2-nitrobenzylthio)-6,7-dihydro-1H-cyclopenta[d]pyrimidin-4(5H)-one**

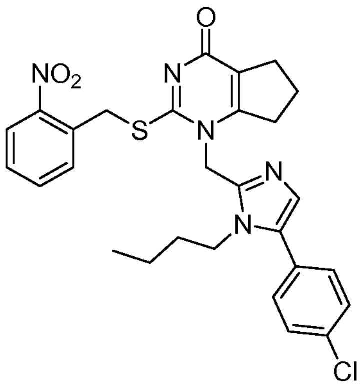

Following a procedure similar to that described for the preparation of example 28 except that intermediate C17 and 1-(chloromethyl)-2-nitrobenzene were used as starting materials in place of C3 and 1-(bromomethyl)-4-fluorobenzene, respectively. $^1$H-NMR ($CDCl_3$, 300 MHz) δ0.81 (t, 3H, *J*=7.2), 1.16 (m, 2H), 1.50 (m, 2H), 2.12 (m, 2H), 2.86 (t, 2H, *J*=7.4), 2.97 (t, 2H, *J*=7.2), 3.92 (t, 2H, *J*=7.6), 4.89 (s, 2H), 5.05 (s, 2H), 6.92 (s, 1H), 7.27 (d, 2H, *J*=8.1), 7.42 (m, 3H), 7.56 (t, 1H, *J*=7.6), 7.99 (m, 2H); MS (ESI): 550(M+H).

**Example 37-1-((5-n-decyl-1-(2-(diethylamino)ethyl)-1H-imidazol-2-yl)methyl)-2-(4-fluorobenz ylthio)-6,7-dihydro-1H-cyclopenta[d]pyrimidin-4(5H)-one**

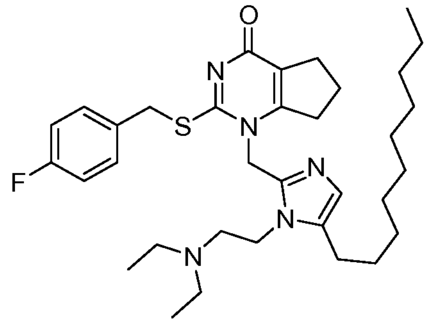

Following a procedure similar to that described for the preparation of example 28 except that intermediate C18 was used in place of intermediate C3. The product is an oil. $^1$H-NMR ($CDCl_3$, 400 MHz) δ0.90 (m, 9H), 1.26 (m, 14H), 1.63 (m, 2H), 2.14 (m, 2H), 2.46 (m, 6H), 2.56 (t, 2H, *J*=7.6), 2.84 (t, 2H, *J*=7.4), 2.91 (t, 2H, *J*=7.5), 3.84 (t, 2H, *J*=7.5), 4.47 (s, 2H), 5.10 (s, 2H), 6.72 (s, 1H), 6.95 (t, 2H, *J*=8.6), 7.32 (dd, 2H, *J*=8.6, 5.6); MS (ESI): 596(M+H).

**Example 38-1-((1-(2-(diethylamino)ethyl)-5-(4'-(trifluoromethyl)biphenyl-4-yl)-1H-imidazol-2 -yl)methyl)-2-(4-fluorobenzylthio)-6,7-dihydro-1H-cyclopenta[d]pyrimidin-4(5H) -one**

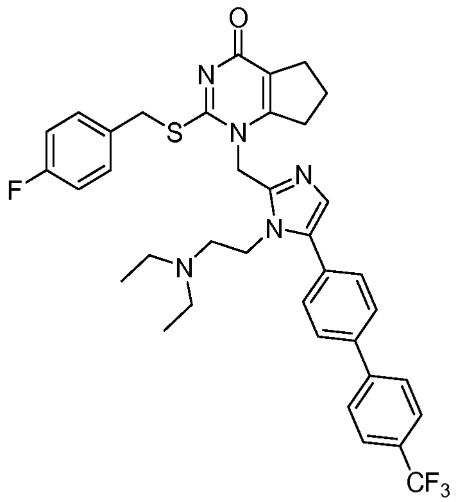

Following a procedure similar to that described for the preparation of example 28 except that intermediate C19 was used in place of intermediate C3. $^1$H-NMR ($CDCl_3$, 300 MHz) δ0.83 (t, 6H, *J*=6.9), 2.13 (m, 2H), 2.43 (m, 6H), 2.88 (t, 2H, *J*=6.9), 3.00 (t, 2H, *J*=7.2), 4.05 (t, 2H, *J*=5.7), 4.51 (s, 2H), 5.22 (s, 2H), 6.96 (t, 2H, *J*=8.6), 7.06 (s, 1H), 7.35 (dd, 2H, *J*=8.1, 5.7), 7.45 (d, 2H, *J*=8.1), 7.71 (m, 6H); MS (ESI): 676(M+H).

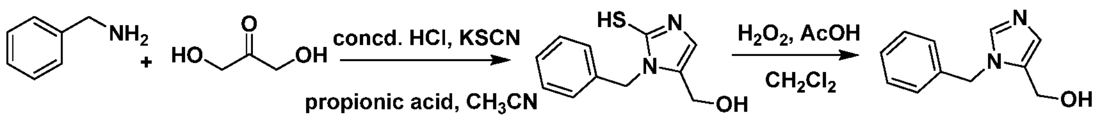


C20 C21

**Intermediate C20-(1-benzyl-2-mercapto-1H-imidazol-5-yl)methanol**

To an ice cold solution of phenylmethanamine (3.3ml, 30mmol) in acetonitrile (30ml) was added concd.HCl (2.55ml, 30mmol) over 3min with vigorous stirring, white solid was formed. Then propionic acid (2.5ml), 1,3-dihydroxypropan-2-one (4.5g, 25mmol) and KSCN (2.9g, 30mmol) were added and the mixture was stirred at 70°C for 2h. After cooled, the mixture was diluted with water (100ml) and stirred for 20min. The precipitate thus obtained was collected by filtration, washed with water and cold industrial ethanol, and then dried to obtain the title compound (4.72g). $^1$H-NMR ($d_6$-DMSO, 300 MHz) δ4.13 (s, 2H), 5.26 (s, 1H, -OH), 5.32 (s, 2H), 6.85 (s, 1H), 7.27 (m, 5H), 12.17 (s, 1H).

**Intermediate C21-(1-benzyl-1H-imidazol-5-yl)methanol**

To a suspension of intermediate C20 (4.72g, 21.45mmol, 1equiv) in DCM (30ml) were added AcOH (2.5ml) and an aqueous solution of hydrogen peroxide (0.5ml, 30% wt). The mixture was heated slightly to initiate the reaction. Then, the rest aqueous solution of hydrogen peroxide (5.5ml) was added dropwise (>2.5 equiv in total) at such a rate that the solution boiled mildly. After the addition, the mixture was stirred at room temperature for 0.5h and then the reaction flask was placed in an ice bath and the mixture was adjusted to pH >10 with a solution of NaOH (10% m/v). The mixture was stayed for a few hours and the precipitate thus obtained was collected by filtration, washed with water and DCM, and then dried in vacuo to give the title compound. Organic layer of the filtrate was then separated and concentrated and solid precipitated and was collected by filtration to give a further batch of product (3.25g in total). $^1$H-NMR ($CDCl_3$, 300 MHz) δ4.49 (s, 2H), 5.22 (s, 2H), 6.92 (s, 1H), 7.12 (m, 2H), 7.32 (m, 3H), 7.44 (s, 1H); MS (EI) *m/z*: 188 ($M^+$).

**Intermediate C22-(1-(4-bromobenzyl)-1H-imidazol-5-yl)methanol**

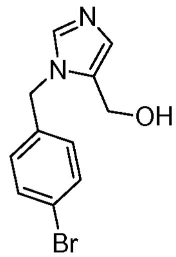

Following a procedure similar to that described for the preparation of intermediate C21 except that (4-bromophenyl)methanamine was used in place of phenylmethanamine. $^1$H-NMR ($d_6$-DMSO, 300 MHz) δ4.30 (d, 2H, *J*=4.8), 5.13 (t, 1H, *J*=5.1, -OH), 5.21 (s, 2H), 6.83 (s, 1H), 7.11 (d, 2H, *J*=8.1), 7.55 (d, 2H, *J*=8.4), 7.69 (s, 1H); MS (ESI): 267/269 (M+H).

**Intermediate C23-1-(4-bromobenzyl)-5-methyl-1H-imidazole**

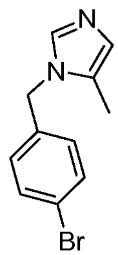

Following a procedure similar to that described for the preparation of intermediate C21 except that 2-hydroxy acetone was used in place of 1,3-dihydroxypropan-2-one. $^1$H-NMR ($CDCl_3$, 300 MHz) δ2.06 (s, 3H), 5.01 (s, 2H), 6.83 (s, 1H), 6.90 (d, 2H, *J*=8.4), 7.45 (d, 2H, *J*=8.1), 7.50 (s, 1H); MS (ESI): 251/253(M+H).

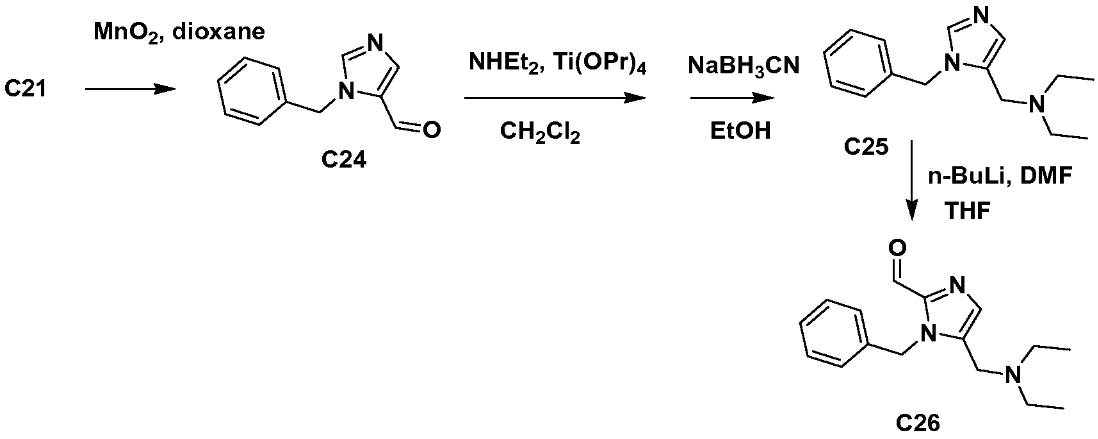


**Intermediate C24-1-benzyl-1H-imidazole-5-carbaldehyde**

To a solution of intermediate C21 (11.5g, lequiv) in dioxane (60ml) was added activated $MnO_2$ (32g, 6equiv) and the mixture was stirred at 60°C for 3h (TLC detection showed that the reaction was complete) before it was filtered to remove $MnO_2$ and washed with EA. The solvent was evaporated to give the title compound as a solid (10.38g). $^1H$-NMR ($CDCl_3$, 300 MHz) δ5.52 (s, 2H), 7.20 (m, 2H), 7.33 (m, 3H), 7.71 (s, 1H), 7.83 (s, 1H), 9.76 (s, 1H); MS (EI) *m/z*: 186 ($M^+$).

**Intermediate C25-N-((1-benzyl-1H-imidazol-5-yl)methyl)-N-ethylethanamine**

A mixture of intermediate C24 (1.5g, lequiv), diethylamine (0.875ml, 1.05equiv), tetrapropoxytitanium (3.1ml, 1.3equiv) in DCM (8ml) was stirred at room temperature for 4h under nitrogen before absolute ethanol (8ml) and sodium cyanoborohydride (507mg, 1equiv) was added. The mixture was stirred at room temperature for 16h and then quenched with 20ml water. The precipitated white solid was removed by filtration and washed with 20ml EA. The filtrate was transferred to a seperatory funnel and the EA layer was separated. The aqueous phase was extracted with 20ml of EA once. The combined organic phase was washed with brine twice, dried over $MgSO_4$ and purified by column chromatography (EA/EtOH=4:1) to give the title compound as an oil (1.32g). $^1H$-NMR ($CDCl_3$, 300 MHz) δ0.94 (t, 6H, *J*=6.9), 2.46 (q, 4H, *J*=7.2), 3.39 (s, 2H), 5.30 (s, 2H), 6.95 (s, 1H), 7.08 (m, 2H), 7.29 (m, 3H), 7.53 (s, 1H); MS (ESI): 244(M+H).

**Intermediate C26-1-benzyl-5-((diethylamino)methyl)-1H-imidazole-2-carbaldehyde**

Following a procedure similar to that described for the preparation of intermediate C17 except that intermediate C25 was used in place intermediate C16. $^1H$-NMR ($CDCl_3$, 300 MHz) 60.96 (t, 6H, *J*=7.1), 2.49 (q, 4H, *J*=7.2), 3.46 (s, 2H), 5.88 (s, 2H), 6.97 (m, 2H), 7.27 (m, 4H), 9.80 (s, 1H).

**Example 39-1-((1-benzyl-5-((diethylamino)methyl)-1H-imidazol-2-yl)methyl)-2-(4-fluorobenz ylthio)-6,7-dihydro-1H-cyclopenta[d]pyrimidin-4(5H)-one**

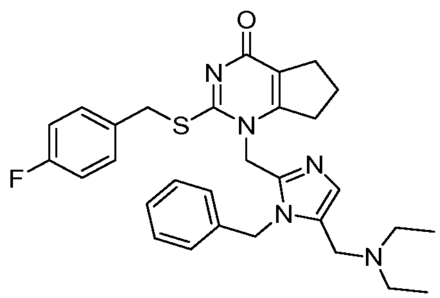

Following a procedure similar to that described for the preparation of example 28 except that intermediate C26 was used in place intermediate C3. $^1H$-NMR ($CDCl_3$, 300 MHz) δ0.93 (t, 6H, *J*=7.2), 2.00 (m, 2H), 2.48 (m, 4H), 2.72

(q, 4H, *J*=7.4), 3.47 (s, 2H), 4.42 (s, 2H), 4.85 (s, 2H), 5.39 (s, 2H), 6.87 (m, 2H), 6.96 (m, 3H), 7.23-7.33 (m, 5H); MS (ESI): 532(M+H).

**Intermediate C27-5-methyl-1-((4'-(trifluoromethyl)biphenyl-4-yl)methyl)-1H-imidazole**

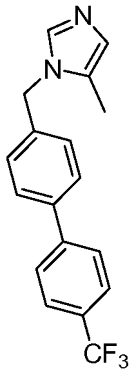

A mixture of intermediate C23 (1.07g, 4.26mmol, lequiv), 4-(trifluoromethyl)phenylboronic acid (0.85g, 1.05equiv), $Cs_2CO_3$ (2.8g, 2equiv) and $Pd(PPh)_3$ (246mg, 0.05equiv) in dioxane (10ml) was refluxed for 6h in a nitrogen atmosphere and then filtered to remove the insoluble substances while hot. The filtrate was mixed with silica gel, evaporated and purified by column chromatography (DCM/MeOH=15:1) to give the title compound as a solid (1.11g). $^1$H-NMR ($CDCl_3$, 300 MHz) δ2.13 (s, 3H), 5.13 (s, 2H), 6.87 (s, 1H), 7.15 (d, 2H, *J*=7.8), 7.57 (d, 2H, *J*=7.8), 7.58 (s, 1H), 7.65 (d, 2H, *J*=9.0), 7.70 (d, 2H, *J*=9.3); MS (ESI): 317(M+H).

**Intermediate C28-(5-methyl-1-((4'-(trifluoromethyl)biphenyl-4-yl)methyl)-1H-imidazol-2-yl)metha** nol

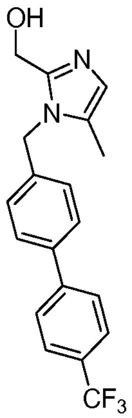

A mixture of intermediate C27 (1.53g, 4.84mmol) and formaldehyde (10ml, 37% aqueous solution) in dioxane (3ml) was heated in a pressure tube at 140~150°C for 36h (the plug was screwed tightly). Then, the heating was stopped and TLC detection showed that the reaction was complete. The reaction mixture was poured into 20ml of saturated brine and extracted with DCM (60mL) three times. The combined organic phase was washed with brine three times, dried over $MgSO_4$ and purified by column chromatography to give the title compound as a white solid (0.9g). $^1$H-NMR ($CDCl_3$, 300 MHz) δ2.10 (s, 3H), 4.62 (s, 2H), 5.29 (s, 2H), 6.73 (s, 1H), 7.10 (d, 2H, *J*=8.4), 7.54 (d, 2H, *J*=8.4), 7.67 (m, 4H); MS (ESI): 347(M+H).

**Example 40-2-(4-fluorobenzylthio)-1-((5-methyl-1-((4'-(trifluoromethyl)biphenyl-4-yl)methyl) -1H-imidazol-2-yl)methyl)-6,7-dihydro-1H-cyclopenta[d]pyrimidin-4(5H)-one**

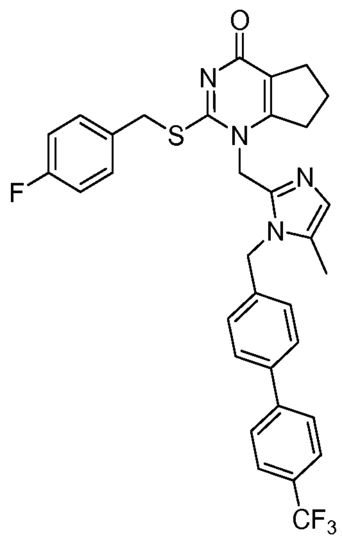

Following a procedure similar to that described for the preparation of example 28 except that intermediate C28 was used in place intermediate C9. $^1$H-NMR ($CDCl_3$, 300 MHz) δ1.93 (m, 2H), 2.22 (s, 3H), 2.60 (t, 2H, *J*=7.2), 2.69 (t, 2H, *J*=7.5), 4.46 (s, 2H), 5.01 (s, 2H), 5.14 (s, 2H), 6.84 (d, 2H, *J*=8.1), 6.88 (s, 1H), 6.95 (t, 2H, *J*=8.4), 7.34 (dd, 2H, *J*=5.4, 8.4), 7.44 (d, 2H, *J*=8.4), 7.63 (d, 2H, *J*=8.4), 7.70 (d, 2H, *J*=8.1); MS (ESI): 605(M+H).

**Example 41-2-(4-fluorobenzylthio)-1-((5-methyl-1-((4'-(trifluoromethyl)biphenyl-4-yl)methyl) -1H-imidazol-2-yl)methyl)-5-((1-methyl-1H-pyrazol-4-yl)methyl)pyrimidin-4(1H) -one**

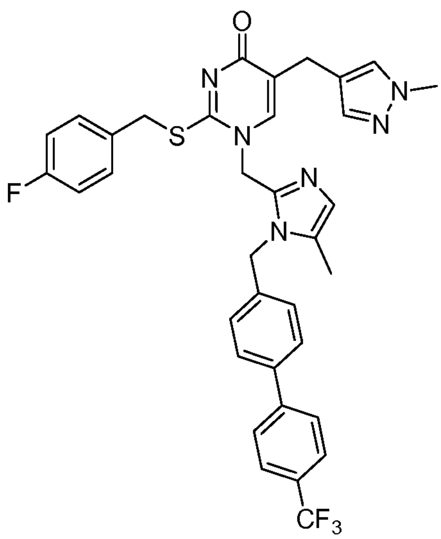

Following a procedure similar to that described for the preparation of example 29 except that intermediate C28 was used in place intermediate C9. $^1$H-NMR ($CDCl_3$, 300 MHz) δ2.20 (s, 3H), 3.39 (s, 2H), 3.80 (s, 3H), 4.40 (s, 2H), 4.89 (s, 2H), 5.09 (s, 2H), 6.74 (d, 2H, *J*=8.1), -6.96 (m, 4H), 7.23 (s, 1H), -7.33 (m, 5H), 7.62 (d, 2H, *J*=8.1), 7.70 (d, 2H, *J*=8.7); MS (ESI): 659(M+H).

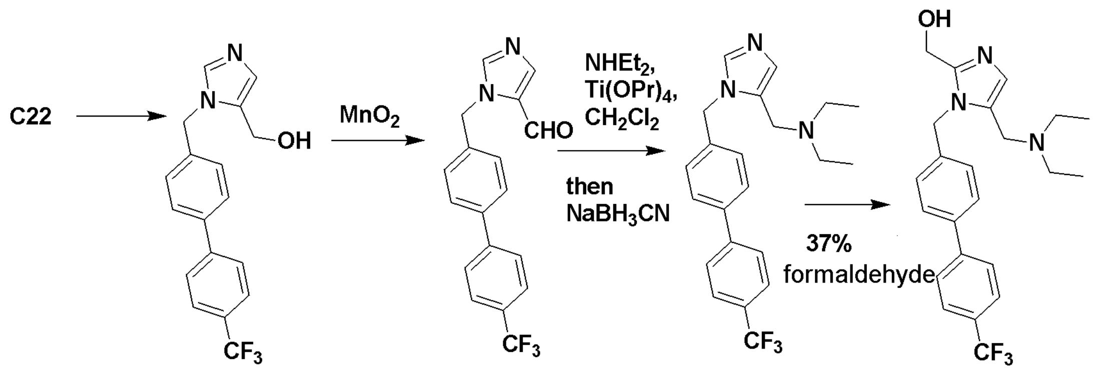


C29 C30 C31 C32

**Intermediate C29-(1-((4'-(trifluoromethyl)biphenyl-4-yl)methyl)-1H-imidazol-5-yl)methanol**

A mixture of intermediate C22 (10mol, lequiv), 4-(trifluoromethyl)phenylboronic acid (1.1equiv), $Cs_2CO_3$ (2equiv) and $Pd(PPh_3)_4$ (0.05equiv) in 30ml dioxane was backfilled with nitrogen several times and heated under reflux for 15h. Then, it was filtered to remove the inorganic salts and washed with dioxane. The filtrate was evaporated under reduced pressure to dryness to give a brown oil or solid which was then dissolved in EA (20ml). The solution was stayed for a few hours to wait for an adequate precipitation of the product. The solid thus obtained was filtered off and dried. The above operation was repeated for the mother liquor. $^1$H-NMR ($d_6$-DMSO, 300 MHz) δ4.35 (s, 2H), 5.30 (s, 2H), 6.85 (s, 1H), 7.29 (d, 2H, *J*=8.1), 7.73 (d, 2H, *J*=7.8), 7.74 (s, 1H), 7.80 (d, 2H, *J*=8.1), 7.88 (d, 2H, *J*=8.4).

**Intermediate C30-1-((4'-(trifluoromethyl)biphenyl-4-yl)methyl)-1H-imidazole-5-carbaldehyde**

Following a procedure similar to that described for the preparation of intermediate C24. $^1$H-NMR ($CDCl_3$, 300 MHz) δ5.58 (s, 2H), 7.30 (d, 2H, *J*=8.1), 7.57 (d, 2H, *J*=8.1), 7.67 (m, 4H), 7.78 (s, 1H), 7.84 (s, 1H), 9.78 (s, 1H).

**Intermediate C31-N-ethyl-N-((1-((4'-(trifluoromethyl)biphenyl-4-yl)methyl)-1H-imidazol-5-yl)meth** yl)ethanamine

Following a procedure similar to that described for the preparation of intermediate C25. $^1$H-NMR ($CDCl_3$, 300 MHz) δ0.99 (t, 6H, *J*=7.2), 2.53 (q, 4H, *J*=6.9), 3.47 (s, 2H), 5.39 (s, 2H), 7.00 (s, 1H), 7.17 (d, 2H, *J*=7.8), 7.55 (d, 2H, *J*=8.1), 7.63 (s, 1H), 7.67 (m, 4H); MS (ESI): 388(M+H).

**Intermediate C32-(5-((diethylamino)methyl)-1-((4'-(trifluoromethyl)biphenyl-4-yl)methyl)-1H-imid** azol-2-yl)methanol

Following a procedure similar to that described for the preparation of intermediate C28. $^1$H-NMR ($CDCl_3$, 300 MHz) δ0.93 (t, 6H, *J*=7.2), 2.46 (q, 4H, *J*=7.2), 3.37 (s, 2H), 4.62 (s, 2H), 5.53 (s, 2H), 6.87 (s, 1H), 7.10 (d, 2H, *J*=8.4), 7.53 (d, 2H, *J*=8.4), 7.67 (m, 4H).

**Intermediate C33-(5-(pyrrolidin-1-ylmethyl)-1-((4'-(trifluoromethyl)biphenyl-4-yl)methyl)-1H-imid** azol-2-yl)methanol

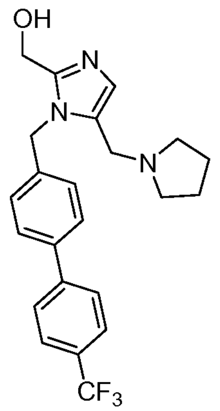

Following a procedure similar to that described for the preparation of intermediate C32 from C30 except that pyrrolidine was used in place of diethylamine. $^1$H-NMR ($CDCl_3$, 300 MHz) δ1.72 (m, 4H), 2.42 (m, 4H), 3.41 (s, 2H), 4.62 (s, 2H), 5.49 (s, 2H), 6.83 (s, 1H), 7.14 (d, 2H, *J*=8.4), 7.53 (d, 2H, *J*=8.1), 7.67 (m, 4H).

C29

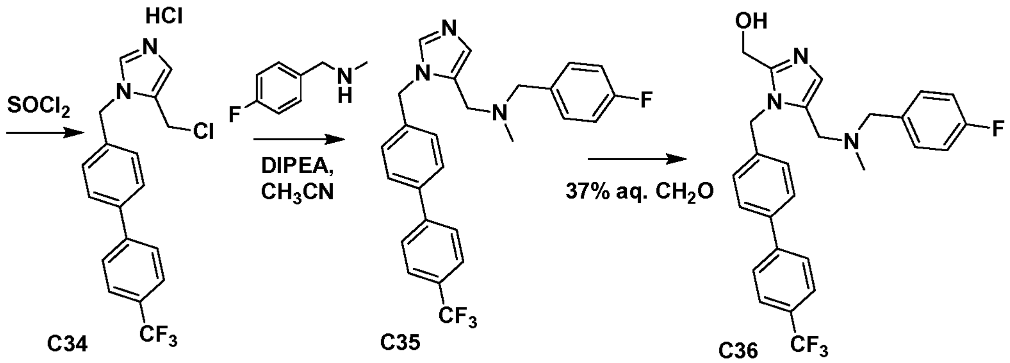


C34 C35 C36

**Intermediate C34-5-(chloromethyl)-1-((4'-(trifluoromethyl)biphenyl-4-yl)methyl)-1H-imidazole** Hydrochloride

To an ice cold mixture of intermediate C29 (5mmol) and DMF (2drops, catalyst) in DCM (20ml) in a 100ml of flask was added a solution of thionyl chloride (0.42ml, 6mmol) in DCM (5ml) dropwise via a constant pressure funnel over 10min. The mixture was stirred at room temperature for 2h until the completion of the reaction monitored by TLC. The solid so formed was collected by filtration with a buchner funnel, washed with DCM three times and then dried at 80 °C to give the title compound as white or light yellow powder. $^1$H-NMR ($d_6$-DMSO, 300 MHz) δ4.96 (s, 2H), 5.61 (s, 2H), 7.52 (d, 2H, *J*=8.4), 7.82 (m, 3H), 7.89 (m, 4H), 9.39 (s, 1H).

**Intermediate C35-N-(4-fluorobenzyl)-N-methyl-1-(1-((4'-(trifluoromethyl)biphenyl-4-yl)methyl)-1H** -imidazol-5-yl)methanamine

A mixture of intermediate C34 (1.38g, 3.57mmol, lequiv), 1-(4-fluorophenyl)-N-methylmethanamine (0.5g, lequiv) and DIPEA (1.47ml, 2.5equiv) in acetonitrile (10ml) was heated at 70~80°C overnight until the completion of the reaction monitored by TLC. Solvent was evaporated and aq. $NH_4Cl$ solution (40ml) was added to the residue. The resulting mixture was extracted with DCM twice and the combined organic phase was washed with brine twice, dried and then purified by column chromatography to give the title compound as an oil (0.9g). $^1$H-NMR ($CDCl_3$, 400 MHz) δ2.16 (s, 3H), 3.38 (s, 2H), 3.46 (s, 2H), 5.31 (s, 2H), 6.97 (t, 2H, *J*=8.4), 7.03 (s, 1H),7.08 (d, 2H, *J*=8.4), 7.21 (dd, 2H, *J*=8.4, 5.6), 7.54 (d, 2H, *J*=8.0), 7.60 (s, 1H), 7.68 (d, 2H, *J*=8.8), 7.72 (d, 2H, *J*=8.0).

**Intermediate C36-(5-(((4-fluorobenzyl)(methyl)amino)methyl)-1-((4'-(trifluoromethyl)biphenyl-4-yl )methyl)-1H-imidazol-2-yl)methanol**

Following a procedure similar to that described for the preparation of intermediate C28 except that intermediate C35 was used in place of intermediate C27. $^1$H-NMR ($CDCl_3$, 300 MHz) δ2.07 (s, 3H), 3.26 (s, 2H), 3.37 (s, 2H), 4.60 (s, 2H), 5.45 (s, 2H), 6.83 (s, 1H), 6.92 (t, 2H, *J*=8.4), 7.01 (d, 2H, *J*=8.4), 7.12 (dd, 2H, *J*=8.4, 5.4), 7.49 (d, 2H, *J*=8.1), 7.64 (d, 2H, *J*=8.7), 7.68 (d, 2H, *J*=8.4).

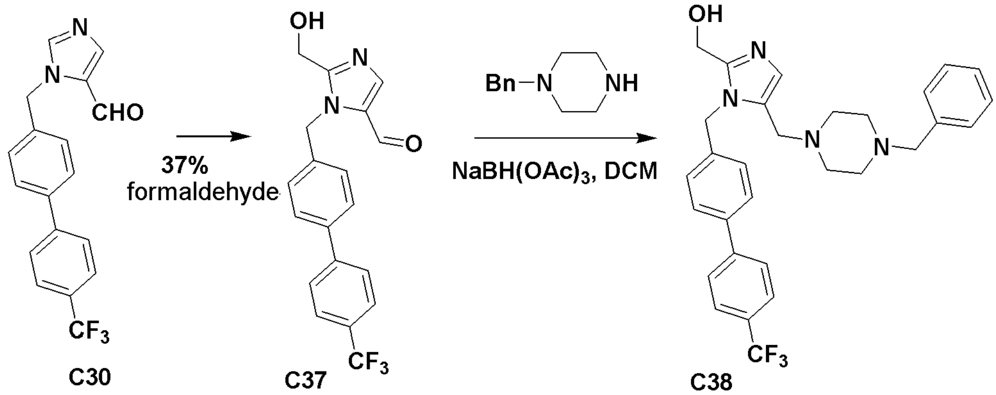


C30 C37 C38

**Intermediate C37-2-(hydroxymethyl)-1-((4'-(trifluoromethyl)biphenyl-4-yl)methyl)-1H-imidazole-5** -carbaldehyde

Following a procedure similar to that described for the preparation of intermediate C28 except that intermediate C30 was used in place of intermediate C27. $^1$H-NMR ($CDCl_3$, 300 MHz) δ4.75 (s, 2H), 5.74 (s, 2H), 7.22 (d, 2H, *J*=8.1), 7.54 (d, 2H, *J*=7.8), 7.63 (d, 2H, *J*=8.7), 7.69 (d, 2H, *J*=8.7), 7.77 (s, 1H), 9.46 (s, 1H).

**Intermediate C38-(5-((4-benzylpiperazin-1-yl)methyl)-1-((4'-(trifluoromethyl)biphenyl-4-yl)methyl) -1H-imidazol-2-yl)methanol**

A mixture of intermediate C37 (0.56g, 1equiv), 1-benzylpiperazine (300μl, 1.1equiv), $NaBH(OAc)_3$ (495mg, 1.5equiv) and AcOH (180μl, 2equiv) in 5ml DCM was stirred at room temperature overnight until the completion of the reaction monitored by TLC. The reaction mixture was washed with brine twice, with saturated $NaHCO_3$ solution twice, dried over $Na_2SO_4$ and then purified by column chromatography to give the title compound as a solid (0.5g). $^1$H-NMR ($CDCl_3$, 400 MHz) δ2.43 (m, 8H), 3.45 (s, 2H), 3.52 (s, 2H), 4.75 (s, 2H), 5.50 (s, 2H), 7.26 (m, 8H), 7.55 (d, 2H, *J*=8.4), 7.65 (d, 2H, *J*=8.4), 7.69 (d, 2H, *J*=8.4).

**Intermediate C39-(5-((dimethylamino)methyl)-1-((4'-(trifluoromethyl)biphenyl-4-yl)methyl)-1H-im** idazol-2-yl)methanol

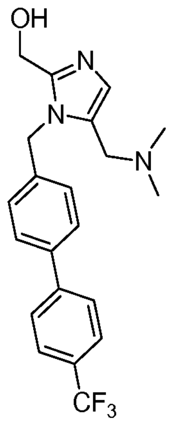

Following a procedure similar to that described for the preparation of intermediate C38 from C37 except that dimethylamine hydrochloride and DIPEA (2equiv) was used in place of 1-benzylpiperazine. $^1$H-NMR ($CDCl_3$, 300 MHz) δ2.15 (s, 6H), 3.19 (s, 2H), 4.62 (s, 2H), 4.63 (vbrs, 1H), 5.49 (s, 2H), 6.83 (s, 1H), 7.14 (d, 2H, *J*=8.1), 7.54 (d, 2H, *J*=8.1), 7.67 (m, 4H); MS (ESI): 390(M+H).

**Example 42-1-((5-((diethylamino)methyl)-1-((4'-(trifluoromethyl)biphenyl-4-yl)methyl)-1H-i midazol-2-yl)methyl)-2-(4-fluorobenzylthio)-6,7-dihydro-1H-cyclopenta[d]pyrimi din-4(5H)-one**

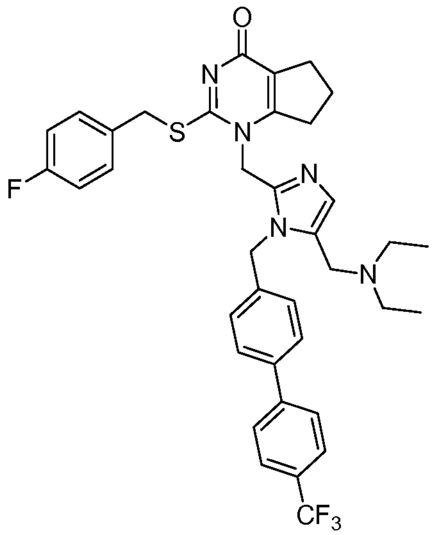

Following a procedure similar to that described for the preparation of example 28 except that intermediate C32 was used in place of intermediate C9. $^1$H-NMR ($CDCl_3$, 300 MHz) δ0.93 (t, 6H, *J*=6.9), 1.97 (m, 2H), 2.47 (q, 4H, *J*=6.9), 2.70 (m, 4H), 3.48 (s, 2H), 4.45 (s, 2H), 4.92 (s, 2H), 5.44 (s, 2H), 6.95 (m, 5H), 7.31 (m, 2H), 7.45 (d, 2H, *J*=7.8), 7.62 (d, 2H, *J*=7.8), 7.70 (d, 2H, *J*=8.1); MS (ESI): 676(M+H).

**Example 43-2-(4-fluorobenzylthio)-1-((5-(pyrrolidin-1-ylmethyl)-1-((4'-(trifluoromethyl)biphe nyl-4-yl)methyl)-1H-imidazol-2-yl)methyl)-6,7-dihydro-1H-cyclopenta[d]pyrimid in-4(5H)-one**

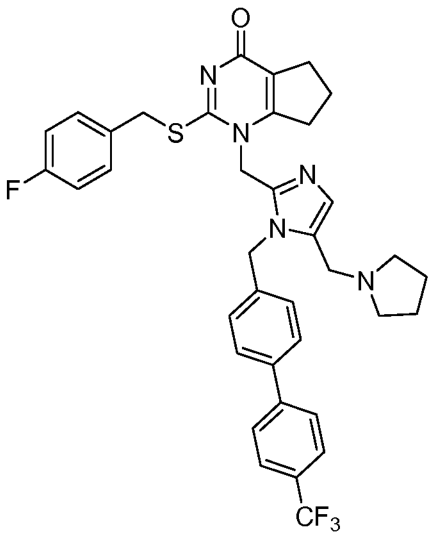

Following a procedure similar to that described for the preparation of example 28 except that intermediate C33 was used in place of intermediate C9. $^1$H-NMR ($CDCl_3$, 300 MHz) δ1.79 (m, 4H), 1.96 (m, 4H), 2.53 (m, 2H), 2.68 (m, 4H), 3.61 (s, 2H), 4.44 (s, 2H), 4.95 (s, 2H), 5.45 (s, 2H), 6.93 (m, 4H), 6.99 (s, 1H), 7.30 (dd, 2H, *J*=7.8, 5.4), 7.43 (d, 2H, *J*=7.8), 7.60 (d, 2H, *J*=7.8), 7.70 (d, 2H, *J*=7.8).

**Example 44-1-((5-(((4-fluorobenzyl)(methyl)amino)methyl)-1-((4'-(trifluoromethyl)biphenyl-4 -yl)methyl)-1H-imidazol-2-yl)methyl)-2-(4-fluorobenzylthio)-6,7-dihydro-1H-cycl openta[d]pyrimidin-4(5H)-one**

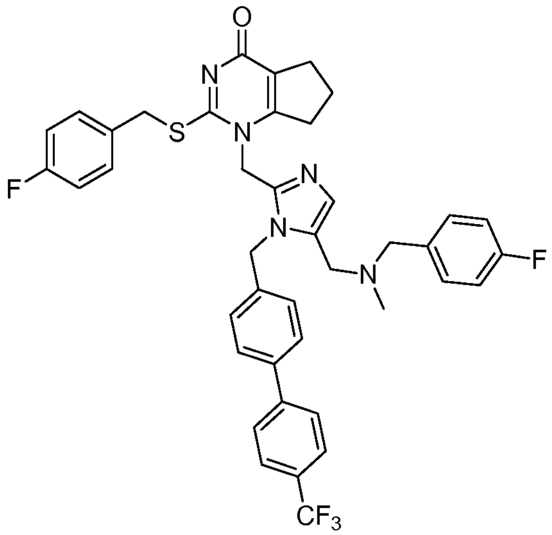

Following a procedure similar to that described for the preparation of example 28 except that intermediate C36 was used in place of intermediate C9. $^1$H-NMR ($CDCl_3$, 400 MHz) δ1.98 (m, 2H), 2.18 (s, 3H), 2.70 (m, 4H), 3.43 (s, 2H), 3.45 (s, 2H), 4.45 (s, 2H), 4.94 (s, 2H), 5.29 (s, 2H), 6.84-6.96 (m, 6H), 7.02 (s, 1H), 7.11 (m, 2H), 7.31 (dd, 2H, *J*=8.4, 6.4), 7.44 (d, 2H, *J*=8.0), 7.64 (d, 2H, *J*=8.4), 7.73 (d, 2H, *J*=8.0); MS (ESI): 742(M+H).

**Example 45-1-((5-((4-benzylpiperazin-1-yl)methyl)-1-((4'-(trifluoromethyl)biphenyl-4-yl)meth yl)-1H-imidazol-2-yl)methyl)-2-(4-fluorobenzylthio)-6,7-dihydro-1H-cyclopenta[ d]pyrimidin-4(5H)-one**

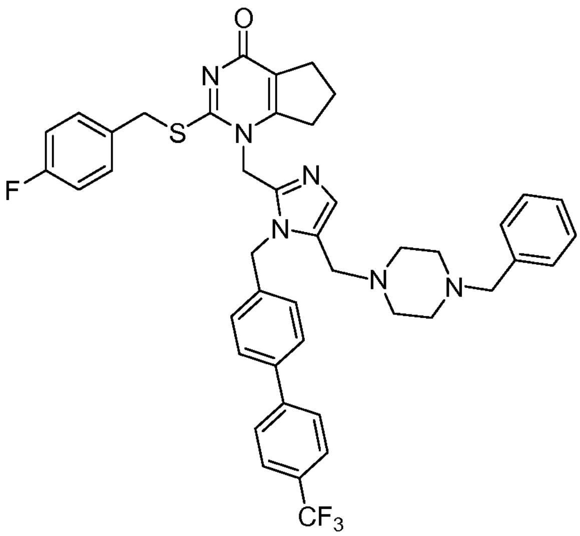

Following a procedure similar to that described for the preparation of example 28 except that intermediate C38 was used in place of intermediate C9. $^1$H-NMR ($CDCl_3$, 400 MHz) δ1.99 (m, 2H), 2.40 (m, 4H), 2.53 (m, 4H), 2.66 (t, 2H), 2.78 (t, 2H), 3.50 (s, 2H), 3.74 (s, 2H), 4.47 (s, 2H), 5.05 (s, 2H), 5.45 (s, 2H), 6.96 (m, 4H), 7.31 (m, 8H), 7.48 (d, 2H), 7.65 (d, 2H), 7.74 (d, 2H).

**Example 46-1-((5-((dimethylamino)methyl)-1-((4'-(trifluoromethyl)biphenyl-4-yl)methyl)-1H-imidazol-2-yl)methyl)-2-(4-fluorobenzylthio)-6,7-dihydro-1H-cyclopenta[d]pyrim idin-4(5H)-one**

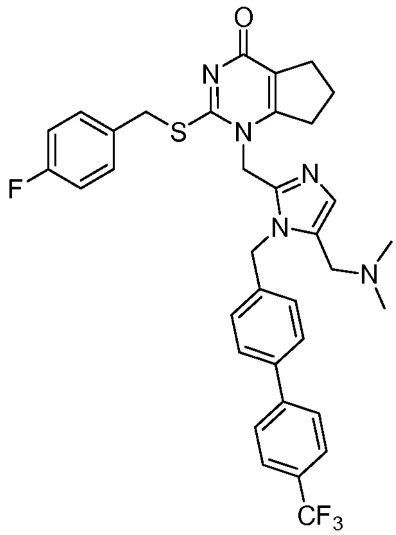

Following a procedure similar to that described for the preparation of example 28 except that intermediate C39 was used in place of intermediate C9. $^1$H-NMR ($CDCl_3$, 300 MHz) δ1.96 (m, 2H), 2.19 (s, 6H), 2.68 (m, 4H), 3.33 (s, 2H), 4.45 (s, 2H), 4.93 (s, 2H), 5.39 (s, 2H), 6.93 (m, 5H), 7.31 (m, 2H), 7.45 (d, 2H, *J*=7.8), 7.62 (d, 2H, *J*=7.8), 7.70 (d, 2H, *J*=7.5); MS (ESI): 648(M+H).

**Example 47-N-(4-fluorobenzyl)-1-(2-((2-(4-fluorobenzylthio)-4-oxo-4,5,6,7-tetrahydro-1H-cycl openta[d]pyrimidin-1-yl)methyl)-1-((4'-(trifluoromethyl)biphenyl-4-yl)methyl)-1 H-imidazol-5-yl)-N,N-dimethylmethanaminium bromide**

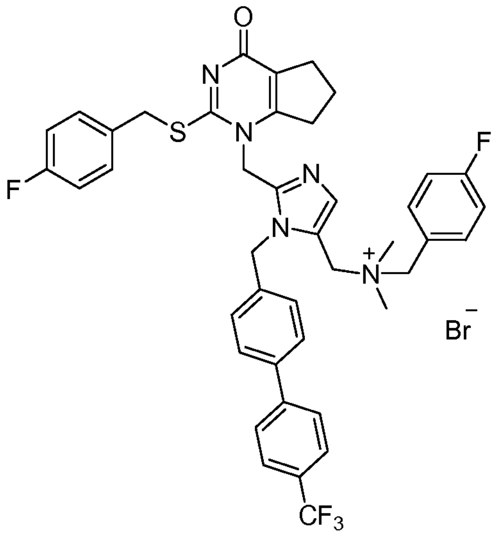

A mixture of example 46 (25mg), 1-(bromomethyl)-4-fluorobenzene (6μl) in acetone (1.5ml) was refluxed for 1h. TLC showed that most of the reaction was complete. The mixture was then stored in refrigerator for a few hours. The precipitate thus obtained was collected by filtration, dried to give the title compound as white powder (10mg). $^1$H-NMR ($CDCl_3$, 300 MHz) δ2.06 (m, 2H), 2.71 (t, 2H, *J*=6.6), 2.85 (t, 2H, *J*=6.6), 2.98 (s, 6H), 4.36 (s, 2H), 4.62 (s, 2H),

4.80 (s, 2H), 5.25 (s, 2H), 5.57 (s, 2H), 6.86 (t, 2H, *J*=8.7), 7.07 (d, 2H, *J*=7.8), 7.27 (m, 4H), 7.74 (s, 1H), 7.59 (m, 4H), 7.71 (d, 2H, *J*=8.1), 7.75 (d, 2H, *J*=8.4); MS (ESI): 756(M-Br).

**Example 48-1-((5-((dimethylamino)methyl)-1-((4'-(trifluoromethyl)biphenyl-4-yl)methyl)-1H-imidazol-2-yl)methyl)-2-(4-fluorobenzylthio)-6,7-dihydro-1H-cyclopenta[d]pyrim idin-4(5H)-one** hydrochloride

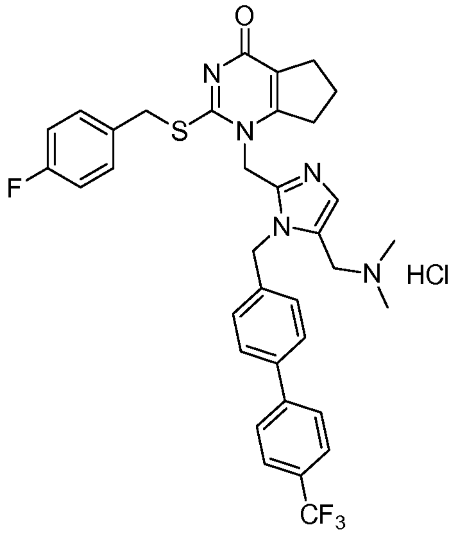

To an ice cold solution of example 46 (100mg, lequiv) in isopropanol (1.5ml) was added concd. HCl (15μl, 1.1equiv) and the mixture was stirred for 20min before ethyl ether (5ml) was added. Then the mixture was stored in refrigerator. The solid thus obtained was collected by filtration, washed with ethyl ether, and then dried to give the title compound (80mg).

**Example 49-1-(4-fluorobenzyl)-1-((2-((2-(4-fluorobenzylthio)-4-oxo-4,5,6,7-tetrahydro-1H-cyc lopenta[d]pyrimidin-1-yl)methyl)-1-((4'-(trifluoromethyl)biphenyl-4-yl)methyl)-1** H-imidazol-5-yl)methyl)pyrrolidinium bromide

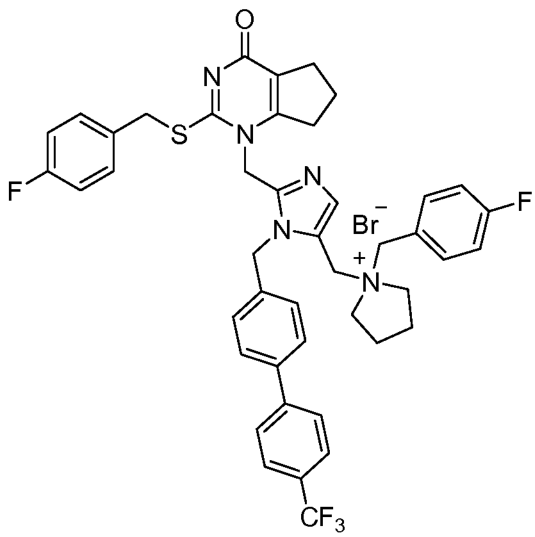

Following a procedure similar to that described for the preparation of example 47 except that example 43 was used in place of example 46.

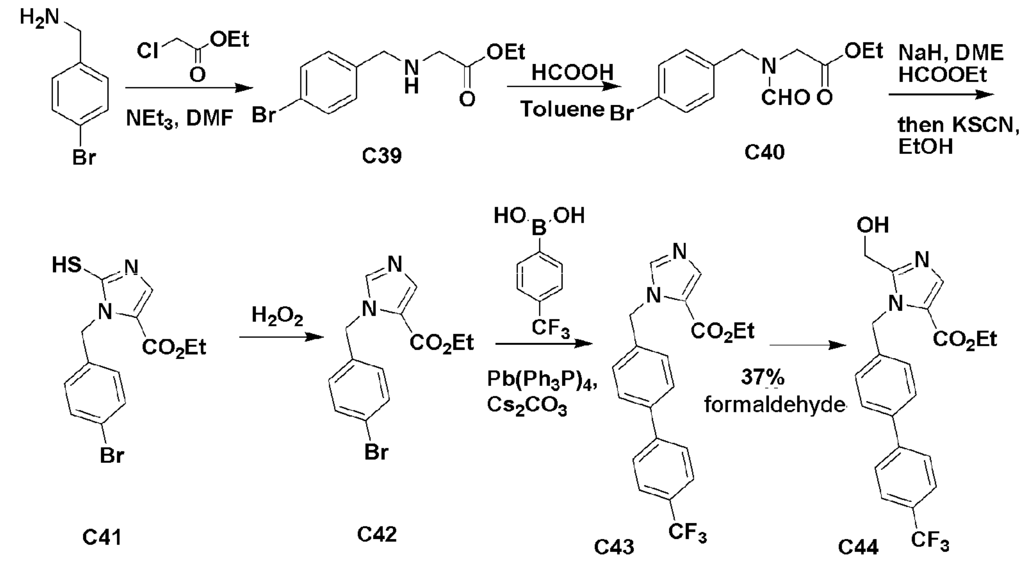


C39 C40 C41 C42 C43 C44

**Intermediate C39-ethyl** 2-(4-bromobenzylamino)acetate

To a mixture of (4-bromophenyl)methanamine (20mmol), triethylamine (20mmol) in DMF (30ml) in a flask placed in a oil bath of 40~50°C was added a solution of ethyl 2-chloroacetate (20mmol) in DMF (10ml) dropwise over 1.5h via a constant pressure funnel. After the addition, the mixture was stirred for 1.5h before it was diluted with EA (150ml). The resulting solution was washed with brine six times (30-40ml each time), dried over anhydrous $NaSO_4$ and then solvent evaporated to give the title compound as a colorless oil which could be used in next step without further purification. $^1$H-NMR ($CDCl_3$, 300 MHz) δ1.28 (t, 3H, *J*=7.2), 2.17 (s, 1H), 3.39 (s, 2H), 3.77 (s, 2H), 4.19 (q, 2H, *J*=7.2), 7.21 (d, 2H, *J*=8.4), 7.44 (d, 2H, *J*=8.4).

**Intermediate C40-ethyl 2-(N-(4-bromobenzyl)formamido)acetate**

A mixture of intermediate C39 (3.4g, 13.6mmol, lequiv) and formic acid (1.5ml, 2.5equiv) in toluene was refluxed in a Dean-Stark trap until no more water could be separated. Toluene was evaporated and the residue was mixed with silica gel and evaporated before purified by column chromatography (PE/EA=2:1) to give the title compound as an oil (2.87g). $^1$H-NMR ($CDCl_3$, 400 MHz, ca 2:1 rotational isomer) δ1.27 (t, 3H, *J*=7.2), 3.96/3.85 (2x s, 2H), 4.19 (m, 2H), 4.51/4.59 (2x s, 2H), 7.12/7.14 (2x d, 2H, *J*=8.0), 7.52/7.47 (2x d, 2H, *J*=8.0), 8.36/8.19 (2x s, 1H).

**Intermediate C41-ethyl 1-(4-bromobenzyl)-2-mercapto-1H-imidazole-5-carboxylate**

To an ice cold solution of intermediate C40 (6.21g, 20.7mmol, 1 equiv) in DME (50ml) was added NaH (1.0g, 55-60% m/m, suspended in mineral oil) in batches under nitrogen and the mixture was stirred for 20min. Then, ethyl formate (5ml) was added and the mixture was stirred for a further 4h at room temperature. DME was evaporated to obatin a yellow solid, which was dissolved with ice water (40ml), acidified to (pH~1 with concd. HCl. Then, KSCN (3.0g) and EtOH (40ml) were added and the mixture was refluxed for 15h. After the solution was cooled in an ice bath, it was adjusted to pH 6~7 with a solution of 10% NaOH (m/v) in an ice bath. The precipitate thus obtained was collected by filtration and dried to give the title compound (3.11g). $^1$H-NMR ($d_6$-DMSO, 400 MHz) δ1.17 (t, 3H, *J*=7.2), 4.15 (q, 2H, *J*=7.2), 5.51 (s, 2H), 7.14 (d, 2H, *J*=8.4), 7.50 (d, 2H, *J*=8.4), 7.88 (s, 1H), 13.10 (s, 1H).

**Intermediate C42-ethyl1-(4-bromobenzyl)-1H-imidazole-5-carboxylate**

To a slurry of intermediate C41 (3.10g, 9.09mmol, 1equiv) in DCM (20ml) was added AcOH (1ml) and hydrogen peroxide (0.2ml, 30% aqueous solution), and the mixture was heated slightly to initiate the reaction. Then, the rest aqueous solution of hydrogen peroxide (2.5ml) was added dropwise (>2.5 equiv in total) at such a rate that the solution boiled mildly. After the addition, the mixture was stirred at room temperature for 0.5h and then reaction flask was place in an ice bath and adjusted to pH 6~7 with a solution of NaOH (10% m/v). The resulting mixture was poured into saturated $NaHCO_3$ solution (15ml) and the DCM layer was separated. The aqueous phase was extracted with DCM once. The combined organic phase was washed with brine twice, dried over anhydrous $NaSO_4$ and purified by flash chromatography to obtain the title compound as a solid (1.85g). $^1$H-NMR ($CDCl_3$, 400 MHz) δ1.31 (t, 3H, *J*=7.2), 4.25 (q, 2H, *J*=7.2), 5.48

(s, 2H), 7.02 (d, 2H, *J*=8.4), 7.44 (d, 2H, *J*=8.8), 7.63 (s, 1H), 7.76 (s, 1H).

**Intermediate C43-ethyl 1-((4'-(trifluoromethyl)biphenyl-4-yl)methyl)-1H-imidazole-5-carboxylate**

Following a procedure similar to that described for the preparation of intermediate C27 except that intermediate C42 was used in place of intermediate C23. $^1$H-NMR ($CDCl_3$, 400 MHz) δ1.33 (t, 3H, *J*=7.2), 4.29 (q, 2H, J=6.8), 5.58 (s, 2H), 7.26 (d, 2H, *J*=8.4), 7.57 (d, 2H, *J*=8.4), 7.68 (m, 5H), 7.79 (s, 1H).

**Intermediate C44-**ethyl **2-(hydroxymethyl)-1-((4'-(trifluoromethyl)biphenyl-4-yl)methyl)-1H-imidazole-5 -carboxylate**

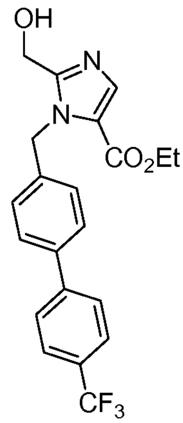

Following a procedure similar to that described for the preparation of intermediate C28 except that intermediate C43 was used in place of intermediate C27. $^1$H-NMR ($CDCl_3$, 300 MHz) δ1.30 (t, 3H, *J*=7.2), 4.27 (q, 2H, *J*=7.2), 4.70 (s, 2H), 5.76 (s, 2H), 7.16 (d, 2H, *J*=7.8), 7.53 (d, 2H, *J*=8.4), 7.66 (m, 5H).

**Example 50-ethyl 2-((2-(4-fluorobenzylthio)-4-oxo-4,5,6,7-tetrahydro-1H-cyclopenta[d]pyrimidin-1 -yl)methyl)-1-((4'-(trifluoromethyl)biphenyl-4-yl)methyl)-1H-imidazole-5-carbox** ylate

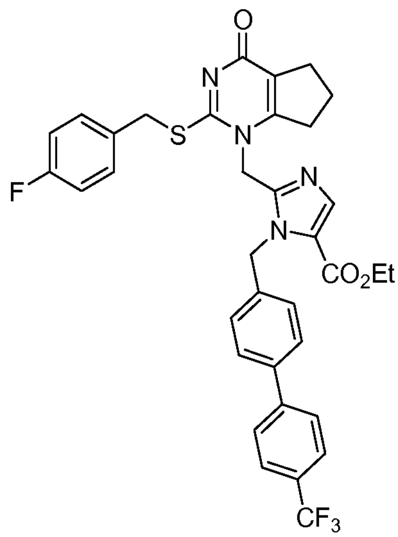

Following a procedure similar to that described for the preparation of example 28 except that intermediate C44 was used in place of intermediate C9. $^1$H-NMR ($CDCl_3$, 300 MHz) δ1.35 (t, 3H, *J*=6.9), 2.02 (m, 2H), 2.71 (m, 4H), 4.32

(q, 2H, *J*=7.2), 4.41 (s, 2H), 5.03 (s, 2H), 5.74 (s, 2H), 6.89 (t, 2H, *J*=8.4), 7.05 (d, 2H, *J*=7.8), 7.27 (dd, 2H, *J*=8.1, 5.4), 7.46 (d, 2H, *J*=7.8), 7.59 (d, 2H, *J*=8.1), 7.69 (d, 2H, *J*=8.1), 7.75 (s, 1H).

**Example 51-2-((2-(4-fluorobenzylthio)-4-oxo-4,5,6,7-tetrahydro-1H-cyclopenta[d]pyrimidin-1 -yl)methyl)-1-((4'-(trifluoromethyl)biphenyl-4-yl)methyl)-1H-imidazole-5-carbox ylic acid**

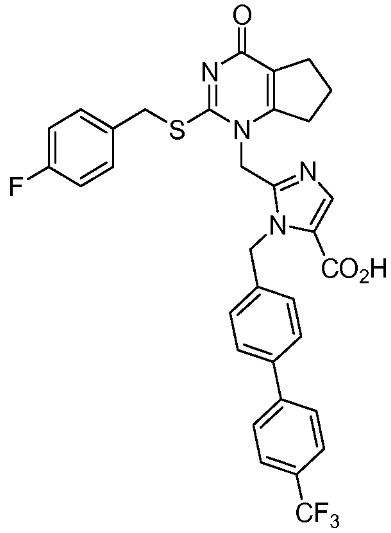

To an ice cooled solution of example 50 (110mg) in EtOH (3ml) was added NaOH (0.5ml, 10% aq. solution) and the mixture was stirred at room temperature for 2h. TLC detection showed that the reaction was complete. Then ice water (15ml) was added and the solution was acidified to pH 5~6 with concd, HCl. The precipitate thus obtained was collected by filtration and dried to give the title compound (60mg). $^1$H-NMR ($d_6$-DMSO, 300 MHz) δ1.91 (m, 2H), 2.56 (t, 2H, *J*=7.2), 2.73 (t, 2H, *J*=7.2), 4.29 (s, 2H), 5.21 (s, 2H), 5.72 (s, 2H), 7.01 (t, 2H, *J*=8.7), 7.14 (d, 2H, *J*=8.1), 7.31 (dd, 2H, *J*=8.4, 5.7), 7.62 (d, 2H, *J*=7.8), 7.83 (m, 5H), 12.98 (s, 1H).

**Example 52-2-((2-(4-fluorobenzylthio)-4-oxo-4,5,6,7-tetrahydro-1H-cyclopenta[d]pyrimidin-1 -yl)methyl)-N,N-dimethyl-1-((4'-(trifluoromethyl)biphenyl-4-yl)methyl)-1H-imid** azole-5-carboxamide

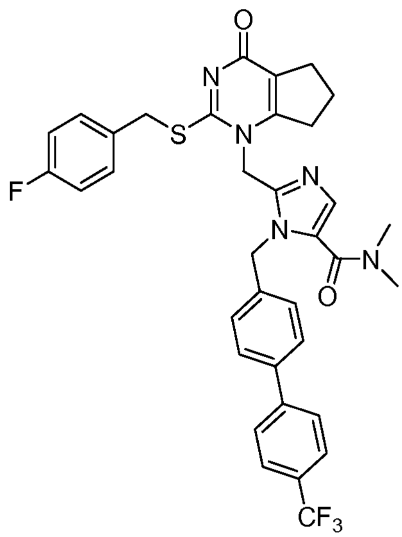

A mixture of example 51 (40mg), EDCI (18mg), HOBt (13mg), dimethylamine hydrochloride (8mg) and DIPEA (17μl) in DCM (5ml) was stirred at room temperature for 4h. Then, the solution was washed with saturated $NH_4Cl$ solution twice, dried over anhydrous $NaSO_4$ and purified by preparative TLC to give the title compound as a white solid (20mg). $^1H$-NMR ($CDCl_3$, 300 MHz) δ2.01 (m, 2H), 2.75 (m, 4H), 3.07 (s, 3H), 3.09 (s, 3H), 4.44 (s, 2H), 4.96 (s, 2H), 5.49 (s, 2H), 6.89 (t, 2H, *J*=8.4), 7.11 (d, 2H, *J*=7.8), 7.27 (m, 3H), 7.46 (d, 2H, *J*=7.8), 7.59 (d, 2H, *J*=8.1), 7.70 (d, 2H, *J*=8.7).

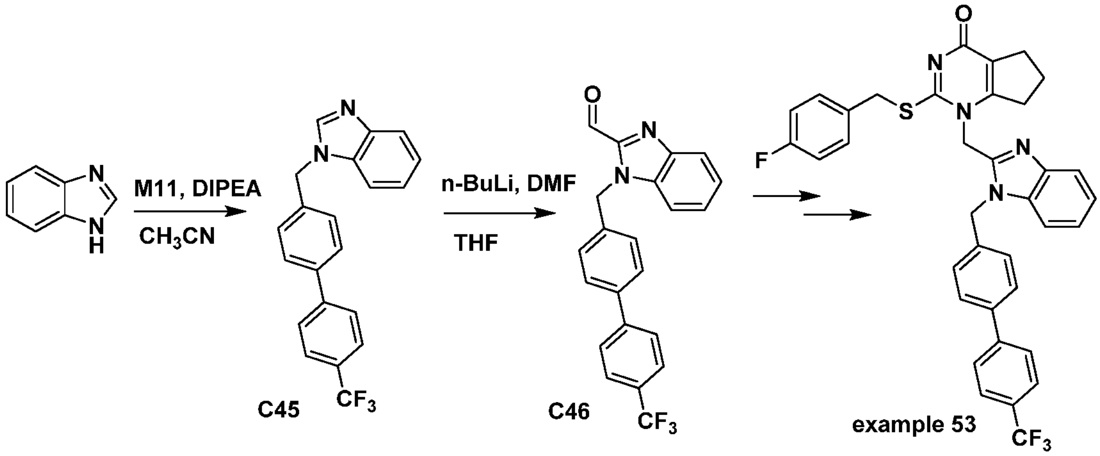


C45 C46 example 53

**Intermediate C45-1-((4'-(trifluoromethyl)biphenyl-4-yl)methyl)-1H-benzo[d]imidazole**

A mixture of 1H-benzo[d]imidazole (519mg, 4.4mmol, 1.1equiv), intermediate M11 (1.26g, 4mmol, lequiv) and DIPEA (730mg, 1.1equiv) in acetonitrile (10ml) was refluxed for 4h under nitrogen. TLC detection showed that the reaction was complete. The mixture was then filtered to remove the insoluble substances and the filtrate was mixed with silica gel, evaporated and then purified by column chromatography (PE/EA=1:2) to give the title compound as a white solid (590mg). $^1H$-NMR ($CDCl_3$, 300 MHz) δ5.43 (s, 2H), -7.29 (m, 5H), 7.57 (d, 2H, *J*=8.1), 7.67 (m, 4H), 7.86 (m, 1H), 8.00 (s, 1H); MS (ESI): 353(M+H).

**Intermediate C46-1-((4'-(trifluoromethyl)biphenyl-4-yl)methyl)-1H-benzo[d]imidazole-2-carbaldeh yde**

Following a procedure similar to that described for the preparation of intermediate C17 except that intermediate C45 was used in place of intermediate C16. $^1H$-NMR ($CDCl_3$, 300 MHz) δ5.92 (s, 2H), 7.27 (d, 2H, *J*=8.1), 7.42-7.53 (m, 5H), 7.62 (d, 2H, *J*=8.7), 7.67 (d, 2H, *J*=8.4), 7.98 (m, 1H), 10.17 (s, 1H).

**Example 53-2-(4-fluorobenzylthio)-1-((1-((4'-(trifluoromethyl)biphenyl-4-yl)methyl)-1H-benz o[d]imidazol-2-yl)methyl)-6,7-dihydro-1H-cyclopenta[d]pyrimidin-4(5H)-one**

Following a procedure similar to that described for the preparation of example 28 except that intermediate C46 was used in place of intermediate C3. $^1H$-NMR ($CDCl_3$, 300 MHz) δ1.95 (m, 2H), 2.68 (m, 4H), 4.46 (s, 2H), 5.22 (s, 2H), 5.44 (s, 2H), 6.90 (t, 2H, *J*=8.7), 7.00 (d, 2H, *J*=7.8), 7.26-7.37 (m, 5H), 7.43 (d, 2H, *J*=7.8), 7.60 (d, 2H, *J*=8.4), 7.69 (d, 2H, *J*=8.4), 7.82 (m, 2H); MS (ESI) found (M+H) = 641.

**Example 54-2-(4-fluorobenzylthio)-5-((1-methyl-1H-pyrazol-4-yl)methyl)-1-((1-((4'-(trifluoro methyl)biphenyl-4-yl)methyl)-1H-benzo[d]imidazol-2-yl)methyl)pyrimidin-4(1H) -one**

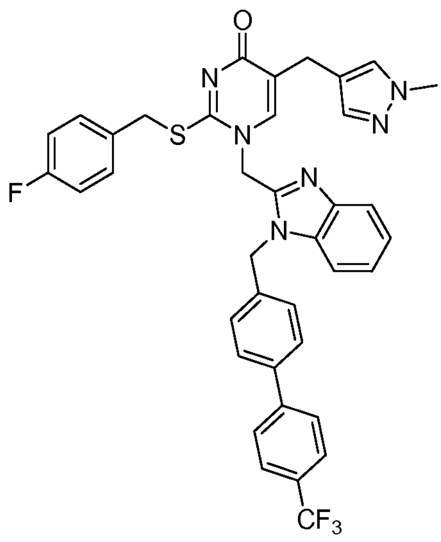

Following a procedure similar to that described for the preparation of example 29 except that intermediate C46 was used in place of intermediate C3. $^1$H-NMR ($CDCl_3$, 300 MHz) $\delta$3.40 (s, 2H), 3.74 (s, 3H), 4.45 (s, 2H), 5.11 (s, 2H), 5.40 (s, 2H), 6.91-6.99 (m, 5H), 7.19 (s, 1H), 7.22 (s, 1H), -7.35 (m, 7H), 7.60 (d, 2H, *J*=7.5), 7.70 (d, 2H, *J*=7.5), 7.84 (m, 1H); MS (ESI): 695(M+H).

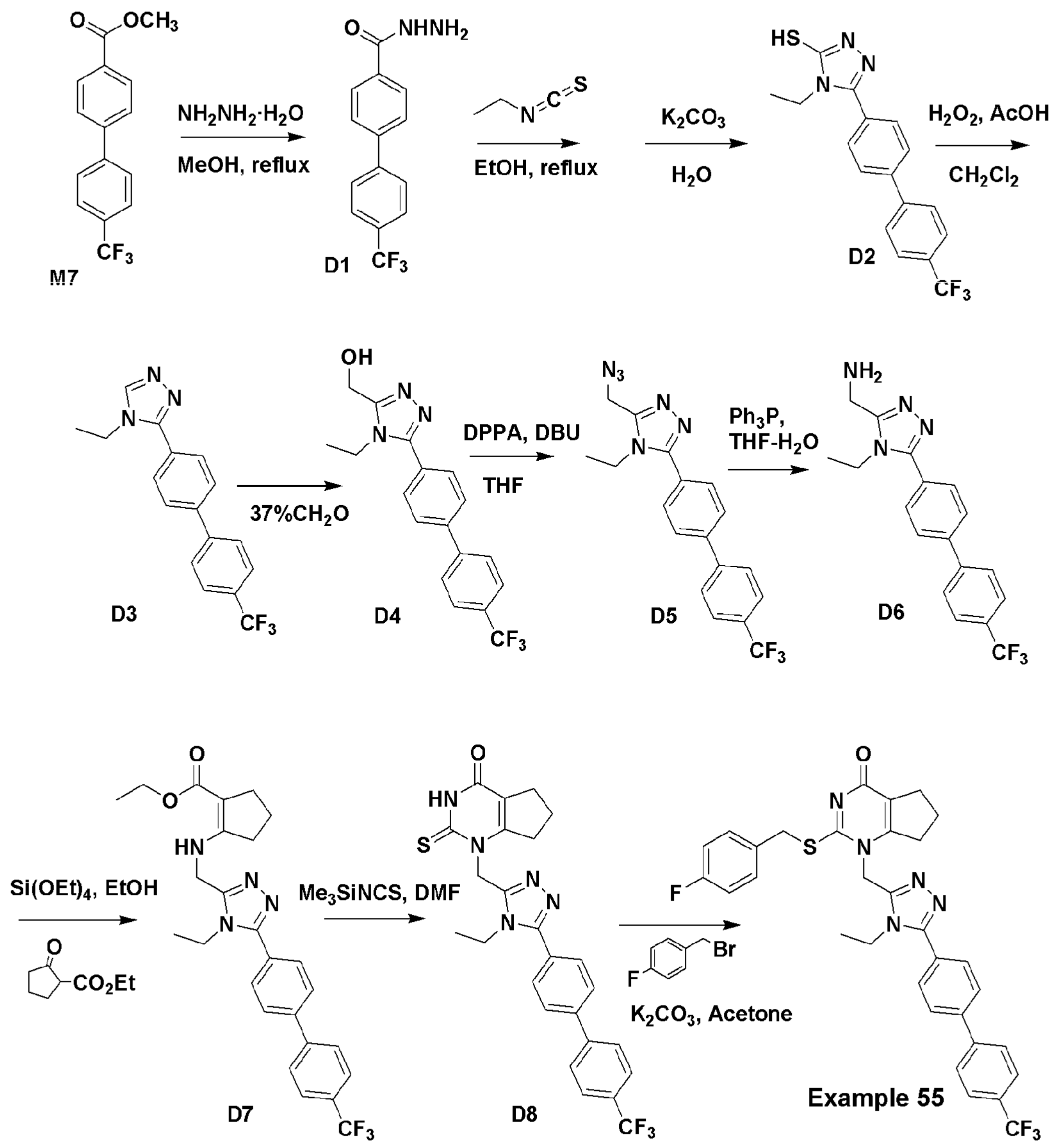


M7, D1, D2, D3, D4, D5, D6, D7, D8, Example 55

**Intermediate D1-4'-(trifluoromethyl)biphenyl-4-carbohydrazide**

To a solution of intermediate M7 (10.5g) in methanol (30ml) was added hydrazine hydrate solution (20ml, 85% m/m) and the mixture was refluxed for 4h before it was evaporated to dryness. Toluene was added and evaporated again to remove the residual water to give the title compound as a white solid. $^1$H-NMR (CDCl$_3$, 300 MHz) δ 4.15 (s, 2H), 7.53 (s, 1H), 7.67 (d, 2H, *J*=8.1), 7.71 (s, 4H), 7.85 (d, 2H, *J*=8.1); MS (EI) *m*/*z*: 280 (M$^+$).

**Intermediate D2-4-ethyl-5-(4'-(trifluoromethyl)biphenyl-4-yl)-4H-1,2,4-triazole-3-thiol**

A mixture of intermediate D1 (5.6g, lequiv) and isothiocyanatoethane (1.76ml, lequiv) in absolute ethanol (30ml) was refluxed for 2.5h. TLC detection showed that the starting material disappeared. Then most of the solvent was evaporated under reduced pressure, followed by addition of K$_2$CO$_3$ (3.26g, 1.1equiv) and water (30ml). The resulting mixture was refluxed for a further 2h and then neutralized with concd.HCl in an ice bath. The white precipitate thus obtained was collected by filtration, washed with water and dried to give the title compound (6.3g). $^1$H-NMR (d$_6$-DMSO, 300 MHz) δ1.90 (t, 3H, *J*=7.2), 4.09 (q, 2H, *J*=7.2), 7.82 (d, 2H, *J*=8.4), 7.85 (d, 2H, *J*=9.3), 7.94 (d, 2H, J=8.4), 7.98 (d, 2H, *J*=8.1), 13.99 (s, 1H); MS (EI) *m*/*z*: 349 (M$^+$).

**Intermediate D3-4-ethyl-3-(4'-(trifluoromethyl)biphenyl-4-yl)-4H-1,2,4-triazole**

To a slurry of intermediate D2 (6.3g, lequiv) in DCM (30ml) was added AcOH (1.5ml) and the mixture was

heated in an oil bath (35°C). An aqueous solution of hydrogen peroxide (5.1ml, 30% wt, >2.5equiv) was added slowly over 0.5h. After the addition, the mixture was stirred at room temperature for a further 0.5h. Then, the solution was adjusted to pH >10 with a solution of NaOH (10%) in an ice bath and extracted with DCM twice. The combined organic phase was washed with brine twice, dried over anhydrous $MgSO_4$ and purified by column chromatography to give the title compound as a solid (4.5g). $^1$H-NMR ($CDCl_3$, 300 MHz) δ1.47 (t, 3H, *J*=7.4), 4.15 (q, 2H, *J*=7.2), 7.73 (d, 8H), 8.30 (s, 1H); MS (EI) *m*/*z*: 317 ($M^+$).

**Intermediate D4-**(4-ethyl-5-(4'-(trifluoromethyl)biphenyl-4-yl)-4H-1,2,4-triazol-3-yl)methanol

A solution of intermediate D3 (4.5g) in formalin (20ml, 37% wt) was refluxed for 12h to obtain a clear solution. After cooled, the solution was diluted with saturated brine (20ml) and extracted with DCM three times (totally 100ml). The combined organic phase was washed with brine four times, dried over $MgSO_4$, filtered and solvent concentrated to let a batch of product precipitate. The solid so obtained was collected by filtration and dried to give the title compound (2.0g). The mother liquid was purified by column chromatography to give another batch of product (2.3g) as a white solid. $^1$H-NMR ($CD_3OD$, 300 MHz) δ1.33 (t, 3H, *J*=7.2), 4.28 (q, 2H, *J*=7.2), 4.85 (s, 2H), 7.78 (d, 4H, *J*=8.1), 7.90 (d, 4H, *J*=7.8); MS (EI) *m*/*z*: 347 ($M^+$).

**Intermediate D5-3-(azidomethyl)-4-ethyl-5-(4'-(trifluoromethyl)biphenyl-4-yl)-4H-1,2,4-triazole**

A mixture of intermediate D4 (1.04g, lequiv), DPPA (0.77ml, 1.2equiv) and DBU (0.67ml, 1.5equiv)) in THF (10ml) was refluxed for 3h. TLC detection showed that the reaction was complete. After cooled, the mixture was then poured into saturated $NH_4Cl$ solution and extracted with EA. The combined organic phase was washed with brine twice, dried and then purified by column chromatography (DCM/MeOH=20:1) to give the title compound as a solid (0.95g). $^1$H-NMR ($CDCl_3$, 300 MHz) δ1.38 (t, 3H, *J*=7.4), 4.15 (q, 2H, *J*=7.4), 4.64 (s, 2H), 7.74 (d, 8H); MS (EI) *m*/*z*: 372 ($M^+$).

**Intermediate D6-**(4-ethyl-5-(4'-(trifluoromethyl)biphenyl-4-yl)-4H-1,2,4-triazol-3-yl)methanamine

To a solution of D5 (0.94g, lequiv) in THF-$H_2O$ (8-0.5ml) was added $Ph_3P$ (990mg, 1.5equiv) and the mixture was stirred at room temperature overnight. After the reaction was complete, the mixture was then mixed with silica gel, evaporated to dryness and loaded on a short silica column. The column was first eluted with EA to get rid of $Ph_3P$ and $Ph_3P$=O and then eluted with methanol to afford the target product portion. The methanol solution so collected was evaporated to dryness and the residue was dissolved in a mixed solvent of DCM/MeOH (15:1). Insoluble silica gel was removed by filtration and the filtrate was evaporated to dryness to give the title compound as a yellow solid (0.68g). $^1$H-NMR ($CDCl_3$, 300 MHz) δ1.35 (t, 3H, *J*=7.5), 1.75 (s, 2H), 4.13 (s, 2H), 4.15 (q, 2H, *J*=7.4), 7.72 (s, 8H); MS (EI) *m*/*z*: 346 ($M^+$).

Intermediate **D7-**ethyl **2-((4-ethyl-5-(4'-(trifluoromethyl)biphenyl-4-yl)-4H-1,2,4-triazol-3-yl)methylami no)cyclopent-1-enecarboxylate**

To a solution of D6 (0.67g, 1equiv) in absolute ethanol (10ml) were added ethyl 2-oxocyclopentanecarboxylate (320μl, 1.1equiv) and tetraethyl orthosilicate (860μl, 2equiv). The mixture was refluxed under nitrogen for 5h until the completion of the reaction monitored by TLC. Silica gel was added to the solution and the mixture was evaporated to dryness, then purified by column chromatography (DCM/MeOH=30:1) to give the title compound as a solid (0.88g). $^1$H-NMR ($CDCl_3$, 300 MHz) δ1.25 (t, 3H, *J*=7.2), 1.34 (t, 3H, *J*=7.2), 1.86 (m, 2H), 2.52 (t, 2H, *J*=7.2), 2.74 (t, 2H, *J*=7.6), 4.13 (q, 2H, *J*=7.2), 4.65 (d, 2H, *J*=6.6), 7.73 (m, 9H); MS (EI) *m*/*z*: 484 ($M^+$).

**Intermediate D8-1-((4-ethyl-5-(4'-(trifluoromethyl)biphenyl-4-yl)-4H-1,2,4-triazol-3-yl)methyl)-2-t hioxo-2,3,6,7-tetrahydro-1H-cyclopenta[d]pyrimidin-4(5H)-one**

A mixture of D7 (0.80g, 1equiv), isothiocyanatotrimethylsilane (0.9ml, 4equiv) in anhydrous DMF (2ml) was heated at 140°C for 8h under nitrogen. The flask was cooled in an ice bath and the mixture was quenched by addition of saturated $NaHCO_3$ solution, diluted with EA (15ml) and water (10ml), and then stirred for 2h. The precipitate thus obtained was collected and dried to give the title compound (0.53g). $^1$H-NMR ($d_6$-DMSO, 300 MHz) δ1.35 (t, 3H, *J*=7.2), 2.06 (m, 2H), 2.67 (t, 2H, *J*=7.5), 3.07 (t, 2H, *J*=7.2), 4.20 (q, 2H, *J*=7.2), 5.68 (s, 2H), 7.79 (d, 2H, *J*=8.4), 7.86 (d, 2H, *J*=8.7), 7.94 (d, 2H, *J*=8.4), 7.99 (d, 2H, *J*=8.1); MS (EI) *m*/*z*: 497 ($M^+$).

**Example 55-1-((4-ethyl-5-(4'-(trifluoromethyl)biphenyl-4-yl)-4H-1,2,4-triazol-3-yl)methyl)-2-( 4-fluorobenzylthio)-6,7-dihydro-1H-cyclopenta[d]pyrimidin-4(5H)-one**

A mixture of D8 (100mg, 1equiv), 1-(bromomethyl)-4-fluorobenzene (28μl, 1.1equiv) and anhydrous $K_2CO_3$ (40mg, 1.5equiv) in acetone (3ml) was refluxed for 1h. Inorganic salts were removed by filtration and the filtrate was purified by column chromatography (DCM/MeOH=10:1) to give the title compound as a solid (60mg). $^1$H-NMR ($CDCl_3$, 300 MHz) δ1.30 (t, 3H, *J*=7.2), 2.13 (m, 2H), 2.86 (t, 2H, *J*=7.4), 3.05 (t, 2H, *J*=7.6), 4.10 (q, 2H, *J*=7.2), 4.53 (s, 2H), 5.24 (s, 2H), 6.97 (t, 2H, *J*=8.7), 7.35 (dd, 2H, *J*=8.7, 5.3), 7.67-7.76 (m, 8H); MS (ESI): 606(M+H).

**Example 56-1-((4-ethyl-5-(4'-(trifluoromethyl)biphenyl-4-yl)-4H-1,2,4-triazol-3-yl)methyl)-2-( 4-methoxybenzylthio)-6,7-dihydro-1H-cyclopenta[d]pyrimidin-4(5H)-one**

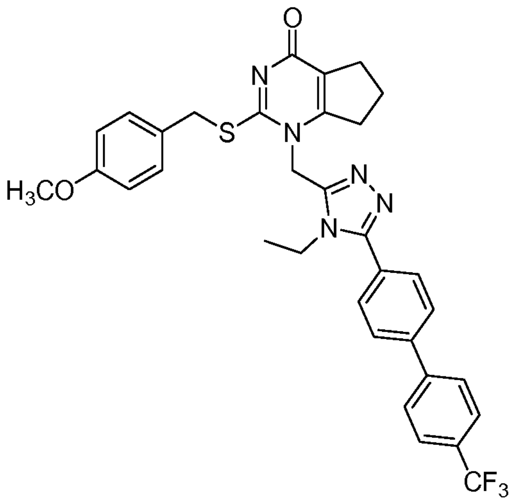

Following a procedure similar to that described for the preparation of example 55 except that 1-(chloromethyl)-4-methoxybenzene was used in place of 1-(bromomethyl)-4-fluorobenzene. $^1$H-NMR ($CDCl_3$, 300 MHz) δ1.28 (t, 3H, *J*=7.2), 2.12 (m, 2H), 2.85 (t, 2H, *J*=7.4), 3.05 (t, 2H, *J*=7.6), 3.76 (s, 3H), 4.10 (q, 2H, *J*=7.2), 4.51 (s, 2H), 5.24 (s, 2H), 6.81 (d, 2H, *J*=8.4), 7.29 (d, 2H, *J*=8.7), 7.73 (m, 8H); MS (ESI): 618(M+H).

**Example 57-2-(2,3-difluorobenzylthio)-1-((4-ethyl-5-(4'-(trifluoromethyl)biphenyl-4-yl)-4H-1, 2,4-triazol-3-yl)methyl)-6,7-dihydro-1H-cyclopenta[d]pyrimidin-4(5H)-one**

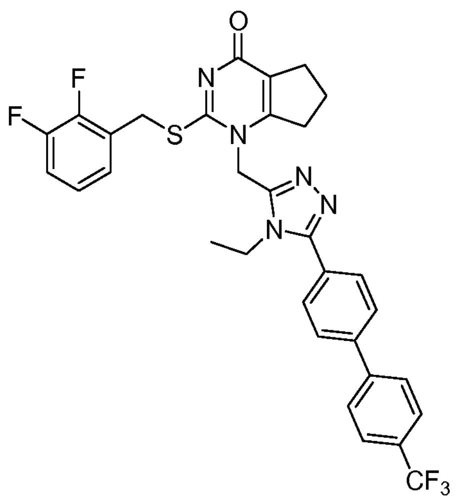

Following a procedure similar to that described for the preparation of example 55 except that 1-(bromomethyl)-2,3-difluorobenzene was used in place of 1-(bromomethyl)-4-fluorobenzene. $^1$H-NMR ($CDCl_3$, 300 MHz) δ 1.31 (t, 3H), 2.13 (m, 2H), 2.86 (t, 2H), 3.06 (t, 2H), 4.13 (q, 2H), 4.61 (s, 2H), 5.23 (s, 2H), 7.04 (m, 2H), 7.35 (t, 1H), 7.74 (m, 8H).

Example **58-1-((4-ethyl-5-(4'-(trifluoromethyl)biphenyl-4-yl)-4H-1,2,4-triazol-3-yl)methyl)-2-( 4-fluorobenzylthio)-5-((1-methyl-1H-pyrazol-4-yl)methyl)pyrimidin-4(1H)-one**

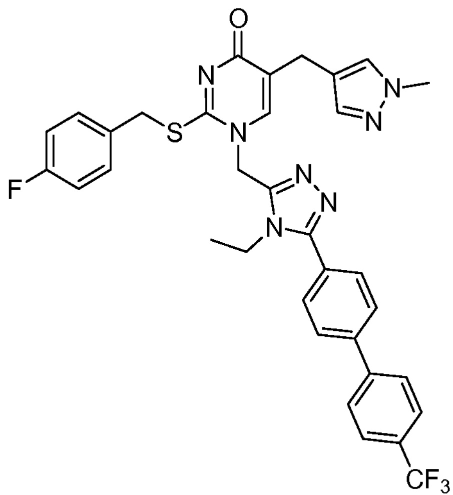

Following a procedure similar to that described for the preparation of example 29 except that intermediate D6 was used in place of intermediate C3. $^1$H-NMR($CDCl_3$, 300MHz) δ 1.16 (t, 3H, *J*=7.2), 3.56 (s, 2H), 3.83 (s, 3H), 4.02 (q, 2H, *J*=7.2), 4.55 (s, 2H), 5.16 (s, 2H), 6.99 (t, 2H, *J*=8.4), 7.21 (s, 1H), 7.30 (s, 1H), 7.32 (s, 1H), 7.40 (m, 2H), 7.72 (m, 8H); MS (ESI): 660(M+H).

**Intermediate D9-methyl 4-phenylbutanoate**

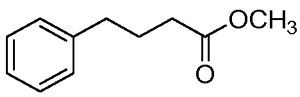

To a stirred solution of 4-phenylbutanoic acid (3.28g, 20mmol) in absolute methanol (20ml) was added concd. $H_2SO_4$ (2ml) dropwise (heat was released) and the mixture was refluxed for 4h. Then, most of the solvent was evaporated and ice water (30ml) was added to the residue. The resulting mixture was neutralized with a solution of NaOH (10%) and extracted with DCM (60ml) three times. The combined organic phase was dried over $MgSO_4$ and solvent evaporated to dryness to give the title compound as an oil which could be used in next step without further purification. $^1$H-NMR ($CDCl_3$, 300MHz) δ 1.96 (m, 2H), 2.33 (t, 2H, *J*=7.5), 2.65 (t, 2H, *J*=7.5), 3.66 (s, 3H), -7.25 (m, 5H).

**Intermediate D10-4-phenylbutanehydrazide**

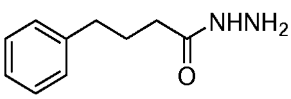

Following a procedure similar to that described for the preparation of intermediate D1 except that intermediate D9 was used in place of intermediate M7. H-NMR ($CD_3OD$, 300 MHz) δ1.97 (m, 2H), 2.14 (t, 2H, *J*=7.2), 2.64 (t, 2H, *J*=7.2), 3.89 (vbrs, 2H), 6.80 (vbrs, 1H), 7.17 (m, 3H), 7.27 (m, 2H).

**Intermediate D11-2-(4'-(trifluoromethyl)biphenyl-4-yl)acetohydrazide**

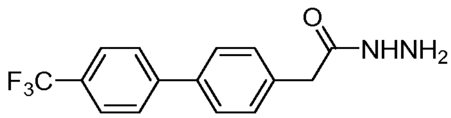

Following a procedure similar to that described for the preparation of intermediate D1 except that intermediate M3 was used in place of intermediate M7. $^1$H-NMR ($d_6$-DMSO, 300 MHz) δ 3.41 (s, 2H), 4.26 (s, 2H, $-NH_2$), 7.39 (d, 2H), 7.67 (d, 2H), 7.80 (d, 2H), 7.87 (d, 2H), 9.26 (s, 1H)

**Example 59-1-((4-ethyl-5-(3-phenylpropyl)-4H-1,2,4-triazol-3-yl)methyl)-2-(4-tluorobenzylthi o)-6,7-dihydro-1H-cyclopenta[d]pyrimidin-4(5H)-one**

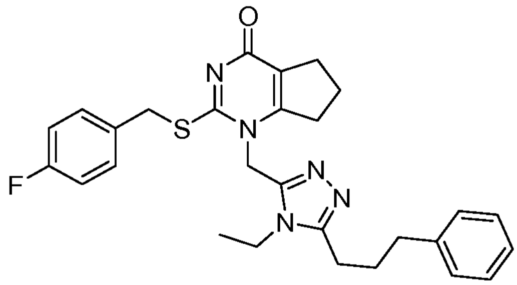

Following a procedure similar to that described for the preparation of example 55 except that intermediate D10 was used in place of intermediate D1. $^1$H-NMR ($CDCl_3$, 300MHz) δ 1.13 (t, 3H), 2.13 (m, 4H), 2.66 (t, 2H), 2.78 (m, 4H), 2.95 (t, 2H), 3.79 (q, 2H), 4.49 (s, 2H), 5.12 (s, 2H), 6.95 (t, 2H), 7.18 (m, 3H), 7.28 (m, 2H), 7.33 (dd, 2H); MS (ESI): 504(M+H).

**Example 60-1-((4-ethyl-5-((4'-(trifluoromethyl)biphenyl-4-yl)methyl)-4H-1,2,4-triazol-3-yl)me thyl)-2-(4-fluorobenzylthio)-6,7-dihydro-1H-cyclopenta[d]pyrimidin-4(5H)-one**

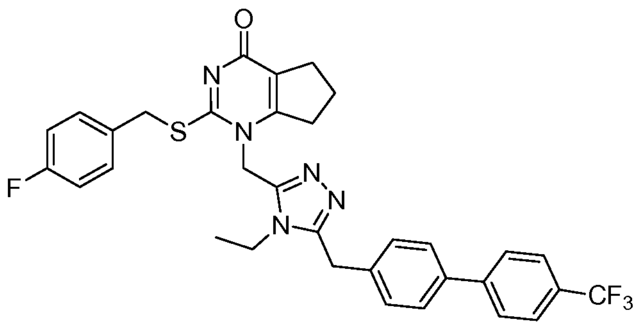

Following a procedure similar to that described for the preparation of example 55 except that intermediate D11 was used in place of intermediate D1. $^1$H-NMR ($CDCl_3$, 300 MHz) δ1.02 (t, 3H, *J*=7.2), 2.11 (m, 2H), 2.83 (t, 2H, *J*=7.5), 2.98 (t, 2H, *J*=7.5), 3.82 (q, 2H, *J*=7.2), 4.23 (s, 2H), 4.49 (s, 2H), 5.11 (s, 2H), 6.95 (t, 2H, *J*=8.7), 7.31 (m, 4H), 7.53 (d, 2H, *J*=8.1), 7.64 (d, 2H, *J*=8.4), 7.69 (d, 2H, *J*=8.7); MS (ESI): 620 (M+H).

**Intermediate D12-1-((4'-(trifluoromethyl)biphenyl-4-yl)methyl)-1H-1,2,4-triazole**

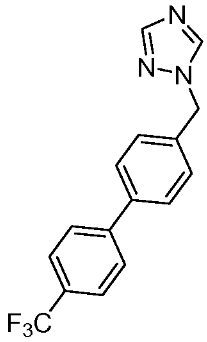

A mixture of 4-(bromomethyl)-4'-(trifluoromethyl)biphenyl (1.25g), 1H-1,2,4-triazole (0.4g) and DIPEA (0.9ml) in acetonitrile (15ml) was refluxed for 3h. Then insoluble substance was filtered off and the filtrate was mixed with silica gel and evaporated t dryness and then purified by column chromatography to give the title compound as a solid (0.54g). $^1$H-NMR ($CD_3OD$, 300 MHz) δ5.49 (s, 2H), 7.42 (d, 2H, *J*=8.1), 7.68 (d, 2H, *J*=8.1), 7.72 (d, 2H, *J*=8.4), 7.79 (d, 2H, *J*=8.4), 8.02 (s, 1H), 8.60 (s, 1H).

**Intermediate D13-(1-((4'-(trifluoromethyl)biphenyl-4-yl)methyl)-1H-1,2,4-triazol-3-yl)methanol**

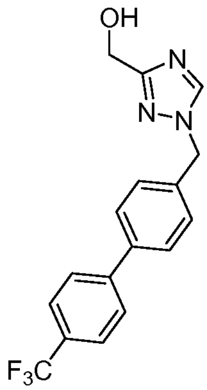

Following a procedure similar to that described for the preparation of intermediate D4. $^1$H-NMR ($CDCl_3$, 300 MHz) δ3.91 (s, 1H), 4.79 (s, 2H), 5.48 (s, 2H), 7.37 (d, 2H, *J*=8.4), 7.58 (d, 2H, *J*=8.4), 7.64 (d, 2H, *J*=8.7), 7.69 (d, 2H, *J*=8.7), 7.84 (s, 1H).

**Example 61-2-(4-fluorobenzylthio)-1-((1-((4'-(trifluoromethyl)biphenyl-4-yl)methyl)-1H-1,2,4-triazol-3-yl)methyl)-6,7-dihydro-1H-cyclopenta[d]pyrimidin-4(5H)-one**

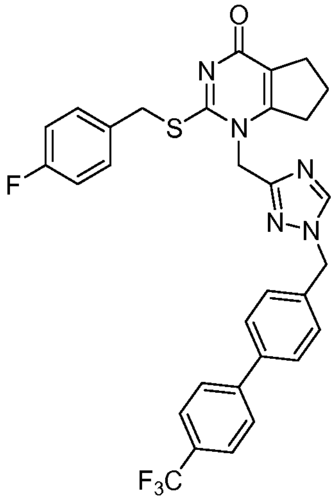

Following a procedure similar to that described for the preparation of example 55 except that intermediate D13 was used in place of intermediate D4. $^1$H-NMR ($CDCl_3$, 300 MHz) δ1.99 (m, 2H), 2.71 (m, 4H), 4.45 (s, 2H), 5.10 (s, 2H), 5.46 (s, 2H), 6.92 (t, 2H, *J*=7.8), 7.18 (d, 2H, *J*=8.1), 7.29 (m, 2H), 7.50 (d, 2H, *J*=7.8), 7.62 (d, 2H, *J*=7.8), 7.70 (d, 2H, *J*=7.8), 7.92 (s, 1H).

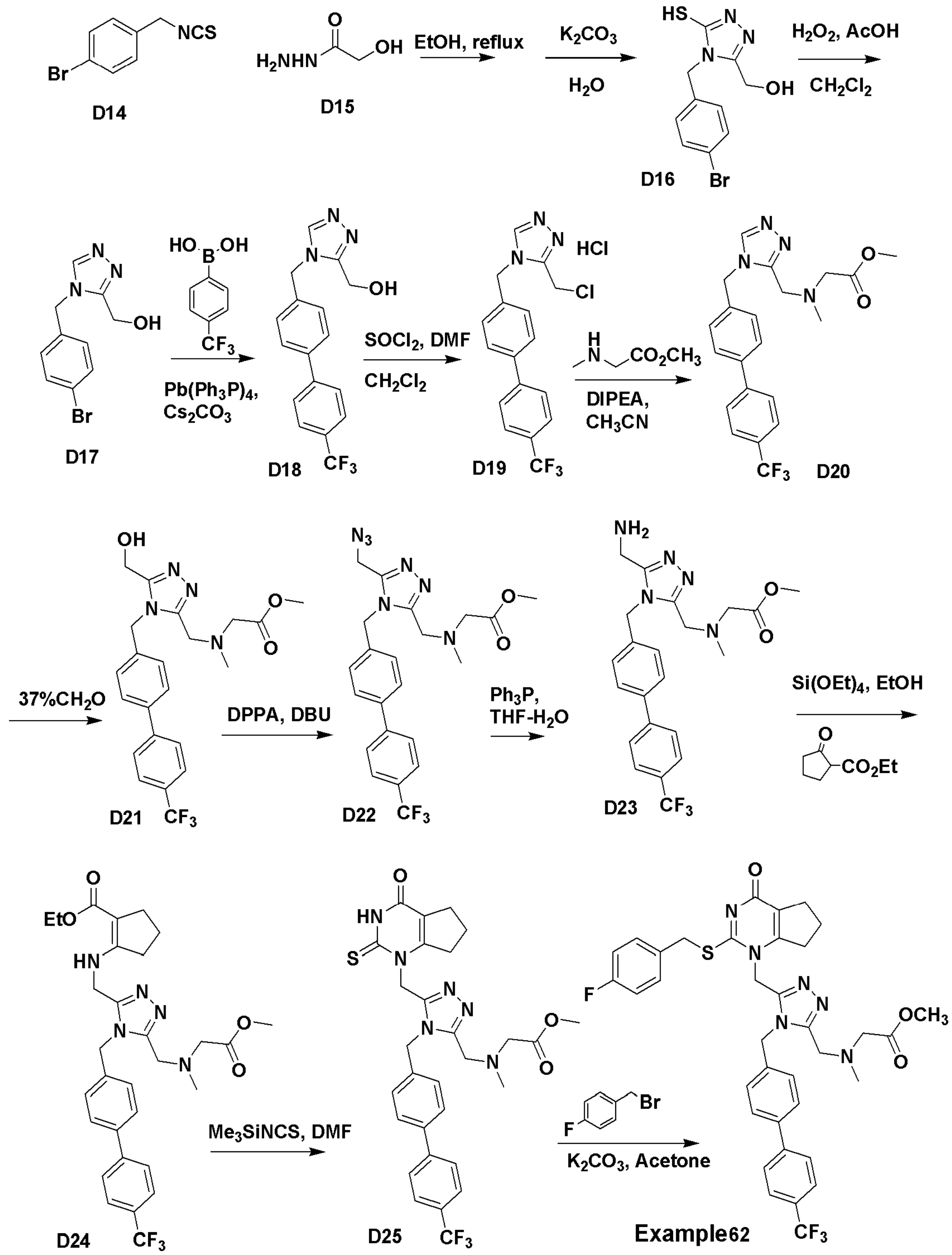


**Intermediate D14-1-bromo-4-(isothiocyanatomethyl)benzene**

To an ice cold mixture of (4-bromophenyl)methanamine (22.8g, 0.1mol, 1equiv) and triethylamine (17.5ml, 3.3equiv) in THF (100ml) in a flask equipped with a drying tube for insulating moisture was added a solution of $CS_2$ (6ml, 1equiv) in THF (25ml) over 30min to obtain a white slurry. The mixture was stirred at room temperature for 1h and then placed in ice bath again. Tosyl chloride (20.9g, 1.1equiv) was added in batches and the mixture was stirred for a further 1h before it was diluted with ice water. The solution was adjusted to pH~5 with concd. HCl and extracted with EA. The combined organic phase was washed with brine, dried and evaporated to give the title compound as a yellow oil which could be used in next step without further purification.

**Intermediate D15-2-hydroxyacetohydrazide**

A mixture of methyl 2-hydroxyacetate (9g, 0.1mol, 1equiv) and hydrazine hydrate (9.6ml, 1.5equiv, 85%) in methanol (100ml) was refluxed for 8h before methanol and exceesive hydrazine hydrate were evaporated. Toluene was added and evaporated again to remove the residual water to give the title compound as a white solid which could be used in next step without further purification.

**Intermediate D16-(4-(4-bromobenzyl)-5-mercapto-4H-1,2,4-triazol-3-yl)methanol**

A mixture of D14 (0.1mol) and D15 (0.1mol) from the previous steps in ethanol (200ml) was refluxed for 2h and a lot of white precipitates separated out. Then most of the solvent was evaporated. Water (200ml) and $K_2CO_3$ (20.7g, 1.5equiv) was added to the residue and the mixture was refluxed for a further 1h. After cooled, the solution was placed in an ice bath and adjusted to pH 8~9 with conc. HCl. A lot of white solid precipitated (if a yellow solid was present, a small amount of DCM could be added) and was collected by filtration, washed with water several times and DCM several times to wash off the color and dried in vacuo to give the title compound as a white solid (2.5g). $^1$H-NMR ($d_6$-DMSO, 300 MHz) δ4.37 (s, 2H), 5.24 (s, 2H), 5.73 (s, 1H, -OH), 7.25 (d, 2H, *J*=8.4), 7.54 (d, 2H, *J*=8.7), 13.80 (s, 1H).

**Intermediate D17-(4-(4-bromobenzyl)-4H-1,2,4-triazol-3-yl)methanol**

To a suspension of D16 (25g, 1equiv) in DCM (100ml) was added AcOH (6ml) and the mixture was heated in an oil bath (35°C). An aqueous solution of hydrogen peroxide (24ml, >2.5equiv) was added slowly over 1h. After the addition, the mixture was stirred at room temperature for a further 0.5h before it was adjusted to pH>10 with a solution of NaOH (10%) in an ice bath. The mixture was stayed for 0.5h and the precipitate so formed was collected by filtration, washed with water and DCM. Organic layer of the filtrate was separated and concentrated. Solids precipitated and was collected by filtration and dried to obtain an off white powder (20.4g in total). $^1$H-NMR ($d_6$-DMSO, 300 MHz) δ4.55 (s, 2H), 5.27 (s, 2H), 5.64 (s, 1H, -OH), 7.22 (d, 2H, *J*=8.4), 7.57 (d, 2H, *J*=8.4), 8.53 (s, 1H).

**Intermediate D18-(4-((4'-(trifluoromethyl)biphenyl-4-yl)methyl)-4H-1,2,4-triazol-3-yl)methanol**

A mixture of intermediate C17 (13.4g, 50mmol, 1equiv), 4-(trifluoromethyl)phenylboronic acid (10.4g, 1.1equiv), $Cs_2CO_3$ (32.6g, 2equiv) and $Pd(PPh)_3$ (2.9g, 0.05equiv) in dioxane (100ml) was refluxed for 18h in a nitrogen atmosphere. The inorganic salt was filtered off, washed with dioxane and the filtrate was evaporated to dryness to give a residue that was recrystallized from DCM/MeOH to give the title compound as a yellow powder (13g). $^1$H-NMR ($d_6$-DMSO, 300 MHz) δ4.59 (d, 2H, *J*=5.4), 5.37 (s, 2H), 5.69 (t, 1H, *J*=5.4), 7.40 (d, 2H, *J*=8.1), 7.75 (d, 2H, *J*=8.4), 7.81 (d, 2H, *J*=8.4), 7.89 (d, 2H, *J*=8.4), 8.59 (s, 1H); MS (ESI): 334(M+H).

**Intermediate D19-3-(chloromethyl)-4-((4'-(trifluoromethyl)biphenyl-4-yl)methyl)-4H-1,2,4-triazole Hydrochloride**

To an ice cold mixture of D18 (5mmol) and DMF (2drops, catalyst) in DCM (20ml) in a 100 ml of flask was added a solution of thionyl chloride(0.42ml, 6mmol) in DCM (5ml) over 10min and the mixture was stirred at room temperature. TLC monitored the progress of the reaction and showed that the reaction was complete about 2 hours later. (Handling Method 1) The solid thus formed was collected by filtration, washed with DCM and dried AT 80 °C to give the title compound as a white or light yellow solid. (Handling Method 2) The filtrate was evaporated to dryness to give the title compound as a yellow solid which could be used in next step without further purification. $^1$H-NMR ($d_6$-DMSO, 300 MHz) δ5.06 (s, 2H), 5.46 (s, 2H), 7.47 (d, 2H, *J*=8.0), 7.77 (d, 2H, *J*=8.4), 7.80 (d, 2H, *J*=8.8), 7.89 (d, 2H, *J*=8.0), 9.12 (s, 1H).

**Intermediate D20-methyl 2-(methyl((4-((4'-(trifluoromethyl)biphenyl-4-yl)methyl)-4H-1,2,4-triazol-3-yl)me thyl)amino)acetate**

A mixture of D19 (2.0g, 1equiv), methyl 2-(methylamino)acetate hydrochloride (0.86g, 1.2equiv) and DIPEA (3ml, 3equiv) in acetonitrile (20ml) was heated at 70~80°C until the completion of the reaction monitored by TLC. Acetonitrile was evaporated and saturated $NH_4Cl$ solution (40ml) was added to the residue. The mixture was extracted with DCM twice and the combined organic phase was washed with brine twice, dried, and then purified by column chromatography to give the title compound as a jelly (1.13g). $^1$H-NMR ($CDCl_3$, 300 MHz) δ2.38 (s, 3H), 3.32 (s, 2H), 3.70 (s, 3H), 3.86 (s, 2H), 5.51 (s, 2H), 7.31 (d, 2H, *J*=8.4), 7.60 (d, 2H, *J*=8.1), 7.68 (m, 4H), 8.16 (s, 1H).

**Intermediate D21-methyl 2-(((5-(hydroxymethyl)-4-((4'-(trifluoromethyl)biphenyl-4-yl)methyl)-4H-1,2,4-tri azol-3-yl)methyl)(methyl)amino)acetate**

A solution of D20 (1.13g) in formalin (15ml, 37% wt) was refluxed for 5h. TLC detection showed that the reaction was complete. The solution was poured into saturated brine (40ml) and extracted with DCM three times (60ml). The combined organic phase was washed with brine three times, dried over $MgSO_4$, and then purified by column chromatography to give the title compound as a jelly (1.0g). $^1$H-NMR ($CDCl_3$, 400 MHz) δ2.33 (s, 3H), 3.26 (s, 2H), 3.66 (s, 3H), 3.75 (s, 2H), 4.74 (s, 2H), 5.62 (s, 2H), 7.26 (d, 2H, *J*=8.0), 7.56 (d, 2H, *J*=8.0), 7.65 (d, 2H, *J*=8.8), 7.69 (d, 2H, *J*=8.8).

**Intermediate D22-methyl 2-(((5-(azidomethyl)-4-((4'-(trifluoromethyl)biphenyl-4-yl)methyl)-4H-1,2,4-triaz ol-3-yl)methyl)(methyl)amino)acetate**

A mixture of D21 (1.0g, 1equiv), DPPA (0.58ml, 1.2equiv) and DBU (0.44ml, 1.3equiv) in THF (10ml) was refluxed for 3h under nitrogen. TLC detection showed that the reaction was complete. After cooled, the mixture was then poured into saturated $NH_4Cl$ solution and extracted with EA. The combined organic phase was washed with brine twice, dried and then purified by flash chromatography to give the title compound as a jelly (0.97g). $^1$H-NMR ($CDCl_3$, 400 MHz) δ2.44 (s, 3H), 3.36 (s, 2H), 3.70 (s, 3H), 3.88 (s, 2H), 4.47 (s, 2H), 5.63 (s, 2H), 7.22 (d, 2H, *J*=8.0), 7.61 (d, 2H, *J*=8.0), 7.69 (d, 2H, *J*=8.4), 7.72 (d, 2H, *J*=8.8).

**Intermediate D23-methyl 2-(((5-(aminomethyl)-4-((4'-(trifluoromethyl)biphenyl-4-yl)methyl)-4H-1,2,4-tria zol-3-yl)methyl)(methyl)amino)acetate**

To a solution of D22 (0.97g, 1equiv) in THF-$H_2O$ (8-0.5ml) was added $Ph_3P$ (806mg, 1.5equiv) and the mixture was stirred at room temperature overnight. After the reaction was complete, the mixture was then mixed with silica gel, evaporated to dryness and loaded on a short silica column. The column was first eluted with EA to get rid of $Ph_3P$ and $Ph_3P$=O and then eluted with methanol to afford the target product portion. The methanol solution so collected was evaporated to dryness and the residue was dissolved in a mixed solvent of DCM/MeOH (15:1). Insoluble silica gel was removed by filtration and the filtrate was evaporated to dryness to give the title compound as a jelly (0.8g). $^1$H-NMR ($CDCl_3$, 400 MHz) δ2.37 (s, 2H +3H), 3.30 (s, 2H), 3.69 (s, 3H), 3.81 (s, 2H), 4.01 (s, 2H), 5.62 (s, 2H), 7.23 (d, 2H, *J*=8.4), 7.59 (d, 2H, *J*=8.0), 7.69 (m, 4H).

**Intermediate D24-ethyl 2-((5-(((2-methoxy-2-oxoethyl)(methyl)amino)methyl)-4-((4'-(trifluoromethyl)biphenyl-4-yl)methyl)-4H-1,2,4-triazol-3-yl)methylamino) cyclopent-1-enecarboxylate**

To a solution of D23 (0.8g, 1equiv) in absolute ethanol (10ml) were added ethyl 2-oxocyclopentanecarboxylate (260μl, 1equiv) and tetraethyl orthosilicate (800μl, 2equiv). The mixture was refluxed under nitrogen for 3h until the completion of the reaction monitored by TLC. Silica gel was added to the solution and the mixture was evaporated to dryness, then purified by column chromatography (DCM/MeOH=40:1) to give the title compound as a jelly (0.89g). $^1$H-NMR ($CDCl_3$, 400 MHz) δ1.21 (t, 3H, *J*=7.2), 1.75 (m, 2H), 2.36 (s, 3H), 2.50 (m, 4H), 3.29 (s, 2H), 3.65 (s, 3H), 3.82 (s, 2H), 4.06 (q, 2H, *J*=7.5), 4.36 (d, 2H, *J*=6.0), 5.56 (s, 2H), 7.15 (d, 2H, *J*=8.1), 7.56 (m, 3H), 7.68 (m, 4H); MS (ESI): 586(M+H).

**Intermediate D25-methyl 2-(methyl((5-((4-oxo-2-thioxo-2,3,4,5,6,7-hexahydro-1H-cyclopenta[d]pyrimidin-1-yl)methyl)-4-((4'-(trifluoromethyl)biphenyl-4-yl)methyl)-4H-1,2,4-triazol-3-yl) methyl)amino)acetate**

A mixture of D24 (0.89g, 1equiv), isothiocyanatotrimethylsilane (0.77ml, 4equiv) in anhydrous DMF (1ml) was heated at 140°C for 6h under nitrogen. After cooled, the mixture was placed in an ice bath and quenched by addition of saturated $NaHCO_3$ solution, and then diluted with EA (15ml) and water (10ml), and then stirred for 2h. The precipitate thus obtained was collected and dried to give the title compound (0.12g). $^1$H-NMR ($d_6$-DMSO, 300 MHz) δ1.94 (m, 2H), 2.24 (s, 3H), 2.50 (t, 2H), 2.83 (t, 2H, *J*=6.9), 3.35 (s, 2H), 3.57 (s, 3H), 3.81 (s, 2H), 5.50 (s, 2H), 5.61 (s, 2H), 7.26 (d, 2H, *J*=8.1), 7.71 (d, 2H, *J*=8.1), 7.81 (d, 2H, *J*=8.4), 7.87 (d, 2H, *J*=9.0), 12.55 (s, 1H).

**Example 62-methyl 2-(((5-((2-(4-fluorobenzylthio)-4-oxo-4,5,6,7-tetrahydro-1H-cyclopenta[d]pyrimid in-1-yl)methyl)-4-((4'-(trifluoromethyl)biphenyl-4-yl)methyl)-4H-1,2,4-triazol-3-y l)methyl)(methyl)amino)acetate**

A mixture of D25 (120mg, 1equiv), 1-(bromomethyl)-4-fluorobenzene (28μl, 1.1equiv) and anhydrous $K_2CO_3$ (55mg, 2equiv) in acetone (3ml) was refluxed for 1h. Inorganic salts were removed by filtration and the filtrate was purified by flash chromatography to give the title compound as a solid (65mg). $^1$H-NMR ($CDCl_3$, 400 MHz) δ1.98 (m, 2H), 2.43

(s, 3H), 2.67 (t, 2H, *J*=7.2), 2.76 (t, 2H, *J*=7.2), 3.38 (s, 2H), 3.66 (s, 3H), 3.95 (s, 2H), 4.43 (s, 2H), 5.03 (s, 2H), 5.64 (s, 2H), 6.92 (t, 2H, *J*=8.0), 7.00 (d, 2H, *J*=7.6), 7.30 (m, 2H), 7.46 (d, 2H, *J*=7.6), 7.61 (d, 2H, *J*=8.0), 7.71 (d, 2H, *J*=8.0).

**Example 63-2-(((5-((2-(4-fluorobenzylthio)-4-oxo-4,5,6,7-tetrahydro-1H-cyclopenta[d]pyrimid in-1-yl)methyl)-4-((4'-(trifluoromethyl)biphenyl-4-yl)methyl)-4H-1,2,4-triazol-3-y l)methyl)(methyl)amino)acetic acid**

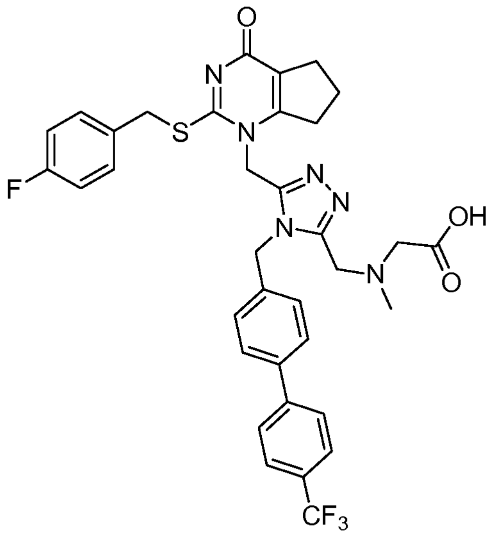

To an ice cold solution of example 62 (40mg) in the solvent of *i*-PrOH-$CH_2Cl_2$-$H_2O$ (1ml-0.5ml-3 ml) was added 10% NaOH solution (0.1ml) and the mixture was stirred for 1h. Then it was diluted with water (5ml), transferred into a separating funnel and washed with DCM (3ml). The aqueous phase was collected and neutralized with a diluted solution of AcOH. The precipitate thus obtained was collected by filtration, washed with an aqueous acetone solution (50%, v/v) twice and dried to give the title compound as a white solid (12mg). $^1$H-NMR ($CD_3OD$, 300 MHz) δ2.06 (m, 2H), 2.41 (s, 3H), 2.70 (t, 2H, *J*=7.2), 2.84 (t, 2H, *J*=6.9), 3.38 (s, 2H), 4.00 (s, 2H), 4.35 (s, 2H), 5.26 (s, 2H), 5.67 (s, 2H), 6.86 (t, 2H, *J*=8.7), 7.23-7.31 (m, 4H), 7.56 (d, 2H, *J*=7.8), 7.73 (m, 4H); MS (ESI): 691(M-H).

**Intermediate D26-4-(4-bromobenzyl)-3-methyl-4H-1,2,4-triazole**

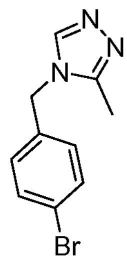

A mixture of 1,1-dimethoxy-N,N-dimethylmethanamine (2.6ml) and acethydrazide (2g) in acetonitrile (3ml) was heated at 60 °C for 0.5h before (4-bromophenyl)methanamine (3.7g) and AcOH (4.5ml) were added. The mixture was stirred at 120°C for a further 3h. After cooled, the mixture was poured into water and extracted with EA twice. The combined organic phase was dried over $MgSO_4$ and then purified by column chromatography to give the title compound as a white solid (1.1g). $^1$H-NMR ($CDCl_3$, 300 MHz) δ2.36 (s, 3H), 5.04 (s, 2H), 6.97 (d, 2H, *J*=8.4), 7.50 (d, 2H, *J*=8.4), 8.08 (s, 1H).

**Intermediate D27- 3-methyl-4-((4'-(trifluoromethyl)biphenyl-4-yl)methyl)-4H-1,2,4-triazole**

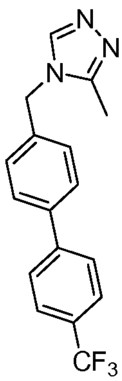

A mixture of intermediate D26 (2.0g, 1equiv), 4-(trifluoromethyl)phenylboronic acid (1.6g, 1.05equiv), $Cs_2CO_3$ (5.2g, 2equiv) and $Pd(PPh)_3$ (0.5g, 0.05equiv) in dioxane (15ml) was refluxed for 12h in a nitrogen atmosphere. The inorganic salts were filtered off and washed with dioxane. The filtrate was concentrated and the precipitate thus obtained was collected, and then dried to give the title compound (1.65g). $^1$H-NMR ($CDCl_3$, 300 MHz) δ2.42 (s, 3H), 5.15 (s, 2H), 7.21 (d, 2H, *J*=8.4), 7.61 (d, 2H, *J*=8.1), 7.66 (d, 2H, *J*=8.7), 7.70 (d, 2H, *J*=9.0), 8.13 (s, 1H).

**Intermediate D28- (5-methyl-4-((4'-(trifluoromethyl)biphenyl-4-yl)methyl)-4H-1,2,4-triazol-3-yl)met hanol**

Following a procedure similar to that described for the preparation of intermediate D21 except that intermediate D27 was used in place of intermediate D20. $^1$H-NMR ($CDCl_3$, 300 MHz) δ2.34 (s, 3H), 4.75 (s, 2H), 5.32 (s, 2H), 7.17 (d, 2H), 7.57 (d, 2H), 7.66 (d, 2H), 7.69 (d, 2H).

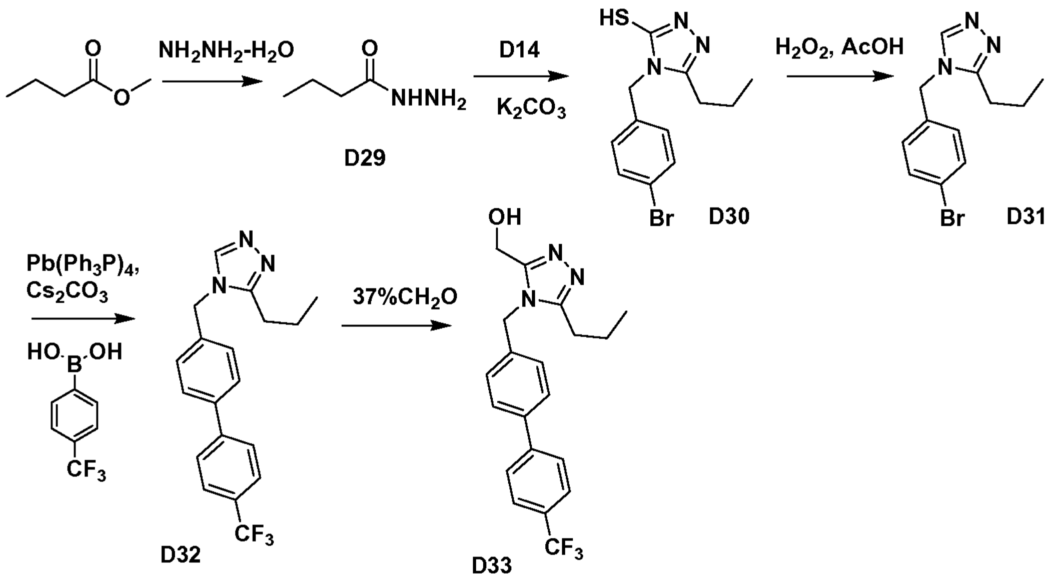


**Intermediate D29-Butyrohydrazide**

Following a procedure similar to that described for the preparation of intermediate D 15 except that methyl butyrate was used in place of methyl 2-hydroxyacetate.

**Intermediate D30-4-(4-bromobenzyl)-5-propyl-4H-1,2,4-triazole-3-thiol**

Following a procedure similar to that described for the preparation of intermediate D16 except that D29 was used in place of D15. H-NMR ($d_6$-DMSO, 300 MHz) δ0.83 (t, 3H, *J*=7.2), 1.49 (m, 2H), 2.48 (t, 2H, *J*=7.2), 5.21 (s, 2H), 7.22 (d, 2H, *J*=8.7), 7.56 (d, 2H, *J*=8.7), 13.69 (s, 1H).

**Intermediate D31-4-(4-bromobenzyl)-3-n-propyl-4H-1,2,4-triazole**

To a slurry of intermediate D30 (1.2g, 1equiv) in DCM (30ml) was added AcOH (0.3ml) and the mixture was heated in an oil bath (35°C). A solution of hydrogen peroxide (1.1ml, >2.5equiv) was added slowly over 10 minutes. After the addition, the mixture was stirred at room temperature for a further 0.5h. Then, the flask was placed in an ice bath and the solution was adjusted to pH>10 with a solution of NaOH (10%) and extracted with DCM twice. The combined organic phase was washed with brine twice, dried over anhydrous $MgSO_4$ and purified by column chromatography to give the title compound as a jelly (630mg). H-NMR ($CDCl_3$, 300 MHz) δ0.98 (t, 3H, *J*=7.2), 1.77 (m, 2H), 2.62 (t, 2H, *J*=7.2), 5.04 (s, 2H), 6.97 (d, 2H, *J*=8.7), 7.51 (d, 2H, *J*=8.7), 8.06 (s, 1H).

**Intermediate D32- 3-n-propyl-4-((4'-(trifluoromethyl)biphenyl-4-yl)methyl)-4H-1,2,4-triazole**

A mixture of intermediate D31 (0.62g, 1equiv), 4-(trifluoromethyl)phenylboronic acid (0.466g, 1.1equiv), $Cs_2CO_3$ (1.45g, 2equiv) and $Pd(PPh)_3$ (0.13g, 0.05equiv) in dioxane (8ml) was refluxed for 12h in a nitrogen atmosphere. The inorganic salts were filtered off and washed with dioxane. The filtrate was concentrated and mixed with silica gel and then purified by column chromatography to give a solid (0.62g). H-NMR ($CDCl_3$, 300 MHz) δ 1.00 (t, 3H), 1.81 (m, 2H), 2.68 (t, 2H), 5.15 (s, 2H), 7.20 (d, 2H), 7.61 (d, 2H), 7.69 (m, 4H), 8.13 (s, 1H).

**Intermediate D33- (5-n-propyl-4-((4'-(trifluoromethyl)biphenyl-4-yl)methyl)-4H-1,2,4-triazol-3-yl)m ethanol**

Following a procedure similar to that described for the preparation of intermediate D21 except that D32 was used in place of D20. H-NMR ($CDCl_3$, 400 MHz) δ0.97 (t, 3H), 1.76 (m, 2H), 2.62 (t, 2H), 4.66 (s, 1H), 4.76 (s, 2H), 5.35 (s, 2H), 7.18 (d, 2H), 7.60 (d, 2H), 7.68 (d, 2H), 7.72 (d, 2H).

**Intermediate D34- (5-isopropyl-4-((4'-(trifluoromethyl)biphenyl-4-yl)methyl)-4H-1,2,4-triazol-3-yl) methanol**

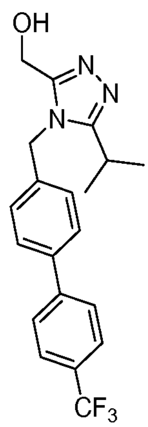

Following a procedure similar to that described for the preparation of intermediate D33 except that ethyl isobutyrate was used in place of methyl butyrate. H-NMR ($CDCl_3$, 400 MHz) δ1.30 (d, 6H), 2.30 (s, 1H), 2.92 (m, 1H), 4.67 (s, 2H), 5.34 (s, 2H), 7.13 (d, 2H), 7.59 (d, 2H), 7.68 (m, 4H).

**Intermediate D35- (5-cyclopropyl-4-((4'-(trifluoromethyl)biphenyl-4-yl)methyl)-4H-1,2,4-triazol-3-yl )methanol**

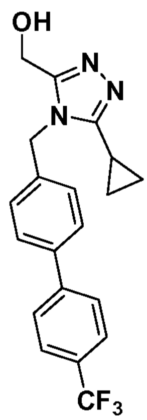

Following a procedure similar to that described for the preparation of intermediate D33 except that ethyl cyclopropanecarboxylate was used in place of methyl butyrate.

**Example 64- 2-(4-fluorobenzylthio)-1-((5-methyl-4-((4'-(trifluoromethyl)biphenyl-4-yl)methyl) -4H-1,2,4-triazol-3-yl)methyl)-6,7-dihydro-1H-cyclopenta[d]pyrimidin-4(5H)-one**

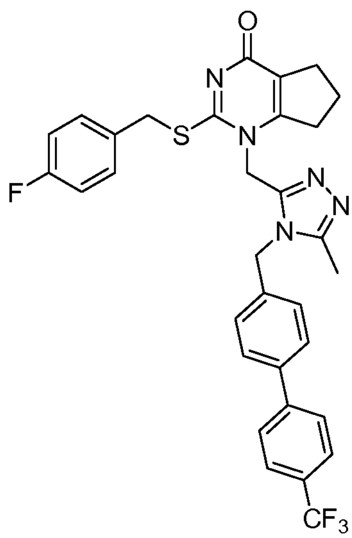

Following a procedure similar to that described for the preparation of example 62 except that D28 was used in place of D21. $^{1}H$-NMR ($CDCl_3$, 300 MHz) $\delta$1.92 (m, 2H), 2.50 (s, 3H), 2.56 (t, 2H, *J*=7.2), 2.73 (t, 2H, *J*=7.2), 4.46 (s, 2H), 5.11 (s, 2H), 5.18 (s, 2H), 6.82 (d, 2H, *J*=7.8), 6.95 (t, 2H, *J*=8.7), 7.34 (dd, 2H, *J*=5.4, 8.7), 7.44 (d, 2H, *J*=8.1), 7.62 (d, 2H, *J*=8.1), 7.70 (d, 2H, *J*=8.7); MS (ESI): 606(M+H).

**Example** 65- **2-(4-fluorobenzylthio)-5-((1-methyl-1H-pyrazol-4-yl)methyl)-1-((5-methyl-4-((4'-( trifluoromethyl)biphenyl-4-yl)methyl)-4H-1,2,4-triazol-3-yl)methyl)pyrimidin-4( 1H)-one**

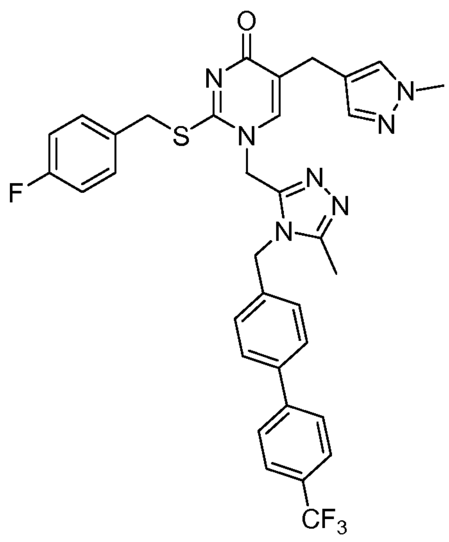

Following a procedure similar to that described for the preparation of example 29 except that D28 was used in place of C3. $^1$H-NMR ($CDCl_3$, 300 MHz) δ2.46 (s, 3H), 3.37 (s, 2H), 3.78 (s, 3H), 4.39 (s, 2H), 5.01 (s, 2H), 5.13 (s, 2H), 6.77 (d, 2H, *J*=8.1), 6.97 (m, 3H), 7.34 (m, 6H), 7.65 (m, 4H); MS (ESI): 660(M+H).

**Example 66- 2-(4-fluorobenzylthio)-1-((5-propyl-4-((4'-(trifluoromethyl)biphenyl-4-yl)methyl) -4H-1,2,4-triazol-3-yl)methyl)-6,7-dihydro-1H-cyclopenta[d]pyrimidin-4(5H)-one**

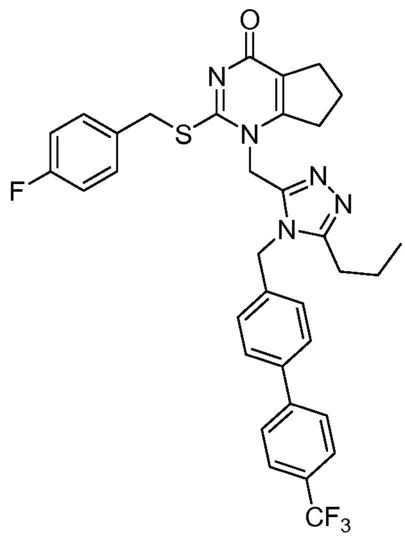

Following a procedure similar to that described for the preparation of example 62 except that D33 was used in place of D21. $^1$H-NMR ($CDCl_3$, 300 MHz) δ1.04 (t, 3H, *J*=7.5), 1.82-2.00 (m, 4H), 2.56 (t, 2H, *J*=7.2), 2.75 (m, 4H), 4.42 (s, 2H), 5.16 (s, 2H), 5.25 (s, 2H), 6.86 (d, 2H, *J*=8.1), 6.93 (t, 2H, *J*=8.7), 7.32 (dd, 2H, *J*=5.7, 8.4), 7.45 (d, 2H, *J*=7.8), 7.61 (d, 2H, *J*=8.1), 7.71 (d, 2H, *J*=8.4).

**Example** 67- **2-(4-fluorobenzylthio)-1-((5-isopropyl-4-((4'-(trifluoromethyl)biphenyl-4-yl)meth yl)-4H-1,2,4-triazol-3-yl)methyl)-6,7-dihydro-1H-cyclopenta[d]pyrimidin-4(5H)-o ne**

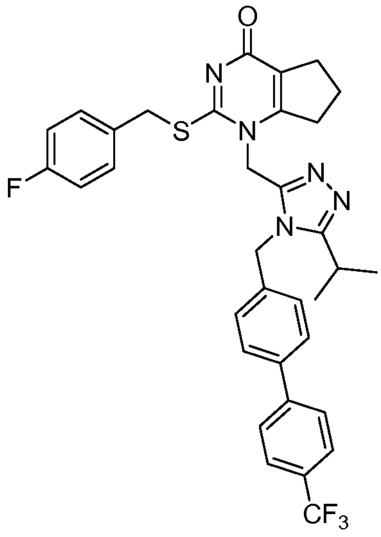

Following a procedure similar to that described for the preparation of example 62 except that D34 was used in place of D21. $^1$H-NMR ($CDCl_3$, 300 MHz) δ1.42 (d, 6H, *J*=6.9), 1.93 (m, 2H), 2.57 (t, 2H, *J*=7.5), 2.76 (t, 2H, *J*=7.5), 2.99 (m, 1H), 4.43 (s, 2H), 5.10 (s, 2H), 5.25 (s, 2H), 6.82 (d, 2H, *J*=8.1), 6.94 (t, 2H, *J*=8.7), 7.33 (dd, 2H, *J*=8.4, 5.4), 7.44 (d, 2H, *J*=7.8), 7.61 (d, 2H, *J*=8.4), 7.70 (d, 2H, *J*=8.7).

**Example 68- 1-((5-cyclopropyl-4-((4'-(trifluoromethyl)biphenyl-4-yl)methyl)-4H-1,2,4-triazol-3-yl)methyl)-2-(4-fluorobenzylthio)-6,7-dihydro-1H-cyclopenta[d]pyrimidin-4(5 H)-one**

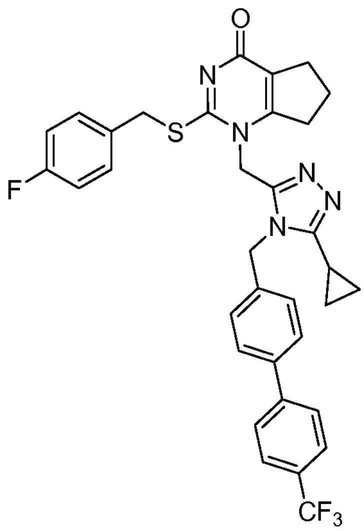

Following a procedure similar to that described for the preparation of example 62 except that D35 was used in place of D21. $^1$H-NMR ($CDCl_3$, 300 MHz) δ1.07 (m, 2H), 1.23 (m, 2H), 1.71 (m, 2H), 1.90 (m, 2H), 2.54 (t, 2H), 2.70 (t, 2H), 2.99 (m, 1H), 4.47 (s, 2H), 5.10 (s, 2H), 5.31 (s, 2H), 6.87 (d, 2H), 6.96 (t, 2H), 7.35 (dd, 2H), 7.45 (d, 2H), 7.63 (d, 2H), 7.71 (d, 2H).

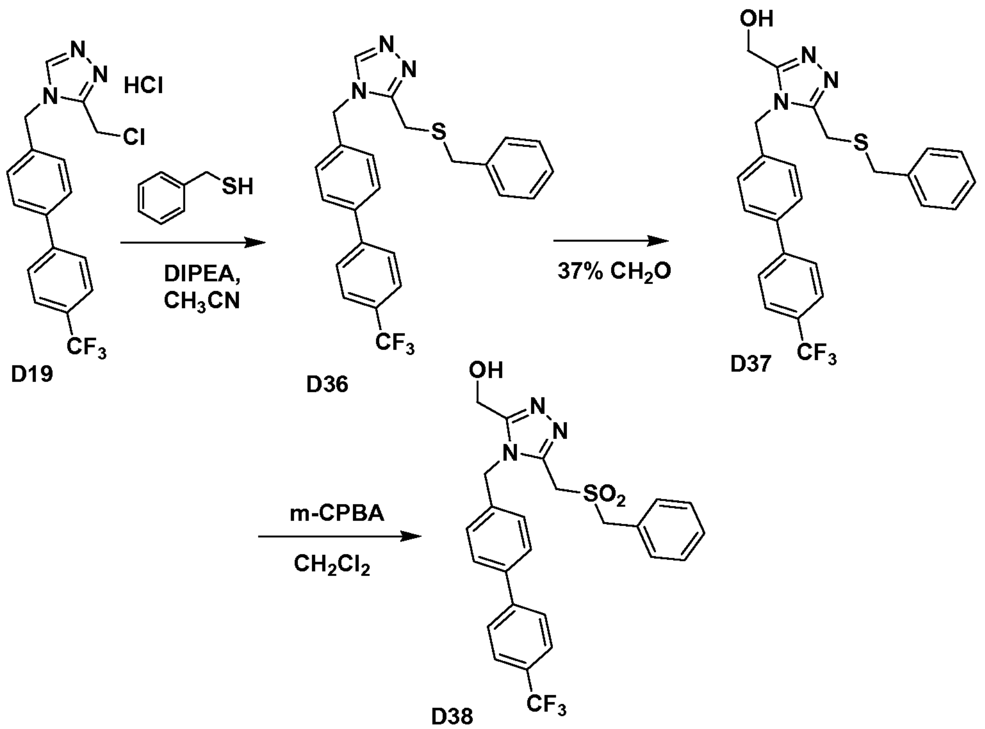


D19 D36 D37 D38

**Intermediate D36- 3-(benzylthiomethyl)-4-((4'-(trifluoromethyl)biphenyl-4-yl)methyl)-4H-1,2,4-tria zole**

A mixture of D19 (610mg), phenylmethanethiol (0.18ml) and DIPEA (0.65ml) in acetonitrile (10ml) was heated at 60°C for 3h. Acetonitrile was evaporated and the residue was dissolved in EA, washed with brine three times, dried over anhydrous $Na_2SO_4$ and purified by flash chromatography to give the title compound as a light yellow solid (630mg). $^1$H-NMR ($CDCl_3$, 300 MHz) δ3.70 (s, 2H), 3.71 (s, 2H), 5.22 (s, 2H), 7.21 (d, 2H, *J*=8.4), 7.33 (m, 5H), 7.65 (d, 2H, *J*=8.4), 7.70 (d, 2H, *J*=8.4), 8.16 (s, 1H).

**Intermediate D37- (5-(benzylthiomethyl)-4-((4'-(trifluoromethyl)biphenyl-4-yl)methyl)-4H-1,2,4-tria zol-3-yl)methanol**

Following a procedure similar to that described for the preparation of D21 except that D36 was used in place of D20. $^1$H-NMR ($d_6$-DMSO, 300 MHz) δ3.65 (s, 2H), 3.70 (s, 2H), 4.52 (d, 2H, *J*=5.7), 5.36 (s, 2H), 5.64 (t, 1H, *J*=5.7), 7.21 (d, 2H, *J*=8.1), 7.29 (m, 5H), 7.70 (d, 2H, *J*=8.4), 7.80 (d, 2H, *J*=8.4), 7.87 (d, 2H, *J*=8.7).

**Intermediate D38- (5-(benzylsulfonylmethyl)-4-((4'-(trifluoromethyl)biphenyl-4-yl)methyl)-4H-1,2,4 -triazol-3-yl)methanol**

To an ice cold suspension of D37 (0.7g, 1equiv) in DCM (15ml) was added m-CPBA (1.07g, 70-75 % pure, 3equiv) and the mixture was stirred at room temperature for 2h before it was quenched with saturated $NaHCO_3$ solution until no more bubbles was generated. The organic layer was separated and the aqueous phase was extracted with DCM again. The combined organic phase was washed with saturated brine, dried over anhydrous $Na_2SO_4$ and purified by flash chromatography to give the title compound as a white solid (470mg). $^1$H-NMR ($CDCl_3$, 300 MHz) δ2.90 (s, 1H), 4.18 (s, 2H), 4.37 (s, 2H), 4.87 (s, 2H), 5.54 (s, 2H), 7.19 (d, 2H, *J*=7.8), 7.36 (m, 3H), 7.53 (d, 2H, *J*=8.1), 7.58 (m, 4H), 7.67 (d, 2H, *J*=8.7).

**Example 69- 1-((5-(benzylsulfonylmethyl)-4-((4'-(trifluoromethyl)biphenyl-4-yl)methyl)-4H-1, 2,4-triazol-3-yl)methyl)-2-(4-fluorobenzylthio)-6,7-dihydro-1H-cyclopenta[d]pyri midin-4(5H)-one**

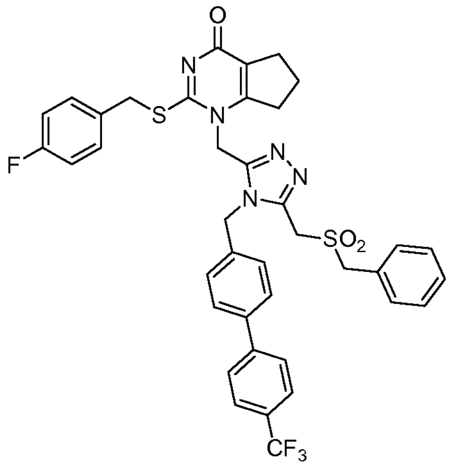

Following a procedure similar to that described for the preparation of example 62 except that D38 was used in place of D21. $^1$H-NMR ($CDCl_3$, 400 MHz) δ2.04 (m, 2H), 2.71 (t, 2H, *J*=7.2), 2.83 (t, 2H, *J*=7.2), 4.28 (s, 2H), 4.40 (s, 2H), 4.44 (s, 2H), 5.08 (s, 2H), 5.42 (s, 2H), 6.91 (t, 2H, *J*=8.4), 6.96 (d, 2H, *J*=8.0), 7.29 (dd, 2H, *J*=8.4, 5.6), 7.43 (m, 5H), 7.58 (d, 2H, *J*=8.4), 7.66 (m, 2H), 7.70 (d, 2H, *J*=8.0).

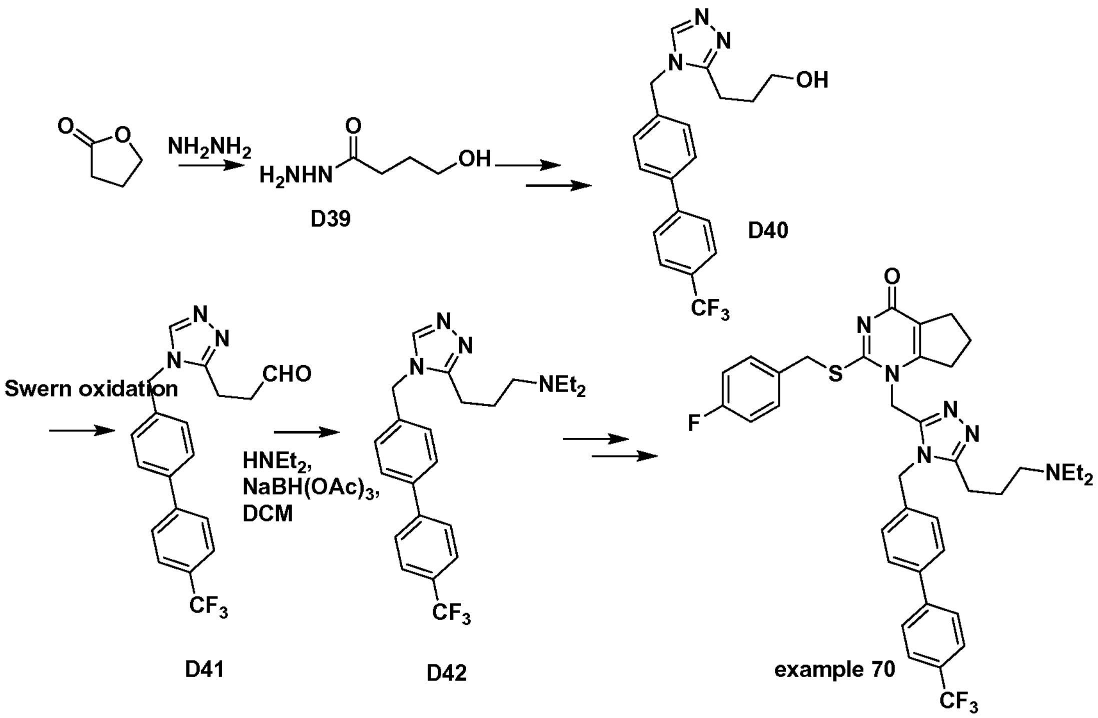


D39 D40 D41 D42 example 70

**Intermediate D39-4-hydroxybutanehydrazide**

Following a procedure similar to that described for the preparation of D15 except that dihydrofuran-2(3H)-one was used in place of methyl 2-hydroxyacetate. $^1$H-NMR ($d_6$-DMSO, 300 MHz) δ 1.61 (m, 2H), 2.03 (t, 2H), 3.45 (m, 2H), 4.13 (s, 2H), 4.44 (t, 1H, -OH), 8.90 (s, 1H).

**Intermediate D40- 3-(4-((4'-(trifluoromethyl)biphenyl-4-yl)methyl)-4H-1,2,4-triazol-3-yl)propan-1-o l**

Following a procedure similar to that described for the preparation of D32 except that D39 was used in place of D29. H-NMR ($CDCl_3$, 300 MHz) δ 2.06 (m, 2H), 2.85 (t, 2H), 3.74 (t, 2H), 5.18 (s, 2H), 7.23 (d, 2H), 7.62 (d, 2H), 7.68 (m, 4H), 8.14 (s, 1H), 9.86 (s, 1H)

**Intermediate D41- 3-(4-((4'-(trifluoromethyl)biphenyl-4-yl)methyl)-4H-1,2,4-triazol-3-yl)propanal**

To a solution of oxalyl chloride (205μl, 1.5equiv) in DCM (5ml) placed in a cold trap of -60°C under nitrogen was added a solution of DMSO (305μl, 3equiv) in DCM (5ml) over 5min via a constant pressure funnel, followed by addition of a solution of D40 (0.52g, 1equiv) in DCM (5ml) and DMSO (105μl) over 5min. The resulting mixture was stirred for 10min and then a solution of $NEt_3$ (800μl, 4equiv) in DCM (5ml) was added over 5 min. The reaction was stirred for a further 10min before it was quenched with saturated $NH_4Cl$ solution. The DCM layer was separated, washed with brine twice, dried over anhydrous $NaSO_4$ and purified by flash chromatography to give the title compound as a jelly (360mg). H-NMR ($CDCl_3$, 300 MHz) δ 2.93 (t, 2H), 3.19 (t, 2H), 5.24 (s, 2H), 7.21 (d, 2H), 7.61 (d, 2H), 7.68 (m, 4H), 8.13 (s, 1H)

**Intermediate D42-N,N-diethyl-3-(4-((4'-(trifluoromethyl)biphenyl-4-yl)methyl)-4H-1,2,4-triaz ol-3-yl)propan-1-amine**

To an ice cold solution of D41 (360mg, 1equiv) and diethylamine (115μl, 1.1equiv) in DCM (5ml) was added $NaBH(OAc)_3$ (318mg, 1.5equiv) and the mixture was stirred for 1h before it was quenched with saturated $NaHCO_3$ solution. The DCM layer was separated, dried over anhydrous $NaSO_4$ and purified by flash chromatography to give the title compound as a jelly (270mg). H-NMR ($CDCl_3$, 300 MHz) δ 1.12 (t, 6H), 2.13 (m, 2H), 2.78 (m, 8H), 5.19 (s, 2H), 7.22 (d, 2H), 7.60 (d, 2H), 7.68 (m, 4H), 8.14 (s, 1H).

**Example 70- 1-((5-(3-(diethylamino)propyl)-4-((4'-(trifluoromethyl)biphenyl-4-yl)methyl)-4H-1,2,4-triazol-3-yl)methyl)-2-(4-fluorobenzylthio)-6,7-dihydro-1H-cyclopenta[d]py rimidin-4(5H)-one**

Following a procedure similar to that described for the preparation of example 62 except that D42 was used in place of D20. $^1$H-NMR ($CDCl_3$, 300 MHz) δ 1.35 (t, 6H), 2.00 (m, 2H), 2.41 (m, 2H), 2.69 (t, 2H), 2.83 (t, 2H), 3.02 (t, 2H), 3.10 (q, 4H), 3.22 (t, 2H), 4.42 (s, 2H), 5.07 (s, 2H), 5.33 (s, 2H), 6.91 (t, 2H), 6.97 (d, 2H), 7.29 (dd, 2H), 7.44 (d, 2H), 7.59 (d, 2H), 7.70 (d, 2H); MS (ESI): 705(M+H).

**Example 71 1-((5-(4-(diethylamino)butyl)-4-((4'-(trifluoromethyl)biphenyl-4-yl)methyl)-4H-1, 2,4-triazol-3-yl)methyl)-2-(4-fluorobenzylthio)-6,7-dihydro-1H-cyclopenta[d]pyri midin-4(5H)-one**

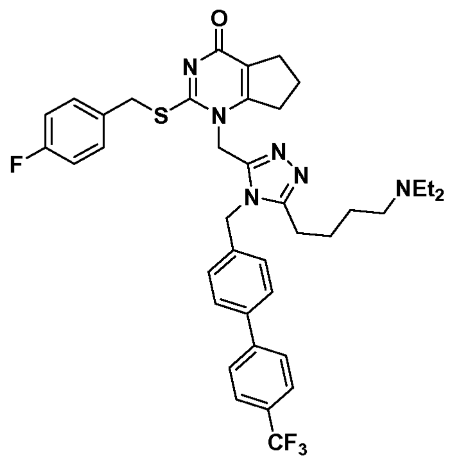

Following a procedure similar to that described for the preparation of example 70 except that tetrahydro-2H-pyran-2-one was used in place of dihydrofuran-2(3H)-one. $^1$H-NMR ($CDCl_3$, 300 MHz) δ 1.23 (t, 6H), 1.89 (m, 6H), 2.63 (t, 2H), 2.79 (m, 6H), 2.89 (q, 4H), 4.42 (s, 2H), 5.08 (s, 2H), 5.24 (s, 2H), 6.88 (d, 2H), 6.91 (t, 2H), 7.30 (dd, 2H), 7.44 (d, 2H), 7.59 (d, 2H), 7.70 (d, 2H); MS (ESI): 719$(M+H)^+$.

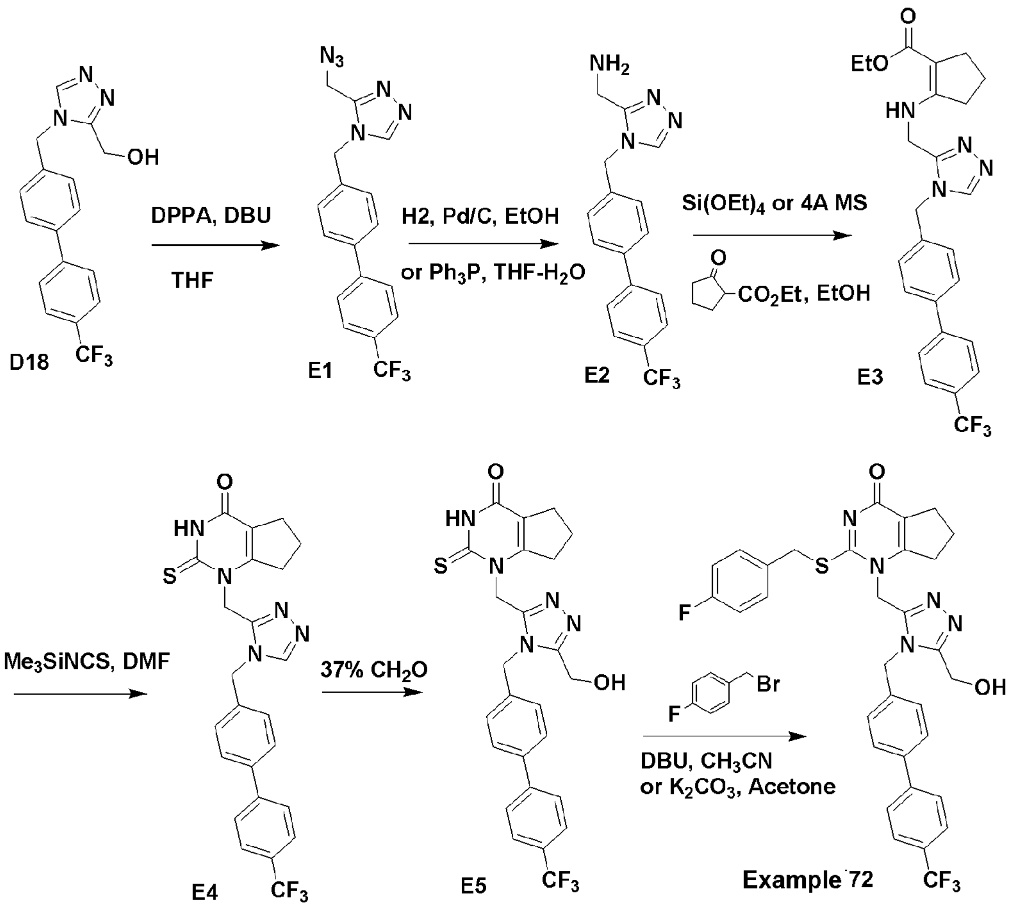


**Intermediate E1- 3-(azidomethyl)-4-((4'-(trifluoromethyl)biphenyl-4-yl)methyl)-4H-1,2,4-triazole**

A mixture of D18 (9.6g, 1equiv), DPPA (7.5ml, 1.2equiv) and DBU (5.6ml, 1.3equiv) in THF (100ml) was refluxed for 3h under nitrogen. TLC detection showed that the reaction was complete. After cooled, the mixture was then poured into saturated $NH_4Cl$ solution and extracted with EA. The combined organic phase was washed with brine twice, dried and then purified by flash chromatography to give the title compound as a jelly (8.7g). $^1$H-NMR ($CDCl_3$, 300 MHz) $\delta$ 4.54 (s, 2H), 5.31 (s, 2H), 7.28 (d, 2H), 7.62 (d, 2H), 7.66 (d, 2H), 7.70 (d, 2H), 8.40 (s, 1H).

**Intermediate E2- (4-((4'-(trifluoromethyl)biphenyl-4-yl)methyl)-4H-1,2,4-triazol-3-yl)methanamin e**

**(Method 1)** To a solution of E1 (10.6g, 1equiv) in THF-$H_2O$ (100-10ml) was added $Ph_3P$ (11.6g, 1.5equiv) and the mixture was stirred at room temperature overnight and the reaction was complete. The mixture was then mixed with silica gel, evaporated to dryness and loaded on a short silica column. The column was first eluted with EA to get rid of $Ph_3P$ and $Ph_3P$=O and then eluted with methanol to afford the target product portion. The methanol solution so collected was evaporated to dryness and the residue was dissolved in a mixed solvent of DCM/MeOH (15:1). Insoluble silica gel was removed by filtration and the filtrate was evaporated to dryness to give the title compound as a solid (8.3g).

**(Method 2)** To a solution of intermediate E1 (8.7g) in absolute ethanol (100ml) was added 10% palladium on charcoal (1.3g) and the mixture was stirred under hydrogen (1 atm.) overnight at room temperature. The mixture was filtered and the filtrate was concentrated to afford the title compound as a solid (8.1 g)

**Intermediate E3- ethyl 2-((4-((4'-(trifluoromethyl)biphenyl-4-yl)methyl)-4H-1,2,4-triazol-3-yl)methylami no)cyclopent-1-enecarboxylate**

**(Method 1)** To a solution of E2 (2.8g, 1equiv) in absolute ethanol (20ml) were added ethyl 2-oxocyclopentanecarboxylate (1.36ml, 1.1equiv) and tetraethyl orthosilicate (3.7ml, 2equiv). The mixture was refluxed under nitrogen for 5h until the completion of the reaction monitored by TLC. Silica gel was added to the solution and the mixture was evaporated to dryness, then purified by column chromatography (DCM/MeOH=25:1) to give the title compound as a solid (3.9g).

**(Method 2)** To a solution of E2 (2.63g, 1equiv) in absolute ethanol (20ml) were added ethyl 2-oxocyclopentanecarboxylate (1.2ml, 1.0equiv) and 4Å dried molecular sieve (10g). The mixture was refluxed for 10h under nitrogen and then filtered to remove the molecular sieve. The filtrate was concentrated to remove most of the solvent and then stored in the refrigerator for a few hours. The precipitate thus obtained was collected by filtration, dried to give the title compound as a solid (3.4g). $^1$H-NMR ($CDCl_3$, 300 MHz) δ1.23 (t, 3H, *J*=7.2), 1.76 (m, 2H), 2.47 (m, 4H), 4.09 (q, 2H, *J*=7.2), 4.44 (d, 2H, *J*=6.3), 5.26 (s, 2H), 7.22 (d, 2H, *J*=8.1), 7.60 (d, 2H, *J*=8.1), 7.68 (m, 5H), 8.17 (s, 1H).

**Intermediate E4- 2-thioxo-1-((4-((4'-(trifluoromethyl)biphenyl-4-yl)methyl)-4H-1,2,4-triazol-3-yl) methyl)-2,3,6,7-tetrahydro-1H-cyclopenta[d]pyrimidin-4(5H)-one**

A mixture of E3 (2.0g, 1equiv), isothiocyanatotrimethylsilane (2.1ml, 3.5equiv) in anhydrous DMF (3ml) was heated at 140°C for 4h under nitrogen. After cooled, the mixture was placed in an ice bath and quenched by addition of saturated $NaHCO_3$ solution, diluted with EA (30ml) and water (20ml), and then stirred for 10min. The organic layer was separated and the aqueous phase was extracted again with EA (30ml) once. The combined organic phase was concentrated to about 20ml and then stored in refrigerator. The precipitate thus obtained was filtered off and dried to give the title compound (1.6g). $^1$H-NMR ($d_6$-DMSO, 300 MHz) δ1.94 (m, 2H), 2.52 (t, 2H), 2.85 (t, 2H, *J*=7.5), 5.48 (s, 2H), 5.60 (s, 2H), 7.33 (d, 2H, *J*=8.1), 7.73 (d, 2H, *J*=8.4), 7.81 (d, 2H, *J*=8.4), 7.88 (d, 2H, *J*=8.7), 8.68 (s, 1H), 12.56 (s, 1H).

**Intermediate E5- 1-((5-(hydroxymethyl)-4-((4'-(trifluoromethyl)biphenyl-4-yl)methyl)-4H-1,2,4-tri azol-3-yl)methyl)-2-thioxo-2,3,6,7-tetrahydro-1H-cyclopenta[d]pyrimidin-4(5H)-one**

A solution of E4 (1.6g) in formalin (15ml, 37%) was refluxed for 8h until the completion of the reaction monitored by TLC. Then the mixture wascooled and precipitates formed, which was collected by filtration, washed with water several times and and dried to give the title compound (1.2g). $^1$H-NMR ($d_6$-DMSO, 300 MHz) δ1.94 (m, 2H), 2.50 (t, 2H), 2.82 (t, 2H), 4.65 (d, 2H), 4.79 (t, 1H, -OH), 5.50 (s, 2H), 5.55 (s, 2H), 7.26 (d, 2H), 7.72 (d, 2H), 7.81 (d, 2H), 7.88 (d, 2H), 12.56 (s, 1H); MS (ESI): 512(M-H).

**Example 72- 2-(4-fluorobenzylthio)-1-((5-(hydroxymethyl)-4-((4'-(trifluoromethyl)biphenyl-4-yl)methyl)-4H-1,2,4-triazol-3-yl)methyl)-6,7-dihydro-1H-cyclopenta[d]pyrimidin-4(5H)-one**

**(Method 1)** A mixture of E5 (1.2g, 1equiv), 1-(bromomethyl)-4-fluorobenzene (0.32ml, 1.1equiv), DBU (0.52ml, 1.5equiv) and KI (20mg, 0.05equiv) in acetonitrile (10ml) was stirred at room temperature overnight and TLC detection showed that the reaction was complete. Acetonitrile was evaporated under reduced pressure and the residue was diluted with EA (10ml) and water (10ml). The resulting mixture was stirred for 0.5h and the precipitate thus obtained was collected by filtration, washed with water and EA, and then dried to give the title compound (1.16g).

**(Method 2)** A mixture of E5 (1.08g, 1.1equiv), 1-(bromomethyl)-4-fluorobenzene (0.29ml, 1.1equiv) and anhydrous $K_2CO_3$ (0.44g, 1.5equiv) in acetone (10ml) was refluxed for 1h and TLC detection showed that the reaction was complete. Acetone was evaporated under reduced pressure and the residue was diluted with EA (10ml) and water (10ml). The resulting mixture was stirred for 0.5h and the precipitate thus obtained was collected by filtration, washed with water and EA, and then dried to give the title compound (0.63g). $^1$H-NMR ($d_6$-DMSO, 400 MHz) δ1.90 (m, 2H), 2.54 (t, 2H), 2.67 (t, 2H), 4.27 (s, 2H), 4.64 (s, 2H), 5.14 (s, 2H), 5.44 (s, 2H), 5.80 (vbrs, 1H), 6.99 (t, 2H, *J*=8.4), 7.28 (d, 2H), 7.31 (dd, 2H), 7.64 (d, 2H), 7.80 (m, 4H).

**Example 73- 2-(4-fluorobenzylthio)-1-((4-((4'-(trifluoromethyl)biphenyl-4-yl)methyl)-4H-1,2,4-triazol-3-yl)methyl)-6,7-dihydro-1H-cyclopenta[d]pyrimidin-4(5H)-one**

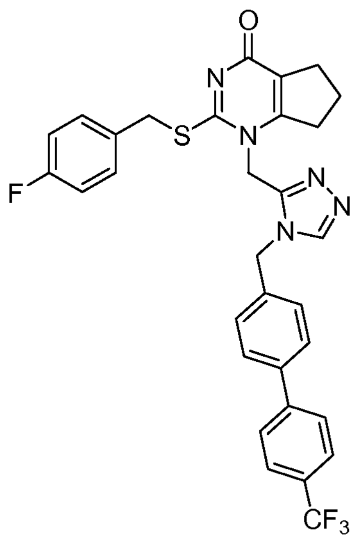

Following a procedure similar to that described for the preparation of example 72 except that E4 was used in place of E5. $^1$H-NMR ($d_6$-DMSO, 400 MHz) δ1.90 (m, 2H), 2.54 (t, 2H, *J*=7.2), 2.69 (t, 2H, *J*=7.2), 4.29 (s, 2H), 5.24 (s, 2H), 5.39 (s, 2H), 7.02 (t, 2H, *J*=8.4), 7.32 (m, 4H), 7.67 (d, 2H, *J*=8.8), 7.82 (m, 4H), 8.74 (s, 1H).

**Example 74- 5-((2-(4-fluorobenzylthio)-4-oxo-4,5,6,7-tetrahydro-1H-cyclopenta[d]pyrimidin-1 -yl)methyl)-4-((4'-(trifluoromethyl)biphenyl-4-yl)methyl)-4H-1,2,4-triazole-3-car baldehyde**

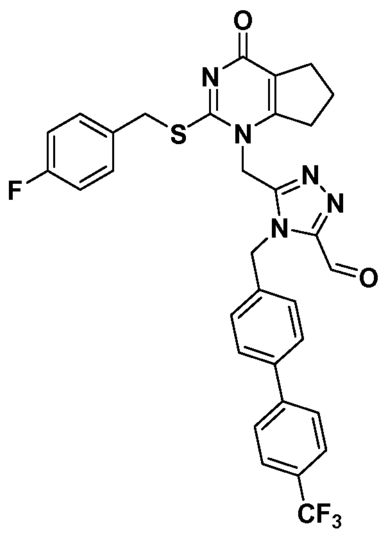

To a solution of the compound of Example 72 (700mg, 1equiv) in dioxane (10ml) was added activated $MnO_2$ (0.6g, 6equiv) and the mixture was stirred at 70~80°C for 3h. $MnO_2$ was then removed by filtration and the filtrate was purified by column chromatography (DCM/MeOH=20:1) to give the title compound as a solid (540mg). $^1$H-NMR ($CDCl_3$, 300 MHz) δ1.99 (m, 2H), 2.71 (m, 4H), 4.42 (s, 2H), 5.13 (s, 2H), 5.73 (s, 2H), 6.90 (m, 2H), 7.03 (d, 2H), 7.28 (dd, 2H), 7.47 (d, 2H), 7.59 (d, 2H), 7.70 (d, 2H).

**Example 75- 1-((5-((dimethylamino)methyl)-4-((4'-(trifluoromethyl)biphenyl-4-yl)methyl)-4H-1,2,4-triazol-3-yl)methyl)-2-(4-fluorobenzylthio)-6,7-dihydro-1H-cyclopenta[d]py rimidin-4(5H)-one**

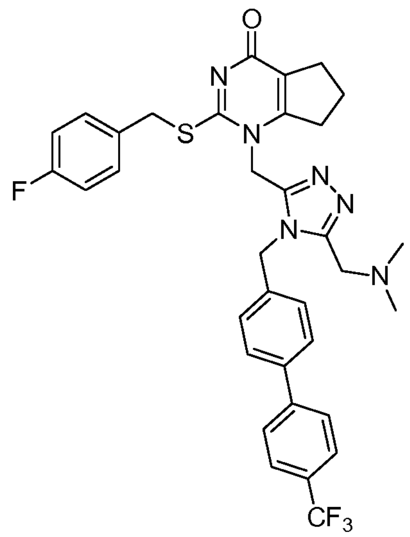

A mixture of the compound of Example 74 (400mg, 1equiv), dimethylamine hydrochloride (105mg, 2equiv), DIPEA (215μl, 2equiv) and $NaHB(OAc)_3$ (205mg, 1.5equiv) in DCM (8ml) was stirred at room temperature. After the completion of the reaction monitored by TLC, the mixture was transferred into a separating funnel, washed with brine three times, dried and purified by column chromatography (DCM/MeOH=10:1) to give the title compound as a solid (280mg). $^1$H-NMR ($CDCl_3$, 300 MHz) δ1.96 (m, 2H), 2.32 (s, 6H), 2.63 (t, 2H, *J*=7.2), 2.75 (t, 2H, *J*=7.2), 3.70 (s, 2H), 4.44 (s, 2H), 5.03 (s, 2H), 5.49 (s, 2H), 6.93 (m, 4H), 7.31 (dd, 2H, *J*=8.4, 5.4), 7.45 (d, 2H, *J*=8.4), 7.61 (d, 2H, *J*=8.1), 7.70 (d, 2H, *J*=8.1); MS (ESI): 649(M+H).

**Example 76- N-(4-fluorobenzyl)-1-(5-((2-(4-fluorobenzylthio)-4-oxo-4,5,6,7-tetrahydro-1H-cycl openta[d]pyrimidin-1-yl)methyl)-4-((4'-(trifluoromethyl)biphenyl-4-yl)methyl)-4 H-1,2,4-triazol-3-yl)-N,N-dimethylmethanaminium bromide**

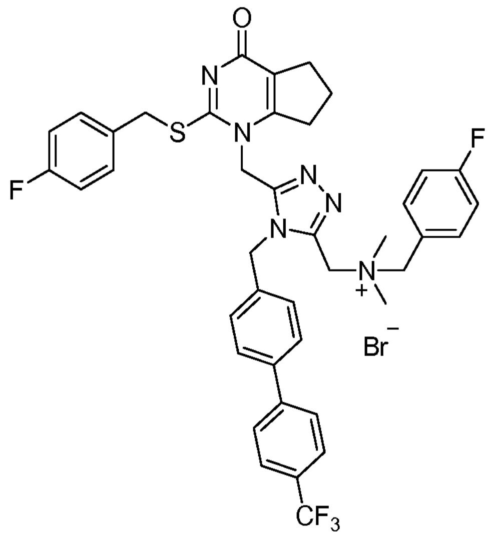

Following a procedure similar to that described for the preparation of example 47 except that example 75 was used in place of example 46. $^1$H-NMR ($d_6$-DMSO, 300 MHz) δ1.92 (m, 2H), 2.55 (t, 2H, *J*=6.6), 2.75 (t, 2H, *J*=6.6), 3.08 (s, 6H), 4.29 (s, 2H), 4.48 (s, 2H), 4.89 (s, 2H), 5.20 (s, 2H), 5.64 (s, 2H), 6.99 (t, 2H, *J*=8.4), 7.21 (d, 2H, *J*=7.8), 7.30 (t, 2H, *J*=7.8), 7.38 (t, 2H, *J*=8.7), 7.65 (d, 2H, *J*=8.4), 7.73 (t, 2H, *J*=7.8), 7.81 (m, 4H); MS (ESI): 757 (M-Br).

**Example 77- 1-(5-((2-(4-fluorobenzylthio)-4-oxo-4,5,6,7-tetrahydro-1H-cyclopenta[d]pyrimidi n-1-yl)methyl)-4-((4'-(trifluoromethyl)biphenyl-4-yl)methyl)-4H-1,2,4-triazol-3-yl )-N,N,N-trimethylmethanaminium iodide**

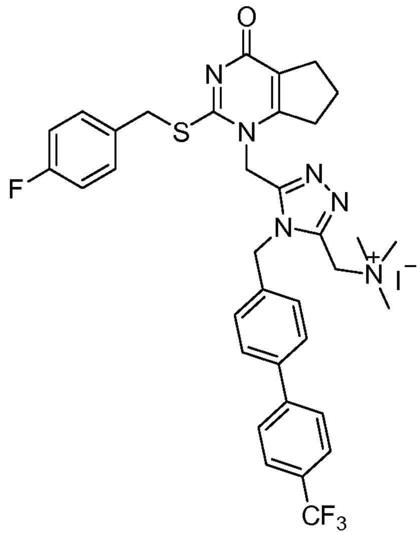

To a solution of the compound of example 75 (65mg, 1equiv) in DCM (3ml) was added $CH_3I$ (19μl, 3equiv)and the plug was plug was screwed tightly. The mixture was stirred at room temperature overnight. Solvent was evaporated under reduced pressure and the residue was recrystallized from acetone to give the title compound as a light yellow solid (40mg). $^1$H-NMR ($d_6$-DMSO, 300 MHz) δ1.91 (m, 2H), 2.54 (t, 2H), 2.71 (t, 2H, *J*=6.9), 3.21 (s, 9H), 4.28 (s, 2H), 4.93 (s, 2H), 5.14 (s, 2H), 5.56 (s, 2H), 6.99 (t, 2H, *J*=8.7), 7.19 (d, 2H, *J*=7.8), 7.30 (dd, 2H, *J*=8.1, 5.7), 7.66 (d, 2H, *J*=8.1), 7.81 (m, 4H).

**Example 78- 1-(5-((2-(4-fluorobenzylthio)-4-oxo-4,5,6,7-tetrahydro-1H-cyclopenta[d]pyrimidi n-1-yl)methyl)-4-((4'-(trifluoromethyl)biphenyl-4-yl)methyl)-4H-1,2,4-triazol-3-yl )-N,N,N-trimethylmethanaminium** bromide

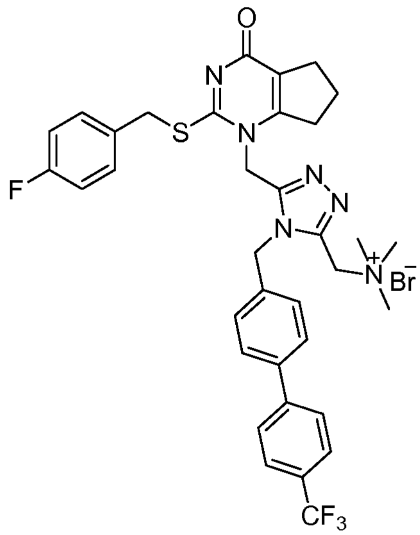

To solution of the compound of Example 75 (90mg) in acetone (2ml) in a flask sealed with rubber plug and placed in a cold trap at -5°C was added $CH_3Br$ (0.5ml) dropwise with an injector. The mixture was stirred at room temperature overnight and the solid thus formed was collected by filtration, washed with acetone, and then dried in

vacuo to give the title compound as a white solid (70mg).

**Example 79- 1-((5-((diethylamino)methyl)-4-((4'-(trifluoromethyl)biphenyl-4-yl)methyl)-4H-1, 2,4-triazol-3-yl)methyl)-2-(4-fluorobenzylthio)-6,7-dihydro-1H-cyclopenta[d]pyri midin-4(5H)-one**

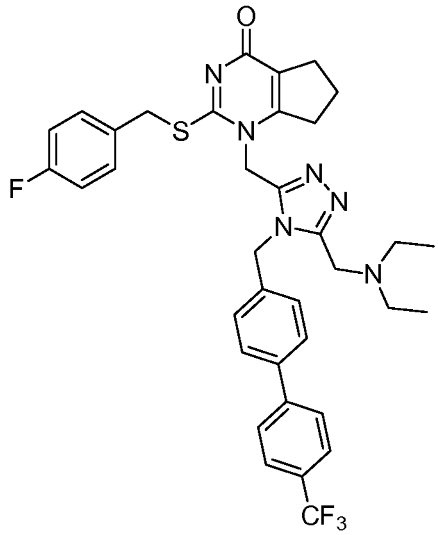

A mixture of the compound of Example 74 (200mg, 1equiv), diethylamine (40µl, 1.2equiv) and $NaHB(OAc)_3$ (103mg, 1.5equiv) in DCM (5ml) was stirred at room temperature. After the completion of the reaction monitored by TLC, the mixture was transferred into a separating funnel, washed with brine three times, dried and purified by column chromatography (DCM/MeOH=15:1) to give the title compound as a colloidal solid (160mg). $^1H$-NMR ($CDCl_3$, 300 MHz) δ0.99 (t, 6H, *J*=7.2), 1.97 (m, 2H), 2.57-2.65 (m, 6H), 2.77 (t, 2H, *J*=7.2), 3.82 (s, 2H), 4.44 (s, 2H), 5.02 (s, 2H), 5.53 (s, 2H), 6.93 (m, 4H), 7.30 (m, 2H), 7.45 (d, 2H, *J*=8.1), 7.63 (d, 2H, *J*=8.4), 7.70 (d, 2H, *J*=8.1); MS (ESI): 677(M+H).

**Example 80- 1-((5-((diethylamino)methyl)-4-((4'-(trifluoromethyl)biphenyl-4-yl)methyl)-4H-1, 2,4-triazol-3-yl)methyl)-2-(4-fluorobenzylthio)-6,7-dihydro-1H-cyclopenta[d]pyri midin-4(5H)-one hydrochloride**

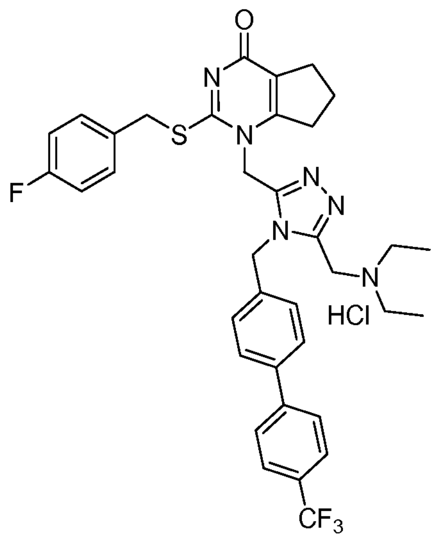

To an ice cold solution of the compound of example 79 (105mg, 1equiv) in isopropanol (2ml) was added concd.

HCl (14μl, about 1equiv), and the mixture was stirred for 0.5h. Solvent was then evaporated under reduced pressure and to the residue was added DCM (3ml) and the residue was smashed by ultrasonic. The solid thus formed was collected by filtration, dried in vacuo to give the title compound as a white solid (60mg). $^1$H-NMR ($d_6$-DMSO, 300 MHz) δ1.23 (m, 6H), 1.89 (m, 2H), 2.55 (t, 2H, *J*=7.2), 2.72 (t, 2H, *J*=7.2), 3.26 (m, 4H), 4.31 (s, 2H), 4.56 (s, 2H), 5.23 (s, 2H), 5.59 (s, 2H), 7.02 (t, 2H, *J*=8.7), 7.22 (d, 2H, *J*=8.1), 7.33 (dd, 2H, J=8.7, 5.4), 7.67 (d, 2H, *J*=8.1), 7.82 (m, 4H), 11.06 (s, 1H).

Example 81- 1-((5-(((4-fluorobenzyl)(methyl)amino)methyl)-4-((4'-(trifluoromethyl)biphenyl-4 -yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one

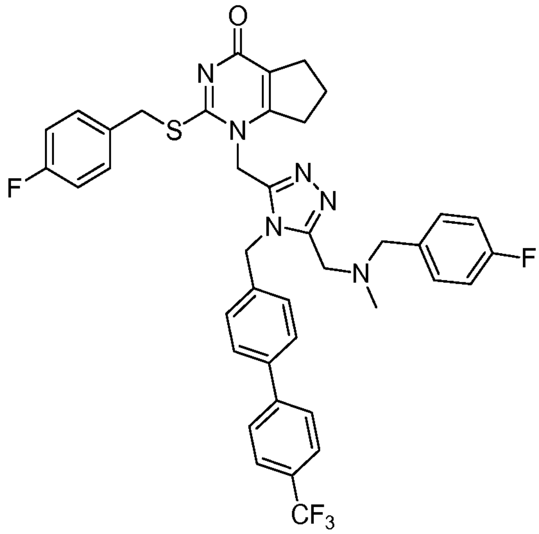

Following a procedure similar to that described for the preparation of example 79 except that 1-(4-fluorophenyl)-N-methylmethanamine was used instead of diethylamine. $^1$H-NMR ($CDCl_3$, 300 MHz) δ1.95 (m, 2H), 2.34 (s, 3H), 2.62 (t, 2H, *J*=7.2), 2.73 (t, 2H, *J*=7.2), 3.61 (s, 2H), 3.75 (s, 2H), 4.44 (s, 2H), 5.01 (s, 2H), 5.31 (s, 2H), 6.80 (d, 2H, *J*=7.8), 6.93 (m, 4H), 7.18 (m, 2H), 7.30 (dd, 2H, *J*=8.1, 5.7), 7.41 (d, 2H, *J*=7.8), 7.62 (d, 2H, *J*=8.1), 7.73 (d, 2H, *J*=8.4); MS (ESI): 743(M+H).

**Example 82- 2-(4-fluorobenzylthio)-1-((5-((isopropyl(methyl)amino)methyl)-4-((4'-(trifluorom ethyl)biphenyl-4-yl)methyl)-4*H*-1,2,4-triazol-3-yl)methyl)-6,7-dihydro-1*H*-cyclop enta[*d*]pyrimidin-4(5*H*)-one**

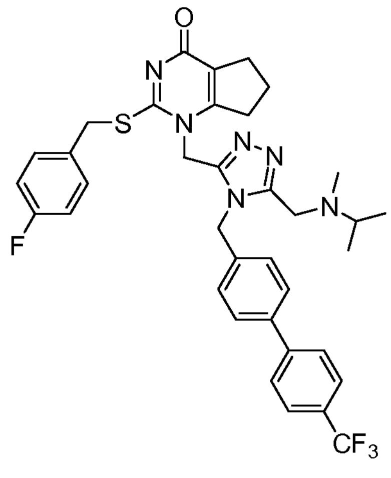

Following a procedure similar to that described for the preparation of example 79 except that N-methylpropan-2-amine was used instead of diethylamine. $^1$H-NMR ($CDCl_3$, 400 MHz) δ 1.01 (d, 6H, *J*=6.8), 1.96 (m, 2H), 2.17 (s, 3H), 2.63 (t, 2H), 2.75 (t, 2H), 2.87 (m, 1H), 3.79 (s, 2H), 4.44 (s, 2H), 5.02 (s, 2H), 5.48 (s, 2H), 6.93 (m, 4H), 7.30 (dd, 2H), 7.45 (d, 2H), 7.61 (d, 2H, *J*=8.0), 7.70 (d, 2H, *J*=8.4).

**Example** 83- **(±)-1-((5-(((1-ethylpyrrolidin-2-yl)methylamino)methyl)-4-((4'-(trifluoromethyl)b iphenyl-4-yl)methyl)-4*H*-1,2,4-triazol-3-yl)methyl)-2-(4-fluorobenzylthio)-6,7-dih ydro-1*H*-cyclopenta[*d*]pyrimidin-4(5*H*)-one**

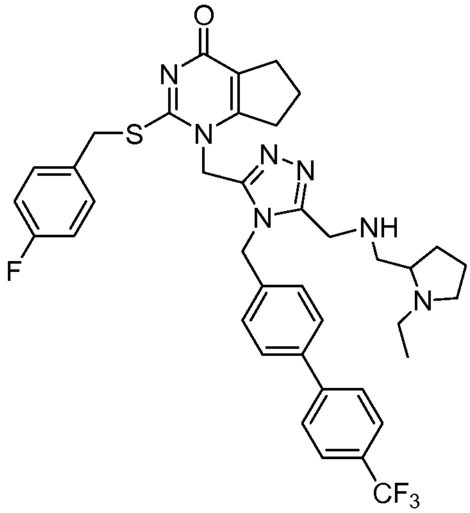

Following a procedure similar to that described for the preparation of example **79** except that (±)-(1-ethylpyrrolidin-2-yl)methanamine was used instead of diethylamine. $^1$H-NMR ($CDCl_3$, 400 MHz) δ 1.30 (t, 3H, *J*=7.2), 1.86 (m, 1H), 2.00 (m, 3H), 2.10 (m, 2H), 2.69 (t, 2H, *J*=7.2), 2.83 (m, 4H), 3.13 (q, 2H), 3.37 (m, 2H), 3.77 (m, 1H), 4.09 (s, 2H), 4.40 (s, 2H), 5.02/5.08 (2x d, 2H, *J*=17.6), 5.44/5.66 (2x d, 2H *J*=16.8), 6.89 (t, 2H, *J*=8.8), 7.02 (d, 2H, *J*=8.0), 7.28 (m, 2H), 7.43 (d, 2H, *J*=7.6), 7.57 (d, 2H, *J*=8.0), 7.69 (d, 2H, *J*=8.0).

**Example 84- 1-((5-(((2*S*,6*R*)-2,6-dimethylmorpholino)methyl)-4-((4'-(trifluoromethyl)biphenyl -4-yl)methyl)-4*H*-1,2,4-triazol-3-yl)methyl)-2-(4-fluorobenzylthio)-6,7-dihydro-1 *H*-cyclopenta[*d*]pyrimidin-4(*5H*)-one**

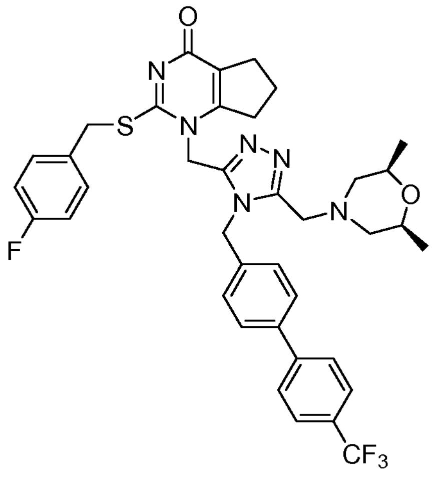

Following a procedure similar to that described for the preparation of example **79** except that (2S,6R)-2,6-dimethylmorpholine was used instead of diethylamine. $^1$H-NMR ($CDCl_3$, 400 MHz) δ 1.11 (d, 6H, *J*=6.0), 1.94 (m, 4H), 2.62 (m, 4H), 2.75 (t, 2H, *J*=7.2), 3.48 (m, 2H), 3.70 (s, 2H), 4.45 (s, 2H), 5.05 (s, 2H), 5.42 (s, 2H), 6.92 (m, 4H), 7.32

(dd, 2H, *J*=8.8, 5.6), 7.45 (d, 2H, *J*=8.0), 7.62 (d, 2H, *J*=8.0), 7.71 (d, 2H, *J*=8.0).

**Example 85- methyl-2-((5-((2-(4-fluorobenzylthio)-4-oxo-4,5,6,7-tetrahydro-1*H*-cyclopenta[*d*]p yrimidin-1-yl)methyl)-4-((4'-(trifluoromethyl)biphenyl-4-yl)methyl)-4*H*-1,2,4-tria zol-3-yl)methylamino)-2-methylpropanoate**

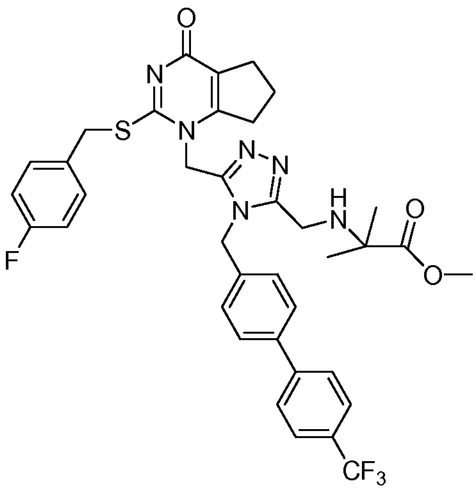

Following a procedure similar to that described for the preparation of example 75 except that methyl-2-amino-2-methylpropanoate hydrochloride was used instead of dimethylamine hydrochloride. $^{1}$H-NMR ($CDCl_3$, 400 MHz) δ1.33 (s, 6H), 1.94 (m, 2H), 2.62 (t, 2H), 2.71 (t, 2H), 3.68 (s, 3H), 3.94 (s, 2H), 4.45 (s, 2H), 5.03 (s, 2H), 5.45 (s, 2H), 6.94 (m, 4H), 7.32 (dd, 2H), 7.45 (d, 2H), 7.62 (d, 2H), 7.71 (d, 2H).

**Example 86- 1-((5-((4-ethylpiperazin-1-yl)methyl)-4-((4'-(trifluoromethyl)biphenyl-4-yl)methy l)-4*H*-1,2,4-triazol-3-yl)methyl)-2-(4-fluorobenzylthio)-6,7-dihydro-1*H*-cyclopent a[*d*]pyrimidin-4(5*H*)-one**

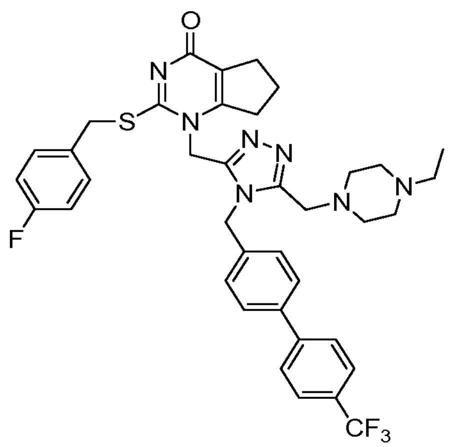

Following a procedure similar to that described for the preparation of example **79** except that 1-ethylpiperazine was used instead of diethylamine. $^{1}$H-NMR ($CDCl_3$, 300 MHz) δ 1.07 (t, 3H, *J*=7.2), 1.96 (m, 4H), 2.39 (m, 4H), 2.54 (m, 4H), 2.64 (t, 2H, *J*=7.2), 2.76 (t, 2H, *J*=7.2), 3.72 (s, 2H), 4.45 (s, 2H), 5.04 (s, 2H), 5.42 (s, 2H), 6.94 (m, 4H), 7.31 (dd, 2H), 7.46 (d, 2H, *J*=8.1), 7.62 (d, 2H), 7.71 (d, 2H).

**Example 87- 2-(4-fluorobenzylthio)-1-((5-((2-methoxyethylamino)methyl)-4-((4'-(trifluoromet hyl)biphenyl-4-yl)methyl)-4*H*-1,2,4-triazol-3-yl)methyl)-6,7-dihydro-1*H*-cyclopen ta[*d*]pyrimidin-4(5*H*)-one**

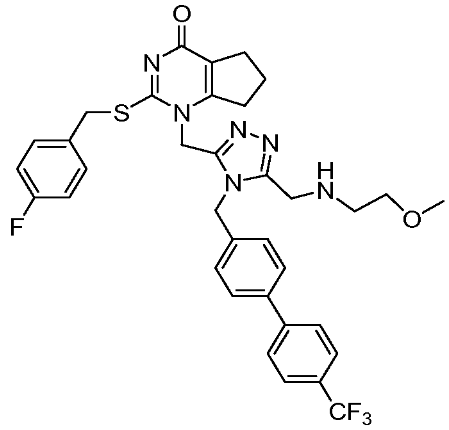

Following a procedure similar to that described for the preparation of example **79** except that 2-methoxyethylamine was used instead of diethylamine. $^1$H-NMR ($CDCl_3$, 300 MHz) δ1.95 (m, 2H), 2.50 (vbrs, 1H), 2.62 (t, 2H), 2.74 (t, 2H), 2.91 (t, 2H), 3.31 (s, 3H), 3.51 (t, 2H), 4.09 (s, 2H), 4.43 (s, 2H), 5.06 (s, 2H), 5.45 (s, 2H), 6.93 (m, 4H), 7.31 (dd, 2H), 7.44 (d, 2H), 7.60 (d, 2H), 7.70 (d, 2H).

**Example 88- methyl-2-(4-((5-((2-(4-fluorobenzylthio)-4-oxo-4,5,6,7-tetrahydro-1*H*-cyclopenta[ *d*]pyrimidin-1-yl)methyl)-4-((4'-(trifluoromethyl)biphenyl-4-yl)methyl)-4*H*-1,2,4-triazol-3-yl)methyl)piperazin-1-yl)-2-methyl-propanoate**

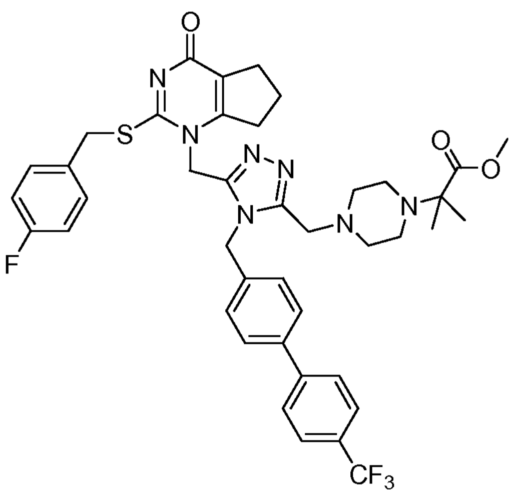

Following a procedure similar to that described for the preparation of example **75** except that **M20** was used instead of dimethylamine hydrochloride. $^1$H-NMR ($CDCl_3$, 400 MHz) δ1.29 (s, 6H), 1.96 (m, 2H), 2.53 (m, 8H), 2.63 (t, 2H), 2.75 (t, 2H), 3.64 (s, 3H), 3.71 (s, 2H), 4.44 (s, 2H), 5.03 (s, 2H), 5.42 (s, 2H), 6.94 (m, 4H), 7.31 (dd, 2H), 7.45 (d, 2H), 7.62 (d, 2H), 7.70 (d, 2H).

**Example 89- 2-(4-((5-((2-(4-fluorobenzylthio)-4-oxo-4,5,6,7-tetrahydro-1*H*-cyclopenta[d]pyrim idin-1-yl)methyl)-4-((4'-(trifluoromethyl)biphenyl-4-yl)methyl)-4*H*-1,2,4-triazol-3 -yl)methyl)piperazin-1-yl)-2-methylpropanoic acid**

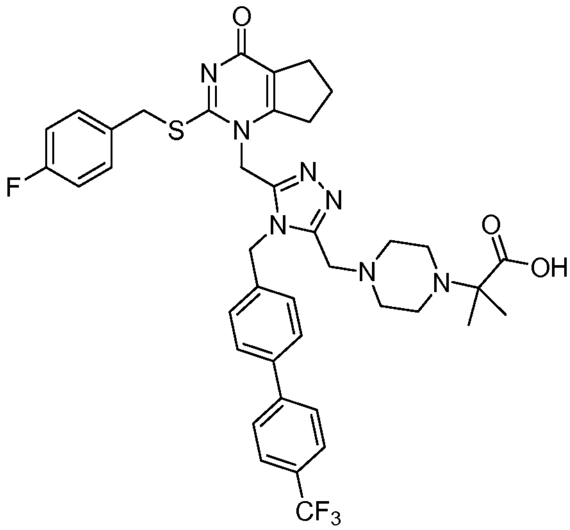

Following a procedure similar to that described for the preparation of example **63** except that example **88** was used instead of example **62.** MS (ESI): 774 (M-H).

**Example 90- 1-((5-(((2-(dimethylamino)ethyl)(methyl)amino)methyl)-4-((4'-(trifluoromethyl)b iphenyl-4-yl)methyl)-4*H*-1,2,4-triazol-3-yl)methyl)-2-(4-fluorobenzylthio)-6,7-dih ydro-1*H*-cyclopenta[*d*]pyrimidin-4(5*H*)-one**

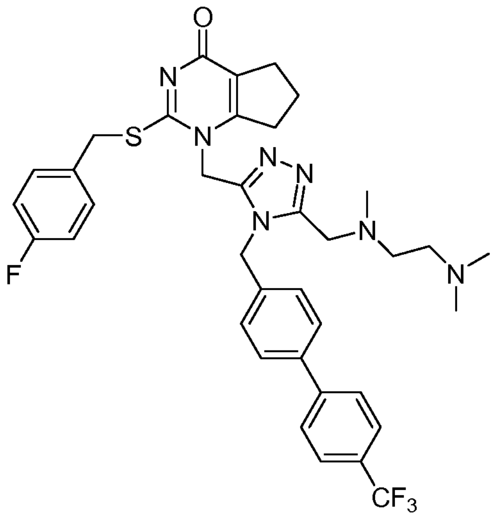

Following a procedure similar to that described for the preparation of example **79** except that *N,N,N'*-trimethylethylenediamine was used instead of diethylamine. $^1$H-NMR ($CDCl_3$, 300 MHz) $\delta$1.93 (m, 2H), 2.07 (s, 6H), 2.24 (s, 3H), 2.33 (t, 2H), 2.53 (t, 2H), 2.59 (t, 2H), 2.70 (t, 2H), 3.78 (s, 2H), 4.45 (s, 2H), 5.02 (s, 2H), 5.57 (s, 2H), 6.87 (d, 2H), 6.94 (t, 2H), 7.32 (dd, 2H), 7.43 (d, 2H), 7.62 (d, 2H), 7.70 (d, 2H).

**Example 91a- 1-((5-(((2-(diethylamino)ethyl)(methyl)amino)methyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one**

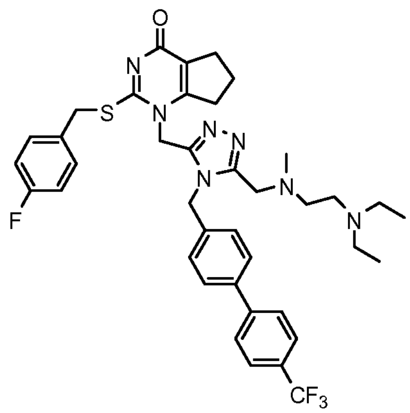

Following a procedure similar to that described for the preparation of example **79** except that *N,N*-Diethyl-N'-methylethylenediamine was used instead of diethylamine. $^1$H-NMR ($CDCl_3$, 300 MHz) δ 1.28 (t, 6H), 2.03 (m, 2H), 2.41 (s, 3H), 2.74 (t, 2H), 2.95 (t, 2H), 3.05 (m, 8H), 3.90 (s, 2H), 4.37 (s, 2H), 5.15 (s, 2H), 5.62 (s, 2H), 6.84 (t, 2H), 7.06 (d, 2H), 7.23 (dd, 2H), 7.40 (d, 2H), 7.55 (d, 2H), 7.70 (d, 2H).

**Example 91b- 1-((5-(((2-(diethylamino)ethyl)(methyl)amino)methyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one tartrate**

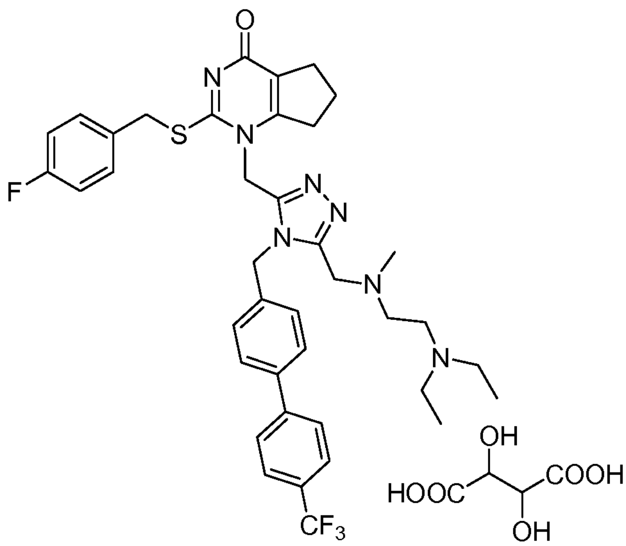

To a solution of **91a** (76mg) in methanol (2ml) was added tartaric acid (15mg, 1 equiv) at room temperature. The reaction was stirred for 5min and then evaporated to dryness under reduced pressure and dried under vacuo to give **91b** as a white solid. $^1$H-NMR ($d_6$-DMSO, 300 MHz) δ 1.09 (t, 6H, *J*=7.2), 1.91 (m, 2H), 2.23 (s, 3H), 2.54 (t, 2H, *J*=6.9), 2.70 (t, 2H, *J*=6.9), 2.75 (t, 2H, *J*=5.7), 2.99 (q, 4H, *J*=7.2), 3.06 (t, 2H, *J*=6.0), 3.77 (s, 2H), 4.26 (s, 2H), 4.30 (s, 2H), 5.17 (s, 2H), 5.45 (s, 2H), 7.02 (t, 2H, *J*=8.7), 7.19 (d, 2H, *J*=8.1), 7.23 (dd, 2H, *J*=8.4, 6.0), 7.65 (d, 2H, *J*=8.4), 7.81 (m, 4H).

**Example 92- 1-((5-(((3-(dimethylamino)propyl)(methyl)amino)methyl)-4-((4'-(trifluoromethyl) biphenyl-4-yl)methyl)-4*H*-1,2,4-triazol-3-yl)methyl)-2-(4-fluorobenzylthio)-6,7-di hydro-1*H*-cyclopenta[*d*]pyrimidin-4(5*H*)-one**

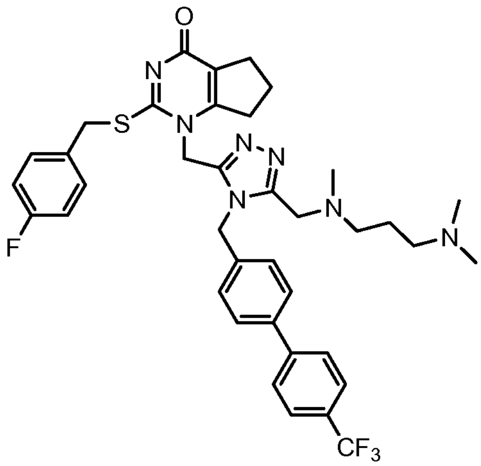

Following a procedure similar to that described for the preparation of example **79** except that *N,N,N'*-trimethyl-1,3-propanediamine was used instead of diethylamine. $^1$H-NMR ($CDCl_3$, 300 MHz) δ1.89 (m, 2H), 2.00 (m, 2H), 2.39 (s, 3H), 2.64 (s, 6H), 2.70 (m, 4H), 2.89 (m, 4H), 3.86 (s, 2H), 4.39 (s, 2H), 5.17 (s, 2H), 5.58 (s, 2H), 6.87 (t, 2H), 7.04 (d, 2H), 7.27 (m, 2H), 7.42 (d, 2H), 7.58 (d, 2H), 7.70 (d, 2H).

**Example 93- 2-(4-fluorobenzylthio)-1-((5-((2-morpholinoethylamino)methyl)-4-((4'-(trifluoro methyl)biphenyl-4-yl)methyl)-4*H*-1,2,4-triazol-3-yl)methyl)-6,7-dihydro-1*H*-cyclo penta[*d*]pyrimidin-4(5*H*)-one**

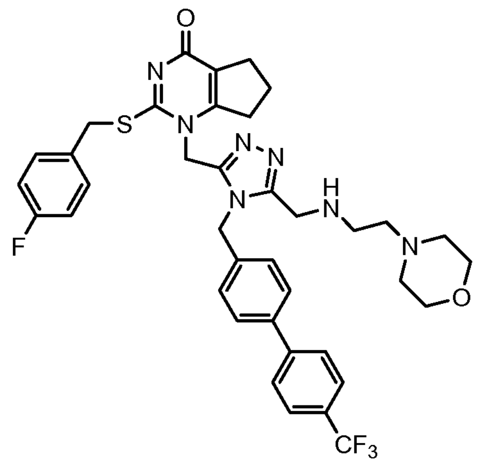

Following a procedure similar to that described for the preparation of example **79** except that 4-(2-aminoethyl)morpholine was used instead of diethylamine. $^1$H-NMR ($CDCl_3$, 300 MHz) δ 1.94 (m, 2H), 2.50 (m, 6H), 2.61 (t, 2H), 2.72 (t, 2H), 2.79 (t, 2H), 3.70 (t, 4H), 4.03 (s, 2H), 4.46 (s, 2H), 5.04 (s, 2H), 5.45 (s, 2H), 6.92 (m, 4H), 7.33 (m, 2H), 7.44 (d, 2H), 7.61 (d, 2H), 7.71 (d, 2H).

**Example 94- (*R*)-1-((5-((3-(dimethylamino)pyrrolidin-1-yl)methyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one**

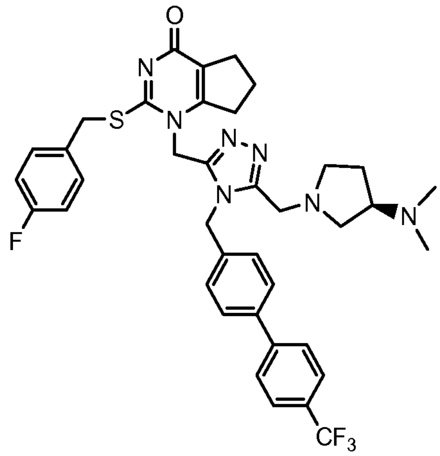

Following a procedure similar to that described for the preparation of example **79** except that (*R*)-*N,N*-dimethylpyrrolidin-3-amine was used instead of diethylamine. $^1$H-NMR ($CDCl_3$, 400 MHz) $\delta$ 1.72 (m, 1H), 1.95 (m, 3H), 2.17 (s, 6H), 2.50 (m, 1H), 2.61 (m, 4H), 2.74 (m, 4H), 3.83 (s, 2H), 4.46 (s, 2H), 5.05 (s, 2H), 5.43 (2x d, 2H), 6.88 (d, 2H), 6.95 (t, 2H), 7.33 (dd, 2H), 7.44 (d, 2H), 7.63 (d, 2H), 7.71 (d, 2H).

**Example** 95- **(*S*)-1-((5-((3-(dimethylamino)pyrrolidin-1-yl)methyl)-4-((4'-(trifluoromethyl)biph enyl-4-yl)methyl)-4*H*-1,2,4-triazol-3-yl)methyl)-2-(4-fluorobenzylthio)-6,7-dihydr o-1*H*-cyclopenta[*d*]pyrimidin-4(5*H*)-one**

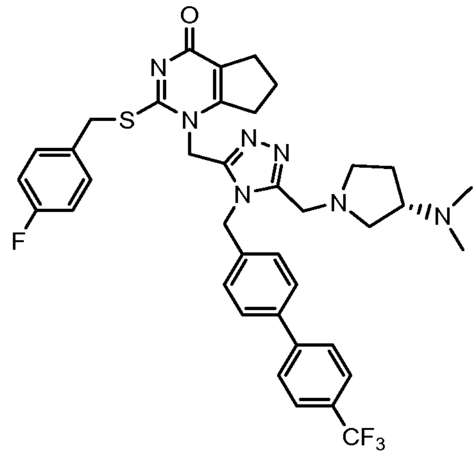

Following a procedure similar to that described for the preparation of example **79** except that (*S*)-*N*,*N*-dimethylpyrrolidin-3-amine was used instead of diethylamine. $^1$H-NMR ($CDCl_3$, 400 MHz) $\delta$ 1.70 (m, 1H), 1.94 (m, 3H), 2.17 (s, 6H), 2.49 (m, 1H), 2.61 (m, 4H), 2.73 (m, 4H), 3.83 (s, 2H), 4.46 (s, 2H), 5.04 (s, 2H), 5.43 (2x d, 2H), 6.88 (d, 2H), 6.95 (t, 2H), 7.33 (dd, 2H), 7.44 (d, 2H), 7.62 (d, 2H), 7.71 (d, 2H).

**Example 96- 2-(4-fluorobenzylthio)-1-((5-((2-(piperidin-1-yl)ethylamino)methyl)-4-((4'-(trifluo romethyl)biphenyl-4-yl)methyl)-4*H*-1,2,4-triazol-3-yl)methyl)-6,7-dihydro-1*H*-cy clopenta[*d*]pyrimidin-4(5*H*)-one**

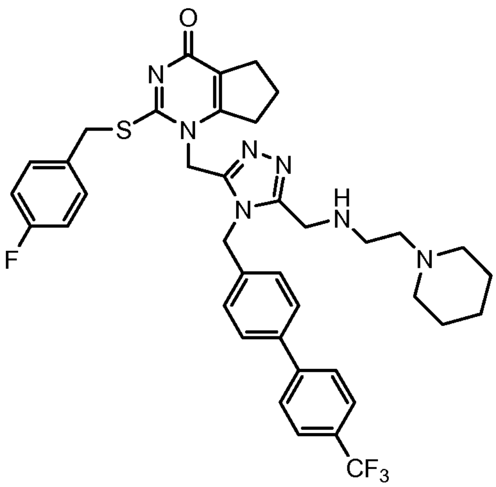

Following a procedure similar to that described for the preparation of example **79** except that *N*-(2-aminoethyl)piperidine was used instead of diethylamine. $^1$H-NMR ($CDCl_3$, 300 MHz) $\delta$1.40 (m, 2H), 1.51 (m, 4H), 1.93 (m, 2H), 2.03 (t, 2H), 2.31 (t, 2H), 2.38 (t, 2H), 2.59 (t, 2H), 2.71 (t, 4H), 4.02 (s, 2H), 4.46 (s, 2H), 5.04 (s, 2H), 5.46 (s, 2H), 6.90 (d, 2H), 6.95 (t, 2H), 7.33 (dd, 2H), 7.43 (d, 2H), 7.62 (d, 2H), 7.71 (d, 2H).

**Example 97- 2-(4-fluorobenzylthio)-1-((5-((2-(pyrrolidin-1-yl)ethylamino)methyl)-4-((4'-(triflu oromethyl)biphenyl-4-yl)methyl)-4*H*-1,2,4-triazol-3-yl)methyl)-6,7-dihydro-1H-c yclopenta[*d*]pyrimidin-4(5*H*)-one**

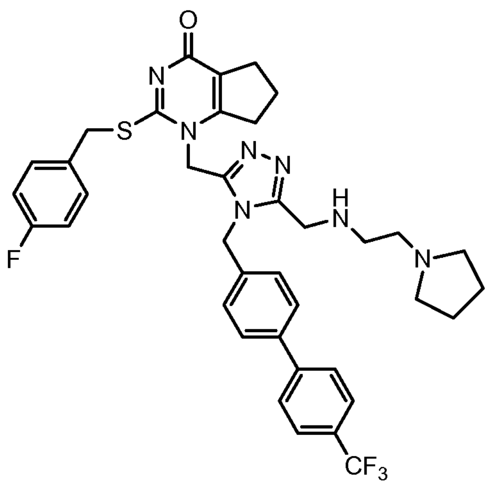

Following a procedure similar to that described for the preparation of example **79** except that 2-(pyrrolidin-1-yl)ethanamine was used instead of diethylamine. $^1$H-NMR ($CDCl_3$, 300 MHz) $\delta$1.73 (m, 4H), 1.94 (m, 2H), 2.47 (m, 4H), 2.58 (m, 4H), 2.73 (m, 4H), 4.02 (s, 2H), 4.46 (s, 2H), 5.04 (s, 2H), 5.46 (s, 2H), 6.90 (d, 2H), 6.94 (t, 2H), 7.33 (dd, 2H), 7.43 (d, 2H), 7.62 (d, 2H), 7.71 (d, 2H).

**Example 98- 1-((5-((2-(diisopropylamino)ethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H* -cyclopenta[*d*]pyrimidin-4(5*H*)-one**

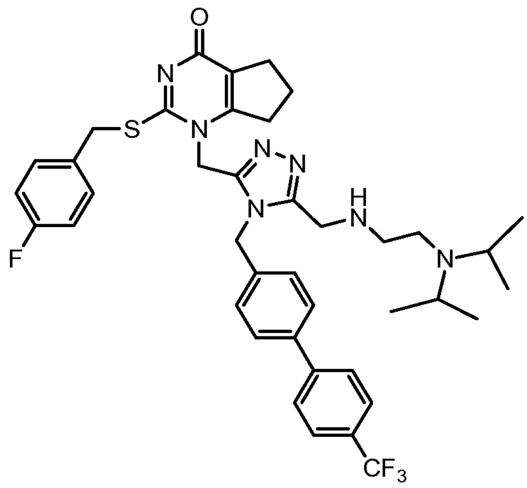

Following a procedure similar to that described for the preparation of example **79** except that *N,N*-di(propan-2-yl)ethane-1,2-diamine was used instead of diethylamine. $^{1}$H-NMR ($CDCl_3$, 300 MHz) δ 1.11 (d, 12H), 1.96 (m, 2H), 2.64 (t, 2H), 2.75 (m, 6H), 3.17 (m, 2H), 4.01 (s, 2H), 4.43 (s, 2H), 5.04 (s, 2H), 5.47 (s, 2H), 6.94 (m, 4H), 7.31 (dd, 2H), 7.43 (d, 2H), 7.60 (d, 2H), 7.70 (d, 2H).

**Example 99- 1-((5-((2-(diethylamino)ethylamino)methyl)-4-((4'-(trifluoromethyl)biphenyl-4-yl )methyl)-4*H*-1,2,4-triazol-3-yl)methyl)-2-(4-fluorobenzylthio)-6,7-dihydro-1H-cyc lopenta[*d*]pyrimidin-4(5*H*)-one**

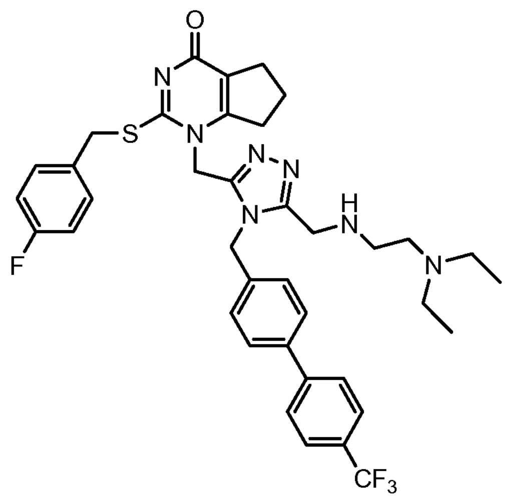

Following a procedure similar to that described for the preparation of example **79** except that *N,N*-Diethylethylenediamine was used instead of diethylamine. $^{1}$H-NMR ($CDCl_3$, 300 MHz) δ 0.96 (t, 6H), 1.94 (m, 2H), 2.49 (m, 6H), 2.66 (m, 6H), 4.01 (s, 2H), 4.46 (s, 2H), 5.03 (s, 2H), 5.46 (s, 2H), 6.94 (m, 4H), 7.32 (dd, 2H), 7.44 (d, 2H), 7.61 (d, 2H), 7.71 (d, 2H).

**Example 100- 2-(4-fluorobenzylthio)-1-((5-((methyl(pyridin-2-ylmethyl)amino)methyl)-4-((4'-(t rifluoromethyl)biphenyl-4-yl)methyl)-4*H*-1,2,4-triazol-3-yl)methyl)-6,7-dihydro-1 *H*-cyclopenta[*d*]pyrimidin-4(5*H*)-one**

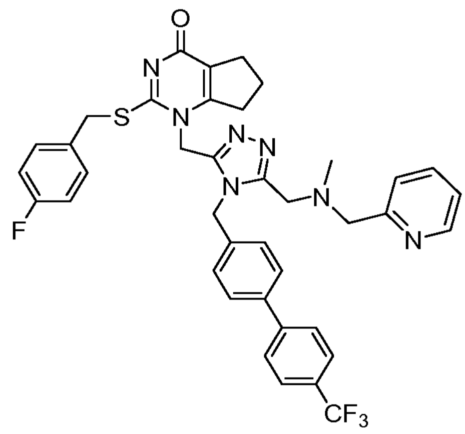

Following a procedure similar to that described for the preparation of example **79** except that *N*-methyl-1-(pyridin-2-yl)methanamine was used instead of diethylamine. $^1$H-NMR (MeOD, 300 MHz) δ 2.04 (m, 2H), 2.26 (s, 3H), 2.67 (t, 2H), 2.82 (t, 2H), 3.73 (s, 2H), 3.84 (s, 2H), 4.36 (s, 2H), 5.25 (s, 2H), 5.49 (s, 2H), 6.87 (t, 2H), 7.04 (d, 2H), 7.20 (m, 1H), 7.29 (m, 3H), 7.51 (d, 2H), 7.57 (d, 1H), 7.62 (m, 1H), 7.72 (d, 2H), 7.75 (d, 2H), 8.36 (d, 1H).

**Example 101- 1-((5-((cyclopropyl(methyl)amino)methyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cycl openta[*d*]pyrimidin-4(5*H*)-one**

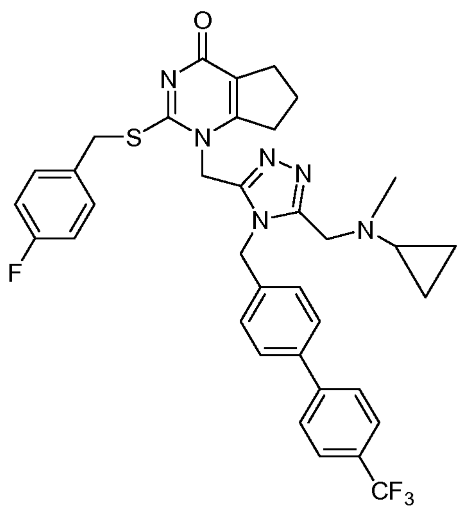

Following a procedure similar to that described for the preparation of example **79** except that *N*-methylcyclopropanamine was used instead of diethylamine. $^1$H-NMR ($CDCl_3$, 400 MHz) δ 0.19 (m, 2H), 0.45 (m, 2H), 1.80 (m, 1H), 1.95 (m, 2H), 2.30 (s, 3H), 2.62 (t, 2H, *J*=7.2), 2.74 (t, 2H, *J*=7.2), 3.90 (s, 2H), 4.40 (s, 2H), 5.01 (s, 2H), 5.33 (s, 2H), 6.88 (d, 2H, *J*=8.0), 6.94 (t, 2H, *J*=8.8), 7.31 (dd, 2H, *J*=8.8, 5.2), 7.45 (d, 2H, *J*=8.4), 7.62 (d, 2H, *J*=8.0), 7.70 (d, 2H, *J*=8.4).

**Example 102- 2-(4-fluorobenzylthio)-1-((5-((2-(2-oxoimidazolidin-1-yl)ethylamino)methyl)-4-((4 '-(trifluoromethyl)biphenyl-4-yl)methyl)-4*H*-1,2,4-triazol-3-yl)methyl)-6,7-dihydr o-1*H*-cyclopenta[*d*]pyrimidin-4(5*H*)-one**

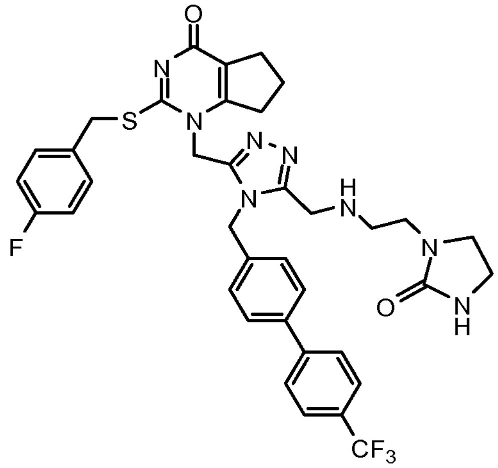

Following a procedure similar to that described for the preparation of example **79** except that 1-(2-aminoethyl)imidazolidin-2-one was used instead of diethylamine. $^1$H-NMR ($CDCl_3$, 300 MHz) $\delta$1.94 (m, 2H), 2.64 (t, 2H), 2.69 (t, 2H), 3.40 (t, 2H), 3.45 (t, 2H), 4.43 (s, 2H), 4.61 (s, 2H), 5.03 (s, 2H), 5.54 (s, 2H), 6.33 (s, 1H), 6.85 (t, 2H), 7.05 (d, 2H), 7.22 (dd, 2H), 7.33 (s, 1H), 7.46 (d, 2H), 7.58 (d, 2H), 7.69 (d, 2H).

The following compounds were also prepared by a procedure similar to that described for the preparation of example 79:

| Name | Structure | Reactant |
| --- | --- | --- |
| **Example 103:** **1-((5-((4-(dimethylamino)piperidin-1-yl)methyl)-4-((4'-(trifluoromethyl)biphenyl-4-yl)methyl)-4*H*-1, 2,4-triazol-3-yl)methyl)-2 -(4-fluorobenzylthio)-6,7-dihydro-1*H*-cyclopenta[ *d*]pyrimidin-4(5*H*)-one** | 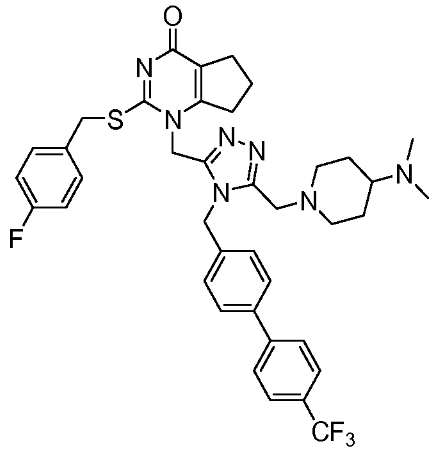 | *N*,*N*-dimethylpiperidin-4-amine |
| **Example 104:** **1-((5-((3,3-difluoropyrrolidin-1-yl)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one** | 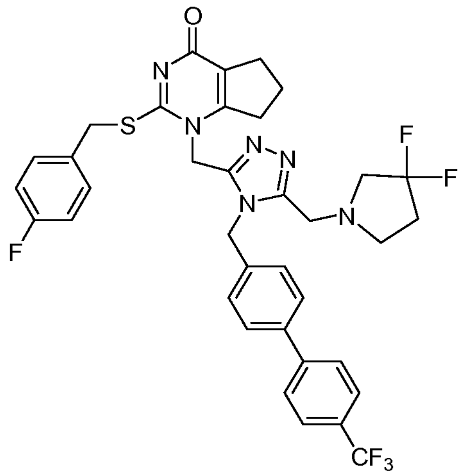 | 3,3-difluoropyrrolidine hydrochloride, DIPEA |

(continued)

| Name | Structure | Reactant |
|---|---|---|
| **Example 105:**<br>**1-((5-((2-(dimethylamino)ethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*] pyrimidin-4(5*H*)-one** | 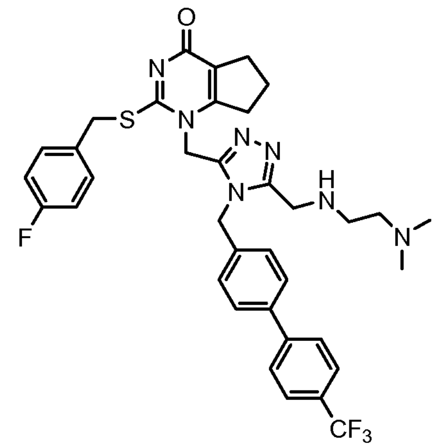 | *N*,*N*-dimethyleth ylenediamine |
| **Example 106:**<br>**(*S*)-1-((5-(((1-ethylpyrrolidin-2-yl)methylamino)methyl)-4-((4'-(trifluorome thyl)biphenyl-4-yl)methy 1)-4*H*-1,2,4-triazol-3-yl)methyl)-2-(4-fluorobenzylt hio)-6,7-dihydro-1*H*-cyclopenta[*d*]pyrimidin-4(5 *H*)-one** | 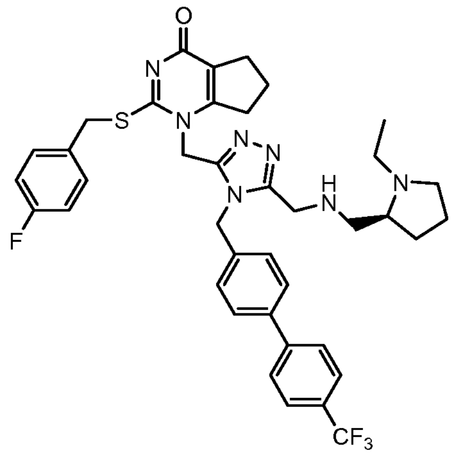 | (*S*)-(1-ethylpyrrolidin-2-yl)methanamine |

(continued)

| Name | Structure | Reactant |
| --- | --- | --- |
| **Example 107:**<br>**2-(4-fluorobenzylthio)-1-((5-((4-methylpiperazin-1-yl)methyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one** | 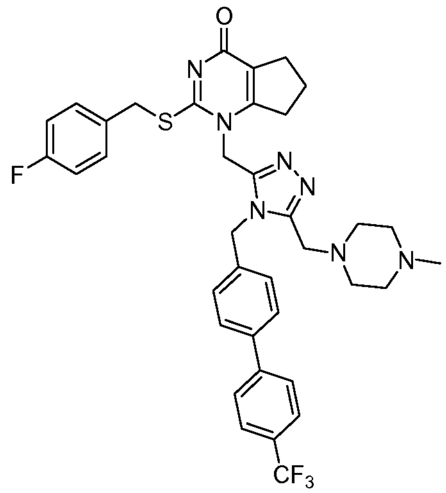 | 1-methylpiperazine |

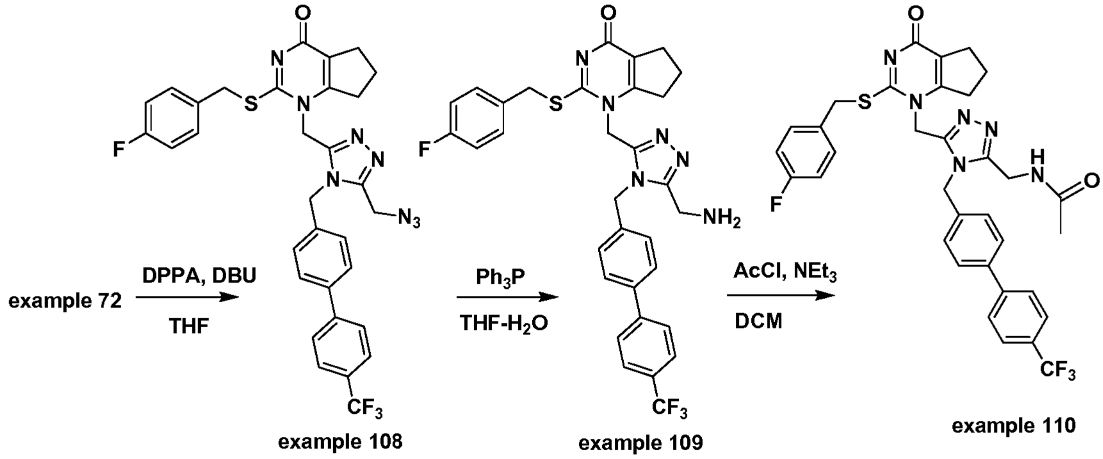


example 108 example 109 example 110

**Example 108-1-((5-(azidomethyl)-4-((4'-(trifluoromethyl)biphenyl-4-yl)methyl)-4*H*-1,2,4-triazo 1-3-yl)methyl)-2-(4-fluorobenzylthio)-6,7-dihydro-1*H*-cyclopenta[*d*]pyrimidin-4(5 H)-one**

Following a procedure similar to that described for the preparation of intermediate **E1** except that example **72** was used instead of **D18**. $^1$H-NMR ($CDCl_3$, 400 MHz) δ1.99 (m, 2H), 2.66 (t, 2H), 2.80 (t, 2H), 4.42 (s, 2H), 4.60 (s, 2H), 5.11 (s, 2H), 5.33 (s, 2H), 6.91 (t, 2H), 6.91 (d, 2H), 7.29 (dd, 2H), 7.47 (d, 2H), 7.60 (d, 2H), 7.71 (d, 2H).

**Example 109-1-((5-(aminomethyl)-4-((4'-(trifluoromethyl)biphenyl-4-yl)methyl)-4*H*-1,2,4-triaz ol-3-yl)methyl)-2-(4-fluorobenzylthio)-6,7-dihydro-1*H*-cyclopenta[*d*]pyrimidin-4( 5H)-one**

Following a procedure similar to that described for the preparation of intermediate **E2** except that example **108** was used instead of **E1**. $^1$H-NMR ($CDCl_3$, 400 MHz) δ1.95 (m, 2H), 2.60 (t, 2H), 2.72 (t, 2H), 4.09 (s, 2H), 4.43 (s, 2H), 5.03 (s, 2H), 5.40 (s, 2H), 6.92 (m, 4H), 7.30 (dd, 2H), 7.44 (d, 2H), 7.60 (d, 2H), 7.70 (d, 2H).

**Example 110-*N*-((5-((2-(4-fluorobenzylthio)-4-oxo-4,5,6,7-tetrahydro-1*H*-cyclopenta[*d*]pyrimid in-1-yl)methyl)-4-((4'-(trifluoromethyl)biphenyl-4-yl)methyl)-4*H*-1,2,4-triazol-3-y l)methyl)acetamide**

To the compound of example **109** (20mg, 1equiv) in $CH_2Cl_2$ (2ml) were added triethylamine (10μl, 2equiv) and acetyl chloride (5μl, 2equiv) (a $CaCl_2$ drying tube was used to insulate moisture) and the mixture was stirred at room temperature for 1h. Then the mixture was transferred to a separating funnel and washed with brine (X3). The organic phase was dried and concentrated. The crude product was purified by preparative TLC to afford **110** (15mg) as a solid. $^1$H-NMR ($CDCl_3$, 400 MHz) δ2.00 (m, 2H), 2.29 (s, 3H), 2.67 (t, 2H), 2.79 (t, 2H), 4.39 (s, 2H), 4.66 (s, 2H), 5.09 (s, 2H), 5.48 (s, 2H), 6.90 (t, 2H), 6.99 (d, 2H), 7.07 (vbrs, 1H), 7.28 (dd, 2H), 7.46 (d, 2H), 7.58 (d, 2H), 7.70 (d, 2H).

**Example 111-*N*-((5-((2-(4-fluorobenzylthio)-4-oxo-4,5,6,7-tetrahydro-1*H*-cyclopenta[*d*]pyrimid in-1-yl)methyl)-4-((4'-(trifluoromethyl)biphenyl-4-yl)methyl)-4*H*-1,2,4-triazol-3-y l)methyl)ethanesulfonamide**

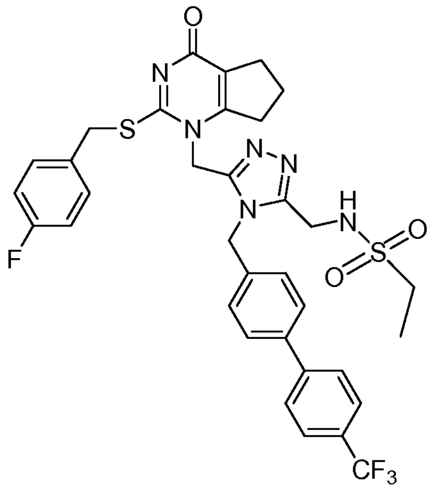

Following a procedure similar to that described for the preparation of example **110** except that ethanesulfonyl chloride was used instead of ethanoyl chloride. $^1$H-NMR ($CDCl_3$, 400 MHz) δ 1.37 (t, 3H), 2.00 (m, 2H), 2.69 (t, 2H), 2.77 (t, 2H), 3.10 (q, 2H), 4.40 (s, 2H), 4.47 (s, 2H), 5.03 (s, 2H), 5.41 (s, 2H), 6.11 (vbrs, 1H), 6.90 (t, 2H), 7.02 (d, 2H), 7.27 (m, 2H), 7.46 (d, 2H), 7.58 (d, 2H), 7.70 (d, 2H).

**Example 112-2-(4-fluorobenzylthio)-1-((5-((2-oxoimidazolidin-1-yl)methyl)-4-((4'-(trifluoromet hyl)biphenyl-4-yl)methyl)-4*H*-1,2,4-triazol-3-yl)methyl)-6,7-dihydro-1*H*-cyclopen ta[*d*]pyrimidin-4(5*H*)-one**

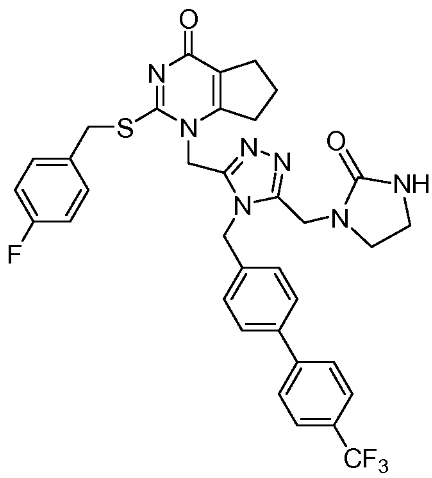

To a mixture of **109** (25mg) in EtOH (2ml) were added triethylamine (12μl) and 2-chloroethyl isocyanate (6μl).The mixture was stirred over night at room temperature and then concentrated under reduced pressure. The residue was dissolved in $CH_2Cl_2$ (5ml), transferred to a separating funnel, washed with brine (X3). The organic phase was dried and concentrated. The crude product was purified by preparative TLC to afford **112** (15mg) as a solid. $^1$H-NMR ($CDCl_3$, 400 MHz) δ 1.94 (m, 2H), 2.66 (m, 4H), 3.40 (t, 2H), 3.45 (t, 2H), 4.34 (s, 2H), 4.61 (s, 2H), 5.03 (s, 2H), 5.54 (s, 2H), 6.33 (vbrs, 1H), 6.85 (t, 2H), 7.05 (d, 2H), 7.22 (dd, 2H), 7.32 (vbrs, 1H), 7.46 (d, 2H), 7.58 (d, 2H), 7.69 (d, 2H).

**Example 113-1-((5-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)-4*H*-1,2,4-triazol-3-yl )methyl)-3-methylthiourea**

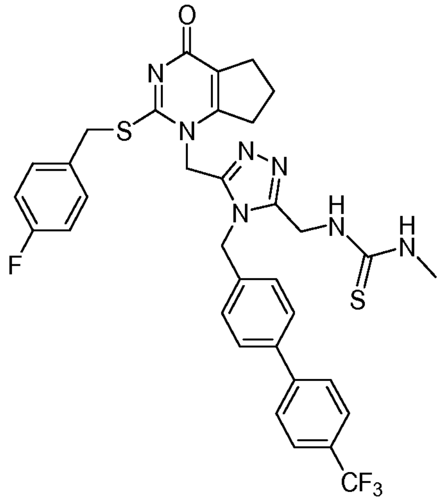

To a solution of the compound of Example **109** (20mg) in $CH_2Cl_2$ (2ml) was added isothiocyanatomethane (5mg). The mixture was stirred under reflux for 2h and then purified by preparative TLC to afford **113** (12mg) as a solid. $^1$H-NMR ($CDCl_3$, 400 MHz) δ 1.98 (m, 2H), 2.69 (m, 4H), 2.91 (s, 3H), 4.35 (s, 2H), 5.00 (s, 2H), 5.09 (s, 2H), 5.71 (s, 2H), 6.83 (t, 2H), 7.13 (d, 2H), 7.20 (dd, 2H), 7.50 (m, 3H), 7.59 (d, 2H), 7.70 (d, 2H), 9.07 (vbrs, 1H).

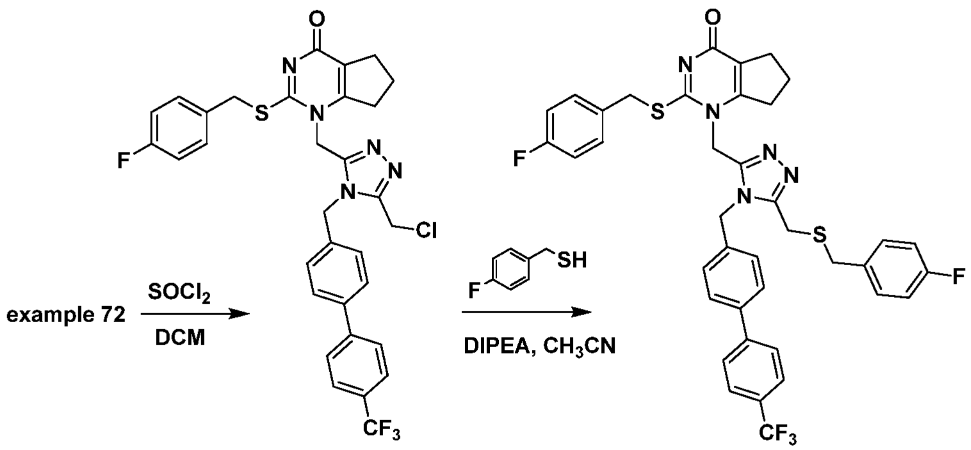


**example 114** **example 115**

**Example 114-1-((5-(chloromethyl)-4-((4'-(trifluoromethyl)biphenyl-4-yl)methyl)-4*H*-1,2,4-triaz ol-3-yl)methyl)-2-(4-fluorobenzylthio)-6,7-dihydro-1*H*-cyclopenta[*d*]pyrimidin-4( 5*H*)-one**

To the compound of Example **72** (200mg, 1equiv) in dry $CH_2Cl_2$ (5ml) in a flaskwas added DMF (1ml). $CaCl_2$ drying tube was used to insulate moisture and the flask was placed in an ice bath. To the mixture was added thionyl chloride (30μl, 1.2equiv). The mixture was stirred for 0.5h before it was quenched with saturated $NaHCO_3$ solution. The organic phase was separated and washed with brine (X1), dried over $Na_2SO_4$ and concentrated to afford **114** (180mg) as a solid which could be used in next step without further purification.

**Example 115-2-(4-fluorobenzylthio)-1-((5-((4-fluorobenzylthio)methyl)-4-((4'-(trifluoromethyl) biphenyl-4-yl)methyl)-4*H*-1,2,4-triazol-3-yl)methyl)-6,7-dihydro-1*H*-cyclopenta[*d* ]pyrimidin-4(5*H*)-one**

Following a procedure similar to that described for the preparation of intermediate **D36** except that the compound of example **114** was used instead **of D19** and "4-fluoro benzyl mercaptan" was used in place of "benzyl mercaptan". $^1$H-NMR ($CDCl_3$, 300 MHz) δ1.98 (m, 2H), 2.63 (t, 2H), 2.76 (t, 2H), 2.91 (t, 2H), 3.69 (s, 4H), 4.46 (s, 2H), 5.04 (s, 2H), 5.26 (s, 2H), 6.95 (m, 6H), 7.34 (m, 4H), 7.43 (d, 2H), 7.60 (d, 2H), 7.71 (d, 2H).

**Example 116-(*E*)-1-((5-((2,2-dimethylhydrazono)methyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cycl openta[*d*]pyrimidin-4(5*H*)-one**

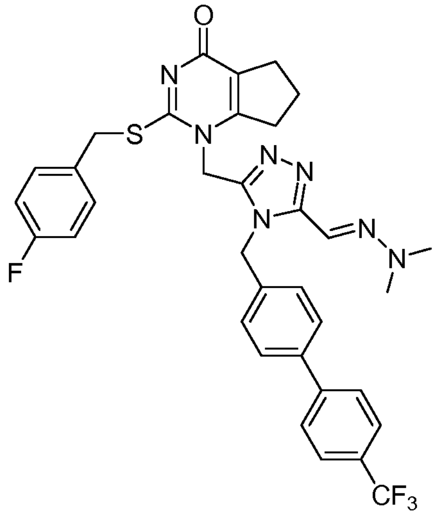

A mixture of example **74** (30mg, 1equiv), DIPEA (16μl, 2equiv), 1,1-dimethylhydrazine monohydrochloride (7mg, 1.5equiv) and 4Å molecular sieve (0.5g) in dry $CH_2Cl_2$ (3ml) was shaked occasionally for 2h. The mixture was then filtrated to remove the molecular sieve and the filtrate was washed with brine (X3), dried and then purified by preparative TLC to afford example **116** (22mg) as a solid. $^1$H-NMR ($CDCl_3$, 300 MHz) δ1.90 (m, 2H), 2.57 (t, 2H), 2.65 (t, 2H), 2.97 (s, 6H), 4.45 (s, 2H), 5.08 (s, 2H), 5.71 (s, 2H), 6.93 (t, 2H), 7.96 (d, 2H), 7.32 (dd, 2H), 7.35 (s, 1H), 7.42 (d, 2H), 7.62 (d, 2H), 7.69 (d, 2H); MS (ESI): 662 (M+H).

**Example 117-(*E*)-2-(4-fluorobenzylthio)-1-((5-((piperidin-1-ylimino)methyl)-4-((4'-(trifluorome thyl)biphenyl-4-yl)methyl)-4*H*-1,2,4-triazol-3-yl)methyl)-6,7-dihydro-1*H*-cyclope nta[*d*]pyrimidin-4(5*H*)-one**

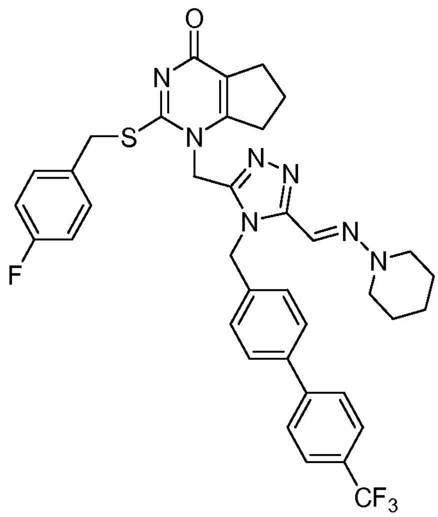

Following a procedure similar to that described for the preparation of example **116** except that piperidin-1-amine was used instead of 1,1-dimethylhydrazine monohydrochloride. $^1$H-NMR ($CDCl_3$, 300 MHz) δ1.53 (m, 2H), 1.67 (m, 4H), 1.91 (m, 2H), 2.58 (t, 2H), 2.65 (t, 2H), 3.11 (t, 4H), 4.45 (s, 2H), 5.08 (s, 2H), 5.74 (s, 2H), 6.93 (t, 2H), 7.97 (d, 2H), 7.31 (dd, 2H), 7.42 (d, 2H), 7.62 (d, 2H), 7.68 (s, 1H), 7.69 (d, 2H).

The following compounds were also prepared by a procedure similar to that described for the preparation of example 116:

| Name | Structure | Reactant |
| --- | --- | --- |
| **Example 118:**<br>**(*E*)-1-((5-((2-tert-butylhydrazono)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one** | 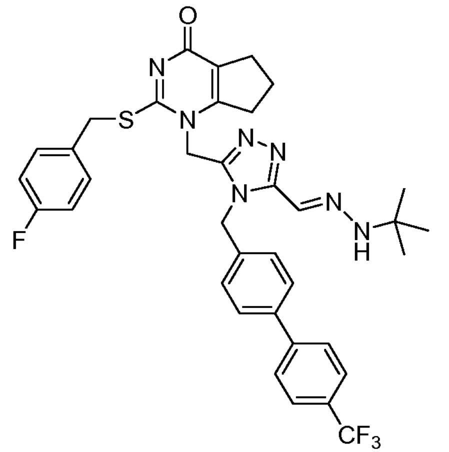 | tert-Butylhydrazine monohydrochloride, DIPEA |

**Example 119-1-(5-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)-4*H*-1,2,4-triazol-3-yl )vinyl acetate**

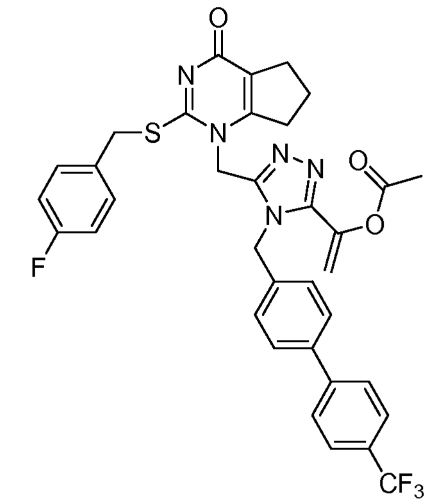

To a mixture of example **73** (920mg, 1equiv) and triethylamine (0.65ml, 3equiv) in $CH_2Cl_2$ (10ml) placed in an ice bath was added acetyl chloride (0.28ml, 2.5 equiv) slowly in a nitrogen atmosphere. The mixture was stirred for 2h before it was transferred to a separating funnel and washed with brine (X3). The organic phase was dried and concentrated. The crude product was purified by flash chromatography to afford example **119** (0.78g) as a jelly.$^1$H-NMR ($CDCl_3$, 400 MHz) $\delta$ 1.99 (m, 2H), 2.07 (s, 3H), 2.66 (t, 2H), 2.77 (t, 2H), 4.42 (s, 2H), 5.03 (s, 2H), 5.42 (s, 2H), 5.43 (d, 1H, *J*=2.4), 5.49 (d, 1H, *J*=2.8), 6.95 (m, 4H), 7.31 (dd, 2H, *J*=8.8, 5.6), 7.47 (d, 2H, *J*=8.0), 7.61 (d, 2H), 7.71 (d, 2H).

**Example 120-2-(4-fluorobenzylthio)-1-((5-(1-hydroxyethyl)-4-((4'-(trifluoromethyl)biphenyl-4-yl)methyl)-4*H*-1,2,4-triazol-3-yl)methyl)-6,7-dihydro-1*H*-cyclopenta[*d*]pyrimidin-4(5*H*)-one**

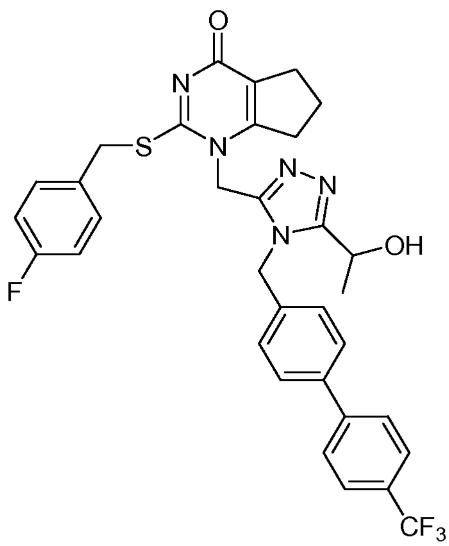

(**Method 1**) To a solution of the compound of example **74** (200mg, 1equiv) in sufficiently dried THF (3ml) placed in an ice bath was added methylmagnesium bromide solution (0.36ml, 1mol/L in THF,1.1equiv) in a nitrogen atmosphere. The mixture was stirred for 2h before it was quenched with saturated aqueous $NH_4Cl$ solution and extracted with EA. The combined organic phase was dried and then concentrated. The crude product was purified by column chromatog-

raphy ($CH_2Cl_2/CH_3OH$=15/1) to afford example **120** (100mg) as a solid.

(**Method 2**) To a solution of the compound of example **119** (760mg) and $NaBH_4$ (85mg) in EtOH (5ml) in an ice bath was stirred for 1h and then evaporated to remove the solvent. The residue was diluted with water, placed in an ice bath followed by addition of concd. HCl under stirring until no more bubbles was generated. Then the mixture was quenched with saturated aqueous $NaHCO_3$ solution and extracted with DCM (X3). The combined organic phase was dried over $Na_2SO_4$ and concentrated. The crude product was purified by flash chromatography to afford example **120** (550mg) as a solid.$^1$H-NMR ($d_6$-DMSO, 300 MHz) δ 1.53 (d, 3H), 1.90 (m, 2H), 2.54 (t, 2H), 2.67 (t, 2H), 4.27 (s, 2H), 4.91 (m, 1H), 5.10 (2x d, 2H), 5.48 (s, 2H), 6.99 (t, 2H), 7.24 (d, 2H), 7.31 (t, 2H), 7.63 (d, 2H), 7.80 (m, 4H).

**Example 121-1-((5-acetyl-4-((4'-(trifluoromethyl)biphenyl-4-yl)methyl)-4*H*-1,2,4-triazol-3-yl)m ethyl)-2-(4-fluorobenzylthio)-6,7-dihydro-1*H*-cyclopenta[*d*]pyrimidin-4(5*H*)-one**

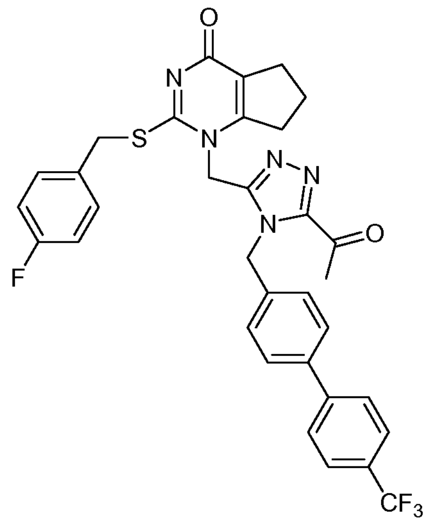

(**Method 1**) To a solution of the compound of example **119** (0.5g) in isopropanol (10ml) was added 6N HCl (1ml) and the mixture was stirred at room temperature over night. Then the mixture was concentrated to remove the solvent. The residue was dissolved in $CH_2Cl_2$ (10ml), washed with brine (X3), dried and concentrated. The crude product was purified by flash chromatography to afford example **121** (230mg) as a solid.

(**Method 2**) To a solution of example **120** (90mg, 1equiv) in 1,4-dioxane (5ml) was added activated $MnO_2$ (123mg, 10equiv). The mixture was stirred for 2h at 70°C and then filtered to remove $MnO_2$. The filtrate was evaporated to give a crude product which was then purified by flash chromatography to afford example **121** (50mg) as a solid. $^1$H-NMR ($CDCl_3$, 400 MHz) δ 1.99 (m, 2H), 2.69 (m, 4H), 2.83 (s, 3H), 4.43 (s, 2H), 5.08 (s, 2H), 5.73 (s, 2H), 6.91 (t, 2H), 7.00 (d, 2H), 7.27(dd, 2H), 7.46 (d, 2H), 7.59 (d, 2H), 7.70 (d, 2H).

**Example 122-*N*-((5-((2-(4-fluorobenzylthio)-4-oxo-4,5,6,7-tetrahydro-1*H*-cyclopenta[*d*]pyrimid in-1-yl)methyl)-4-((4'-(trifluoromethyl)biphenyl-4-yl)methyl)-4*H*-1,2,4-triazol-3-y l)methyl)-2-hydroxy-*N*,*N*-dimethylethanaminium chloride**

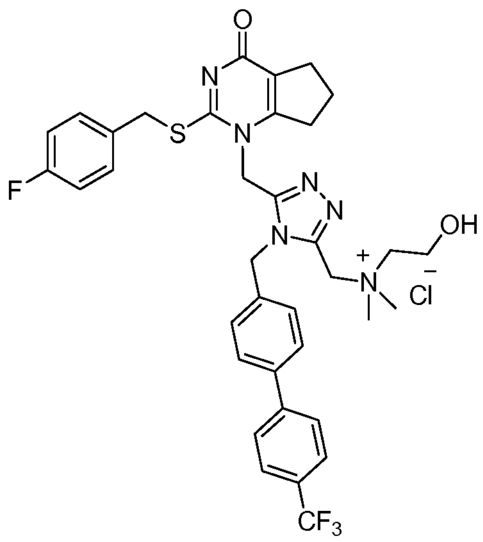

The mixture of **114** (50mg), $Cs_2CO_3$ (50mg) and N,N-Dimethylethanolamine (10μl) in acetonitrile (2ml) was heated under reflux for 2h and the reaction was complete. The mixture was filtered to remove the insoluble materials and the solvent was evaporated under reduced pressure to give a residue which was crystallized from acetone to afford example **122** as a white solid. $^1$H-NMR ($CD_3OD$, 400 MHz) δ 2.08 (m, 2H), 2.71 (t, 2H), 2.91 (t, 2H), 3.39 (s, 6H), 3.77 (t, 2H), 4.08 (t, 2H), 4.36 (s, 2H), 5.09 (s, 2H), 5.36 (s, 2H), 5.62 (s, 2H), 6.87 (t, 2H), 7.20 (d, 2H), 7.29 (dd, 2H), 7.59 (d, 2H), 7.71 (d, 2H), 7.75 (d, 2H).

**Example 123-2-(4-fluorobenzylthio)-1-((5-((2-hydroxyethoxy)methyl)-4-((4'-(trifluoromethyl)bi phenyl-4-yl)methyl)-4*H*-1,2,4-triazol-3-yl)methyl)-6,7-dihydro-1*H*-cyclopenta[*d*]p yrimidin-4(5*H*)-one**

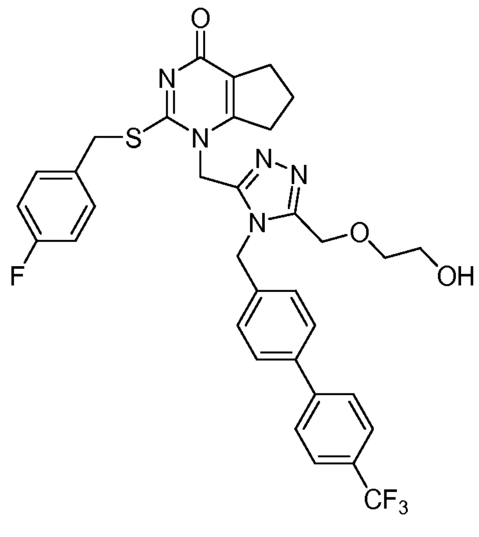

To an ice cooled suspension of NaH (55-65% suspended in mineral oil, 24mg, 1.5equiv) in anhydrous DMF (2ml) was added ethylene glycol (42μl, 2equiv) under a nitrogen atmosphere. The mixture was stirred for 0.5h, followed by addition of a solution of the compound of example **114** (250mg) in DMF (0.5ml) over 2min. The resulting solution was stirred for 15min before it was quenched with saturated aqueous $NH_4Cl$ solution. The mixture was extracted with DCM (X2) and the combined organic phase was washed with brine (X2), dried over $Na_2SO_4$ and concentrated. The crude product was purified by flash chromatography to afford example **123** (80mg) as a solid. $^1$H-NMR ($CDCl_3$, 300 MHz) δ1.96 (m, 2H), 2.63 (t, 2H), 2.73 (t, 2H), 3.67 (m, 4H), 4.42 (s, 2H),4.78 (s, 2H), 5.03 (s, 2H), 5.36 (s, 2H), 6.91 (t, 2H), 6.98 (d, 2H), 7.29 (dd, 2H), 7.45 (d, 2H), 7.60 (d, 2H), 7.70 (d, 2H).

**Example 124-2-((5-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)-4*H*-1,2,4-triazol-3-yl )methoxy)acetaldehyde**

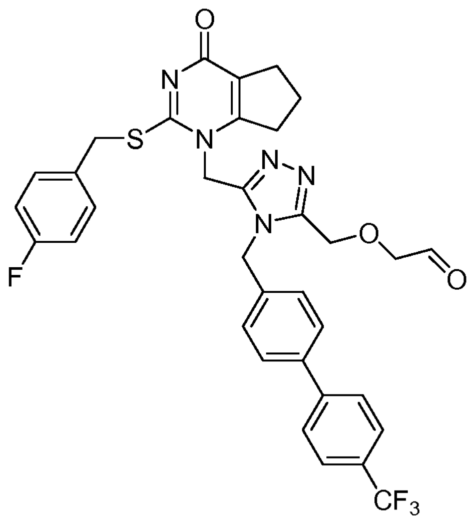

Following a procedure similar to that described for the preparation of intermediate **D41** except that the compound of example **123** was used instead of **D40.**

**Example 125-1-((5-((2-(diethylamino)ethoxy)methyl)-4-((4'-(tritluoromethyl)biphenyl-4-yl)met hyl)-4*H*-1,2,4-triazol-3-yl)methyl)-2-(4-fluorobenzylthio)-6,7-dihydro-1*H*-cyclope nta[*d*]pyrimidin-4(5*H*)-one**

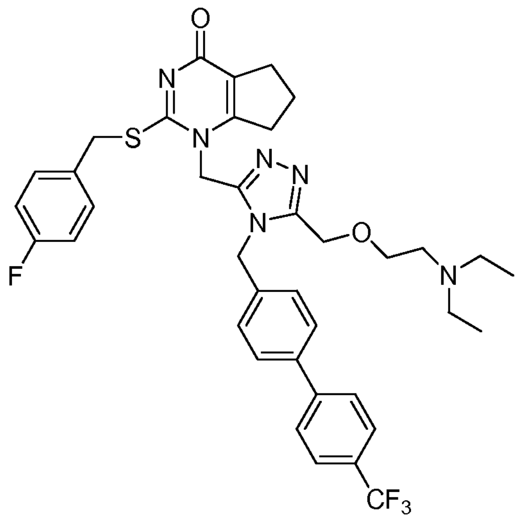

To a mixture of the compound of example **124** (20mg) and diethylamine (5μl) in $CH_2Cl_2$ (2ml) was added $NaBH(OAc)_3$ (10mg) and the mixture was stirred for 0.5h at room temperature. Then the solution was washed with saturated aqueous $NaHCO_3$ (X2) solution, dried over $Na_2SO_4$ and concentrated. The crude product was purified by preparative TLC (spread with DCM/MeOH=5/1) to afford example **125** (10mg) as a solid. $^1$H-NMR ($CDCl_3$, 300 MHz) δ 1.02 (t, 6H), 1.96 (m, 2H), 2.59-2.78 (m, 10H), 3.68 (t, 2H), 4.42 (s, 2H), 4.75 (s, 2H), 5.04 (s, 2H), 5.42 (s, 2H), 6.91 (t, 2H), 6.97 (d, 2H), 7.29 (dd, 2H), 7.43 (d, 2H), 7.59 (d, 2H), 7.70 (d, 2H); MS(ESI): 721(M+H).

**Example 126-1-((5-(n-butoxymethyl)-4-((4'-(trifluoromethyl)biphenyl-4-yl)methyl)-4*H*-1,2,4-tri azol-3-yl)methyl)-2-(4-fluorobenzylthio)-6,7-dihydro-1*H*-cyclopenta[*d*]pyrimidin-4(5*H*)-one**

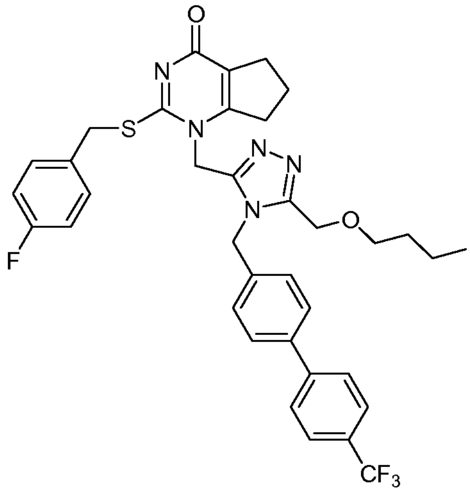

Following a procedure similar to that described for the preparation of example **123** except that 1-butanol was used instead of ethylene glycol. $^1$H-NMR ($CDCl_3$, 400 MHz) δ 0.86 (t, 3H), 1.29 (m, 2H), 1.41 (m, 2H), 2.06 (m, 2H), 2.63 (t, 2H), 3.11 (t, 2H), 3.60 (s, 2H), 3.69 (s, 2H), 5.10 (s, 2H), 5.53 (s, 2H), 6.89 (d, 2H), 6.98 (t, 2H), 7.35 (dd, 2H), 7.46 (d, 2H), 7.60 (d, 2H), 7.69 (d, 2H).

**Example 127-1-((5-(1-chloroethyl)-4-((4'-(tritluoromethyl)biphenyl-4-yl)methyl)-4*H*-1,2,4-triaz ol-3-yl)methyl)-2-(4-fluorobenzylthio)-6,7-dihydro-1*H*-cyclopenta[*d*]pyrimidin-4( 5*H*)-one**

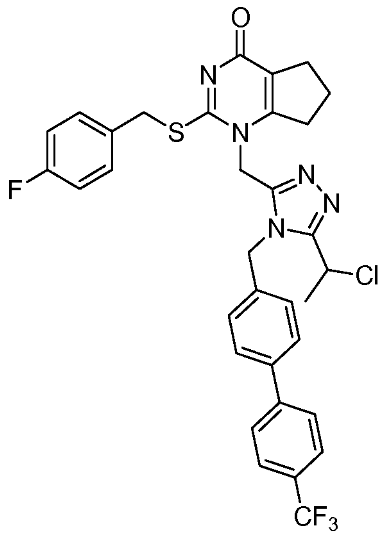

Following a procedure similar to that described for the preparation of example **114** except that example **120** was used instead of example **72.**

**Example 128 1-((5-(1-(2-(dimethylamino)ethylamino)ethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-c yclopenta[*d*]pyrimidin-4(5*H*)-one**

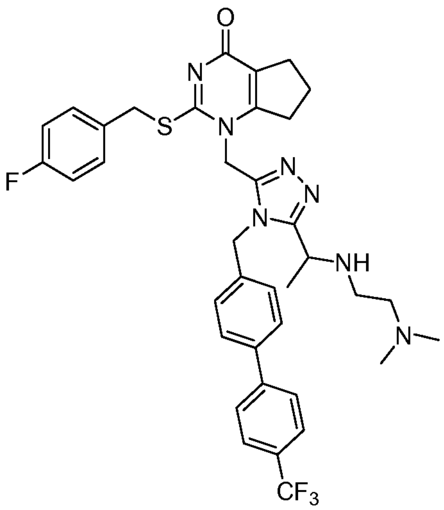

The mixture of the compound of Example **127** (50mg, 1equiv), *N*,*N*-Dimethylethylenediamine (18μl, 2.1equiv) and KI (1mg) in acetonitrile (2ml) was heated under reflux until the completion of the reaction monitored by TLC. Then solvent was removed under reduced pressure and the residue was dissolved in $CH_2Cl_2$, washed with brine (X3), dried over $Na_2SO_4$ and concentrated. The crude product was purified by preparative TLC (spread with DCM/MeOH=5/1) to afford example **128** (35mg) as a solid. $^1$H-NMR ($CDCl_3$, 400 MHz) δ1.53 (d, 3H), 1.98 (m, 2H), 2.24 (s, 6H), 2.37 (m, 2H), 2.55-2.78 (m, 6H), 4.11 (q, 1H), 4.43 (s, 2H), 4.98 (s, 2H), 5.53 (2x d, 2H), 6.92 (m, 4H), 7.29 (dd, 2H), 7.45 (d, 2H), 7.60 (d, 2H), 7.70 (d, 2H).

**Example 129-1-((5-(1-((2-(dimethylamino)ethyl)(methyl)amino)ethyl)-4-((4'-(tritluoromethyl)b iphenyl-4-yl)methyl)-4*H*-1,2,4-triazol-3-yl)methyl)-2-(4-fluorobenzylthio)-6,7-dih ydro-1*H*-cyclopenta[*d*]pyrimidin-4(5*H*)-one**

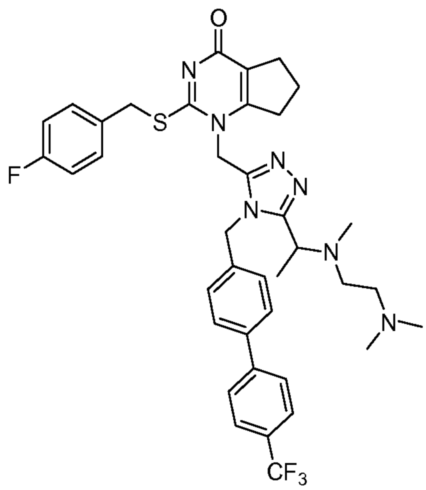

Following a procedure similar to that described for the preparation of example **128** except that *N*,*N*,*N*-trimethylethylenediamine was used instead of *N*,*N*-Dimethylethylenediamine.$^1$H-NMR ($CDCl_3$, 400 MHz) δ1.48 (d, 3H), 2.01 (m, 2H), 2.35 (s, 3H), 2.45 (s, 6H), 2.59-3.00 (m, 8H), 4.01 (q, 1H), 4.40 (2x d, 2H), 5.00 (d, 1H), 5.26 (d, 1H), 5.56 (d, 1H), 5.74 (d, 1H), 6.87 (t, 2H), 6.99 (d, 2H), 7.26 (m, 2H), 7.40 (d, 2H), 7.56 (d, 2H), 7.69 (d, 2H).

**Example 130-1-((5-(1-(2-(diethylamino)ethylamino)ethyl)-4-((4'-(trifluoromethyl)biphenyl-4-yl )methyl)-4*H*-1,2,4-triazol-3-yl)methyl)-2-(4-fluorobenzylthio)-6,7-dihydro-1*H*-cyc lopenta[*d*]pyrimidin-4(5*H*)-one**

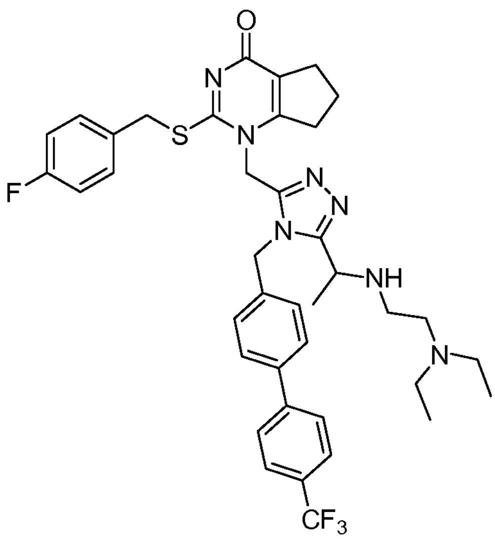

Following a procedure similar to that described for the preparation of example **128** except that *N*,*N*-diethylethylenediamine was used instead of *N*,*N*-Dimethylethylenediamine. $^{1}$H-NMR ($CDCl_3$, 300 MHz) δ1.10 (t, 6H), 1.53 (d, 3H), 1.99 (m, 2H), 2.60-2.85 (m, 12H), 4.21 (q, 1H), 4.43 (s, 2H), 4.98 (s, 2H), 5.57 (2x d, 2H), 6.93 (m, 4H), 7.30 (dd, 2H), 7.45 (d, 2H), 7.60 (d, 2H), 7.70 (d, 2H).

**Example 131a-1-((5-(1-((2-(diethylamino)ethyl)(methyl)amino)ethyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one**

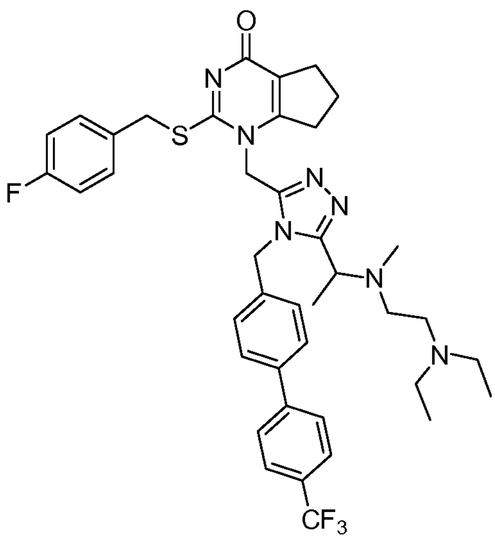

Following a procedure similar to that described for the preparation of example **128** except that *N*,*N*-diethyl-*N*'-methylethylenediamine was used instead of *N*,*N*-Dimethylethylenediamine.$^{1}$H-NMR ($CDCl_3$, 300 MHz) δ1.25 (m, 6H), 1.48 (d, 3H), 2.02 (m, 2H), 2.35 (s, 3H), 2.74 (t, 2H), 2.85-3.03 (m, 10H), 4.10 (q, 1H), 4.38 (2x d, 2H), 5.03 (d, 1H), 5.21 (d, 1H), 5.65 (2x d, 2H), 6.85 (t, 2H), 7.02 (d, 2H), 7.25 (dd, 2H), 7.39 (d, 2H), 7.55 (d, 2H), 7.69 (d, 2H).

**Example 131b-1-((5-(1-((2-(diethylamino)ethyl)(methyl)amino)ethyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one tartrate**

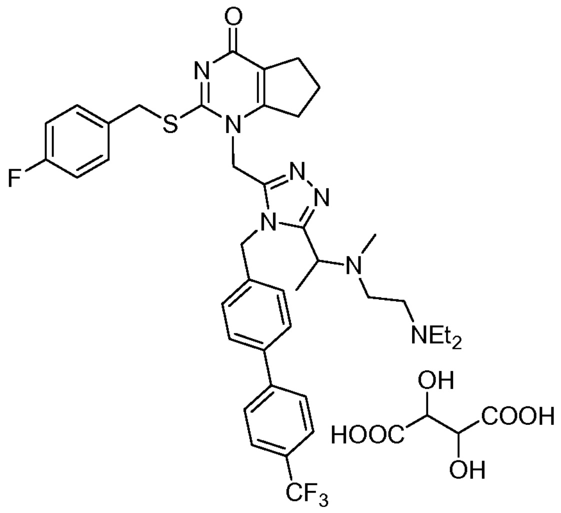

Following a procedure similar to that described for the preparation of example **91b** except that example **131a** was used instead of example **91a.** $^1$H-NMR ($d_6$-DMSO, 300 MHz) δ1.06 (t, 6H, *J*=6.9), 1.35 (d, 3H, *J*=6.0), 1.91 (m, 2H), 2.17 (s, 3H), 2.54 (t, 2H, *J*=7.2), 2.71(m, 4H), 2.91(m, 6H), 4.14 (q, 1H, *J*=6.0), 4.23 (s, 2H), 4.31 (s, 2H), 5.09/5.21 (2x d, 2H, *J*=12.9), 5.44/5.52 (2x d, 2H, *J*=16.5), 7.03 (t, 2H, *J*=8.4), 7.15 (d, 2H, *J*=7.8), 7.34 (dd, 2H, *J*=8.4, 6.0), 7.65 (d, 2H, *J*=7.8), 7.81 (m, 4H).

**Example 132-1-((5-(1-((3-(dimethylamino)propyl)(methyl)amino)ethyl)-4-((4'-(trifluoromethyl) biphenyl-4-yl)methyl)-4*H*-1,2,4-triazol-3-yl)methyl)-2-(4-fluorobenzylthio)-6,7-di hydro-1*H*-cyclopenta[*d*]pyrimidin-4(5*H*)-one**

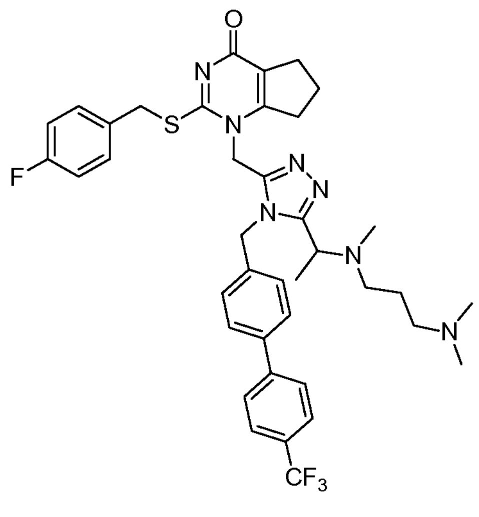

Following a procedure similar to that described for the preparation of example **128** except that *N*,*N*,*N*-trimethyl-1,3-propanediamine was used instead of *N*,*N*-dimethylethylenediamine. $^1$H-NMR ($CDCl_3$, 300 MHz) δ1.48 (d, 3H), 1.75 (m, 2H), 2.01 (m, 2H), 2.30 (s, 3H), 2.59 (m, 9H), 2.70 (t, 2H), 2.85 (m, 3H), 4.05 (q, 1H), 4.36 (2x d, 2H), 5.00 (d, 1H), 5.38 (d, 1H), 5.50 (d, 1H), 5.67 (d, 1H), 6.84 (t, 2H), 7.03 (d, 2H), 7.23 (m, 2H), 7.40 (d, 2H), 7.55 (d, 2H), 7.69 (d, 2H).

**Example 133-1-((5-(((2-(dimethylamino)ethyl)(ethyl)amino)methyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one**

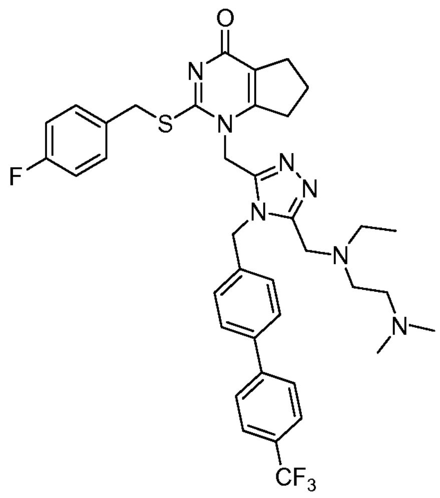

Following a procedure similar to that described for the preparation of example **79** except that *N*,*N*-dimethyl-*N*-ethylethylenediamine was used instead of diethylamine. $^1$H-NMR ($CDCl_3$, 300 MHz) δ1.11 (t, 3H), 2.03 (m, 2H), 2.61 (s, 6H), 2.69 (q, 2H), 2.75 (t, 2H), 2.97 (m, 6H), 3.77 (s, 2H), 4.39 (s, 2H), 5.18 (s, 2H), 5.58 (s, 2H), 6.86 (t, 2H), 7.03 (d, 2H), 7.25 (m, 2H), 7.40 (d, 2H), 7.56 (d, 2H), 7.70 (d, 2H).

**Example 134-ethyl-2-((2-(diethylamino)ethyl)((3-((2-(4-fluorobenzylthio)-4-oxo-4,5,6,7-tetrahy dro-1*H*-cyclopenta[*d*]pyrimidin-1-yl)methyl)-4-((4'-(trifluoromethyl)biphenyl-4-y l)methyl)-4*H*-1,2,4-triazol-5-yl)methyl)amino)acetate**

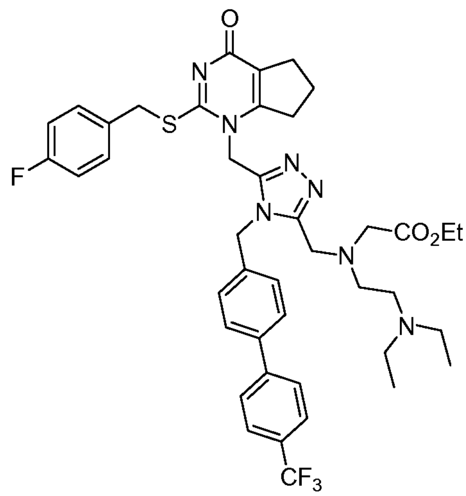

Following a procedure similar to that described for the preparation of example **79** except that ethyl-2-(2-(diethylamino)ethylamino)acetate was used instead of diethylamine. $^1$H-NMR ($CDCl_3$, 300 MHz) δ1.22 (m, 3H), 1.30 (m, 6H), 2.02 (m, 2H), 2.73 (t, 2H), 2.95 (t, 2H), 3.09 (m, 6H), 3.25 (t, 2H), 3.56 (s, 2H), 4.12 (q, 2H), 4.19 (s, 2H), 4.35 (s, 2H), 5.20 (s, 2H), 5.63 (s, 2H), 6.84 (t, 2H), 7.09 (d, 2H), 7.22 (dd, 2H), 7.41 (d, 2H), 7.55 (d, 2H), 7.70 (d, 2H).

**Example 135-2-((2-(diethylamino)ethyl)((3-((2-(4-fluorobenzylthio)-4-oxo-4,5,6,7-tetrahydro-1 *H*-cyclopenta[*d*]pyrimidin-1-yl)methyl)-4-((4'-(trifluoromethyl)biphenyl-4-yl)met hyl)-4*H*-1,2,4-triazol-5-yl)methyl)amino)acetic acid**

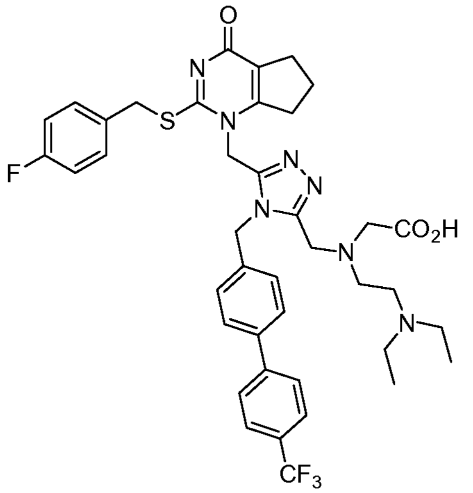

Following a procedure similar to that described for the preparation of example **63** except that example **134** was used instead of example **62.** $^{1}$H-NMR ($d_6$-DMSO, 300 MHz) δ0.98 (t, 6H), 1.91 (m, 2H), 2.54 (t, 2H), 2.65-2.75 (m, 10H), 3.13 (s, 2H), 3.93 (s, 2H), 4.30 (s, 2H), 5.16 (s, 2H), 5.48 (s, 2H), 7.03 (t, 2H), 7.19 (d, 2H), 7.33 (dd, 2H), 7.63 (d, 2H), 7.90 (m, 4H).

**Example 136-2-(4-fluorobenzylthio)-1-((5-((methyl(2-(pyrrolidin-1-yl)ethyl)amino)methyl)-4-(( 4'-(trifluoromethyl)biphenyl-4-yl)methyl)-4*H*-1,2,4-triazol-3-yl)methyl)-6,7-dihyd ro-1*H*-cyclopenta[*d*]pyrimidin-4(5*H*)-one**

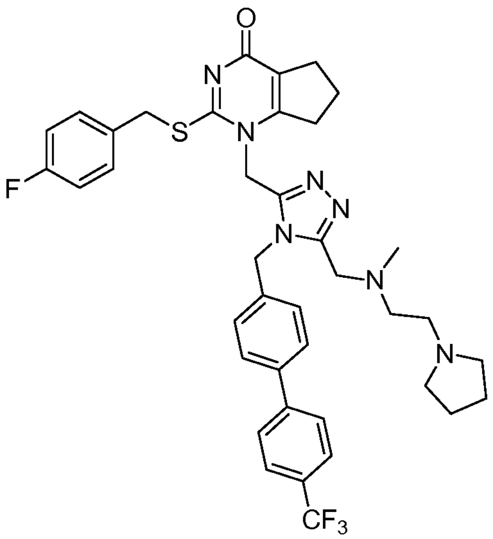

Following a procedure similar to that described for the preparation of example **79** except that *N*-methyl-2-(pyrrolidin-1-yl)ethanamine was used instead of diethylamine. $^{1}$H-NMR ($CDCl_3$, 300 MHz) δ2.00 (m, 6H), 2.37 (s, 3H), 2.73 (t, 2H), 2.91 (t, 2H), 3.07 (t, 2H), 3.77 (s, 2H), 4.39 (s, 2H), 5.14 (s, 2H), 5.59 (s, 2H), 6.86 (t, 2H), 7.02 (d, 2H), 7.26 (dd, 2H), 7.40 (d, 2H), 7.56 (d, 2H), 7.70 (d, 2H).

**Example 137-*N*-(2-(diethylamino)ethyl)-2-((2-(4-fluorobenzylthio)-4-oxo-4,5,6,7-tetrahydro-1*H* -cyclopenta[*d*]pyrimidin-1-yl)methyl)-1-((4'-(trifluoromethyl)biphenyl-4-yl)meth yl)-1*H*-imidazole-5-carboxamide**

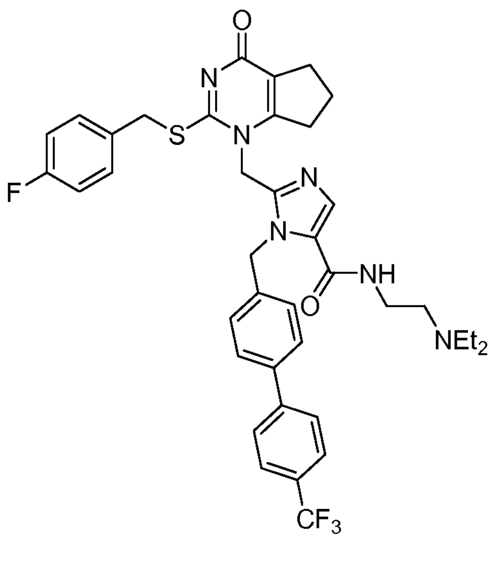

Following a procedure similar to that described for the preparation of example **51** except that *N*,*N*-diethylethylenediamine was used instead of dimethylamine hydrochloride. $^1$H-NMR ($CDCl_3$, 300 MHz) δ 1.38 (t, 6H), 1.96 (m, 2H), 2.66 (t, 4H), 3.17 (m, 6H), 3.78 (m, 2H), 4.42 (s, 2H), 4.96 (s, 2H), 5.74 (s, 2H), 6.90 (t, 2H), 7.01 (d, 2H), 7.29 (dd, 2H), 7.43 (d, 2H), 7.58 (d, 2H), 7.68 (d, 2H), 8.03 (s, 1H), 8.95 (t, 1H, -CONH-).

**Example 138-*N*-(2-(diethylamino)ethyl)-2-((2-(4-fluorobenzylthio)-4-oxo-4,5,6,7-tetrahydro-1*H* -cyclopenta[*d*]pyrimidin-1-yl)methyl)-*N*-methyl-1-((4'-(trifluoromethyl)biphenyl-4-yl)methyl)-1*H*-imidazole-5-carboxamide**

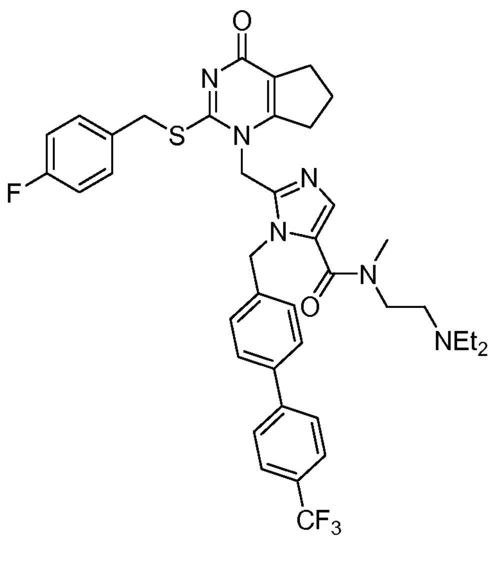

Following a procedure similar to that described for the preparation of example **51** except that *N*,*N*-diethyl-*N*'-methylethylenediamine was used instead of dimethylamine hydrochloride. $^1$H-NMR ($CDCl_3$, 300 MHz) δ 1.20 (t, 6H), 1.99 (m, 2H), 2.69 (m, 6H), 2.89 (m, 4H), 3.22 (s, 3H), 3.70 (t, 3H), 4.36 (s, 2H), 4.92 (s, 2H), 5.43 (s, 2H), 6.84 (t, 2H), 7.08 (d, 2H), 7.22 (dd, 2H), 7.26 (s, 1H), 7.43 (d, 2H), 7.55 (d, 2H), 7.65 (d, 2H).

**Example 139-1-((5-(((2-(diethylamino)ethyl)(methyl)amino)methyl)-1-((4'-(trifluoromethyl)bip henyl-4-yl)methyl)-1*H*-imidazol-2-yl)methyl)-2-(4-fluorobenzylthio)-6,7-dihydro-1*H*-cyclopenta[*d*]pyrimidin-4(5*H*)-one**

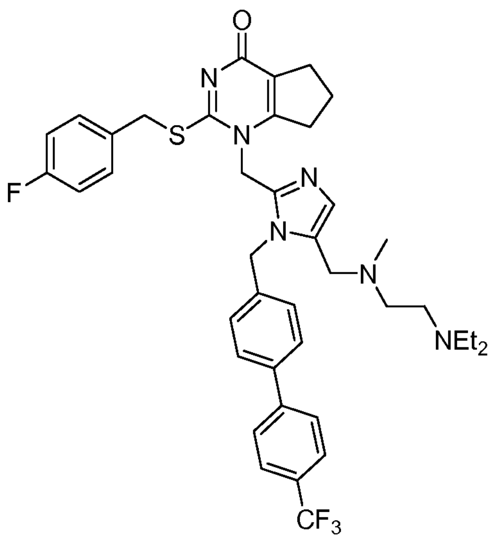

Following a procedure similar to that described for the preparation of example **45** except that *N*,*N*-diethyl-*N*'-methylethylenediamine was used instead of 1-benzylpiperazine. $^1$H-NMR ($CDCl_3$, 300 MHz) δ 1.08 (t, 6H), 1.98 (m, 2H), 2.21 (s, 3H), 2.67-2.85 (m, 12H), 3.47 (s, 2H), 4.42 (s, 2H), 4.95 (s, 2H), 5.46 (s, 2H), 6.89 (t, 2H), 6.95 (d, 2H), 6.96 (s, 1H), 7.27 (dd, 2H), 7.42 (d, 2H), 7.58 (d, 2H), 7.69 (d, 2H).

**Example 140-2-(4-fluorobenzylthio)-1-((5-((methyl(2-(piperidin-1-yl)ethyl)amino)methyl)-1-((4 '-(trifluoromethyl)biphenyl-4-yl)methyl)-1*H*-imidazol-2-yl)methyl)-6,7-dihydro-1 *H*-cyclopenta[*d*]pyrimidin-4(5*H*)-one**

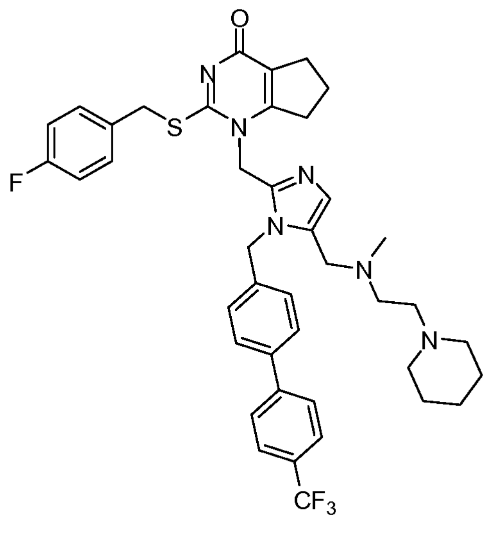

Following a procedure similar to that described for the preparation of example **45** except that *N*-methyl-2-(piperidin-1-yl)ethanamine was used instead of 1-benzylpiperazine. $^1$H-NMR ($CDCl_3$, 300 MHz) δ 1.37 (m, 2H), 1.48 (m, 4H), 1.95 (m, 2H), 2.16 (s, 3H), 2.33 (m, 4H), 2.40 (t, 2H), 2.54 (t, 2H), 2.64 (t, 2H), 2.68 (t, 2H), 3.46 (s, 2H), 4.43 (s, 2H), 4.92 (s, 2H), 5.44 (s, 2H), 6.92 (m, 5H), 7.31 (dd, 2H), 7.42 (d, 2H), 7.60 (d, 2H), 7.69 (d, 2H).

**Example 141-2-(4-fluorobenzylthio)-1-((5-(3-(pyrrolidin-1-yl)propyl)-4-((4'-(trifluoromethyl)bi phenyl-4-yl)methyl)-4*H*-1,2,4-triazol-3-yl)methyl)-6,7-dihydro-1*H*-cyclopenta[*d*]p yrimidin-4(5*H*)-one**

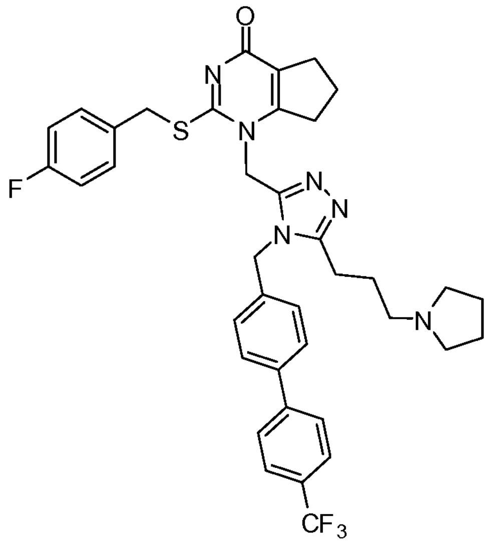

Following a procedure similar to that described for the preparation of example **70** except that pyrrolidine was used instead of diethylamine.$^1$H-NMR ($CDCl_3$, 400 MHz) δ 1.91 (m, 4H), 1.96 (m, 2H), 2.21 (m, 2H), 2.62 (t, 2H), 2.79 (m, 6H), 2.86 (t, 2H), 2.92 (t, 2H), 4.45 (s, 2H), 5.07 (s, 2H), 5.27 (s, 2H), 6.87 (d, 2H), 6.94 (t, 2H), 7.33 (dd, 2H), 7.43 (d, 2H), 7.60 (d, 2H), 7.70 (d, 2H).

**Example 142-2-(4-fluorobenzylthio)-1-((5-(3-(piperidin-1-yl)propyl)-4-((4'-(trifluoromethyl)bip henyl-4-yl)methyl)-4*H*-1,2,4-triazol-3-yl)methyl)-6,7-dihydro-1*H*-cyclopenta[*d*]py rimidin-4(5*H*)-one**

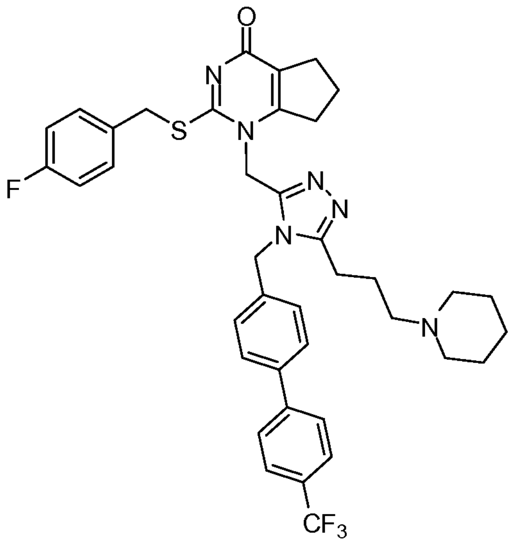

Following a procedure similar to that described for the preparation of example **70** except that piperidine was used instead of diethylamine.$^1$H-NMR ($CDCl_3$, 300 MHz) δ 1.46 (m, 2H), 1.61 (m, 4H), 1.94 (m, 2H), 2.10 (m, 2H), 2.47 (m, 4H), 2.54 (t, 2H), 2.59 (t, 2H), 2.75 (t, 2H), 2.83 (t, 2H), 4.46 (s, 2H), 5.08 (s, 2H), 5.27 (s, 2H), 6.83 (d, 2H), 6.95 (t, 2H), 7.33 (dd, 2H), 7.43 (d, 2H), 7.61 (d, 2H), 7.70 (d, 2H).

**Example 143-2-(4-fluorobenzylthio)-1-((5-(4-(pyrrolidin-1-yl)butyl)-4-((4'-(trifluoromethyl)bip henyl-4-yl)methyl)-4*H*-1,2,4-triazol-3-yl)methyl)-6,7-dihydro-1*H*-cyclopenta[*d*]py rimidin-4(5*H*)-one**

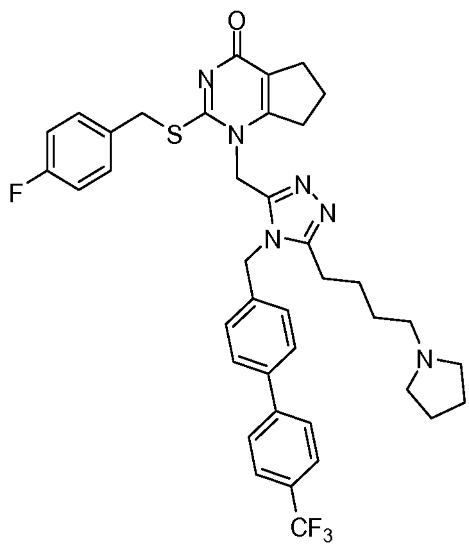

Following a procedure similar to that described for the preparation of example **71** except that pyrrolidine was used instead of diethylamine. $^{1}$H-NMR ($CDCl_3$, 300 MHz) δ 1.70 (m, 2H), 1.80 (m, 4H), 1.91 (m, 4H), 2.58 (m, 8H), 2.76 (m, 4H), 4.46 (s, 2H), 5.07 (s, 2H), 5.20 (s, 2H), 6.82 (d, 2H), 6.95 (t, 2H), 7.34 (dd, 2H), 7.44 (d, 2H), 7.62 (d, 2H), 7.70 (d, 2H).

**Example 144-2-(4-fluorobenzylthio)-1-((5-(4-(piperidin-1-yl)butyl)-4-((4'-(trifluoromethyl)biph enyl-4-yl)methyl)-4*H*-1,2,4-triazol-3-yl)methyl)-6,7-dihydro-1*H*-cyclopenta[*d*]pyr imidin-4(5*H*)-one**

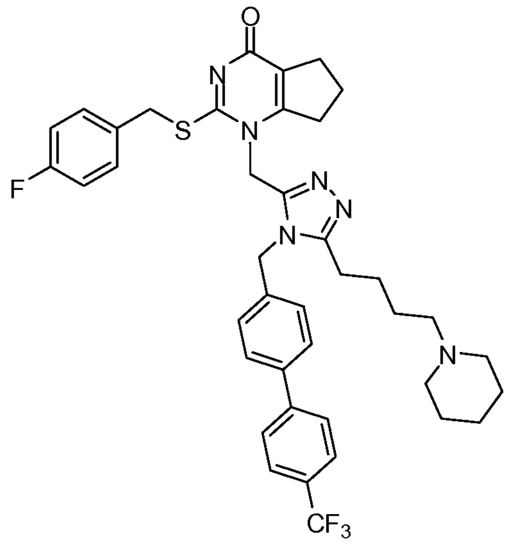

Following a procedure similar to that described for the preparation of example **71** except that piperidine was used instead of diethylamine. $^{1}$H-NMR ($CDCl_3$, 300 MHz) δ 1.80 (m, 2H), 2.00 (m, 2H), 2.18 (m, 6H), 2.58 (t, 2H), 2.66 (t, 2H), 2.85 (m, 4H), 2.95 (m, 2H), 3.50 (m, 2H), 4.40 (s, 2H), 5.11 (s, 2H), 5.29 (s, 2H), 6.89 (t, 2H), 6.95 (d, 2H), 7.28 (dd, 2H), 7.44 (d, 2H), 7.59 (d, 2H), 7.69 (d, 2H).

**Example 145-2-(4-fluorobenzylthio)-1-((5-((methyl(2-(piperidin-1-yl)ethyl)amino)methyl)-4-((4 '-(trifluoromethyl)biphenyl-4-yl)methyl)-4*H*-1,2,4-triazol-3-yl)methyl)-6,7-dihydr o-1*H*-cyclopenta[*d*]pyrimidin-4(5*H*)-one**

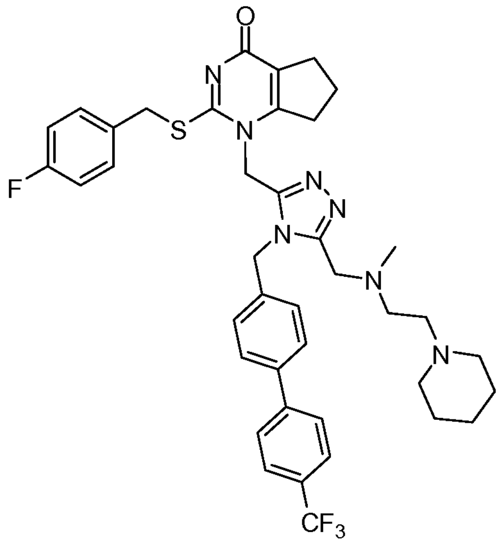

Following a procedure similar to that described for the preparation of example **79** except that *N*-methyl-2-(piperidin-1-yl)ethanamine was used instead of diethylamine. $^1$H-NMR ($CDCl_3$, 300 MHz) δ 1.47 (m, 2H), 1.66 (m, 4H), 1.98 (m, 2H), 2.29 (s, 3H), 2.67 (m, 4H), 2.80 (m, 8H), 3.82 (s, 2H), 4.40 (s, 2H), 5.07 (s, 2H), 5.57 (s, 2H), 6.88 (t, 2H), 6.98 (d, 2H), 7.29 (m, 2H), 7.41 (d, 2H), 7.58 (d, 2H), 7.70 (d, 2H).

**Example 146-1-((5-(((2-(diethylamino)ethyl)(methyl)amino)methyl)-1-((4'-(trifluoromethyl)bip henyl-4-yl)methyl)-1*H*-imidazol-2-yl)methyl)-2-(4-fluorobenzylthio)-6,7-dihydro-1*H*-cyclopenta[*d*]pyrimidin-4(5*H*)-one tartrate**

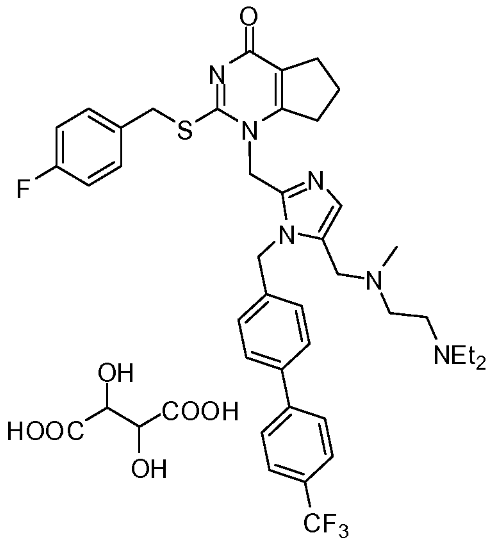

Following a procedure similar to that described for the preparation of example **91b** except that example **139** was used instead of example **91a.** $^1$H-NMR ($d_6$-DMSO, 400 MHz) δ 1.04 (t, 6H), 1.88 (m, 2H), 2.12 (s, 3H), 2.53 (t, 2H), 2.59 (t, 2H), 2.67 (t, 2H), 2.90 (q, 4H), 2.99 (t, 2H), 3.43 (s, 2H), 4.20 (s, 2H), 4.29 (s, 2H), 5.05 (s, 2H), 5.38 (s, 2H), 6.86 (s, 1H), 7.02 (d, 2H), 7.07 (t, 2H), 7.33 (dd, 2H ), 7.63 (d, 2H), 7.79 (m, 4H).

**Example 147-2-(4-fluorobenzylthio)-1-((5-((methyl(2-(piperidin-1-yl)ethyl)amino)methyl)-1-((4 '-(trifluoromethyl)biphenyl-4-yl)methyl)-1*H*-imidazol-2-yl)methyl)-6,7-dihydro-1 *H*-cyclopenta[*d*]pyrimidin-4(5*H*)-one tartrate**

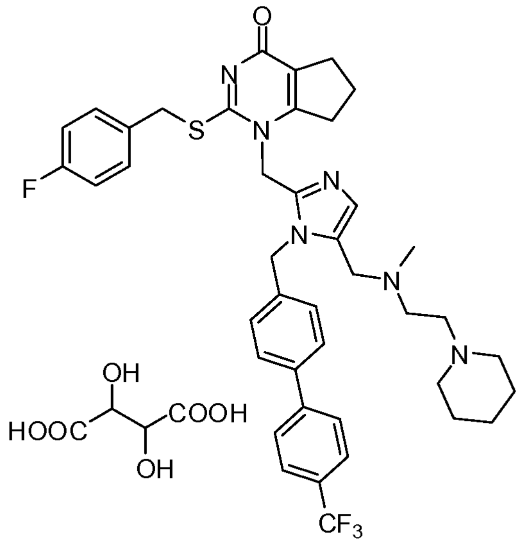

Following a procedure similar to that described for the preparation of example **91b** except that example **140** was used instead of example **91a.** $^1$H-NMR ($d_6$-DMSO, 300 MHz) δ 1.36 (m, 2H), 1.46 (m, 4H), 1.89 (m, 2H), 2.11 (s, 3H), 2.53 (m, 4H), 2.66 (m, 8H), 3.42 (s, 2H), 4.08 (s, 2H), 4.30 (s, 2H), 5.04 (s, 2H), 5.43 (s, 2H), 6.86 (s, 1H), 7.04 (t, 2H), 7.10 (d, 2H), 7.34 (dd, 2H), 7.64 (d, 2H), 7.81 (m, 2H).

**Example 148-1-((5-(3-(diethylamino)propyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]py rimidin-4(5*H*)-one tartrate**

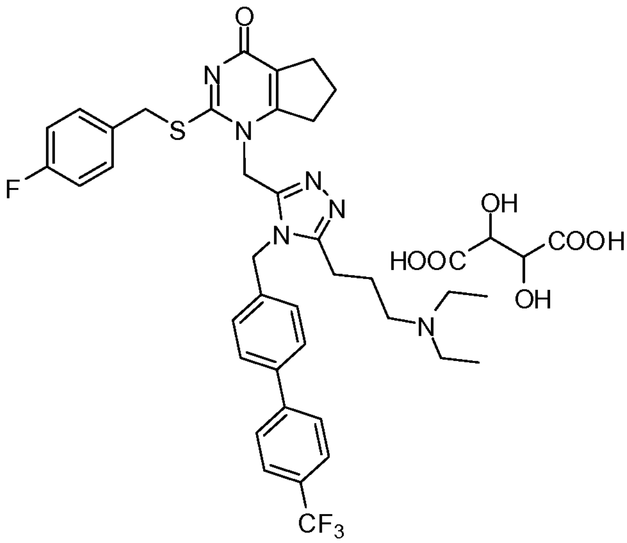

Following a procedure similar to that described for the preparation of example **91b** except that example **70** was used instead of example **91a.** $^1$H-NMR ($d_6$-DMSO, 300 MHz) δ 1.09 (t, 6H), 1.92 (m, 4H), 2.55 (t, 2H), 2.74 (q, 4H), 2.93 (m, 6H), 4.08 (s, 2H), 4.33 (s, 2H), 5.23 (s, 2H), 5.36 (s, 2H), 7.04 (t, 2H), 7.16 (d, 2H), 7.36 (dd, 2H), 7.65 (d, 2H), 7.81 (m, 4H).

**Example 149-2-(4-fluorobenzylthio)-1-((5-(3-(piperidin-1-yl)propyl)-4-((4'-(trifluoromethyl)bip henyl-4-yl)methyl)-4*H*-1,2,4-triazol-3-yl)methyl)-6,7-dihydro-1*H*-cyclopenta[*d*]py rimidin-4(5*H*)-one tartrate**

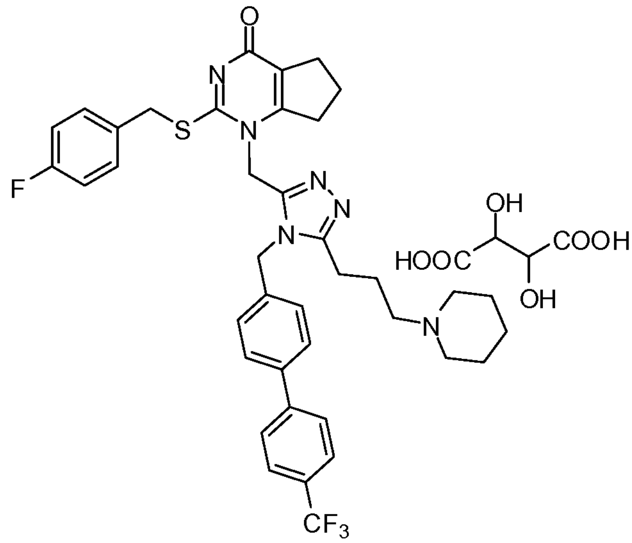

Following a procedure similar to that described for the preparation of example **91b** except that example **142** was used instead of example **91a.** $^1$H-NMR ($d_6$-DMSO, 300 MHz) δ 1.45 (m, 2H), 1.62 (m, 4H), 1.95 (m, 4H), 2.55 (t, 2H), 2.73 (m, 4H), 2.83 (m, 6H), 4.13 (s, 2H), 4.33 (s, 2H), 5.23 (s, 2H), 5.36 (s, 2H), 7.04 (t, 2H), 7.15 (d, 2H), 7.36 (dd, 2H), 7.65 (d, 2H), 7.81 (d, 2H).

**Example 150-2-(4-fluorobenzylthio)-1-((5-(4-(piperidin-1-yl)butyl)-4-((4'-(trifluoromethyl)biph enyl-4-yl)methyl)-4*H*-1,2,4-triazol-3-yl)methyl)-6,7-dihydro-1*H*-cyclopenta[*d*]pyr imidin-4(5*H*)-one tartrate**

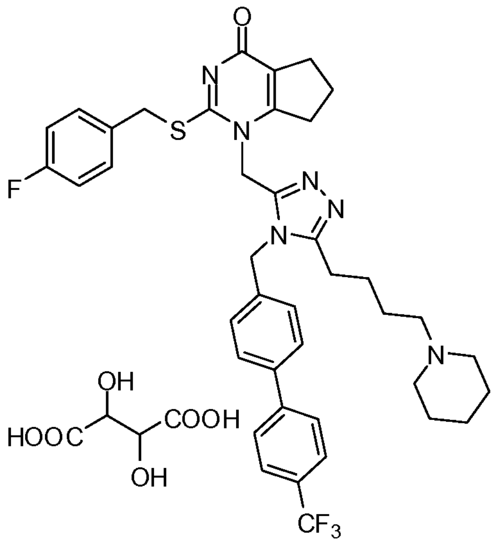

Following a procedure similar to that described for the preparation of example **91b** except that example **144** was used instead of example **91a.** $^1$H-NMR ($d_6$-DMSO, 400 MHz) δ 1.49 (m, 2H), 1.71 (m, 8H), 1.91 (m, 2H), 2.55 (t, 2H), 2.72 (m, 4H), 2.80-3.20 (m, 6H), 4.25 (s, 2H), 4.32 (s, 2H), 5.21 (s, 2H), 5.37 (s, 2H), 7.04 (t, 2H), 7.14 (d, 2H), 7.36 (dd, 2H), 7.68 (d, 2H), 7.81 (m, 4H).

**Example 151-1-((5-(((2-(diethylamino)ethyl)(methyl)amino)methyl)-4-((4'-(trifluoromethyl)bip henyl-4-yl)methyl)-4*H*-1,2,4-triazol-3-yl)methyl)-2-(4-fluorobenzylthio)-6,7-dihyd ro-1*H*-cyclopenta[*d*]pyrimidin-4(5*H*)-one hydrochloride**

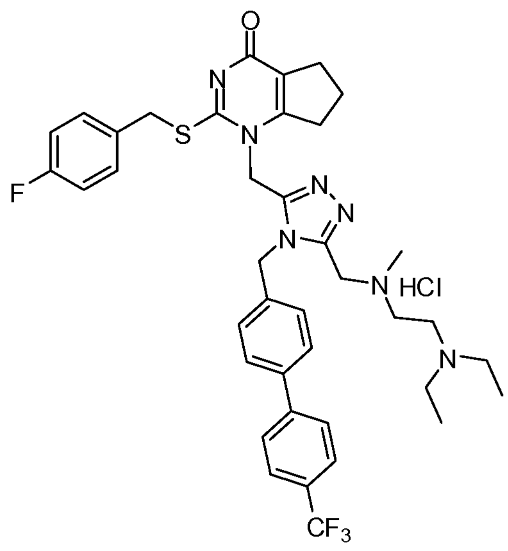

Following a procedure similar to that described for the preparation of example **80** except that example **91a** was used instead of example **79.** $^1$H-NMR ($d_6$-DMSO, 300 MHz) δ 1.17 (t, 6H), 1.91 (m, 2H), 2.55 (m, 4H), 2.71 (t, 2H), 3.08 (q, 4H), 3.28 (s, 3H), 3.45 (t, 2H), 4.21 (s, 2H), 4.30 (s, 2H), 5.19 (s, 2H), 5.53 (s, 2H), 7.01 (t, 2H), 7.21 (d, 2H), 7.32 (dd, 2H), 7.66 (d, 2H), 7.81 (m, 4H).

**Example 152-2-(4-fluorobenzylthio)-1-((5-((methyl(2-(pyrrolidin-1-yl)ethyl)amino)methyl)-4-(( 4'-(trifluoromethyl)biphenyl-4-yl)methyl)-4*H*-1,2,4-triazol-3-yl)methyl)-6,7-dihyd ro-1*H*-cyclopenta[*d*]pyrimidin-4(5*H*)-one tartrate**

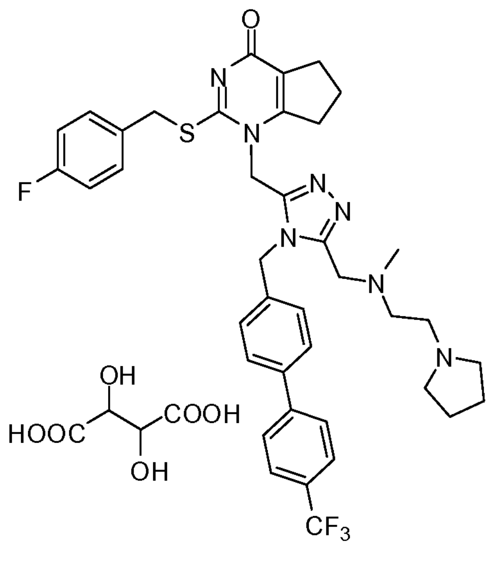

Following a procedure similar to that described for the preparation of example **91b** except that example **136** was used instead of example **91a.** $^1$H-NMR ($d_6$-DMSO, 400 MHz) δ 1.78 (m, 4H), 1.91 (m, 2H), 2.20 (s, 3H), 2.55 (t, 2H), 2.70 (m, 4H), 2.99 (m, 4H), 3.10 (t, 2H), 3.73 (s, 2H), 4.18 (s, 2H), 4.31 (s, 2H), 5.17 (s, 2H), 5.48 (s, 2H), 7.03 (t, 2H), 7.19 (d, 2H), 7.34 (dd, 2H), 7.66 (d, 2H), 7.81 (m, 4H).

**Example 153-2-(4-fluorobenzylthio)-1-((5-((methyl(2-(piperidin-1-yl)ethyl)amino)methyl)-4-((4 '-(trifluoromethyl)biphenyl-4-yl)methyl)-4*H*-1,2,4-triazol-3-yl)methyl)-6,7-dihydr o-1*H*-cyclopenta[*d*]pyrimidin-4(5*H*)-one**

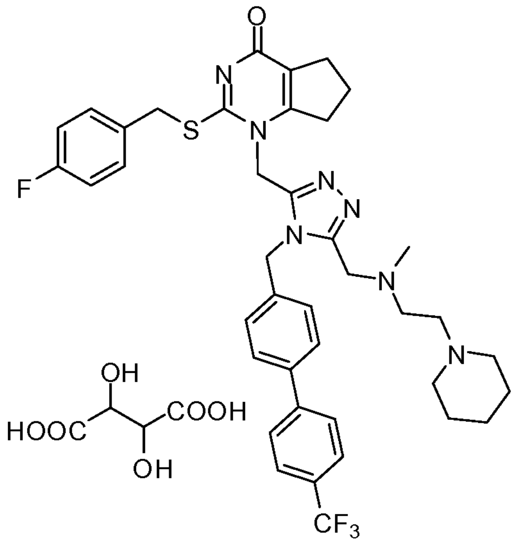

Following a procedure similar to that described for the preparation of example **91b** except that example **145** was used instead of example **91a**. $^1$H-NMR ($d_6$-DMSO, 400 MHz) δ 1.38 (m, 2H), 1.54 (m, 4H), 1.90 (m, 2H), 2.20 (s, 3H), 2.53 (t, 2H), 2.69 (t, 2H), 2.79 (t, 2H), 3.05 (m, 6H), 3.74 (s, 2H), 4.15 (s, 2H), 4.30 (s, 2H), 5.13 (s, 2H), 5.49 (s, 2H), 7.01 (d, 2H), 7.19 (t, 2H), 7.32 (m, 2H), 7.65 (d, 2H), 7.79 (d, 2H), 7.82 (d, 2H).

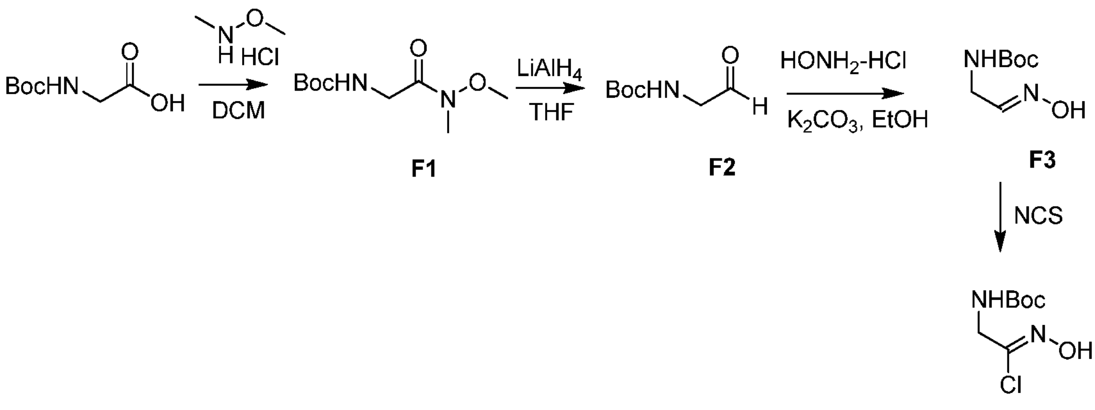


F1 F2 F3 F4

A mixture of Boc-glycine (8.75g, 50mmol, 1 equiv), EDCI (11.5g, 60mmol) and HOBt (7.43g, 55mmol) in DCM (100ml) was stirred in an ice bath for 15min to give a clear solution. Then *N*,*O*-Dimethylhydroxylamine hydrochloride (5.1g, 52.5mmol) and triethylamine (5.85ml, 55mmol) were added and the mixture was stirred for 2h at room temperature. Water (100ml) was added and the solution was stirred tempestuously. The precipitate so formed was filtered off and the filtrate was transferred into a separating funnel. The organic phase was separated and the aqueous phase was extracted with $CH_2Cl_2$ once again. The combined organic phase was washed with brine (X2), dried over $MgSO_4$ and concentrated. The residue was recrystallized from PE-EA to give **F1** (6.0g) as a crystal.

To an ice cooled suspension of $LiAlH_4$ (2.13g, 56mmol) in dry THF (20ml) was added **F1** (12.2g, 56mmol) in small batches to avoid drastic reaction. After the addition, the mixture was stirred for a further 1h. Then the reaction was quenched cautiously by addition of saturated $Na_2CO_3$ solution dropwise until no gas bubbles were generated, followed by addition of water dropwise with vigorous stirring. The solid so formed was filtered off under reduced pressure and washed with THF. The filtrate was concentrated to afford **F2** (9.6g) as a jelly which could be used in next step without further purification.

A mixture of **F2** (9.6g, 60mmol, 1 equiv), hydroxylamine hydrochloride (4.6g, 1.1 equiv) and $K_2CO_3$ (12.5g, 1.5 equiv) in EtOH (100ml) was stirred over night at room temperature. Then the mixture was concentrated under reduced pressure to remove the solvent and the residue was diluted with water, extracted with DCM (X3). The combined organic phase was washed with brine, dried over $MgSO_4$. After $MgSO_4$ was filtered off, the filtrate was concentrated to afford **F3** (7.7g) as a jelly. $^1$H-NMR ($CDCl_3$, 300 MHz) δ 1.45(s, 9H), 3.91/4.04(2x t, 2H, *J*=5.1, $-CH_2-$), 4.93 (vbrs, 1H, -NH-), 7.45/6.79(2x t, 2H, *J*=4.8, -CH=), 7.72/8.04(2x s, 1H, -OH).

To a solution of **F3** (7.7g, 44.25mmol, 1 equiv) in dry DMF (40ml) was added NCS (5.9g, 1 equiv) and the mixture was stirred for 3h at room temperature. EA (100ml) was added and the mixture was transferred to a separating funnel, washed with brine (X5). The organic phase was dried over $MgSO_4$. After $MgSO_4$ was filtered off, the filtrate was concentrated to afford **F4** (8.7g) as a jelly.

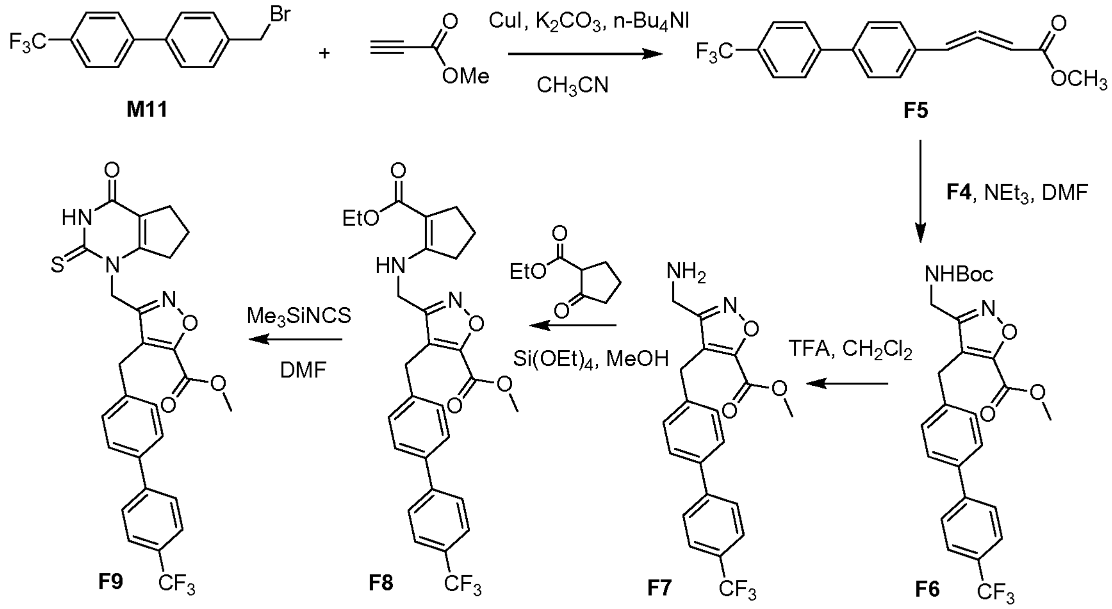


**Intermediate F5-methyl 4-(4'-(trifluoromethyl)biphenyl-4-yl)buta-2,3-dienoate**

A mixture of **M11** (3.66g, 11.62mmol, 1 equiv), methyl propiolate (1.93ml, 23.24mmol, 2 equiv), CuI (2.43g, 12.78mmol, 1.1 equiv), $K_2CO_3$ (3.21g, 23.24mmol, 2 equiv) and tetran-butylammonium iodide (4.29g, 11.62mmol, 1 equiv) in acetonitrle (30ml) were stirred for 4h at 40°C under a nitrogen atmosphere. Then the mixture was concentrated under reduced pressure to remove the solvent and the residue was diluted with water, extracted with EA (X2). The combined organic phase was dried over $Na_2SO_4$ and concentrated. The crude product was purified by column chromatography to afford **F5** (1.63g) as a white solid. **M11** (1.19g) was recycled. $^1$H-NMR ($CDCl_3$, 400 MHz) δ3.78 (s, 3H), 6.08 (d, 1H, *J*=6.4), 6.68 (d, 1H, *J*=6.4), 7.41 (d, 2H, *J*=8.4), 7.58 (d, 2H, *J*=8.4), 7.69 (m, 4H).

**Intermediate F6-methyl 3-((tert-butoxycarbonylamino)methyl)-4-((4'-(trifluoromethyl)biphenyl-4-yl)met hyl)isoxazole-5-carboxylate**

To a two-neck flask containing dry DMF (10ml) were added **F5** (1.02g, 3.2mmol, 1 equiv) and triethylamine (0.67ml, 4.8mmol, 1.5 equiv) and the mixture was placed in an oil bath of 70°C under a nitrogen atmosphere. Then a solution of **F4** (0.95g, 4.2mmol) in DMF (10ml) was added dropwise over 2h through a constant pressure drop funnel. After the addition, the mixture was stirred for a further 1h before it was cooled and diluted with EA (100ml). The mixture was transferred to a separating funnel, washed with brine several times, dried over $Na_2SO_4$ and concentrated. The residue was recrystallized from PE-EA to give **F6** (0.53g) as a white solid. **F5** (0.43g) was recycled by column chromatography. $^1$H-NMR ($CDCl_3$, 400 MHz) δ1.41 (s, 9H), 3.99 (s, 3H), 4.21 (s, 2H), 4.32 (d, 2H, *J*=5.4), 4.98 (vbrs, 1H), 7.28 (d, 2H, *J*=7.8), 7.51 (d, 2H, *J*=8.1), 7.66 (m, 4H).

**Intermediate F8-methyl 3-((2-(ethoxycarbonyl)cyclopent-1-enylamino)methyl)-4-((4'-(trifluoromethyl)bip henyl-4-yl)methyl)isoxazole-5-carboxylate**

To a solution of **F6** (0.64g) in DCM (5ml) was added $CF_3COOH$ (1ml) and the mixture was stirred for 2h at room temperature. Then the mixture was concentrated to remove the solvent and the residue was quenched with saturated aqueous $NaHCO_3$ solution. The resulting mixture was extracted with DCM and the organic phase was washed with brine, dried over $MgSO_4$ and concentrated to afford **Intermediate F7** (0.51g) as a jelly.

To a solution of **F7** (0.51g, 1equiv) in anhydrous MeOH (10ml) was added ethyl 2-oxocyclopentanecarboxylate (0.2ml, 1equiv) and tetraethyl orthosilicate (0.58ml, 2equiv) . The resulting mixture was refluxed for 5h and then concentrated. The crude product was purified by flash chromatography to afford **F8** (0.52g) as a jelly. $^1$H-NMR ($CDCl_3$, 300

MHz) δ1.24 (t, 3H, *J*=6.9) 1.74 (m, 2H), 2.39 (t, 2H, *J*=7.2), 2.45 (t, 2H, *J*=7.2), 4.00 (s, 3H), 4.11 (q, 2H, *J*=6.9), 4.21 (d, 2H, *J*=6.3), 4.26 (s, 2H), 7.25 (d, 2H, *J*=7.8), 7.52 (d, 2H, *J*=8.1), 7.59 (t, 1H, -NH-), 7.67 (m, 4H).

**Intermediate F9-methyl 3-((4-oxo-2-thioxo-2,3,4,5,6,7-hexahydro-1*H*-cyclopenta[*d*]pyrimidin-1-yl)methyl) -4-((4'-(trifluoromethyl)biphenyl-4-yl)methyl)isoxazole-5-carboxylate**

To a solution of **F8** (0.52g, 1equiv) in dry DMF (1ml) was added $Me_3SiNCS$ (0.56ml, 4equiv). The mixture was stirred for 4h at 140°C before it was quenched with saturated aqueous $NaHCO_3$ solution in an ice bath. The resulting mixture was extracted with EA (X2) and the combined organic phase was washed with brine, dried and concentrated. The crude product was purified by column chromatography to afford **F9** (0.18g) as a solid. $^1$H-NMR ($d_6$-DMSO, 300 MHz) δ1.86 (m, 2H), 2.41 (t, 2H, *J*=7.2), 2.69 (t, 2H, *J*=7.2), 3.92 (s, 3H), 4.28 (s, 2H), 5.57 (s, 2H), 7.29 (d, 2H, *J*=8.1), 7.61 (d, 2H, *J*=8.4), 7.80 (d, 2H, *J*=8.4), 7.85 (d, 2H, *J*=8.4), 12.57 (s, 1H).

**Example 154-methyl-3-((2-(4-fluorobenzylthio)-4-oxo-4,5,6,7-tetrahydro-1*H*-cyclopenta[d]pyri midin-1-yl)methyl)-4-((4'-(trifluoromethyl)biphenyl-4-yl)methyl)isoxazole-5-carb oxylate**

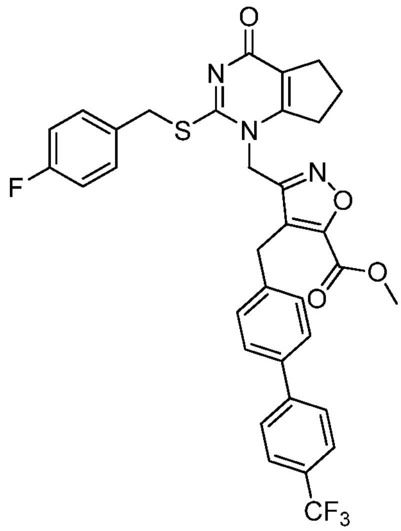

A mixture of **F9** (0.15g, 1equiv), 4-fluorobenzyl bromide (38μl, 1.1equiv) and DBU (50μl, 1.2equiv) in acetonitrile (3ml) was stirred at room temperature for 1h and then concentrated to remove the solvent. The residue was dissolved in DCM, washed with water, dried and concentrated. The crude product was purified by column chromatography to afford **154** (0.15g) as a solid. $^1$H-NMR ($CDCl_3$, 300 MHz) δ 1.93 (m, 2H), 2.58 (t, 2H, *J*=7.5), 2.66 (t, 2H, *J*=7.5), 4.02 (s, 3H), 4.22 (s, 2H), 4.43 (s, 2H), 4.86 (s, 2H), 6.92 (t, 2H, *J*=8.7), 7.10 (d, 2H, *J*=8.1), 7.28 (dd, 2H, *J*=9.0, 5.4), 7.44 (d, 2H, *J*=8.1), 7.61 (d, 2H, *J*=8.1), 7.70 (d, 2H, *J*=8.4).

**Example 155-2-(4-fluorobenzylthio)-1-((5-(hydroxymethyl)-4-((4'-(trifluoromethyl)biphenyl-4-yl)methyl)isoxazol-3-yl)methyl)-6,7-dihydro-1*H*-cyclopenta[*d*]pyrimidin-4(5*H*)-o ne**

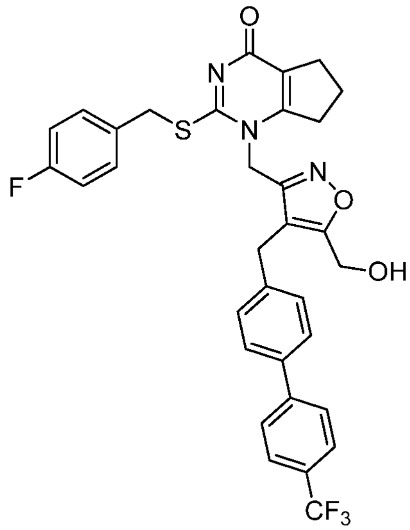

To a solution of the compound of Example **154** (0.15g, 1 equiv) in MeOH (3ml) was added $NaBH_4$ (10mgX4) portion-wise over a period of 0.5h. Then the solution was evaporated to dryness, diluted with water and extracted with DCM (X2). The combined organic phase was washed with brine (X1), dried over $Na_2SO_4$ and concentrated. The crude product was purified by column chromatography to afford **155** (100mg) as a white solid. $^1$H-NMR ($CDCl_3$, 400 MHz) δ1.91 (m, 2H), 2.60 (m, 4H), 2.95 (s, 1H), 3.98 (s, 2H), 4.41 (s, 2H), 4.81 (s, 2H), 4.84 (s, 2H), 6.91 (t, 2H, *J*=8.4), 7.10 (d, 2H, *J*=8.0), 7.29 (m, 2H), 7.43 (d, 2H, *J*=8.0), 7.61 (d, 2H, *J*=7.6), 7.69 (d, 2H, *J*=8.0).

**Example 156-1-((5-(chloromethyl)-4-((4'-(trifluoromethyl)biphenyl-4-yl)methyl)isoxazol-3-yl)m ethyl)-2-(4-fluorobenzylthio)-6,7-dihydro-1*H*-cyclopenta[*d*]pyrimidin-4(5*H*)-one**

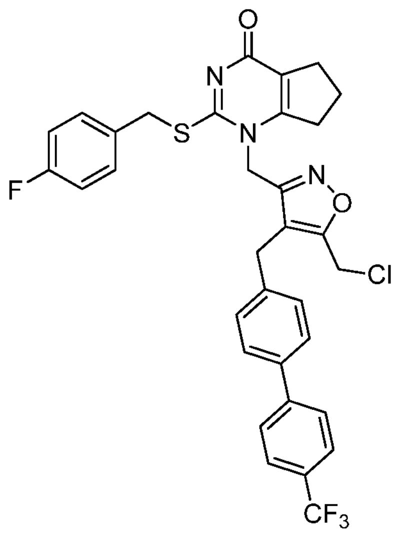

To a solution of the compound of Example **155** (90mg, 1equiv) in DCM (2ml) was added thionyl chloride (12μl, 1.2equiv) in an ice bath. The mixture was stirred for 1h before it was quenched with saturated aqueous $NaHCO_3$ solution. The organic phase was separated, dried over $MgSO_4$ and concentrated to afford **156** (90mg) as a solid. $^1$H-NMR ($CDCl_3$, 400 MHz) δ1.94 (m, 2H), 2.64 (m, 4H), 3.87 (s, 2H), 4.45 (s, 2H), 4.63 (s, 2H), 4.84 (s, 2H), 6.93 (t, 2H, *J*=8.4), 7.10 (d, 2H, *J*=8.4), 7.31 (dd, 2H, *J*=8.8, 5.2), 7.46 (d, 2H, *J*=8.4), 7.63 (d, 2H, *J*=8.4), 7.70 (d, 2H, *J*=8.4).

**Example 157-1-((5-((diethylamino)methyl)-4-((4'-(trifluoromethyl)biphenyl-4-yl)methyl)isoxaz ol-3-yl)methyl)-2-(4-fluorobenzylthio)-6,7-dihydro-1*H*-cyclopenta[*d*]pyrimidin-4( 5*H*)-one**

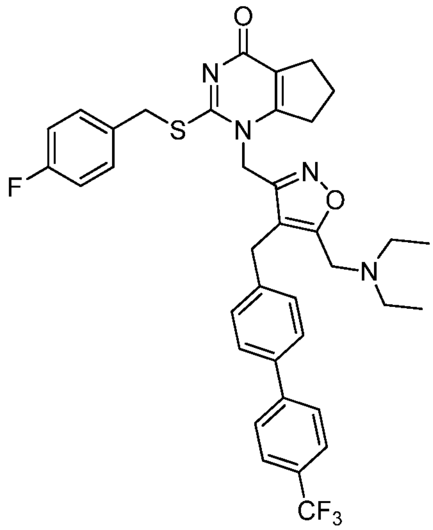

A mixture of the compound of Example **156** (53mg, 0.083mmol, 1equiv) and diethylamine (11μl, 1.3equiv), $K_2CO_3$ (23mg, 2equiv) and KI (1mg, cat.) in acetonitrile (2ml) was stirred under reflux for 1h, and then filtered to remove the insoluble salts and concentrated. The crude product was purified by column chromatography to afford **157** (50mg) as a solid. $^1$H-NMR ($CDCl_3$, 400 MHz) δ 1.07 (t, 6H, *J*=7.2), 1.94 (m, 2H), 2.62 (m, 8H), 3.81 (s, 2H), 3.91 (s, 2H), 4.43 (s, 2H), 4.81 (s, 2H), 6.92 (t, 2H, *J*=8.4), 7.08 (d, 2H, *J*=8.0), 7.30 (dd, 2H, *J*=8.0, 5.6), 7.43 (d, 2H, *J*=8.0), 7.62 (d, 2H, *J*=8.0), 7.69 (d, 2H, *J*=8.0); MS(ESI): 677 (M+H).

**Example 158-1-((5-(((2-(dimethylamino)ethyl)(methyl)amino)methyl)-4-((4'-(trifluoromethyl)b iphenyl-4-yl)methyl)isoxazol-3-yl)methyl)-2-(4-fluorobenzylthio)-6,7-dihydro-1*H*-cyclopenta[*d*]pyrimidin-4(5*H*)-one**

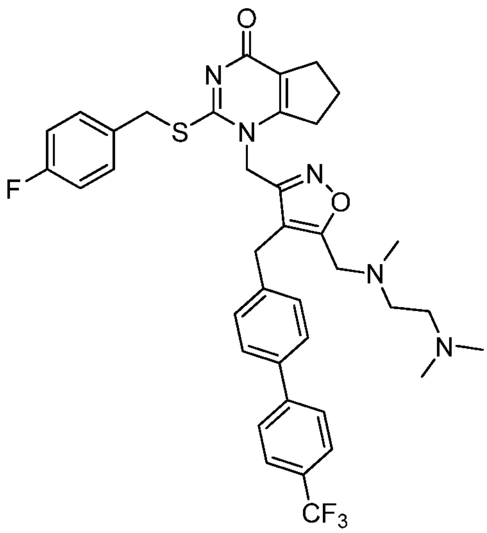

Following a procedure similar to that described for the preparation of example **157** except that *N*,*N*,*N*'-trimethylethylenediamine was used instead of diethylamine. $^1$H-NMR ($CDCl_3$, 400 MHz) δ1.98 (m, 2H), 2.35 (s, 3H), 2.60 (s, 6H), 2.70 (m, 4H), 2.89 (m, 4H), 3.80 (s, 2H), 3.95 (s, 2H), 4.41 (s, 2H), 4.86 (s, 2H), 6.89 (t, 2H, *J*=8.4), 7.11 (d, 2H, *J*=8.0), 7.27 (m, 2H), 7.42 (d, 2H, *J*=8.4), 7.60 (d, 2H, *J*=8.4), 7.69 (d, 2H, *J*=8.0).

**Example 159-1-((5-(((2-(diethylamino)ethyl)(methyl)amino)methyl)-4-((4'-(trifluoromethyl)bip henyl-4-yl)methyl)isoxazol-3-yl)methyl)-2-(4-fluorobenzylthio)-6,7-dihydro-1*H*-c yclopenta[*d*]pyrimidin-4(5*H*)-one**

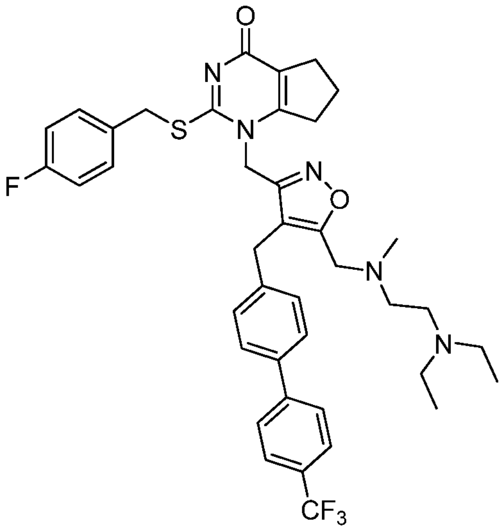

Following a procedure similar to that described for the preparation of example **157** except that *N*,*N*-diethyl-*N*'-methylethylenediamine was used instead of diethylamine. $^1$H-NMR ($CDCl_3$, 300 MHz) δ1.25 (t, 6H, *J*=7.2), 1.97 (m, 2H), 2.34 (s, 3H), 2.70 (m, 4H), 2.94-3.03 (m, 8H), 3.80 (s, 2H), 3.95 (s, 2H), 4.40 (s, 2H), 4.86 (s, 2H), 6.88 (t, 2H, *J*=8.7), 7.10 (d, 2H, *J*=7.8), 7.27 (m, 2H), 7.42 (d, 2H, *J*=8.1), 7.59 (d, 2H, *J*=8.1), 7.69 (d, 2H, *J*=7.8).

**Example 160-1-((5-(((2-(diethylamino)ethyl)(methyl)amino)methyl)-4-((4'-(trifluoromethyl)bip henyl-4-yl)methyl)isoxazol-3-yl)methyl)-2-(4-fluorobenzylthio)-6,7-dihydro-1*H*-c yclopenta[*d*]pyrimidin-4(5*H*)-one single-tartrate**

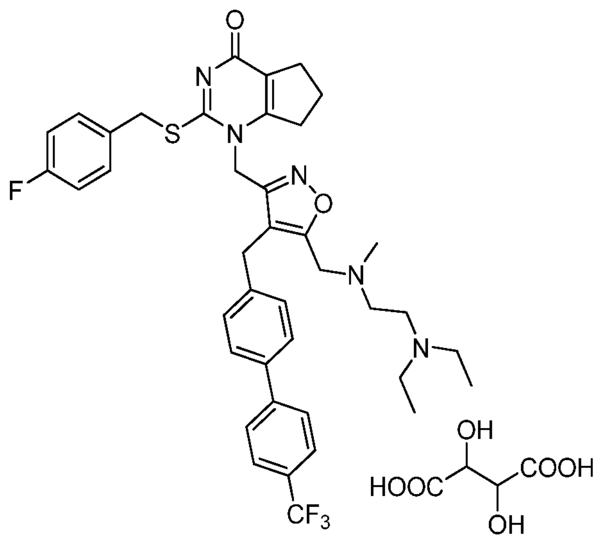

Following a procedure similar to that described for the preparation of example **91b** except that example **159** was used instead of example **91a.** $^1$H-NMR ($d_6$-DMSO, 300 MHz) δ1.14 (t, 6H), 1.87 (m, 2H), 2.22 (s, 3H), 2.67 (t, 2H), 2.75 (t, 2H), 3.05-3.13 (m, 8H), 3.85 (s, 2H), 3.95 (s, 2H), 4.24 (s, 2H), 4.32 (s, 2H), 5.03 (s, 2H), 7.04 (t, 2H), 7.22 (d, 2H), 7.35 (m, 2H), 7.60 (d, 2H), 7.80 (m, 4H).

**Example 161-2-(4-fluorobenzylthio)-1-((5-((methyl(2-(piperidin-1-yl)ethyl)amino)methyl)-4-((4 '-(trifluoromethyl)biphenyl-4-yl)methyl)isoxazol-3-yl)methyl)-6,7-dihydro-1*H*-cy clopenta[*d*]pyrimidin-4(5*H*)-one**

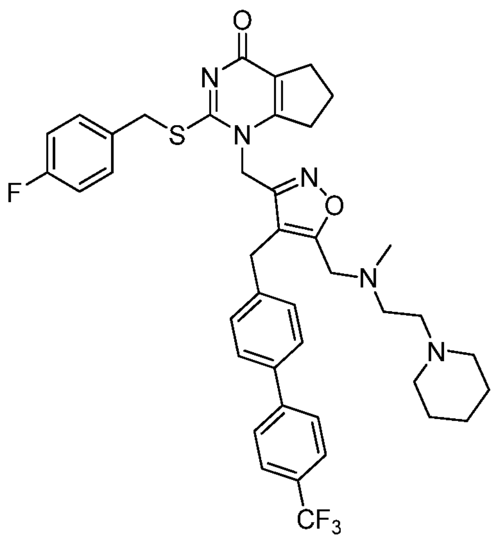

Following a procedure similar to that described for the preparation of example **157** except that *N*-methyl-2-(piperidin-1-yl)ethanamine was used instead of diethylamine. $^1$H-NMR ($CDCl_3$, 400 MHz) $\delta$1.55 (m, 2H), 1.81 (m, 4H), 1.97 (m, 2H), 2.34 (s, 3H), 2.68 (t, 2H, *J*=7.2), 2.74 (t, 2H, *J*=7.2), 2.82 (m, 4H), 2.90 (m, 4H), 3.78 (s, 2H), 3.95 (s, 2H), 4.40 (s, 2H), 4.88 (s, 2H), 6.88 (t, 2H, *J*=8.4), 7.11 (d, 2H, *J*=8.0), 7.26 (m, 2H), 7.41 (d, 2H, *J*=8.1), 7.59 (d, 2H, *J*=8.0), 7.69 (d, 2H, *J*=8.4).

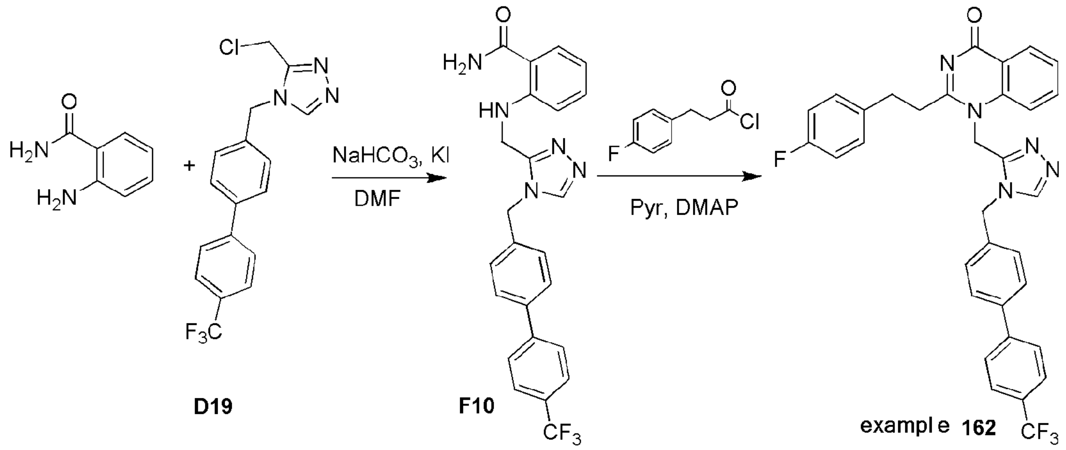

D19 F10 exampl e 162

**Example 162-2-(4-fluorophenethyl)-1-((4-((4'-(trifluoromethyl)biphenyl-4-yl)methyl)-4*H*-1,2,4-triazol-3-yl)methyl)quinazolin-4(1*H*)-one**

The mixture of 2-aminobenzamide (320mg, 2.3mmol, 1equiv), **D19** (1g, 1.1equiv), $NaHCO_3$ (580mg, 3equiv) and KI (38mg, 0.1 equiv) in DMF (10ml) was heated for 2h at 80°C. Then it was poured into water and extracted with EA. The organic phase was dried and concentrated. The crude product was purified by column chromatography to afford **F10** (380mg) as a solid.

The mixture of **F10** (370mg, 0.82mmol, 1equiv), 3-(4-fluorophenyl)propanoyl chloride (185mg, 1.2equiv), pyridine (132$\mu$l, 2equiv) and DMAP (4mg) in dry 1,4-dioxacyclohexane (5ml) was refluxed over night and then mixed with silica gel and concentrated to remove the solvent. The crude product was purified by column chromatography to afford **161** (50mg) as a solid. $^1$H-NMR ($CDCl_3$, 400 MHz) $\delta$3.25 (t, 2H, *J*=6.8), 3.35 (t, 2H, *J*=6.8), 5.30 (s, 2H), 5.32 (s, 2H), 6.93 (t, 2H, *J*=8.8), 7.20 (dd, 2H, *J*=8.4, 5.6), 7.31 (d, 2H, *J*=8.0), 7.44 (t, 1H, *J*=7.2), 7.56 (d, 2H, *J*=8.4),7.65 (m, 4H), 7.70 (d, 2H, *J*=8.4), 8.06 (s, 1H), 8.37 (d, 1H, *J*=8.0); MS(ESI): 584(M+H).

**Pharmacological Examples**

**1. Test of *in vitro* inhibitory activities of the compounds towards the enzyme Lp-PLA$_2$ in rabbit serum**

1.1 Test method

The activities of compounds were measured using [$^3$H] labeled platelet activating factor ([$^3$H]PAF, Perkinelmer, Lot NET910) as the substrate. The reaction was carried out in a system containing 50 mmol/L HEPES and 150 mmol/L NaCl (pH 7.4) in a total volume of 200 μl. First, 10 μL rabbit serum and 10 μl DMSO solution of the test compound were added to the system and preincubated for 10 min at 37°C.Then 20 nM [$^3$H]PAF was added as listed in table 1 to start the reaction and incubated for a further 10 min at 37°C. After that, 600 μl of $CHCl_3$/MeOH (2:1) was added and the resulting mixture was vortex mixed and the reaction was stopped. After settled for a while, the mixture was centrifugated at 12000xg for 15 min. The supernatant was transferred to a new tube and 200 μl of $CHCl_3$ was added. The mixture was mixed by vortexing and then settled or centrifugated for 2 min. A 100 μl portion of the supernatant was then collected for radioactivity intensity measurement.

Table 1: reaction system lists

| | blank tube(μl) | control tube(μl) | compound tube(μl) |
|---|---|---|---|
| reaction buffer | 180 | 170 | 170 |
| test compound | | | 10 |
| DMSO (solvent) | 10 | 10 | |
| substrate ([$^3$H] PAF) | 10 | 10 | 10 |
| enzyme source (rabbit serum) | | 10 | 10 |

**The inhibition rate was determined by the following equation:**

$$\text{Inhibitory rate (\%)} = 1-(DPM_{\text{compound tube}} - DPM_{\text{blank tube}})/(DPM_{\text{control tube}} - DPM_{\text{blank tube}})*100\%$$

(Note: DPM is radioactivity intensity unit.)

1.2 Test results are listed in Table 2.

Table 2: inhibitory activities of part of the compounds towards the enzyme Lp-PLA$_2$ in rabbit serum at different concentration

| **compound** | **inhibition, %** | | |
|---|---|---|---|
| | **10μM** | **1μM** | **100nM** |
| Example 1 | 62.6 | 10.0 | NT |
| Example 2 | 82.7 | 26.6 | NT |
| Example 3 | 12.5 | NT | NT |
| Example 6 | 74.0 | 16.2 | NT |
| Example 7 | 60.3 | 25.0 | NT |
| Example 18 | 41.9 | 7.4 | NT |
| Example 19 | 76.8 | 29 | NT |
| Example 20 | 86.6 | 31 | NT |
| Example 21 | 51.2 | - | NT |
| Example 22 | 63.0 | - | NT |
| Example 23 | 82.4 | 33 | NT |
| Example 24 | 78.5 | 16.3 | NT |
| Example 25 | 74.3 | 23 | NT |

(continued)

| compound | inhibition, % | | |
|---|---|---|---|
| | 10μM | 1μM | 100nM |
| Example 26 | 7.4 | NT | NT |
| Example 28 | 96.5 | 79.6 | 16.9 |
| Example 29 | NT | 64.9 | 12.6 |
| Example 30 | 87.5 | 42.3 | - |
| Example 32 | NT | 50.0 | 2.1 |
| Example 34 | NT | 14.6 | NT |
| Example 38 | 20.4 | 16.0 | NT |
| Example 40 | NT | 90.7 | 53.1 |
| Example 41 | NT | 64.9 | 12.6 |
| Example 42 | NT | 97.7 | 88.3 |
| Example 43 | NT | NT | 78.6 |
| Example 44 | NT | NT | 89.1 |
| Example 45 | NT | NT | 87 |
| Example 46 | NT | 97.2 | 87.1 |
| Example 47 | NT | 98.6 | 96.9 |
| Example 49 | NT | NT | 96.6 |
| Example 50 | NT | NT | 57 |
| Example 52 | NT | NT | 90 |
| Example 53 | NT | 90.1 | 43.2 |
| Example 54 | NT | 88.8 | 35.9 |
| Example 55 | 33.6 | 21 | NT |
| Example 57 | 72.4 | 13 | NT |
| Example 58 | 94.8 | 32.0 | NT |
| Example 59 | 9.3 | - | NT |
| Example 60 | 40.0 | - | NT |
| Example 61 | NT | NT | 61.0 |
| Example 62 | NT | NT | 81.9 |
| Example 62 | NT | NT | 73.8 |
| Example 64 | NT | NT | 78.9 |
| Example 65 | NT | NT | 60.0 |
| Example 66 | NT | NT | 88 |
| Example 67 | NT | NT | 91 |
| Example 68 | NT | 87 | 34 |
| Example 69 | NT | NT | 89 |
| Example 70 | NT | NT | 96 |
| Example 72 | NT | NT | 73 |
| Example 75 | NT | NT | 88.3 |
| Example 76 | NT | NT | 97.0 |
| Example 77 | NT | NT | 93 |
| Example 79 | NT | NT | 85.3 |
| Example 81 | NT | NT | 90 |
| Example 82 | NT | NT | 85 |
| Example 83 | NT | NT | 93 |
| Example 84 | NT | NT | 76 |
| Example 85 | NT | NT | 77 |
| Example 86 | NT | NT | 90 |
| Example 87 | NT | NT | 75 |

(continued)

| compound | inhibition, % | | |
|---|---|---|---|
| | 10μM | 1μM | 100nM |
| Example 88 | NT | NT | 67 |
| Example 89 | NT | NT | 13.4 |
| Example 90 | NT | NT | 96 |
| Example 91a | NT | NT | 98 |
| Example 92 | NT | NT | 98 |
| Example 93 | NT | NT | 98 |
| Example 94 | NT | NT | 98 |
| Example 95 | NT | NT | 99 |
| Example 96 | NT | NT | 97 |
| Example 97 | NT | NT | 98 |
| Example 98 | NT | NT | 97 |
| Example 99 | NT | NT | 97 |
| Example 100 | NT | NT | 91 |
| Example 101 | NT | NT | 86 |
| Example 102 | NT | NT | 97 |
| Example 103 | NT | NT | 82 |
| Example 104 | NT | NT | 67 |
| Example 105 | NT | NT | 98 |
| Example 106 | NT | NT | 96 |
| Example 107 | NT | NT | 68 |
| Example 110 | NT | NT | 67 |
| Example 111 | NT | NT | 75 |
| Example 112 | NT | NT | 63 |
| Example 113 | NT | NT | 64 |
| Example 115 | NT | NT | 78 |
| Example 116 | NT | NT | 94 |
| Example 117 | NT | NT | 80 |
| Example 118 | NT | NT | 68 |
| Example 119 | NT | NT | 93 |
| Example 120 | NT | NT | 90 |
| Example 122 | NT | NT | 95 |
| Example 123 | NT | NT | 88 |
| Example 125 | NT | NT | 92 |
| Example 128 | NT | NT | 94 |
| Example 129 | NT | NT | 96 |
| Example 130 | NT | NT | 94 |
| Example 131a | NT | NT | 96 |
| Example 132 | NT | NT | 96 |
| Example 133 | NT | NT | 97 |
| Example 135 | NT | NT | 83 |
| Example 157 | NT | NT | 89 |
| Example 158 | NT | NT | 98 |
| Example 159 | NT | NT | 97 |
| Example 161 | NT | NT | 99 |

Note: "—", inactive; "NT", not tested.

**2. Test of *in vitro* inhibitory activities of the compounds towards the enzyme Lp-PLA$_2$ in human serum**

2.1 Test method

The activities of compounds were measured using [$^3$H] labeled platelet activating factor ([$^3$H]PAF, Perkinelmer, Lot NET910) as the substrate. The reaction was carried out in a system containing 50 mmol/l HEPES and 150 mmol/l NaCl (pH 7.4) in a total volume of 200 μl. First, 10 μL human plasma and 10 μl DMSO solution of the test compound were added to the system and preincubated for 10 min at 37°C.Then 20 nM [$^3$H]PAF was added as listed in table 1 to start the reaction and incubated for a further 10 min at 37°C. After that, 600 μl of $CHCl_3$/MeOH (2:1) was added and the resulting mixture was mixed with vortex. After settled for a while, the mixture was centrifugated at 12000xg for 15 min. The supernatant was transferred to a new tube and 200 μl of $CHCl_3$ was added and mixed by vortexing. The mixture was settled or centrifugated for 2 min. A 100 μl portion of the supernatant was then collected for radioactivity intensity measurement.

2.2 Test results are listed in Table3.

Table 3: inhibitory activities of part of the compounds towards the enzyme Lp-PLA$_2$ in human serum at different concentration

| compound | inhibition, % | |
|---|---|---|
| | 100nM | 10nM |
| Example 42 | 92 | 75 |
| Example 43 | 92 | 67 |
| Example 44 | 95 | 69 |
| Example 45 | 90 | 56 |
| Example 46 | 89 | 75 |
| Example 47 | 99 | 93 |
| Example 49 | 98 | 97 |
| Example 52 | 99 | 97 |
| Example 61 | 91 | 59 |
| Example 62 | 96 | 83 |
| Example 62 | 94 | 76 |
| Example 64 | 93 | 73 |
| Example 65 | 96 | 82 |
| Example 66 | 98 | 88 |
| Example 67 | 98 | 92 |
| Example 68 | 97 | 93 |
| Example 69 | 97 | 87 |
| Example 70 | 97 | 91 |
| Example 72 | 91 | 65 |
| Example 75 | 97 | 85 |
| Example 76 | 100 | 100 |
| Example 77 | 98 | 97 |
| Example 79 | 93 | 68 |
| Example 81 | 98 | 93 |
| Example 82 | 98 | 77 |
| Example 83 | 95 | 81 |
| Example 84 | 91 | 69 |
| Example 85 | 91 | 59 |
| Example 86 | 96 | 82 |
| Example 87 | 87 | 76 |
| Example 88 | 91 | 59 |

(continued)

| compound | inhibition, % | |
|---|---|---|
| | 100nM | 10nM |
| Example 90 | 100 | 95 |
| Example 91a | NT | 94 |
| Example 92 | NT | 93 |
| Example 93 | NT | 85 |
| Example 94 | NT | 89 |
| Example 95 | NT | 86 |
| Example 96 | NT | 78 |
| Example 97 | NT | 81 |
| Example 98 | NT | 82 |
| Example 99 | NT | 81 |
| Example 100 | 98 | 91 |
| Example 101 | 96 | 77 |
| Example 102 | NT | 77 |
| Example 103 | 98 | 71 |
| Example 104 | 86 | 53 |
| Example 105 | NT | 72 |
| Example 106 | NT | 80 |
| Example 107 | 91 | 70 |
| Example 110 | 83 | 67 |
| Example 111 | 92 | 68 |
| Example 112 | 87 | 56 |
| Example 113 | 91 | 56 |
| Example 115 | NT | 77 |
| Example 116 | 100 | 88 |
| Example 117 | 92 | 67 |
| Example 118 | 88 | 58 |
| Example 119 | 98 | 94 |
| Example 120 | 98 | 89 |
| Example 122 | NT | 92 |
| Example 123 | NT | 76 |
| Example 125 | NT | 76 |
| Example 128 | NT | 90 |
| Example 129 | NT | 94 |
| Example 130 | NT | 92 |
| Example 131a | NT | 92 |
| Example 132 | NT | 93 |
| Example 133 | NT | 92 |
| Example 135 | NT | 66 |
| Example 157 | NT | 96 |
| Example 158 | NT | 98 |
| Example 159 | NT | 100 |
| Example 161 | NT | 100 |
| Note: "NT", not tested. | | |

3. Analysis on the pharmacological results

From the *in vitro* activity test results, it can be seen that most compounds have a relatively good Lp-PLA$_2$ inhibitory activity. The inhibitory rate of compounds 47, 49, 52, 67, 70, 76, 77, 81, 83, 86, 90-100, 106, 116, 119, 120,

122, 128, 133, 158, 159, 161 at 100nM towards the rabbit serum derived Lp-$PLA_2$ enzyme are all greater than 90%, and at 10nM towards the human serum derived Lp-$PLA_2$ enzyme are all greater than 80%. It can be seen that the compounds of formula (I), (II) or (III) of the present application are potent Lp-$PLA_2$ inhibitors.

All literatures mentioned in the present application are incorporated by reference herein, as though individually incorporated by reference. Additionally, it should be understood that after reading the description above, many variations and modifications may be made by the skilled in the art, and these equivalents also fall within the scope as defined by the appended claims.

## Claims

**1.** An azole heterocyclic compound of Formula (I), (II) or (III), *cis-trans* isomers, enantiomers, diastereoisomers, racemes, solvates, hydrates, or pharmaceutically acceptable salts thereof:

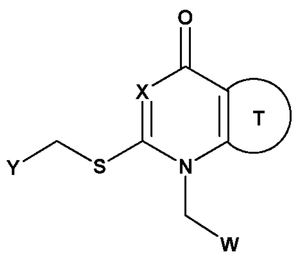
(I) or

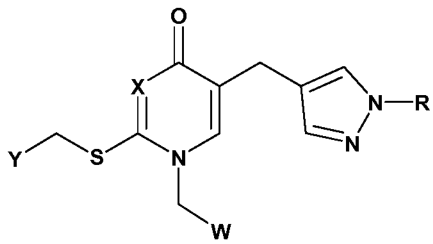
(II) or

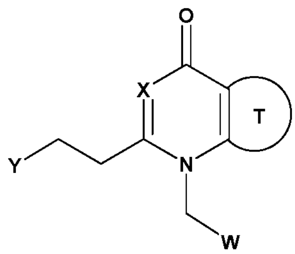
(III)

in which:

T is 4 to 6-membered aliphatic ring or benzene ring;
R is $C_1$-$C_6$ alkyl;
X is CH or N;
Y is phenyl group, optionally substituted by one or more substituents selected from halogen, $C_1$-$C_6$ alkoxyl, $C_1$-$C_6$ alkyl or halogenated $C_1$-$C_6$ alkyl;
W is selected from 12 structures of Formulae (a-1) as follows:

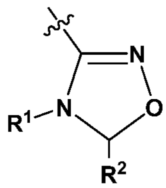
(a)

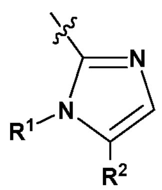
(b)

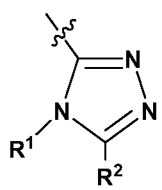
(c)

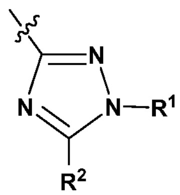
(d)

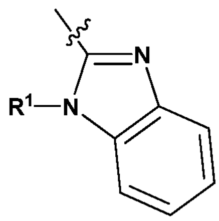
(e)

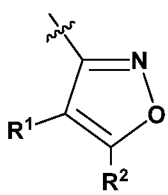
(f)

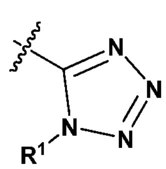
(g )

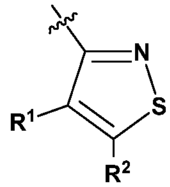
(h)

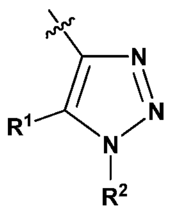
(i)

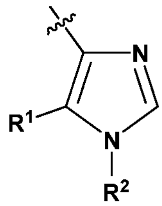
(j)

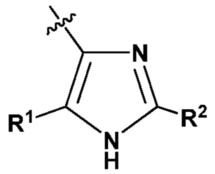 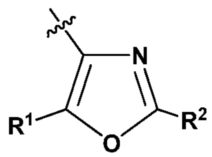

(k) (l)

$R^1$ is selected from H,

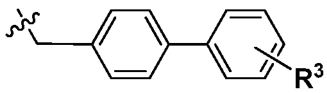,

$C_3$-$C_{12}$ alkenyl, $C_1$-$C_{12}$ alkyl,- $NR^4R^5$ substituted $C_2$-$C_4$ alkyl, benzyl or piperidyl which is optionally substituted by $-COOR^4$;
$R^2$ is selected from H,

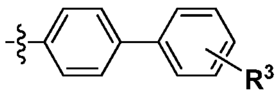, 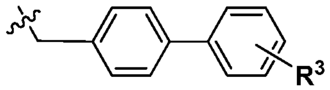,

$-COR^4$, $-COOR^4$, $-CONR^4R^5$, $-CH=NNR^4R^5$, $-C(=CH_2)-OC(=O)R^4$, $C_1$-$C_{12}$ alkyl, $C_3$-$C_7$ cycloalkyl, phenyl, wherein alkyl, cycloalkyl and phenyl are optionally substituted by halogen, $-NR^4R^5$, $-OR^4$, $-SR^4$, $-SO_2R^4$, $-NHCOR^4$, $-NHSO_2R^4$, $-NHCSNHR^4$,

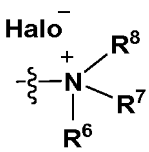,

$-N_3$ or phenyl;
$R^3$, optionally at ortho-, meta- or para-positon of the benzene ring, is selected from H, halogen, $C_1$-$C_6$ alkyl or partially or fully halogenated $C_1$-$C_6$ alkyl;
$R^4$ and $R^5$ are independently selected from H, $C_3$-$C_7$ cycloalkyl, straight or branched $C_1$-$C_6$ alkyl, wherein alkyl and cycloalkyl are optionally substituted by $-COOR^9$, $-NR^9R^{10}$, $-OR^9$, $-COR^9$, phenyl, benzyl, aromatic or non-aromatic heterocycle, wherein phenyl, benzyl, aromatic and nonaromatic heterocycle are optionally further substituted by halogen or $C_1$-$C_6$ alkyl; or
$R^4$ and $R^5$ together with the N-atom to which they are attached form 5 to 8-membered nonaromatic heterocycle which may contain another heteroatom selected from the group consisting of N, O and S, and is optionally substituted by halogen, $C_1$-$C_6$ alkyl, $-NR^{11}R^{12}$, $-OR^{11}$, =O, or benzyl, wherein $C_1$-$C_6$ alkyl is optionally substituted by $-COOR^4$;
$R^6$, $R^7$ and $R^8$ are independently selected from $C_1$-$C_6$ alkyl, hydroxyl substituted $C_2$-$C_4$ alkyl or benzyl, wherein benzyl is optionally substituted by halogen or $C_1$-$C_6$ alkyl;
$R^9$ and $R^{10}$ are independently selected from H, $C_1$-$C_6$ alkyl; or
$R^9$, $R^{10}$ together with the N-atom to which they are attached form 5 to 8-membered nonaromatic heterocycle which may contain another heteroatom selected from the group consisting ofN, O and S;
$R^{11}$ and $R^{12}$ are independently selected from H, $C_1$-$C_6$ alkyl;
Halo is an abbreviation of halogen.

**2.** The azole heterocyclic compound, *cis-trans* isomers, enantiomers, diastereoisomers, racemes, solvates, hydrates, or pharmaceutically acceptable salts thereof as claimed in claim 1, wherein in Formula (I) or (III), T is 5-membered

aliphatic ring or benzene ring; X is CH or N.

3. The azole heterocyclic compound, *cis-trans* isomers, enantiomers, diastereoisomers, racemes, solvates, hydrates, or pharmaceutically acceptable salts thereof as claimed in claim 1, wherein in Formula (I), when T is 5-membered aliphatic ring, X is N; when T is benzene ring, X is CH.

4. The azole heterocyclic compound, *cis-trans* isomers, enantiomers, diastereoisomers, racemes, solvates, hydrates, or pharmaceutically acceptable salts thereof as claimed in claim 1, wherein in Formula (I), (II) or (III), Y is phenyl substituted by fluorine atoms.

5. The azole heterocyclic compound, *cis-trans* isomers, enantiomers, diastereoisomers, racemes, solvates, hydrates, or pharmaceutically acceptable salts thereof as claimed in claim 1, wherein in Formula (I), (II) or (III), Y is 4-fluorophenyl or 2,3-difluorophenyl.

6. The azole heterocyclic compound, *cis-trans* isomers, enantiomers, diastereoisomers, racemes, solvates, hydrates, or pharmaceutically acceptable salts thereof as claimed in claim 1, wherein in Formula (I), (II) or (III), W is selected from 6 structures of Formulae (a-f) as follows:

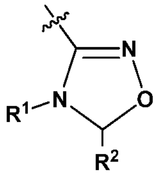
(a)

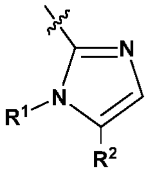
(b)

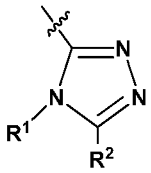
(c)

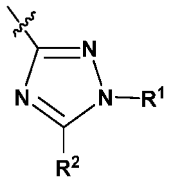
(d)

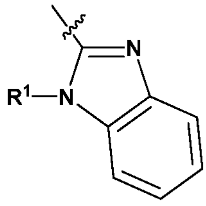
(e)

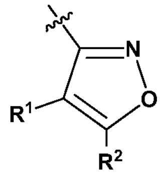
(f)

in which $R^1$ and $R^2$ are as defined in claim 1.

7. The azole heterocyclic compound, *cis-trans* isomers, enantiomers, diastereoisomers, racemes, solvates, hydrates, or pharmaceutically acceptable salts thereof as claimed in claim 6, wherein in Formula (I), (II) or (III), $R^1$ is

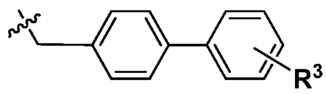

or $-NR^4R^5$ substituted $C_2$-$C_4$ alkyl.

8. The azole heterocyclic compound, *cis-trans* isomers, enantiomers, diastereoisomers, racemes, solvates, hydrates, or pharmaceutically acceptable salts thereof as claimed in claim 6, wherein in Formula (I), (II) or (III), $R^1$ is (4-(trifluoromethyl)biphenyl-4-yl)methyl.

9. The azole heterocyclic compound, *cis-trans* isomers, enantiomers, diastereoisomers, racemes, solvates, hydrates, or pharmaceutically acceptable salts thereof as claimed in claim 6, wherein in Formula (I), (II) or (III), $R^2$ is $-COR^4$, $-CONR^4R^5$, $C_1$-$C_5$ alkyl or $C_3$-$C_5$ cycloalkyl, wherein alkyl and cycloalkyl are optionally substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

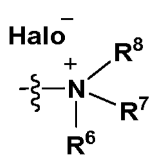
.

10. The azole heterocyclic compound, *cis-trans* isomers, enantiomers, diastereoisomers, racemes, solvates, hydrates, or pharmaceutically acceptable salts thereof as claimed in claim 6, wherein in Formula (I), (II) or (III), $R^2$ is $-COR^4$,-

$CONR^4R^5$, cyclopropyl or $C_1$-$C_5$ alkyl, wherein alkyl is substituted by $-NR^4R^5$, $-OR^4$, $-SR^4$, $-SO_2R^4$, $=NNR^4R^5$, $-NHCOR^4$, $-NHSO_2R^4$, $-NHCSNHR^4$ or

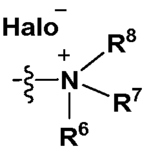

.

**11.** The azole heterocyclic compound, *cis-trans* isomers, enantiomers, diastereoisomers, racemes, solvates, hydrates, or pharmaceutically acceptable salts thereof as claimed in claim 6, wherein in Formula (I), (II) or (III), $R^2$ is $-CONR^4R^5$, cyclopropyl or $C_1$-$C_5$ alkyl, wherein $C_1$-$C_5$ alkyl is substituted by $-NR^4R^5$, $-OR^4$, $-SR^4$, $=NNR^4R^5$, or

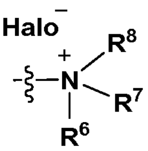

.

**12.** The azole heterocyclic compound, *cis-trans* isomers, enantiomers, diastereoisomers, racemes, solvates, hydrates, or pharmaceutically acceptable salts thereof as claimed in claim 6, wherein $C_1$-$C_5$ $R^2$ is selected from dimethylcarbamoyl, 2-(diethylamino)ethylcarbamoyl, (2-(diethylamino)ethyl)(methyl)carbamoyl, (dimethylamino)methyl, (diethylamino)methyl, pyrrolidin-1-ylmethyl, ((4-fluorobenzyl)(methyl)amino)methyl, isopropyl, cyclopropyl, 3-(diethylamino)propyl, 4-(diethylamino)butyl, hydroxymethyl, 1-hydroxyethyl, (4-fluorobenzylthio)methyl, (isopropyl(methyl)amino)methyl, ((1-ethylpyrrolidin-2-yl)methylamino)methyl, (4-ethylpiperazin-1-yl)methyl, ((2-(dimethylamino)ethyl)(methyl)amino)methyl, ((2-(diethylamino)ethyl)(methyl)amino)methyl, (((2-(dimethylamino)ethyl)(ethyl)amino)methyl, (((3-(dimethylamino)propyl)(methyl)amino)methyl), (methyl(pyridin-2-ylmethyl)amino)methyl, (4-(dimethylamino)piperidin-1-yl)methyl, (2,2-dimethylhydrazono)methyl, (2-hydroxyethoxy)methyl, (2-(diethylamino)ethoxy)methyl, 1-(2-(dimethylamino)ethylamino)ethyl, 1-((2-(dimethylamino)ethyl)(methyl)amino)ethyl, 1-(2-(diethylamino)ethylamino)ethyl, 1-((2-(diethylamino)ethyl)(methyl)amino)ethyl, 1-((3-(dimethylamino)propyl)(methyl)amino)ethyl, ((methyl(2-(pyrrolidin-1-yl)ethyl)amino)methyl, ((methyl(2-(piperidin-1-yl)ethyl)amino)methyl, 3-(pyrrolidin-1-yl)propyl, 3-(piperidin-1-yl)propyl, 4-(pyrrolidin-1-yl)butyl, 4-(piperidin-1-yl)butyl,

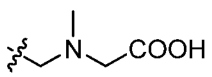 , 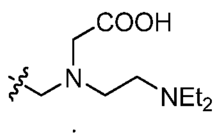 , 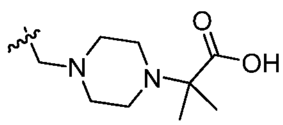 , 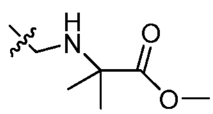 ,

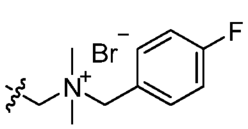 , 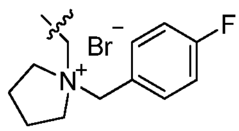 , 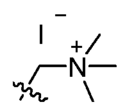 , 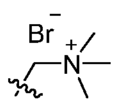 , 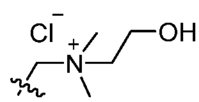 .

**13.** The azole heterocyclic compound, *cis-trans* isomers, enantiomers, diastereoisomers, racemes, solvates, hydrates, or pharmaceutically acceptable salts thereof as claimed in claim 1 having the structure of Formula (**IA**)-(**IG**), (**IIA**) or (**IIIA**):

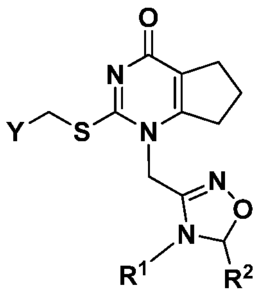

(IA)

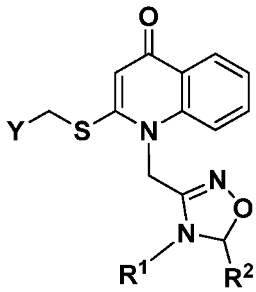

(IB)

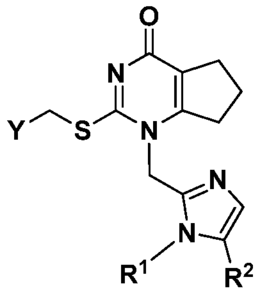

(IC)

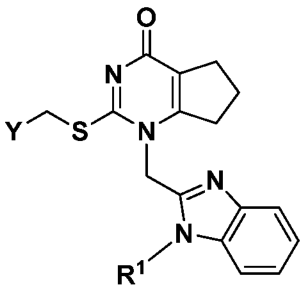

(ID)

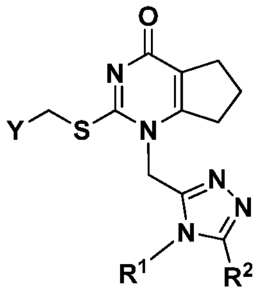

(IE)

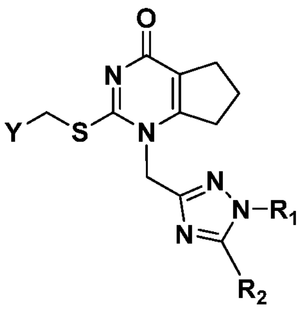

(IF)

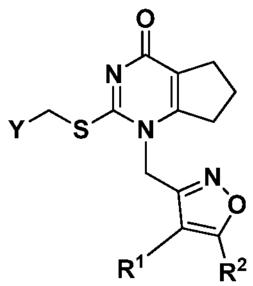

(IG)

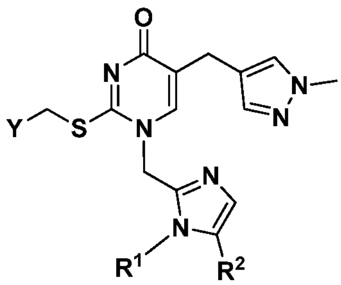

(IIA)

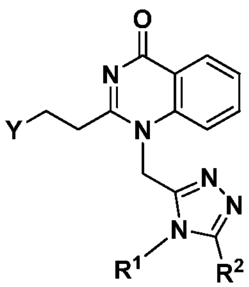

(IIIA)

in which Y, $R^1$ and $R^2$ are as defined in claim 1.

**14.** The azole heterocyclic compound, *cis-trans* isomers, enantiomers, diastereoisomers, racemes, solvates, hydrates, or pharmaceutically acceptable salts thereof as claimed in claim 1, wherein the compound is selected from:

2-((4-fluorobenzylthio)-1-((5-n-heptyl-4,5-dihydro-1,2,4-oxadiazol-3-yl)methyl)-6,7-dihydro-1*H*-cyclopenta[d]pyrimidin-4(5*H*)-one;
1-(((5-n-decyl-4,5-dihydro-1,2,4-oxadiazol-3-yl)methyl)-2-(4-fluorobenzylthio)-6,7-dihydro-1*H*-cyclopenta[d]pyrimidin-4(5*H*)-one;
2-((4-fluorobenzylthio)-1-((5-n-heptyl-4,5-dihydro-1,2,4-oxadiazol-3-yl)methyl)quinol in-4(1*H*)-one;
2-((4-fluorobenzylthio)-1-((5-(4'-(trifluoromethyl)biphenyl-4-yl)-4,5-dihydro-1,2,4-oxadiazol-3-yl)methyl)-6,7-dihydro-1*H*-cyclopenta[d]pyrimidin-4(5*H*)-one;
(*E*)-2-(4-fluorobenzylthio)-1-((5-n-heptyl-4-(n-oct-1-enyl)-4,5-dihydro-1,2,4-oxadiazol-3-yl)methyl)-6,7-dihydro-1*H*-cyclopenta[d]pyrimidin-4(5*H*)-one;
2-((2,3-difluorobenzylthio)-1-((4-(2-morpholinoethyl)-5-(4'-(trifluoromethyl)biphenyl-4-yl)-4,5-dihydro-1,2,4-oxadiazol-3-yl)methyl)-6,7-dihydro-1*H*-cyclopenta[d]pyrimid in-4(5*H*)-one;
ethyl 4-(3-((2-(2,3-difluorobenzylthio)-4-oxo-4,5,6,7-tetrahydro-1*H*-cyclopenta[d]pyrimidin-1-yl)methyl)-5-(4'-(trifluoromethyl)biphenyl-4-yl)-1,2,4-oxadiazol-4(5*H*)-yl)piperid ine-1-carboxylate;

ethyl 4-(3-((2-(4-fluorobenzylthio)-4-oxo-4,5,6,7-tetrahydro-1*H*-cyclopenta[*d*]pyrimidin-1-yl)methyl)-5-(4'-(trifluoromethyl)biphenyl-4-yl)-1,2,4-oxadiazol-4(5*H*)-yl)piperidine-1-carboxylate;
2-((4-fluorobenzylthio)-1-((4-(2-(piperidin-1-yl)ethyl)-5-(4'-(trifluoromethyl)biphenyl-4-yl)-4,5-dihydro-1,2,4-oxadiazol-3-yl)methyl)-6,7-dihydro-1*H*-cyclopenta[*d*]pyrimid in-4(5*H*)-one;
1-(((4-(2-(diethylamino)ethyl)-5-(4'-(trifluoromethyl)biphenyl-4-yl)-4,5-dihydro-1,2,4-oxadiazol-3-yl)methyl)-2-(4-fluorobenzylthio)-6,7-dihydro-1*H*-cyclopenta[*d*]pyrimidi n-4(5*H*)-one;
2-((2,3-difluorobenzylthio)-1-((4-(2-(piperidin-1-yl)ethyl)-5-(4'-(trifluoromethyl)biphe nyl-4-yl)-4,5-dihydro-1,2,4-oxadiazol-3-yl)methyl)-6,7-dihydro-1*H*-cyclopenta[d]pyri midin-4(5*H*)-one;
2-((4-fluorobenzylthio)-1-((5-phenyl-1*H*-imidazol-2-yl)methyl)-6,7-dihydro-1*H*-cyclo penta[*d*]pyrimidin-4(5H)-one;
1-(((1*H*-tetrazol-5-yl)methyl)-2-(4-fluorobenzylthio)-6,7-dihydro-1*H*-cyclopenta[d]pyr imidin-4(5*H*)-one;
2-((4-fluorobenzylthio)-1-((1-((4'-(trifluoromethyl)biphenyl-4-yl)methyl)-1*H*-imidazol -2-yl)methyl)-6,7-dihydro-1*H* cyclopenta[d]pyrimidin-4(5H)-one;
1-(((1-n-dodecyl-1*H*-imidazol-2-yl)methyl)-2-(4-fluorobenzylthio)-6,7-dihydro-1H-cy clopenta[*d*]pyrimidin-4(5*H*)-one;
1-(((1-n-butyl-1*H*-imidazol-2-yl)methyl)-2-(4-fluorobenzylthio)-6,7-dihydro-1*H*-cyclo penta[*d*]pyrimidin-4(5*H*)-one;
1-(((1-((4'-chlorobiphenyl-4-yl)methyl)-1*H*-imidazol-2-yl)methyl)-2-(4-fluorobenzylth io)-6,7-dihydro-1*H*-cyclopenta[*d*]pyrimidin-4(5*H*)-one;
1-(((1-(biphenyl-4-ylmethyl)-1*H*-imidazol-2-yl)methyl)-2-(4-fluorobenzylthio)-6,7-dih ydro-1*H*-cyclopenta[*d*]pyrimidin-4(5H)-one;
1-(((1-n-butyl-5-(4-chlorophenyl)-1*H*-imidazol-2-yl)methyl)-2-(4-fluorobenzylthio)-6, 7-dihydro-1*H*-cyclopenta[*d*]pyrimidin-4(5*H*)-one;
1-(((1-n-butyl-5-(4-chlorophenyl)-1*H*-imidazol-2-yl)methyl)-2-(2-nitrobenzylthio)-6,7-dihydro-1*H*-cyclopenta[d]pyrimidin-4(5*H*)-one;
1-(((5-n-decyl-1-(2-(diethylamino)ethyl)-1*H*-imidazol-2-yl)methyl)-2-(4-fluorobenzylt hio)-6,7-dihydro-1*H*-cyclopenta[d]pyrimidin-4(5*H*)-one;
1-(((1-(2-(diethylamino)ethyl)-5-(4'-(trifluoromethyl)biphenyl-4-yl)-1*H*-imidazol-2-yl) methyl)-2-(4-fluorobenzylthio)-6,7-dihydro-1*H*-cyclopenta[d]pyrimidin-4(5*H*)-one;
1-(((1-benzyl-5-((diethylamino)methyl)-1*H*-imidazol-2-yl)methyl)-2-(4-fluorobenzylth io)-6,7-dihydro-1*H*-cyclopenta[*d*]pyrimidin-4(5*H*)-one;
2-((4-fluorobenzylthio)-1-((5-methyl-1-((4'-(trifluoromethyl)biphenyl-4-yl)methyl)-1*H* -imidazol-2-yl)methyl)-6,7-dihydro-1*H*-cyclopenta[d]pyrimidin-4(5*H*)-one;
1-(((5-((diethylamino)methyl)-1-((4'-(trifluoromethyl)biphenyl-4-yl)methyl)-1*H*-imida zol-2-yl)methyl)-2-(4-fluorobenzylthio)-6,7-dihydro-1*H*-cyclopenta[d]pyrimidin-4(5 *H*)-one;
2-((4-fluorobenzylthio)-1-((5-(pyrrolidin-1-ylmethyl)-1-((4'-(trifluoromethyl)biphenyl-4-yl)methyl)-1*H*-imidazol-2-yl)methyl)-6,7-dihydro-1*H*-cyclopenta[*d*]pyrimidin-4(5*H* )-one;
1-(((5-(((4-fluorobenzyl)(methyl)amino)methyl)-1-((4'-(trifluoromethyl)biphenyl-4-yl) methyl)-1*H*-imidazol-2-yl)methyl)-2-(4-fluorobenzylthio)-6,7-dihydro-1*H*-cyclopenta [d]pyrimidin-4(5*H*)-one;
1-(((5-((4-benzylpiperazin-1-yl)methyl)-1-((4'-(trifluoromethyl)biphenyl-4-yl)methyl)-1*H*-imidazol-2-yl)methyl)-2-(4-fluorobenzylthio)-6,7-dihydro-1*H*-cyclopenta[*d*]pyrim idin-4(5*H*)-one;
1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[*d*]pyrimidin-4( 5*H*)-one;
N-(4-fluorobenzyl)-1-(2-((2-(4-fluorobenzylthio)-4-oxo-4,5,6,7-tetrahydro-1*H*-cyclop enta[d]pyrimidin-1-yl)methyl)-1-((4'-(trifluoromethyl)biphenyl-4-yl)methyl)-1*H*-imid azol-5-yl)-*N,N*-dimethylmethanaminium bromide;
1-(((5-((dimethylamino)methyl)-1-((4'-(trifluoromethyl)biphenyl-4-yl)methyl)-1*H*-imi dazol-2-yl)methyl)-2-(4-fluorobenzylthio)-6,7-dihydro-1*H*-cyclopenta[*d*]pyrimidin-4( 5*H*)-one;
1-((4-fluorobenzyl)-1-((2-((2-(4-fluorobenzylthio)-4-oxo-4,5,6,7-tetrahydro-1*H*-cyclop enta[*d*]pyrimidin-1-yl)methyl)-1-((4'-(trifluoromethyl)biphenyl-4-yl)methyl)-1*H*-imid azol-5-yl)methyl)pyrrolidinium bromide;
ethyl 2-((2-(4-fluorobenzylthio)-4-oxo-4,5,6,7-tetrahydro-1*H*-cyclopenta[d]pyrimidin-1-yl) methyl)-1-((4'-(trifluoromethyl)biphenyl-4-yl)methyl)-1*H* imidazole-5-carboxylate;
2-(((2-(4-fluorobenzylthio)-4-oxo-4,5,6,7-tetrahydro-1*H*-cyclopenta[d]pyrimidin-1-yl) methyl)-1-((4'-(trifluoromethyl)biphenyl-4-yl)methyl)-1*H*-imidazole-5-carboxylic acid;
2-(((2-(4-fluorobenzylthio)-4-oxo-4,5,6,7-tetrahydro-1*H*-cyclopenta[*d*]pyrimidin-1-yl) methyl)-N,N-dimethyl-1-((4'-(trifluoromethyl)biphenyl-4-yl)methyl)-1*H*-imidazole-5-carboxamide;
2-((4-fluorobenzylthio)-1-((1-((4'-(trifluoromethyl)biphenyl-4-yl)methyl)-1*H*-benzo[*d*] imidazol-2-yl)methyl)-6,7-dihydro-1*H*-cyclopenta[*d*]pyrimidin-4(5*H*)-one;
1-(((4-ethyl-5-(4'-(trifluoromethyl)biphenyl-4-yl)-4*H*-1,2,4-triazol-3-yl)methyl)-2-(4-fl uorobenzylthio)-6,7-dihydro-1*H*-cyclopenta[d]pyrimidin-4(5*H*)-one;
1-(((4-ethyl-5-(4'-(trifluoromethyl)biphenyl-4-yl)-4*H*-1,2,4-triazol-3-yl)methyl)-2-(4-methoxybenzylthio)-6,7-dihydro-1*H*-cyclopenta[d]pyrimidin-4(5*H*)-one;
2-((2,3-difluorobenzylthio)-1-((4-ethyl-5-(4'-(trifluoromethyl)biphenyl-4-yl)-4*H*-1,2,4-triazol-3-yl)methyl)-6,7-dihydro-1*H*-cyclopenta[d]pyrimidin-4(5H)-one;
1-(((4-ethyl-5-(3-phenylpropyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((4-ethyl-5-((4'-(trifluoromethyl)biphenyl-4-yl)methyl)-4*H*-1,2,4-triazol-3-yl)methy 1)-2-(4-fluorobenzylthio)-6,7-dihydro-1*H*-cyclopenta[d]pyrimidin-4(5*H*)-one;
2-((4-fluorobenzylthio)-1-((1-((4'-(trifluoromethyl)biphenyl-4-yl)methyl)-1*H*-1,2,4-tria zol-3-yl)methyl)-6,7-dihydro-1*H*-cyclopenta[d]pyrimidin-4(5*H*)-one;
methyl 2-(((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)meth yl)(methyl)amino)acetate;
2-((((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)meth yl)(methyl)amino)acetic acid;
2-((4-fluorobenzylthio)-1-((5-methyl-4-((4'-(trifluoromethyl)biphenyl-4-yl)methyl)-4*H* -1,2,4-triazol-3-yl)methyl)-6,7-dihydro-1*H*-cyclopenta[d]pyrimidin-4(5*H*)-one;
2-((4-fluorobenzylthio)-1-((5-n-propyl-4-((4'-(trifluoromethyl)biphenyl-4-yl)methyl)-4 *H*-1,2,4-triazol-3-yl)methyl)-6,7-dihydro-1*H*-cyclopenta[d]pyrimidin-4(5*H*)-one;
2-((4-fluorobenzylthio)-1-((5-isopropyl-4-((4'-(trifluoromethyl)biphenyl-4-yl)methyl)-4*H*-1,2,4-triazol-3-yl)methyl)-6,7-dihydro-1*H*-cyclopenta[d]pyrimidin-4(5*H*)-one;
1-(((5-cyclopropyl-4-((4'-(trifluoromethyl)biphenyl-4-yl)methyl)-4*H*-1,2,4-triazol-3-yl )methyl)-2-(4-fluorobenzylthio)-6,7-dihydro-1*H*-cyclopenta[d]pyrimidin-4(5*H*)-one;
1-(((5-(benzylsulfonylmethyl)-4-((4'-(trifluoromethyl)biphenyl-4-yl)methyl)-4H-1,2,4-triazol-3-yl)methyl)-2-(4-fluorobenzylthio)-6,7-dihydro-1*H*-cyclopenta[d]pyrimidin-4 (5*H*)-one;
1-(((5-(3-(diethylamino)propyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;
1-(((5-(4-(diethylamino)butyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;
2-((4-fluorob enzylthio)-1-((5-(hydroxymethyl)-4-((4'-(trifluoromethyl)biphenyl-4-yl)m ethyl)-4*H*-1,2,4-triazol-3-yl)methyl)-6,7-dihydro-1*H*-cyclopenta[d]pyrimidin-4(5*H*)-o ne;
2-((4-fluorobenzylthio)-1-((4-((4'-(trifluoromethyl)biphenyl-4-yl)methyl)-4*H*-1,2,4-tria zol-3-yl)methyl)-6,7-dihydro-1*H*-cyclopenta[d]pyrimidin-4(5*H*)-one;
5-(((2-(4-fluorobenzylthio)-4-oxo-4,5,6,7-tetrahydro-1*H*-cyclopenta[d]pyrimidin-1-yl) methyl)-4-((4'-(trifluoromethyl)biphenyl-4-yl)methyl)-4*H*-1,2,4-triazole-3-carbaldehy de;
1-(((5-((dimethylamino)methyl)-4-((4'-(trifluoromethyl)biphenyl-4-yl)methyl)-4*H*-1,2, 4-triazol-3-yl)methyl)-2-(4-fluorobenzylthio)-6,7-dihydro-1*H*-cyclopenta[d]pyrimidin -4(5*H*)-one;
N-(4-fluorobenzyl)-1-(5-((2-(4-fluorobenzylthio)-4-oxo-4,5,6,7-tetrahydro-1*H*-cyclop enta[d]pyrimidin-1-yl)methyl)-4-((4'-(trifluoromethyl)biphenyl-4-yl)methyl)-4*H*-1,2,4 -triazol-3-yl)-N,N-dimethylmethanaminium bromide;
1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium iodide;
1-((5-((2-(4-fluorobenzylthio)-4-oxo-4,5,6,7-tetrahydro-1*H*-cyclopenta[d]pyrimidin-1-yl)methyl)-4-((4'-(trifluoromethyl)biphenyl-4-yl)methyl)-4*H*-1,2,4-triazol-3-yl)-N,N, N-trimethylmethanaminium bromide;
1-(((5-((diethylamino)methyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[d]pyrimidin-4 (5*H*)-one;
1-(((5-(((4-fluorobenzyl)(methyl)amino)methyl)-4-((4'-(trifluoromethyl)biphenyl-4-yl) methyl)-4*H*-1,2,4-triazol-3-yl)methyl)-2-(4-fluorobenzylthio)-6,7-dihydro-1H-cyclope nta[d]pyrimidin-4(5*H*)-one;
2-((4-fluorobenzylthio)-1-((5-((isopropyl(methyl)amino)methyl)-4-((4'-(trifluoromethy 1)biphenyl-4-yl)methyl)-4*H*-1,2,4-triazol-3-yl)methyl)-6,7-dihydro-1*H*-cyclopenta[d]p yrimidin-4(5*H*)-one;
(±) 1-((5-(((1-ethylpyrrolidin-2-yl)methylamino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[d]pyrimidin-4(5*H*)-one;
1-(((5-(((2*S*,6*R*)-2,6-dimethylmorpholino)methyl)-4-((4'-(trifluoromethyl)biphenyl-4-y 1)methyl)-4*H*-1,2,4-triazol-3-yl)methyl)-2-(4-fluorobenzylthio)-6,7-dihydro-1H-cyclo penta[d]pyrimidin-4(5*H*)-one;
methyl-2-((5-((2-(4-fluorobenzylthio)-4-oxo-4,5,6,7-tetrahydro-1*H*-cyclopenta[d]pyri midin-1-yl)methyl)-4-((4'-(trifluoromethyl)biphenyl-4-yl)methyl)-4*H*-1,2,4-triazol-3-yl)methylamino)-2-methylpropanoate;
1-(((5-((4-ethylpiperazin-1-yl)methyl)-4-((4'-(trifluoromethyl)biphenyl-4-yl)methyl)-4 *H*-1,2,4-triazol-3-yl)methyl)-2-(4-fluorobenzylthio)-6,7-dihydro-1*H*-cyclopenta[d]pyr imidin-4(5*H*)-one;
2-((4-fluorobenzylthio)-1-((5-((2-methoxyethylamino)methyl)-4-((4'-(trifluoromethyl) biphenyl-4-yl)methyl)-4*H*-

1,2,4-triazol-3-yl)methyl)-6,7-dihydro-1H-cyclopenta[d]py rimidin-4(5*H*)-one;

methyl-2-(4-((5-((2-(4-fluorobenzylthio)-4-oxo-4,5,6,7-tetrahydro-1*H*-cyclopenta[d]p yrimidin-1-yl)methyl)-4-((4'-(trifluoromethyl)biphenyl-4-yl)methyl)-4*H*-1,2,4-triazol-3-yl)methyl)piperazin-1-yl)-2-methylpropanoate;

2-((4-((5-((2-(4-fluorobenzylthio)-4-oxo-4,5,6,7-tetrahydro-1*H*-cyclopenta[d]pyrimidi n-1-yl)methyl)-4-((4'-(trifluoromethyl)biphenyl-4-yl)methyl)-4*H*-1,2,4-triazol-3-yl)m ethyl)piperazin-1-yl)-2-methylpropanoic acid;

1-(((5-(((2-(dimethylamino)ethyl)(methyl)amino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 H-cyclopenta[d]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[d]pyrimidin-4(5*H*)-one;

1-(((5-(((2-(diethylamino)ethyl)(methyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H* cyclopenta[d]pyrimidin-4(5*H*)-one tartrate;

1-(((5-(((3-(dimethylamino)propyl)(methyl)amino)methyl)-4-((4'-(trifluoromethyl)biph enyl-4-yl)methyl)-4*H*-1,2,4-triazol-3-yl)methyl)-2-(4-fluorobenzylthio)-6,7-dihydro-1 *H*-cyclopenta[*d*]pyrimidin-4(5*H*)-one;

2-((4-fluorobenzylthio)-1-((5-((2-morpholinoethylamino)methyl)-4-((4'-(trifluorometh yl)biphenyl-4-yl)methyl)-4*H*-1,2,4-triazol-3-yl)methyl)-6,7-dihydro-1*H*-cyclopenta[*d*] pyrimidin-4(5*H*)-one;

(*R*)-1-((5-((3-(dimethylamino)pyrrolidin-1-yl)methyl)-4-((4'-(trifluoromethyl)bipheny 1-4-yl)methyl)-4*H*-1,2,4-triazol-3-yl)methyl)-2-(4-fluorobenzylthio)-6,7-dihydro-1*H*-c yclopenta[d]pyrimidin-4(5*H*)-one;

(*S*)-1-((5-((3-(dimethylamino)pyrrolidin-1-yl)methyl)-4-((4'-(trifluoromethyl)biphenyl -4-yl)methyl)-4*H*-1,2,4-triazol-3-yl)methyl)-2-(4-fluorobenzylthio)-6,7-dihydro-1*H*-c yclopenta[d]pyrimidin-4(5*H*)-one;

2-((4-fluorobenzylthio)-1-((5-((2-(piperidin-1-yl)ethylamino)methyl)-4-((4'-(trifluorom ethyl)biphenyl-4-yl)methyl)-4*H*-1,2,4-triazol-3-yl)methyl)-6,7-dihydro-1*H*-cyclopenta [*d*]pyrimidin-4(5*H*)-one;

2-((4-fluorobenzylthio)-1-((5-((2-(pyrrolidin-1-yl)ethylamino)methyl)-4-((4'-(trifluoro methyl)biphenyl-4-yl)methyl)-4*H*-1,2,4-triazol-3-yl)methyl)-6,7-dihydro-1*H*-cyclope nta[*d*]pyrimidin-4(5*H*)-one;

1-(((5-((2-(diisopropylamino)ethylamino)methyl)-4-((4'-(trifluoromethyl)biphenyl-4-yl )methyl)-4*H*-1,2,4-triazol-3-yl)methyl)-2-(4-fluorobenzylthio)-6,7-dihydro-1*H*-cyclop enta[d]pyrimidin-4(5*H*)-one;

1-(((5-((2-(diethylamino)ethylamino)methyl)-4-((4'-(trifluoromethyl)biphenyl-4-yl)met hyl)-4*H*-1,2,4-triazol-3-yl)methyl)-2-(4-fluorobenzylthio)-6,7-dihydro-1*H*-cyclopenta [*d*]pyrimidin-4(5*H*)-one;

2-((4-fluorobenzylthio)-1-((5-((4-methylpiperazin-1-yl)methyl)-4-((4'-(trifluoromethyl )biphenyl-4-yl)methyl)-4*H*-1,2,4-triazol-3-yl)methyl)-6,7-dihydro-1*H*-cyclopenta[*d*]p yrimidin-4(5*H*)-one;

2-((4-fluorobenzylthio)-1-((5-((methyl(pyridin-2-ylmethyl)amino)methyl)-4-((4'-(triflu oromethyl)biphenyl-4-yl)methyl)-4H-1,2,4-triazol-3-yl)methyl)-6,7-dihydro-1*H*-cyclo penta[*d*]pyrimidin-4(5*H*)-one;

1-(((5-((cyclopropyl(methyl)amino)methyl)-4-((4'-(trifluoromethyl)biphenyl-4-yl)meth yl)-4H-1,2,4-triazol-3-yl)methyl)-2-(4-fluorobenzylthio)-6,7-dihydro-1*H*-cyclopenta[ *d*]pyrimidin-4(5*H*)-one;

1-(((5-((4-(dimethylamino)piperidin-1-yl)methyl)-4-((4'-(trifluoromethyl)biphenyl-4-yl )methyl)-4*H*-1,2,4-triazol-3-yl)methyl)-2-(4-fluorobenzylthio)-6,7-dihydro-1*H*-cyclop enta[*d*]pyrimidin-4(5*H*)-one;

1-(((5-((3,3-difluoropyrrolidin-1-yl)methyl)-4-((4'-(trifluoromethyl)biphenyl-4-yl)meth yl)-4*H*-1,2,4-triazol-3-yl)methyl)-2-(4-fluorobenzylthio)-6,7-dihydro-1*H*-cyclopenta[ d]pyrimidin-4(5*H*)-one;

1-(((5-((2-(dimethylamino)ethylamino)methyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[d]pyrimidin-4(5*H*)-one;

(*S*)-1-((5-(((1-ethylpyrrolidin-2-yl)methylamino)methyl)-4-((4'-(trifluoromethyl)biphe nyl-4-yl)methyl)-4*H*-1,2,4-triazol-3-yl)methyl)-2-(4-fluorobenzylthio)-6,7-dihydro-1 *H*-cyclopenta[d]pyrimidin-4(5*H*)-one;

2-((4-fluorobenzylthio)-1-((5-((2-(2-oxoimidazolidin-1-yl)ethylamino)methyl)-4-((4'-(t rifluoromethyl)biphenyl-4-yl)methyl)-4*H*-1,2,4-triazol-3-yl)methyl)-6,7-dihydro-1*H*-c yclopenta[d]pyrimidin-4(5*H*)-one;

1-(((5-(azidomethyl)-4-((4'-(trifluoromethyl)biphenyl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one ;

1-(((5-(aminomethyl)-4-((4'-(trifluoromethyl)biphenyl-4-yl)methyl)-4*H*-1,2,4-triazol-3 -yl)methyl)-2-(4-fluorobenzylthio)-6,7-dihydro-1*H*-cyclopenta[d]pyrimidin-4(5*H*)-on e;

*N*-((5-((2-(4-fluorobenzylthio)-4-oxo-4,5,6,7-tetrahydro-1*H*-cyclopenta[d]pyrimidin-1 -yl)methyl)-4-((4'-(trifluoromethyl)biphenyl-4-yl)methyl)-4*H*-1,2,4-triazol-3-yl)methy 1)ethanesulfonamide;

2-((4-fluorobenzylthio)-1-((5-((2-oxoimidazolidin-1-yl)methyl)-4-((4'-(trifluoromethyl )biphenyl-4-yl)methyl)-4*H*-1,2,4-triazol-3-yl)methyl)-6,7-dihydro-1*H*-cyclopenta[*d*]p yrimidin-4(5*H*)-one;

1-(((5-((2-(4-fluorobenzylthio)-4-oxo-4,5,6,7-tetrahydro-1*H*-cyclopenta[*d*]pyrimidin-1 -yl)methyl)-4-((4'-(trifluoromethyl)biphenyl-4-yl)methyl)-4*H*-1,2,4-triazol-3-yl)methy 1)-3-methylthiourea;

1-(((5-(chloromethyl)-4-((4'-(trifluoromethyl)biphenyl-4-yl)methyl)-4*H*-1,2,4-triazol-3 -yl)methyl)-2-(4-fluorobenzylthio)-6,7-dihydro-1*H*-cyclopenta[*d*]pyrimidin-4(5*H*)-on e;

2-((4-fluorobenzylthio)-1-((5-((4-fluorobenzylthio)methyl)-4-((4'-(trifluoromethyl)bip henyl-4-yl)methyl)-4*H*-1,2,4-triazol-3-yl)methyl)-6,7-dihydro-1*H*-cyclopenta[*d*]pyrim idin-4(5*H*)-one;

(*E*)-1-((5-((2,2-dimethylhydrazono)methyl)-4-((4'-(trifluoromethyl)biphenyl-4-yl)met hyl)-4*H*-1,2,4-triazol-3-

yl)methyl)-2-(4-fluorobenzylthio)-6,7-dihydro-1*H*-cyclopenta [*d*]pyrimidin-4(5*H*)-one;
(*E*)-1-((5-((2-tert-butylhydrazono)methyl)-4-((4'-(trifluoromethyl)biphenyl-4-yl)meth yl)-4*H*-1,2,4-triazol-3-yl)methyl)-2-(4-fluorobenzylthio)-6,7-dihydro-1*H*-cyclopenta[ *d*]pyrimidin-4(5*H*)-one;
(*E*)-2-(4-fluorobenzylthio)-1-((5-((piperidin-1-ylimino)methyl)-4-((4'-(trifluoromethyl )biphenyl-4-yl)methyl)-4*H*-1,2,4-triazol-3-yl)methyl)-6,7-dihydro-1*H*-cyclopenta[*d*]p yrimidin-4(5*H*)-one;
1-((5-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl)-4*H*-1,2,4-triazol-3-yl)vinyl acetate;
2-((4-fluorobenzylthio)-1-((5-(1-hydroxyethyl)-4-((4'-(trifluoromethyl)biphenyl-4-yl) methyl)-4*H*-1,2,4-triazol-3-yl)methyl)-6,7-dihydro-1*H*-cyclopenta[*d*]pyrimidin-4(5H) -one;
1-(((5-acetyl-4-((4'-(trifluoromethyl)biphenyl-4-yl)methyl)-4*H*-1,2,4-triazol-3-yl)meth yl)-2-(4-fluorobenzylthio)-6,7-dihydro-1*H*-cyclopenta[*d*]pyrimidin-4(5*H*)-one;
*N*-((5-((2-(4-fluorobenzylthio)-4-oxo-4,5,6,7-tetrahydro-1H-cyclopenta[d]pyrimidin-1 -yl)methyl)-4-((4'-(trifluoromethyl)biphenyl-4-yl)methyl)-4*H*-1,2,4-triazol-3-yl)methy 1)-2-hydroxy-*N,N*-dimethylethanaminium chloride;
2-((4-fluorobenzylthio)-1-((5-((2-hydroxyethoxy)methyl)-4-((4'-(trifluoromethyl)biphe nyl-4-yl)methyl)-4*H*-1,2,4-triazol-3-yl)methyl)-6,7-dihydro-1*H*-cyclopenta[*d*]pyrimid in-4(5*H*)-one;
1-(((5-((2-(diethylamino)ethoxy)methyl)-4-((4'-(trifluoromethyl)biphenyl-4-yl)methyl) -4*H*-1,2,4-triazol-3-yl)methyl)-2-(4-fluorobenzylthio)-6,7-dihydro-1*H*-cyclopenta[*d*]p yrimidin-4(5*H*)-one;
1-(((5-(n-butoxymethyl)-4-((4'-(trifluoromethyl)biphenyl-4-yl)methyl)-4*H*-1,2,4-triazo 1-3-yl)methyl)-2-(4-fluorobenzylthio)-6,7-dihydro-1*H*-cyclopenta[*d*]pyrimidin-4(5*H*)-one;
1-(((5-(1-(2-(dimethylamino)ethylamino)ethyl)-4-((4'-(trifluoromethyl)biphenyl-4-yl) methyl)-4*H*-1,2,4-triazol-3-yl)methyl)-2-(4-fluorobenzylthio)-6,7-dihydro-1*H*-cyclope nta[*d*]pyrimidin-4(5*H*)-one;
1-(((5-(1-((2-(dimethylamino)ethyl)(methyl)amino)ethyl)-4-((4'-(trifluoromethyl)biphe nyl-4-yl)methyl)-4*H*-1,2,4-triazol-3-yl)methyl)-2-(4-fluorobenzylthio)-6,7-dihydro-1 H-cyclopenta[d]pyrimidin-4(5*H*)-one;
1-(((5-(1-(2-(diethylamino)ethylamino)ethyl)-4-((4'-(trifluoromethyl)biphenyl-4-yl)me thyl)-4*H*-1,2,4-triazol-3-yl)methyl)-2-(4-fluorobenzylthio)-6,7-dihydro-1*H*-cyclopenta [*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
1-(((5-(1-((2-(diethylamino)ethyl)(methyl)amino)ethyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[d]pyrimidin-4(5*H*)-one tartrate;
1-(((5-(1-((3-(dimethylamino)propyl)(methyl)amino)ethyl)-4-((4'-(trifluoromethyl)bip henyl-4-yl)methyl)-4*H*-1,2,4-triazol-3-yl)methyl)-2-(4-fluorobenzylthio)-6,7-dihydro-1H-cyclopenta[d]pyrimidin-4(5*H*)-one;
1-(((5-(((2-(dimethylamino)ethyl)(ethyl)amino)methyl)-4-((4'-(trifluoromethyl)biphen yl-4-yl)methyl)-4*H*-1,2,4-triazol-3-yl)methyl)-2-(4-fluorobenzylthio)-6,7-dihydro-1*H*-cyclopenta[*d*]pyrimidin-4(5*H*)-one;
ethyl-2-((2-(diethylamino)ethyl)((3-((2-(4-fluorobenzylthio)-4-oxo-4,5,6,7-tetrahydro-1*H*-cyclopenta[*d*]pyrimidin-1-yl)methyl)-4-((4'-(trifluoromethyl)biphenyl-4-yl)methyl )-4*H*-1,2,4-triazol-5-yl)methyl)amino)acetate;
2-(((2-(diethylamino)ethyl)((3-((2-(4-fluorobenzylthio)-4-oxo-4,5,6,7-tetrahydro-1*H*-c yclopenta[d]pyrimidin-1-yl)methyl)-4-((4'-(trifluoromethyl)biphenyl-4-yl)methyl)-4*H* -1,2,4-triazol-5-yl)methyl)amino)acetic acid;
*N*-(2-(diethylamino)ethyl)-2-((2-(4-fluorobenzylthio)-4-oxo-4,5,6,7-tetrahydro-1*H*-cy clopenta[d]pyrimidin-1-yl)methyl)-1-((4'-(trifluoromethyl)biphenyl-4-yl)methyl)-1*H*-i midazole-5-carboxamide;
*N*-(2-(diethylamino)ethyl)-2-((2-(4-fluorobenzylthio)-4-oxo-4,5,6,7-tetrahydro-1*H*-cy clopenta[*d*]pyrimidin-1-yl)methyl)-*N*-methyl-1-((4'-(trifluoromethyl)biphenyl-4-yl)me thyl)-1*H*-imidazole-5-carboxamide;
1-(((5-(((2-(diethylamino)ethyl)(methyl)amino)methyl)-1-((4'-(trifluoromethyl)biphen yl-4-yl)methyl)-1*H*-imidazol-2-yl)methyl)-2-(4-fluorobenzylthio)-6,7-dihydro-1*H*-cyc lopenta[d]pyrimidin-4(5*H*)-one;
2-((4-fluorobenzylthio)-1-((5-((methyl(2-(pyrrolidin-1-yl)ethyl)amino)methyl)-4-((4'-(t rifluoromethyl)biphenyl-4-yl)methyl)-4*H*-1,2,4-triazol-3-yl)methyl)-6,7-dihydro-1*H*-c yclopenta[d]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-1*H*-imidazol-2-yl)methyl)-6,7-dihydro-1H-cyclo penta[d]pyrimidin-4(5*H*)-one;
2-((4-fluorobenzylthio)-1-((5-(3-(pyrrolidin-1-yl)propyl)-4-((4'-(trifluoromethyl)biphe nyl-4-yl)methyl)-4H-1,2,4-triazol-3-yl)methyl)-6,7-dihydro-1*H*-cyclopenta[d]pyrimid in-4(5*H*)-one;
2-((4-fluorobenzylthio)-1-((5-(3-(piperidin-1-yl)propyl)-4-((4'-(trifluoromethyl)biphen yl-4-yl)methyl)-4*H*-1,2,4-triazol-3-yl)methyl)-6,7-dihydro-1*H*-cyclopenta[d]pyrimidi n-4(5*H*)-one;
2-((4-fluorobenzylthio)-1-((5-(4-(pyrrolidin-1-yl)butyl)-4-((4'-(trifluoromethyl)biphen yl-4-yl)methyl)-4*H*-1,2,4-triazol-3-yl)methyl)-6,7-dihydro-1*H*-cyclopenta[d]pyrimidi n-4(5*H*)-one;
2-((4-fluorobenzylthio)-1-((5-(4-(piperidin-1-yl)butyl)-4-((4'-(trifluoromethyl)biphenyl -4-yl)methyl)-4*H*-1,2,4-triazol-3-yl)methyl)-6,7-dihydro-1*H*-cyclopenta[d]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)-4*H*-1,2,4-triazol-3-yl)methyl)-6,7-dihydro-1*H*-c yclopenta[*d*]pyrimidin-4(5*H*)-one;
methyl-3-((2-(4-fluorobenzylthio)-4-oxo-4,5,6,7-tetrahydro-1*H*-cyclopenta[*d*]pyrimidi n-1-yl)methyl)-4-((4'-(trifluoromethyl)biphenyl-4-yl)methyl)isoxazole-5-carboxylate;
2-((4-fluorobenzylthio)-1-((5-(hydroxymethyl)-4-((4'-(trifluoromethyl)biphenyl-4-yl)m ethyl)isoxazol-3-yl)methyl)-6,7-dihydro-1*H*-cyclopenta[d]pyrimidin-4(5*H*)-one;
1-(((5-(chloromethyl)-4-((4'-(trifluoromethyl)biphenyl-4-yl)methyl)isoxazol-3-yl)meth yl)-2-(4-fluorobenzylthio)-6,7-dihydro-1*H*-cyclopenta[d]pyrimidin-4(5*H*)-one;
1-(((5-((diethylamino)methyl)-4-((4'-(trifluoromethyl)biphenyl-4-yl)methyl)isoxazol-3 -yl)methyl)-2-(4-fluorobenzylthio)-6,7-dihydro-1*H*-cyclopenta[d]pyrimidin-4(5H)-on e;
1-(((5-(((2-(dimethylamino)ethyl)(methyl)amino)methyl)-4-((4'-(trifluoromethyl)biphe nyl-4-yl)methyl)isoxazol-3-yl)methyl)-2-(4-fluorobenzylthio)-6,7-dihydro-1*H*-cyclop enta[d]pyrimidin-4(5*H*)-one;
1-(((5-(((2-(diethylamino)ethyl)(methyl)amino)methyl)-4-((4'-(trifluoromethyl)biphen yl-4-yl)methyl)isoxazol-3-yl)methyl)-2-(4-fluorobenzylthio)-6,7-dihydro-1*H*-cyclope nta[d]pyrimidin-4(5*H*)-one;
2-((4-fluorobenzylthio)-1-((5-((methyl(2-(piperidin-1-yl)ethyl)amino)methyl)-4-((4'-(tr ifluoromethyl)biphenyl-4-yl)methyl)isoxazol-3-yl)methyl)-6,7-dihydro-1*H*-cyclopent a[d]pyrimidin-4(5*H*)-one;
2-((4-fluorophenethyl)-1-((4-((4'-(trifluoromethyl)biphenyl-4-yl)methyl)-*4*H-1,2,4-tria zol-3-yl)methyl)quinazolin-4(1*H*)-one.

**15.** A pharmaceutical composition comprising a therapeutically effective amount of one or more of the azole heterocyclic compounds, *cis-trans* isomers, enantiomers, diastereoisomers, racemes, solvates, hydrates, or pharmaceutically acceptable salts thereof as claimed in any one of claims 1 to 14, and a pharmaceutically acceptable auxiliary.

**16.** The pharmaceutical composition as claimed in claim 15, further comprising one or more agents selected from the group consisting of anti-hyperlipidaemic, anti-atherosclerotic, anti-diabetic, anti-anginal, antiinflammatory, anti-hypertension agents and agents for lowering Lp(a).

**17.** A use of the azole heterocyclic compounds, *cis-trans* isomers, enantiomers, diastereoisomers, racemes, solvates, hydrates, or pharmaceutically acceptable salts thereof as claimed in any one of claims 1 to 14 in manufacturing a medicament as Lp-$PLA_2$ inhibitor.

**18.** A use of the azole heterocyclic compounds, *cis-trans* isomers, enantiomers, diastereoisomers, racemes, solvates, hydrates, or pharmaceutically acceptable salts thereof as claimed in any one of claims 1 to 14 in manufacturing a medicament for preventing, curing or ameliorating diseases associated with activity of the enzyme Lp-$PLA_2$.

**19.** The use as claimed in claim 18, wherein the diseases include atherosclerosis, stroke, coronary heart disease, diabetes, asthma, psoriasis, rheumatoid arthritis, or acute and chronic inflammation.

**20.** A method of preventing, curing or ameliorating a disease associated with activity of the enzyme Lp-$PLA_2$, wherein the method involves administering the compounds, *cis-trans* isomers, enantiomers, diastereoisomers, racemes, solvates, hydrates, or pharmaceutically acceptable salts thereof as claimed in any one of claims 1 to 14, or the pharmaceutical composition as claimed in any one of claims 15 to 16.

**21.** The method as claimed in claim 20, wherein the diseases include atherosclerosis, stroke, coronary heart disease, diabetes, asthma, psoriasis, rheumatoid arthritis, or acute and chronic inflammation.

**22.** A preparation method of the azole heterocyclic compound of Formula (I), *cis-trans* isomers, enantiomers, diastereoisomers, racemes, solvates, hydrates, or pharmaceutically acceptable salts thereof, wherein one of the processes illustrated by synthetic route (1-4) as follows is employed:

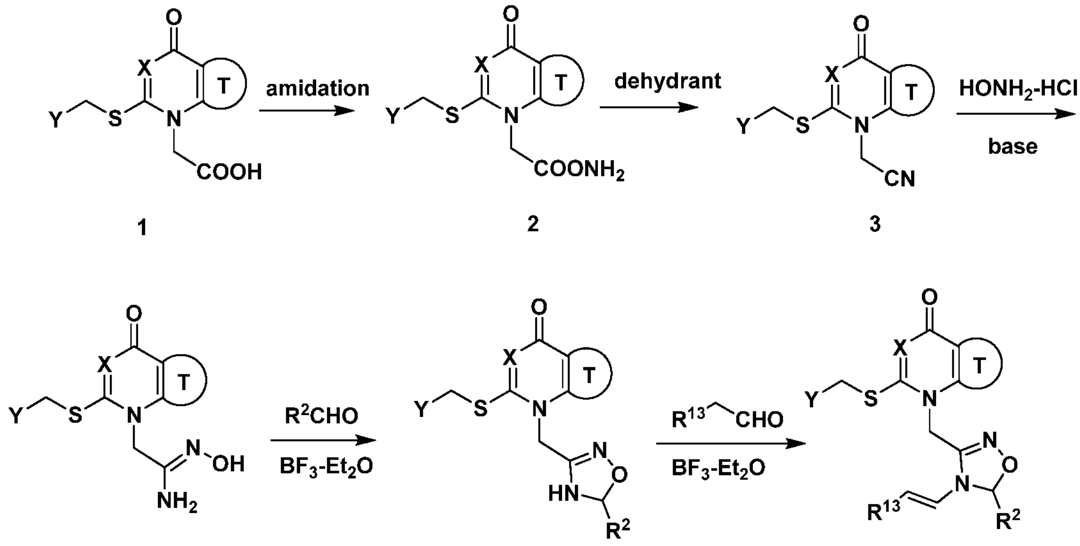


1 2 3 4 5 6

**Synthetic Route 1**

when W is Formula (a) meanwhile $R^1$ is alkenyl in the compound of Formula (I), the compound of Formula (I) (i.e. compound **6**) can be prepared according to the method illustrated by synthetic route 1, in which: $R^{13}$ is $C_1$-$C_{10}$ alkyl; T, X, Y and $R^2$ are as defined in claim 1;

compound **1** is converted to compound **2** by amidation which is then dehydrated to give compound **3;** compound **3** reacts with hydroxylamine hydrochloride in the presence of a base to produce compound **4;** catalyzed by boron trifluoride-diethyl ether complex, the reaction of compound **4** with $R^2CHO$ yields compound **5** in an aprotic solvent; compound **5** is then transformed into compound **6** by reaction with $R^{13}CH_2CHO$ in the presence of boron trifluoride-diethyl ether complex as a catalyst;

or,

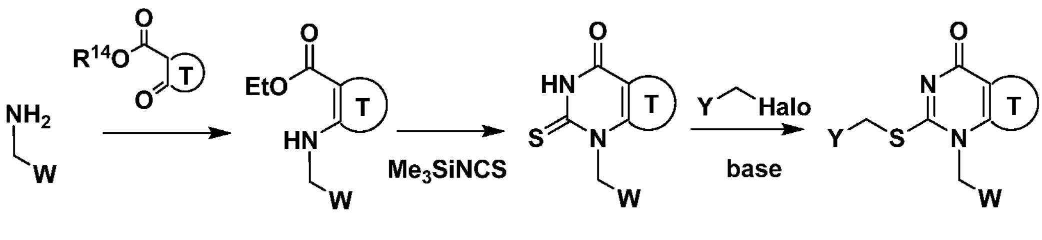


7 8 9 10

**Synthetic Route 2**

when T is 4 to 6-membered aliphatic ring and X=N, meanwhile $R^1$ is not alkenyl in the compound of Formula (I), the compound of Formula (I) (i.e. compound **10**) can be prepared according to the method illustrated by synthetic route 2, in which: $R^{14}$ is methyl or ethyl; Halo, T, W and Y are as defined in claim 1;

in a polar solvent, compound 7 is condensed with a cycloalkanone carboxylate

d to

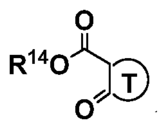

to yield compound **8** in the presence of a dehydrant; compound **8** reacts with $Me_3SiNCS$ to produce compound **9** which is then converted to compound 10 by reaction with

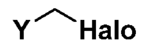

in a polar solvent in the presence of a base;
or,

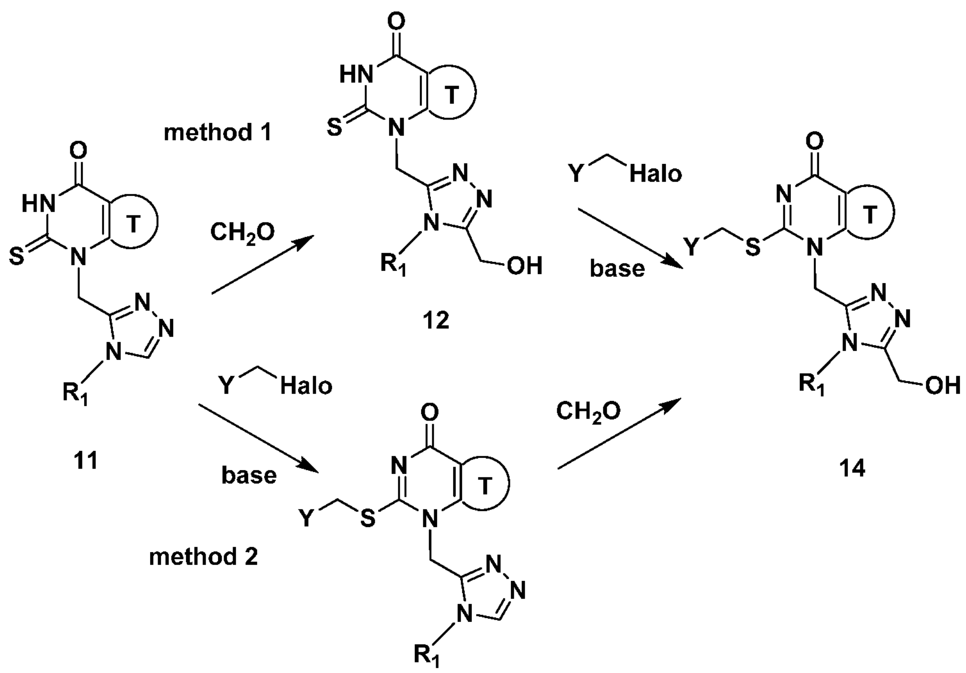


13

**Synthetic Route 3**

when W is structure (c) meanwhile $R^2$ is hydroxymethyl in the above mentioned compound **10,** the compound, i.e. compound 14, can be prepared according to either of the methods illustrated by synthetic route 3, in which Halo, T, Y and $R^1$ are as defined in claim 1;
according to the first method, compound **11** is heated with aqueous formaldehyde to afford compound **12** which then reacts in the presence of a base with

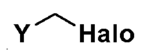

in a polar solvent to give compound **14**;
according to the second method, compound **11** first reacts with

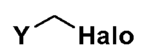

to give compound **13** which is then heated with aqueous formaldehyde to afford compound **14;**

or,

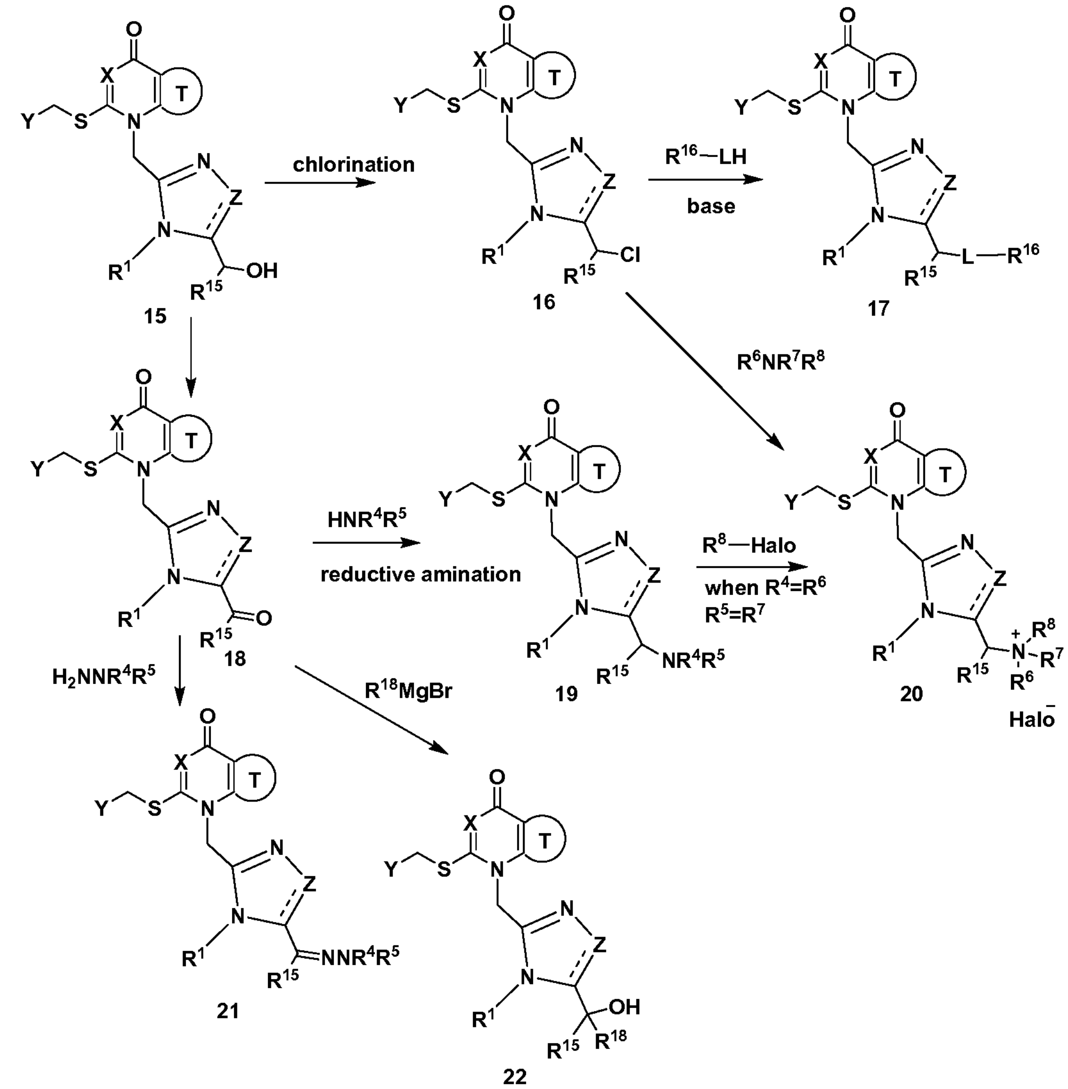


## Synthetic Route 4

a compound of Formula (I) can be converted into another structure of the compound of Formula (I) by functional transformation, which is illustrated by route 4 as follows:

when $R^2$ in Formula (I) is $\alpha$-hydroxy substituted alkyl, the compound of Formula (I) can be represented by the structure of compound **15,** in which $R^{15}$ is H or $C_1$-$C_6$ alkyl; $R^{16}$ is $C_1$-$C_6$ alkyl that is optionally substituted by $NR^4R^5$ or phenyl, wherein phenyl is optionally substituted by halogen; $R^{18}$ is $C_1$-$C_6$ alkyl; L is $NR^4$, O or S; Z is CH, N or O; T, X, Y, $R^1$, $R^4$, $R^5$, $R^6$, $R^7$ and $R^8$ are as defined in claim 1;

Compound **15** is chlorinated to afford compound **16** which then reacts with $R^{16}$LH to give compound 17 in the presence of a base;

Compound **15** is oxidized to afford compound **18** which then reacts with $HNR^4R^5$ to give compound 19 in the presence of a reductant;

the condensation reaction of compound **16** with $R^6NR^7R^8$ yields compound **20;**

the condensation reaction of compound **19** with $R^8$-Halo also yields compound **20;**

the condensation reaction of compound **18** with $H_2NNR^4R^5$ yields compound **21;**

the reaction of compound **18** with $R^{18}$BrMg yields compound **22**.

## INTERNATIONAL SEARCH REPORT

International application No.
**PCT/CN2012/000661**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 20-21
because they relate to subject matter not required to be searched by this Authority, namely:
Claims 20-21 relate to a method for prevention, treatment or improvement of diseases concerned with Lp-$PLA_2$ enzymatic activity, belong to a method for treatment of the human or animal body, and do not meet the requirement of PCT Rule 39 (iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

# INTERNATIONAL SEARCH REPORT

International application No. **PCT/CN2012/000661**

**A. CLASSIFICATION OF SUBJECT MATTER**

See the extra sheet

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

WPI, EPODOC, CPRS, CNKI, CHINESE PATENT FULL-TEXT DATABASE, REGISTRY, CAPLUS, MARPAT: azotic heterocyclic, cyclopentapyrimidine; WANG, Kai; Pyrroles, cyclopent, pyrimidine, Pyrimidones, Lp-PLA$_2$, structure search

**C. DOCUMENTS CONSIDERED TO BE RELEVANT**

| Category* | Citation of document, with indication, where appropriate, of the relevant passages | Relevant to claim No. |
|---|---|---|
| A | WO 9924420 A1 (SMITHKLINE BEECHAM PLC et al.), 20 May 1999 (20.05.1999), the whole document, especially abstract, and claims 1-19 | 1-19, 22 |
| A | CN 1418199 A (SMITHKLINE BEECHAM P.L.C.), 14 May 2003 (14.05.2003), the whole document, especially abstract, and claims 1-25 | 1-19, 22 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 02 August 2012 (02.08.2012) | **16 August 2012 (16.08.2012)** |
| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br>**LIN, Guan**<br>Telephone No.: (86-10) **62411194** |

Form PCT/ISA/210 (second sheet) (July 2009)

## INTERNATIONAL SEARCH REPORT
Information on patent family members

International application No.
**PCT/CN2012/000661**

| Patent Documents referred in the Report | Publication Date | Patent Family | Publication Date |
|---|---|---|---|
| WO 9924420 A1 | 20.05.1999 | US 2002120139 A1 | 29.08.2002 |
| | | EP 1028955 B1 | 16.07.2003 |
| | | CA 2309177 A1 | 20.05.1999 |
| | | ES 2203988 T3 | 16.04.2004 |
| | | US 6417192 B1 | 09.07.2002 |
| | | DE 69816471 D1 | 21.08.2003 |
| | | AU 1157599 A | 31.05.1999 |
| | | ZA 9810112 A | 28.06.2000 |
| | | EP 1028955 A1 | 23.08.2000 |
| | | JP 2001522844 T | 20.11.2001 |
| CN 1418199 A | 14.05.2003 | KR 20030011772 A | 11.02.2003 |
| | | EP 1686119 A1 | 02.08.2006 |
| | | ES 2267714 T3 | 16.03.2007 |
| | | US 2009118313 A1 | 07.05.2009 |
| | | US 7652019 B2 | 26.01.2010 |
| | | US 2010144765 A1 | 10.06.2010 |
| | | GC 221 B | 29.03.2006 |
| | | ZA 200206528 A | 28.05.2003 |
| | | RU 2235722 C2 | 10.09.2004 |
| | | DE 60121550 T2 | 21.06.2007 |
| | | KR 100781425 B1 | 03.12.2007 |
| | | US 2009170877 A1 | 02.07.2009 |
| | | EP 1686119 B1 | 29.07.2009 |
| | | DE 60139429 D1 | 10.09.2009 |
| | | AU 2001235466 B2 | 22.04.2004 |
| | | EP 1263740 B1 | 19.07.2006 |
| | | IL 151236 A | 11.02.2009 |
| | | ES 2330552 T3 | 11.12.2009 |
| | | US 2002103213 A1 | 01.08.2002 |
| | | SK 11772002 A3 | 04.02.2003 |
| | | US 6649619 B1 | 18.11.2003 |
| | | NZ 520752 A | 26.03.2004 |
| | | US 2004097525 A1 | 20.05.2004 |
| | | WO 0160805 A1 | 23.08.2001 |
| | | NO 20023828 A | 30.09.2002 |
| | | CZ 20022768 A3 | 14.05.2003 |
| | | CN 1179952 C | 15.12.2004 |
| | | DE 60121550 D1 | 31.08.2006 |

Form PCT/ISA/210 (patent family annex) (July 2009)

**INTERNATIONAL SEARCH REPORT**
Information on patent family members

International application No.
**PCT/CN2012/000661**

| Patent Documents referred in the Report | Publication Date | Patent Family | Publication Date |
|---|---|---|---|
| | | US 7638520 B2 | 29.12.2009 |
| | | IN 246780 B | 18.03.2011 |
| | | EP 1263740 A1 | 11.12.2002 |
| | | JP 4095804 B2 | 04.06.2008 |
| | | US 7470694 B2 | 30.12.2008 |
| | | SK 287296 B6 | 07.06.2010 |
| | | MX 2002008062 A1 | 01.12.2002 |
| | | MX 228834 B | 01.07.2005 |
| | | NO 324691 B1 | 03.12.2007 |
| | | PL 209824 B1 | 31.10.2011 |
| | | IN 200201086 P3 | 04.03.2005 |
| | | US 7153861 B2 | 26.12.2006 |
| | | AU 3546601 A | 27.08.2001 |
| | | BR 0108396 A | 11.03.2003 |
| | | HU 200204410 A2 | 28.05.2003 |
| | | JP 2003523335 A | 05.08.2003 |
| | | TW 550259 B | 01.09.2003 |
| | | US 2007123549 A1 | 31.05.2007 |
| | | US 2007155762 A1 | 05.07.2007 |
| | | PH 1200100325 B1 | 21.12.2007 |
| | | CA 2400554 C | 07.04.2009 |

Form PCT/ISA/210 (patent family annex) (July 2009)

# INTERNATIONAL SEARCH REPORT

| International application No. |
| --- |
| PCT/CN2012/000661 |

**CLASSIFICATION OF SUBJECT MATTER:**

C07D 401/06 (2006.01) i

C07D 401/14 (2006.01) i

C07D 403/06 (2006.01) i

C07D 403/14 (2006.01) i

C07D 413/06 (2006.01) i

C07D 413/14 (2006.01) i

C07D 417/06 (2006.01) i

C07D 417/14 (2006.01) i

A61K 31/506 (2006.01) i

A61K 31/517 (2006.01) i

A61P 9/10 (2006.01) i

A61P 3/10 (2006.01) i

A61P 17/06 (2006.01) i

A61P 29/00 (2006.01) i

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9613484 A
- WO 9619451 A
- WO 9702242 A
- WO 9721765 A
- WO 9721766 A
- WO 9741098 A
- WO 9741099 A
- WO 9924420 A
- WO 0160805 A
- WO 0230911 A
- WO 03016287 A
- WO 03042179 A
- WO 03042206 A
- WO 08048867 A
- US 7642291 B


### Non-patent literature cited in the description


- **ZALEWSKI A et al.** *Arterioscler Thromb Vasc Biol,* 2005, vol. 25 (5), 923-931
- **MACPHEE CH ; MOORES KE ; BOYD HF et al.** *Biochem J,* 1999, vol. 338, 479-87
- **ROSENSON RS ; VRACAR-GRABAR M ; HELENOWSKI I.** *Cardiovasc Drugs Ther,* 2008, vol. 22, 55-8
- **PACKARD CJ ; O'REILLY DS ; CASLAKE MJ et al.** West of Scotland Coronary Prevention Group. *N Engl J Med,* 2000, vol. 343, 1148-55
- **TEW et al.** *Biochemistry,* 1998, vol. 37, 10087
- *Comprehensive Org. Syn.,* vol. 7, 251-327
- Comprehensive Org. Trains. Wiley-VCH, 1999, 835-840
- *Tetrahedron Letters,* 2001, vol. 42, 315-317
- **GOLOLOBOV, Y. G.** Sixty years of Staudinger reaction. *Tetrahedron,* 1981, vol. 37, 437
- **A. J. MANCUSO ; S-L. HUANG ; D. SWERN.** *J. Org. Chem.,* 1978, vol. 43, 2480
- *J. Amer. Chem. Soc.,* 1959, vol. 81, 3108
- *Eur. J. Org. Chem.,* 2008, 895-913
- *J. Het. Chem.,* 1995, vol. 32, 611